# EUROPEAN PATENT APPLICATION

(11) **EP 3 939 968 A1**
(43) Date of publication of application: **19.01.2022**
(21) Application number: 20784904.3
(22) Date of filing: 30.03.2020
(51) Int. Cl.: C07D 295/18, A61K 31/5375, A61K 31/216, A61K 31/36, A61K 31/40, A61K 31/404, A61K 31/4168, A61K 31/4196, A61K 31/426, A61K 31/4453

(54) **NOVEL COMPOUND HAVING ANTICANCER ACTIVITY, AND METHOD FOR PRODUCING SAME**

(30) Priority: 29.03.2019 KR 20190037271
(71) Applicant: Medicinal Bioconvergence Research Center, Suwon-si, Gyeonggi-do 16229 (KR); Dongguk University Industry-Academic Cooperation Foundation, Seoul 04620 (KR)
(72) Inventor: KIM, Sunghoon, Suwon-si Gyeonggi-do 16229 (KR); KIM, Dae Gyu, Suwon-si Gyeonggi-do 16229 (KR); LEE, Kyeong, Seoul 04620 (KR); KIM, Minkyoung, Seoul 04620 (KR)
(74) Representative: Richardt Patentanwälte PartG mbB
(86) International application number: PCT/KR2020/004364
(87) International publication number: WO 2020/204548

(57) **Abstract**

The present invention pertains to a novel compound having anticancer activity, and a method for producing same, and more specifically, to a novel compound that exhibits excellent anticancer activity by inhibiting the expression of AIMP2-DX2, and a method for producing same. The compound represented by chemical formula 1 according to the present invention is highly effective in inhibiting the expression of AIMP2-DX2, and thus can be very advantageously used for the development of agents for treating various diseases, in particular cancer, caused by AIMP2-DX2.

## Description

### THECHNICAL FIELD

This application claims priority to Korean Patent Application No. 10-2019-0037271 filed on March 29, 2019, and the entire specifications of which are incorporated herein by reference in their entireties.

The present invention pertains to a novel compound having anticancer activity, and a method for producing same, and more specifically, to a novel compound that exhibits excellent anticancer activity by inhibiting the expression of AIMP2-DX2, and a method for producing same.

### BACKGROUND OF THE INVENTION

\AIMP2 (aminoacyl-tRNA synthetase complex-interacting multifunctional protein 2) is one of the proteins involved in the formation of the aminoacyl-tRNA synthetase (ARS) complex. AIMP2 is known to have a function of enhancing TGF-β signaling through direct interaction with Smad2/3 as a novel tumor suppressor in addition to its original function in protein synthesis.

As known in Korean Patent No. 10-0762995 by the present inventor, it was observed that AIMP2-DX2, a mutant in which exon 2 of AIMP2 is missing, is overexpressed in various cancer cell lines and tissues such as lung cancer, liver cancer, skin cancer, breast cancer, kidney cancer, osteosarcoma. On the other hand, when normal cells are transformed to overexpress AIMP2-DX2, the level of wild-type (WT) AIMP2 is greatly reduced, and the activity of AIMP2 is inhibited such as blocking AIMP2 from translocating to the nucleus, and c-myc expression is increased, and it has been shown to cause dysfunction of TGF-β signaling such as cell growth is promoted. This shows that there is a close relationship between AIMP2-DX2 and cancer development and progression.

In addition, in another Korean Patent No. 10-1067816 of the present inventor, AIMP2 mediates the apoptotic activity of TNF-α through interaction with TRAF2, and it has been shown for the first time that this activity is regulated by AIMP2-DX2. In addition, it was confirmed that AIMP2-DX2 also affects the expression of COX-2, an inflammatory marker, and suggested that by inhibiting the activity of AIMP2-DX2, a therapeutic effect for inflammatory diseases can be produced. it was suggested that it can have a therapeutic effect on inflammatory diseases by inhibiting the activity of AIMP2-DX2.

As it was revealed that AIMP2-DX2 protein is involved in the induction and progression of various diseases, AIMP2-DX2 is emerging as a new target material for drug development.

### DETAILED DESCRIPTION OF THE INVENTION

### TECHNICAL PROBLEM

Accordingly, the present inventors repeated intensive research to develop a novel compound capable of inhibiting the expression of AIMP2-DX2, which is closely related to various diseases. it was found that the compound represented by Formula 1 in the present specification suppresses the expression of AIMP2-DX2 to exhibit excellent anticancer activity, and thus the present invention has been completed.

Accordingly, an object of the present invention is to provide a compound represented by the following formula 1 or a pharmaceutically acceptable salt thereof: 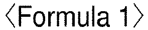 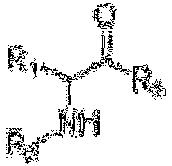 wherein Formula 1,
R1 is hydrogen; C1~C5 straight or branched alkyl substituted or unsubstituted with one or more substituents selected from the group consisting of hydroxy, substituted or unsubstituted phenyl, mercapto, and C1~C5 alkylthio; Indolylmethyl unsubstituted or substituted with one or more substituents selected from the group consisting of C1~C5 alkyl, hydroxy, hydroxycarbonyl and formyl; C1-C5 carboxymethyl; C7-C10 hydroxyphenylalkyl; substituted or unsubstituted C3-C8 cycloalkyl; substituted or unsubstituted C1~C5 aminoalkyl; 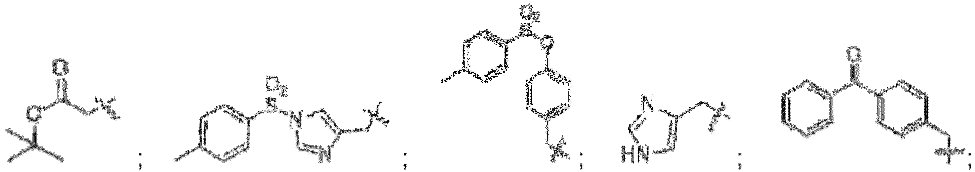 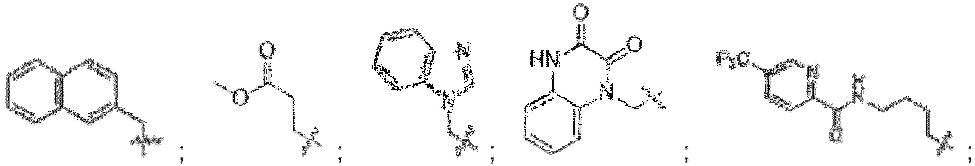 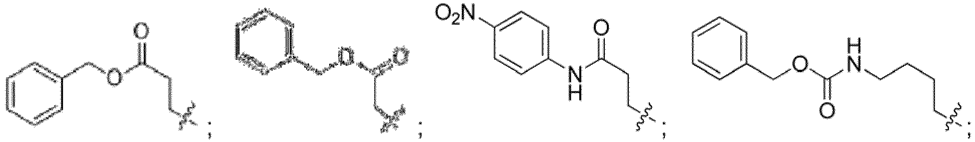 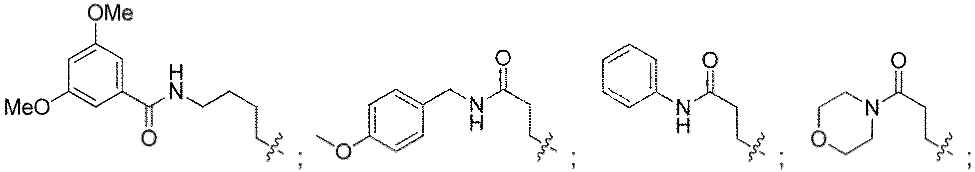 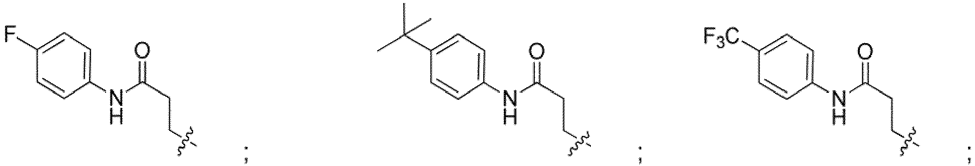 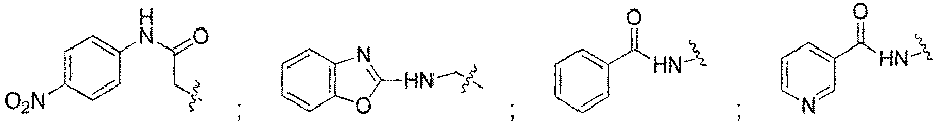 or 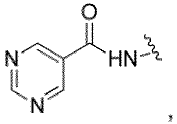
R2 is hydrogen; benzenesulfonyl unsubstituted or substituted with one or more substituents selected from the group consisting of C1~C5 straight or branched alkyl, nitro group, amine group, halo, -CF₃, C1~C5 alkylcarbonylamino, -COOH, C1~C5 alkoxy, phenyl, -OCH₂Ph, hydroxy, -COOCH₂Ph, C1~C5 alkyloxycarbonyl, -CONHCH₃ and nitrile; naphthalenesulfonyl; benzylsulfonyl; benzoyl unsubstituted or substituted with one or more substituents selected from the group consisting of C1~C5 straight or branched alkyl and C1~C5 straight or branched alkylamine groups; thiophenesulfonyl; 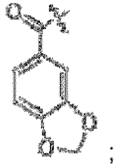 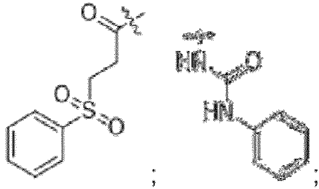 C1~C5 alkyloxycarbonyl; or phenylaminocarbonyl,

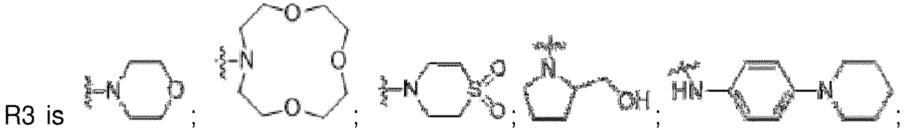

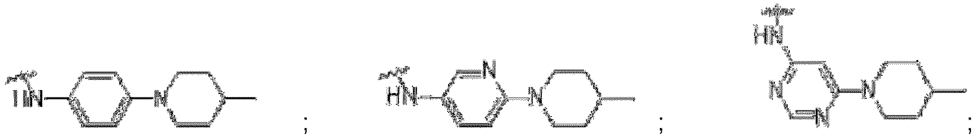

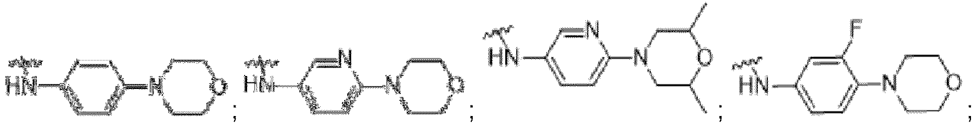

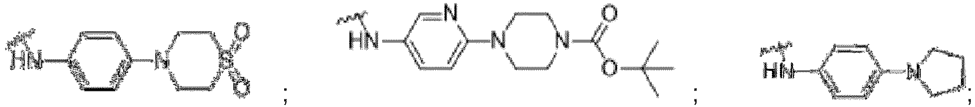

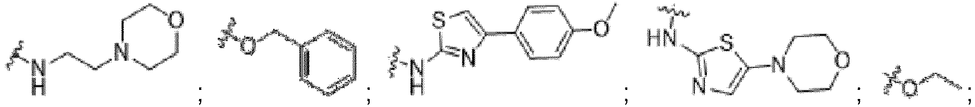

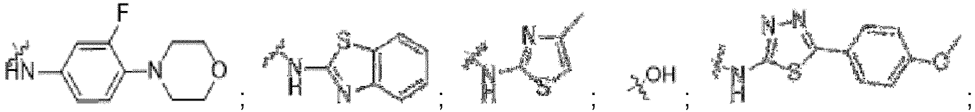

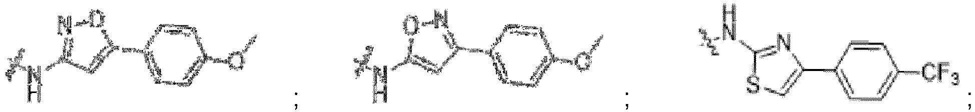

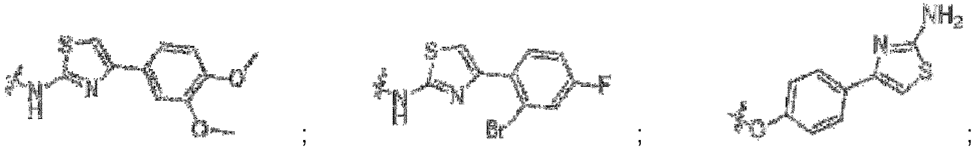

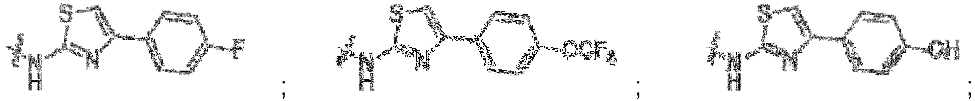

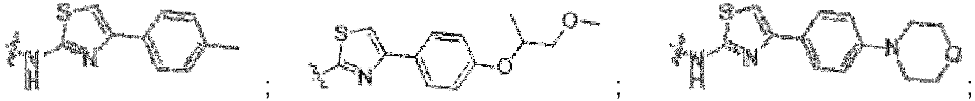

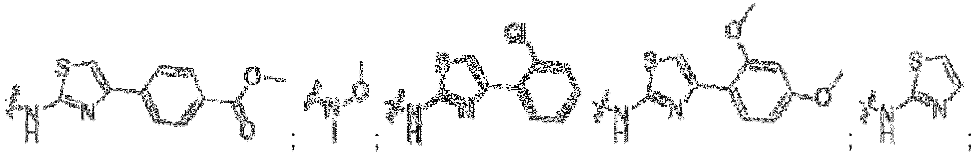

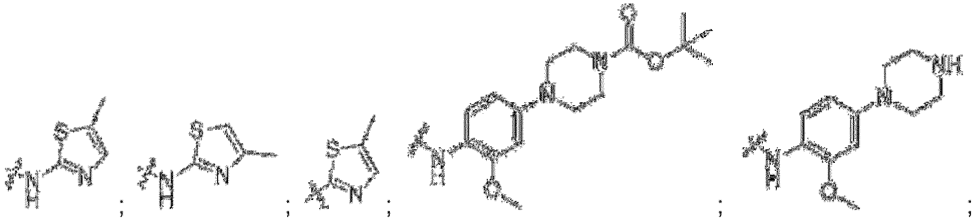

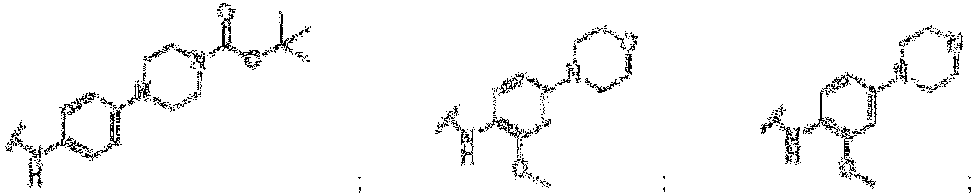

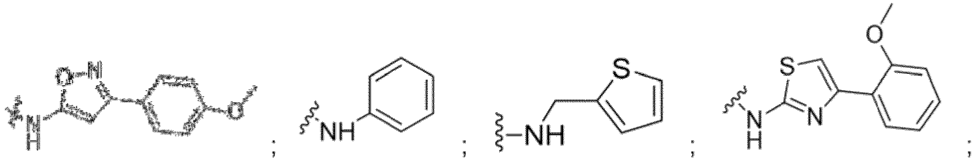

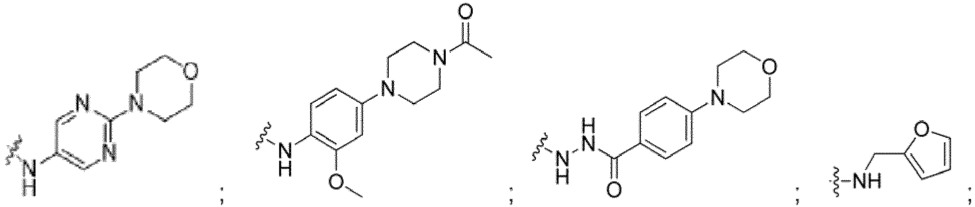

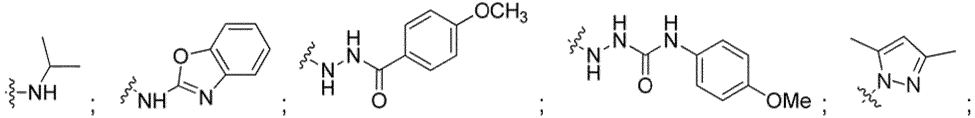

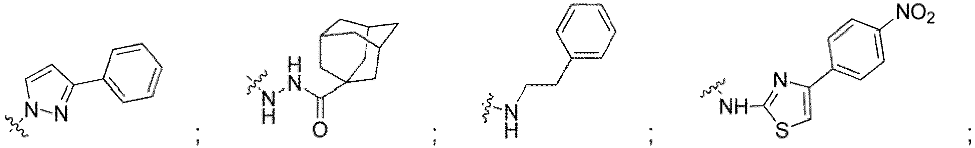

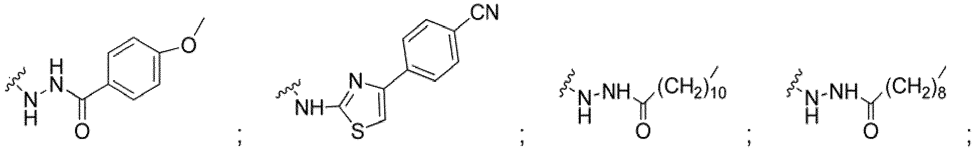

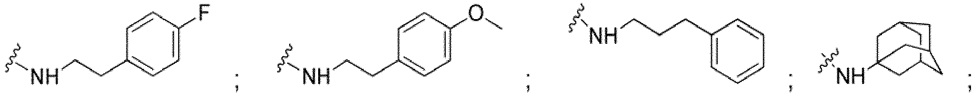

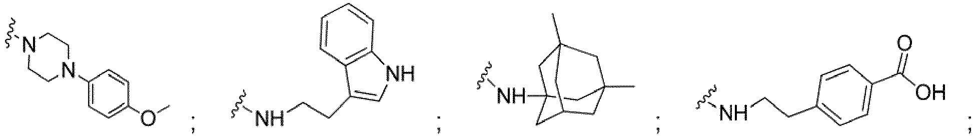

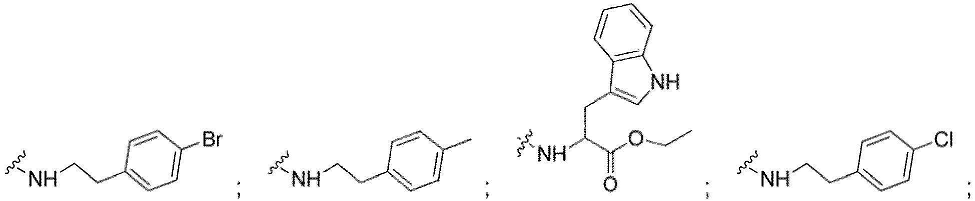

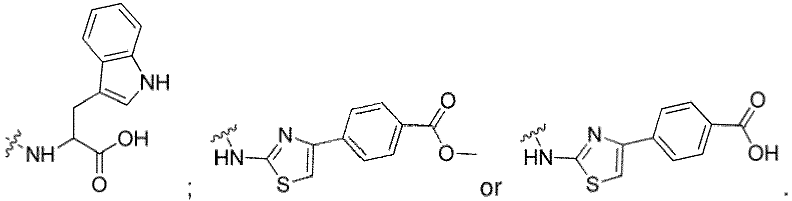

Another object of the present invention is to provide a pharmaceutical composition for preventing or treating cancer comprising the compound of the Formula 1 or a pharmaceutically acceptable salt thereof as an active ingredient.

Another object of the present invention is to provide a pharmaceutical composition for preventing or treating cancer consisting of the compound of the Formula 1 or a pharmaceutically acceptable salt thereof as an active ingredient.

Another object of the present invention is to provide a pharmaceutical composition for preventing or treating cancer essentially consisting of the compound of the Formula 1 or a pharmaceutically acceptable salt thereof as an active ingredient.

Another object of the present invention is to provide a use of the compound of the Formula 1 or a pharmaceutically acceptable salt thereof for preparing an agent for preventing or treating cancer.

Another object of the present invention is to provide a method for treating cancer comprising administering to a subject in need thereof an effective amount of a composition comprising the compound of the Formula 1 or a pharmaceutically acceptable salt thereof as an active ingredient.

Another object of the present invention is to provide a use of the compound of the Formula 1 or a pharmaceutically acceptable salt thereof for preparing an agent for preventing or treating inflammatory diseases.

Another object of the present invention is to provide a method for treating an inflammatory disease comprising administering to a subject in need thereof an effective amount of a composition comprising the compound of the Formula 1 or a pharmaceutically acceptable salt thereof as an active ingredient.

### TECHNICAL SOLUTION

In order to achieve the above object of the present invention, the present invention provides a compound represented by the following formula 1 or a pharmaceutically acceptable salt thereof: 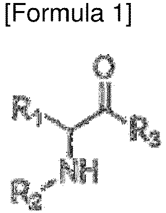 wherein Formula 1,
R1 is hydrogen; C1~C5 straight or branched alkyl substituted or unsubstituted with one or more substituents selected from the group consisting of hydroxy, substituted or unsubstituted phenyl, mercapto, and C1~C5 alkylthio; Indolylmethyl unsubstituted or substituted with one or more substituents selected from the group consisting of C1~C5 alkyl, hydroxy, hydroxycarbonyl and formyl; C1~C5 carboxymethyl; C7~C10 hydroxyphenylalkyl; substituted or unsubstituted C3~C8 cycloalkyl; substituted or unsubstituted C1~C5 aminoalkyl; 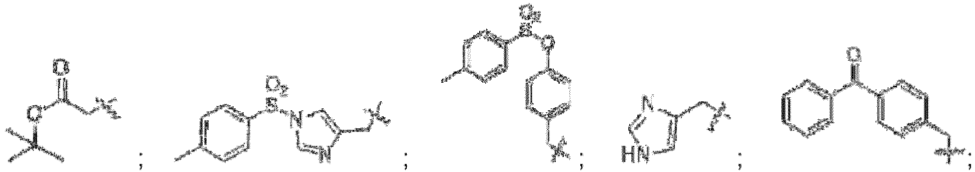 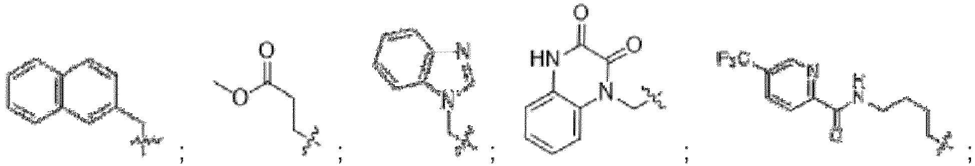 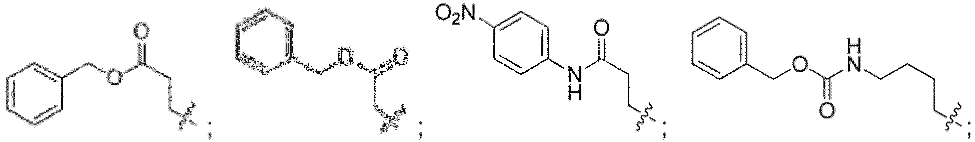 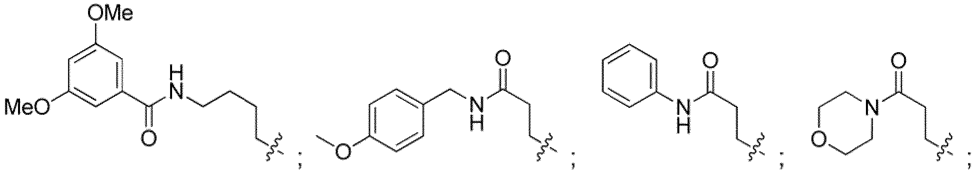 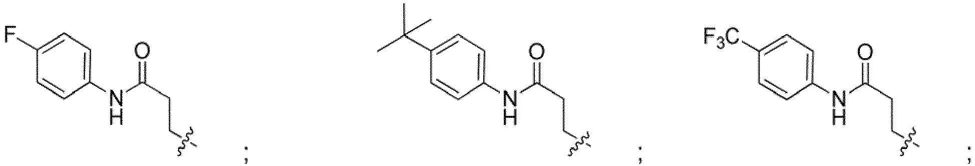 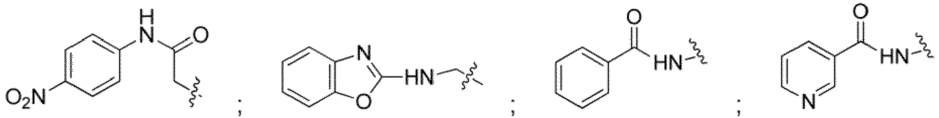 or 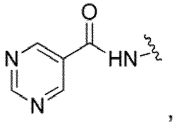
R2 is hydrogen; benzenesulfonyl unsubstituted or substituted with one or more substituents selected from the group consisting of C1~C5 straight or branched alkyl, nitro group, amine group, halo, -CF₃, C1~C5 alkylcarbonylamino, -COOH, C1~C5 alkoxy, phenyl, -OCH₂Ph, hydroxy, -COOCH₂Ph, C1~C5 alkyloxycarbonyl, -CONHCH₃ and nitrile; naphthalenesulfonyl; benzylsulfonyl; benzoyl unsubstituted or substituted with one or more substituents selected from the group consisting of C1~C5 straight or branched alkyl and C1~C5 straight or branched alkylamine groups; thiophenesulfonyl; 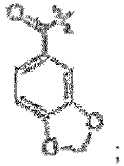 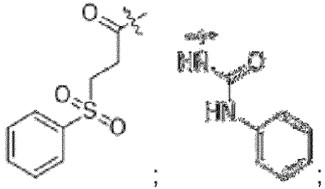 C1~C5 alkyloxycarbonyl; or phenylaminocarbonyl,
R3 is

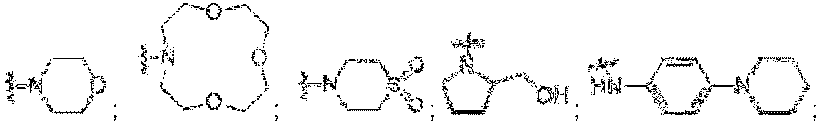

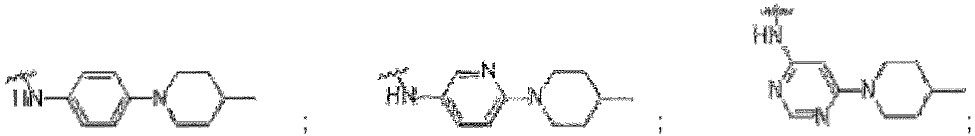

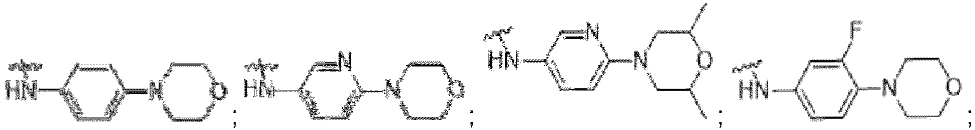

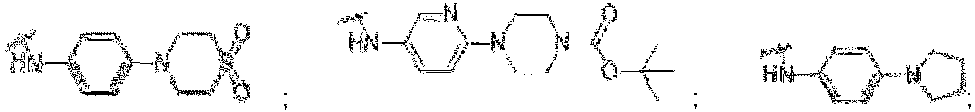

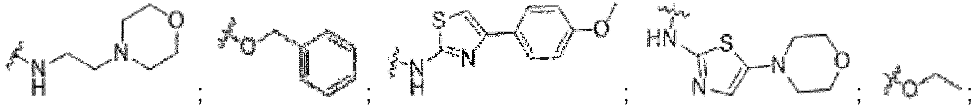

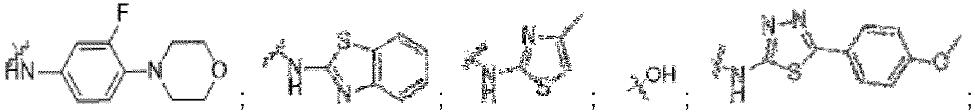

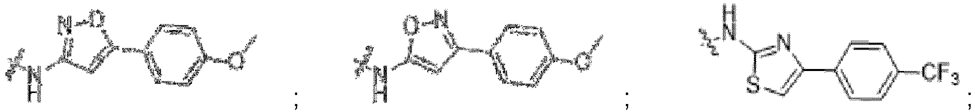

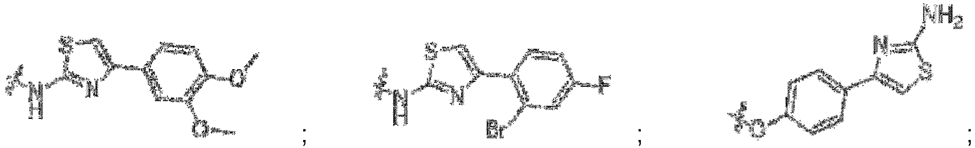

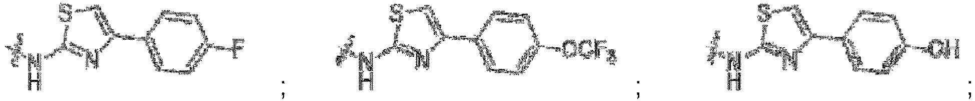

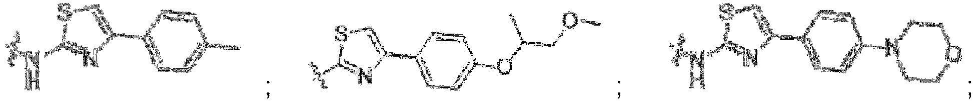

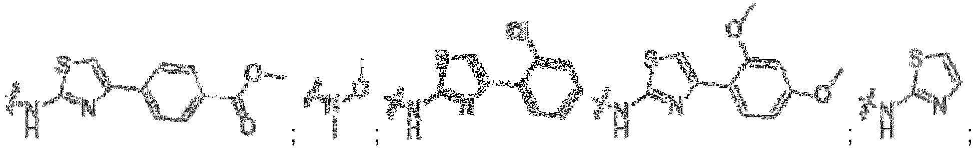

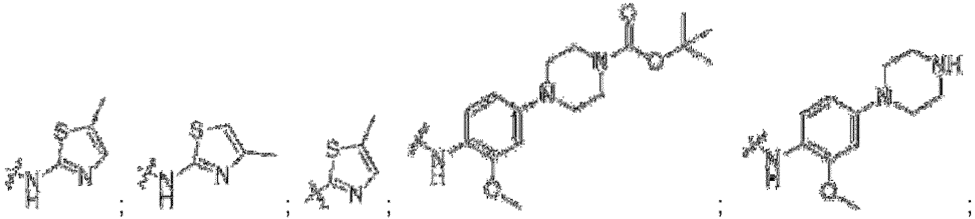

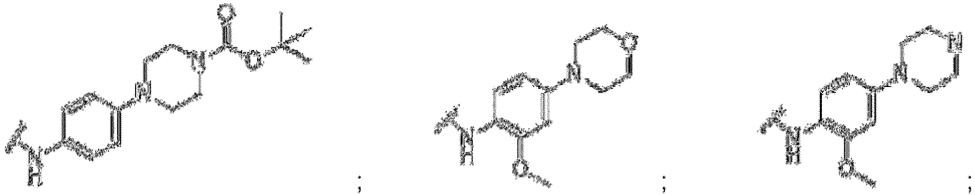

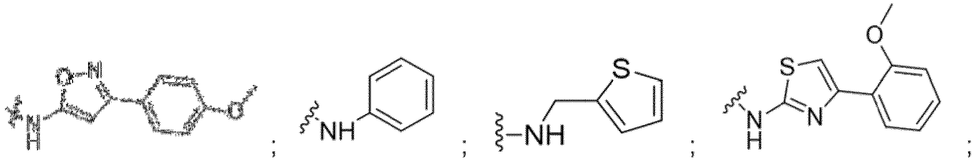

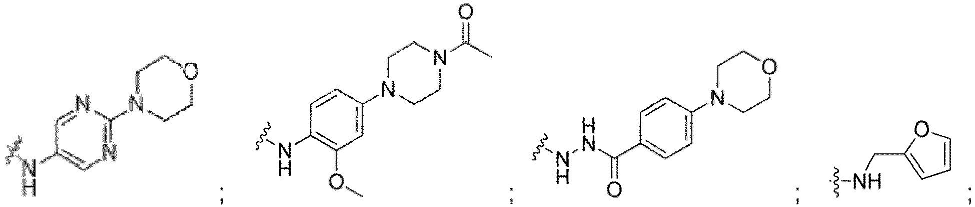

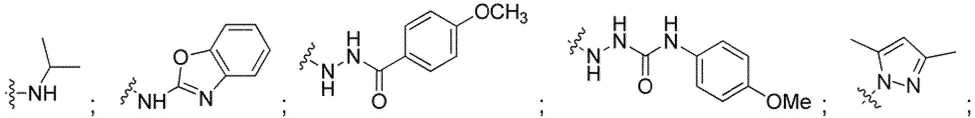

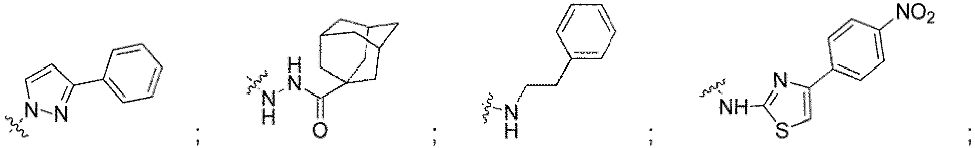

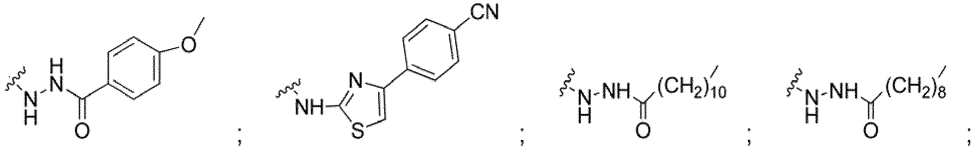

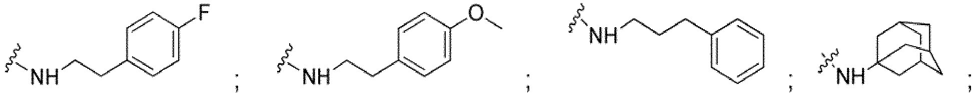

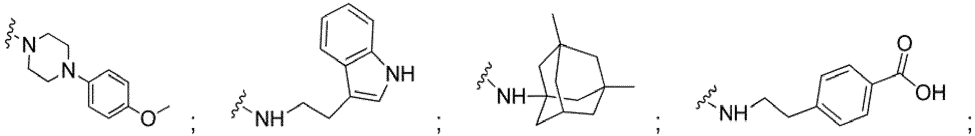

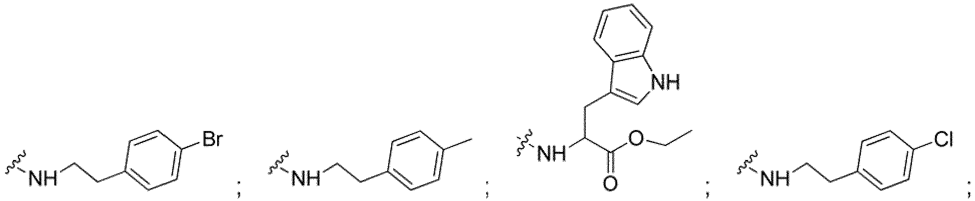

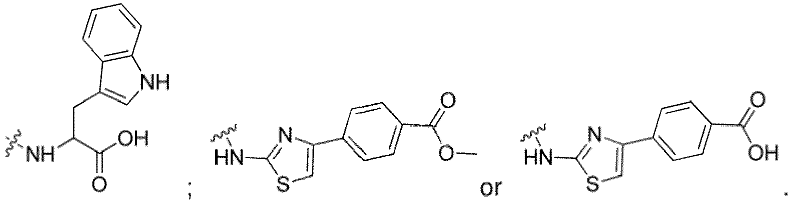

In order to achieve another object of the present invention, the present invention provides a pharmaceutical composition comprising the compound of the Formula 1 or a pharmaceutically acceptable salt thereof as an active ingredient.

In order to achieve another object of the present invention, the present invention provides a pharmaceutical composition consisting of the compound of the Formula 1 or a pharmaceutically acceptable salt thereof as an active ingredient.

In order to achieve another object of the present invention, the present invention provides a pharmaceutical composition essentially consisting of the compound of the Formula 1 or a pharmaceutically acceptable salt thereof as an active ingredient.

In order to achieve another object of the present invention, the present invention provides a use of the compound of the Formula 1 or a pharmaceutically acceptable salt thereof, for preparing an agent for preventing or treating cancer.

In order to achieve another object of the present invention, the present invention provides a method for treating cancer comprising administering to a subject in need thereof an effective amount of a composition comprising the compound of the Formula 1 or a pharmaceutically acceptable salt thereof as an active ingredient.

In order to achieve another object of the present invention, the present invention provides a use of the compound of the Formula 1 or a pharmaceutically acceptable salt thereof, for preparing an agent for preventing or treating inflammatory diseases.

In order to achieve another object of the present invention, the present invention provides a method for treating an inflammatory disease comprising administering to a isubject in need thereof an effective amount of a composition comprising the compound of the Formula 1 or a pharmaceutically acceptable salt thereof as an active ingredient.

Hereinafter, the present invention will be described in detail.

The present invention provides a compound represented by the following formula 1 or a pharmaceutically acceptable salt thereof: 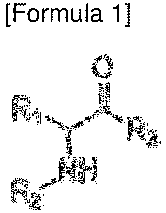 wherein Formula 1,
R1 is hydrogen; C1~C5 straight or branched alkyl substituted or unsubstituted with one or more substituents selected from the group consisting of hydroxy, substituted or unsubstituted phenyl, mercapto, and C1-C5 alkylthio; Indolylmethyl unsubstituted or substituted with one or more substituents selected from the group consisting of C1~C5 alkyl, hydroxy, hydroxycarbonyl and formyl; C1-C5 carboxymethyl; C7-C10 hydroxyphenylalkyl; substituted or unsubstituted C3~C8 cycloalkyl; substituted or unsubstituted C1~C5 aminoalkyl; 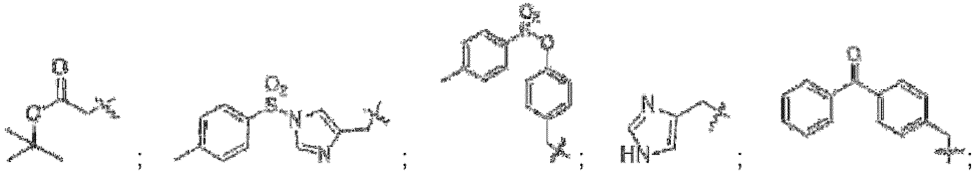 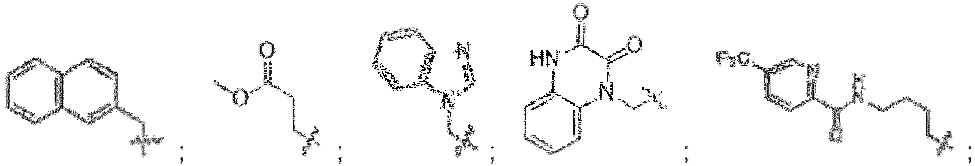 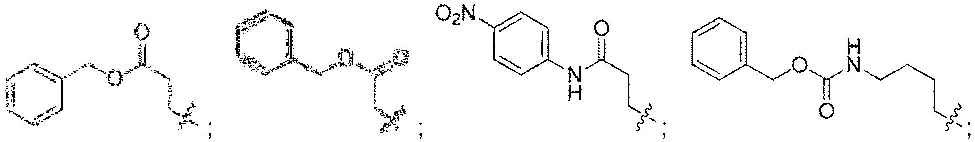 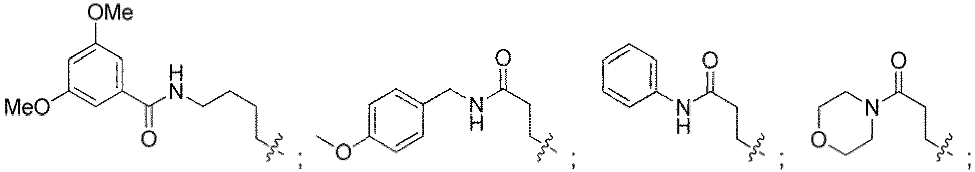 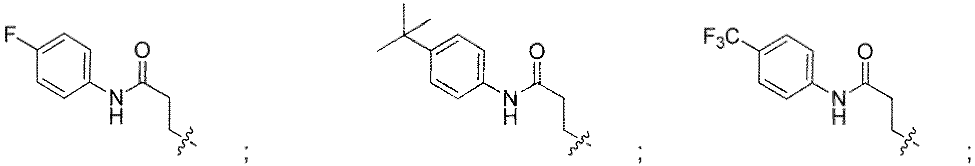 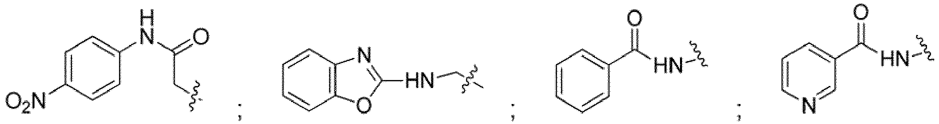 or 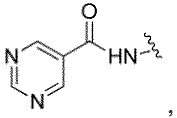
R2 is hydrogen; benzenesulfonyl unsubstituted or substituted with one or more substituents selected from the group consisting of C1~C5 straight or branched alkyl, nitro group, amine group, halo, -CF₃, C1~C5 alkylcarbonylamino, -COOH, C1~C5 alkoxy, phenyl, -OCH₂Ph, hydroxy, -COOCH₂Ph, C1~C5 alkyloxycarbonyl, -CONHCH₃ and nitrile; naphthalenesulfonyl; benzylsulfonyl; benzoyl unsubstituted or substituted with one or more substituents selected from the group consisting of C1~C5 straight or branched alkyl and C1-C5 straight or branched alkylamine groups; thiophenesulfonyl; 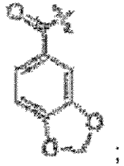 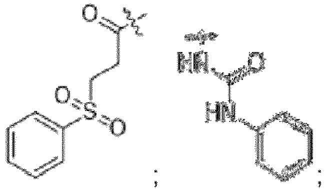 C1~C5 alkyloxycarbonyl; or phenylaminocarbonyl,
R3 is

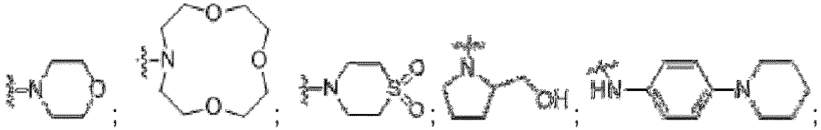

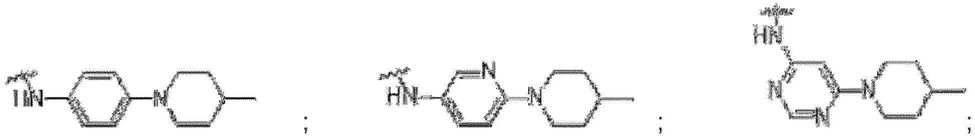

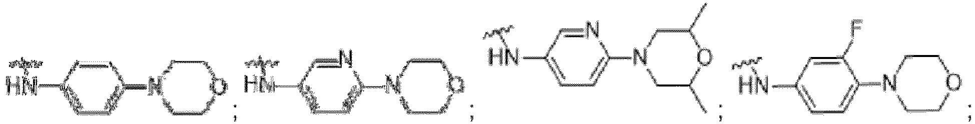

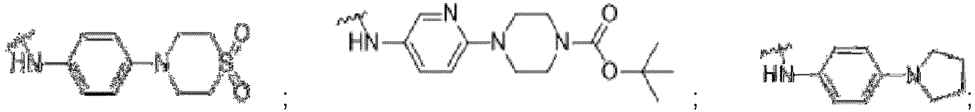

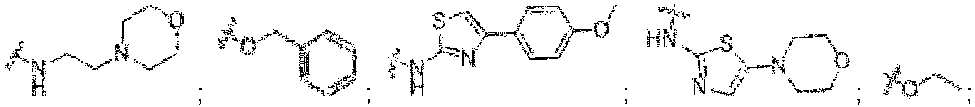

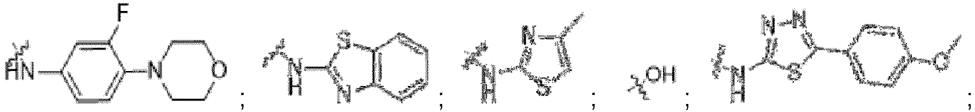

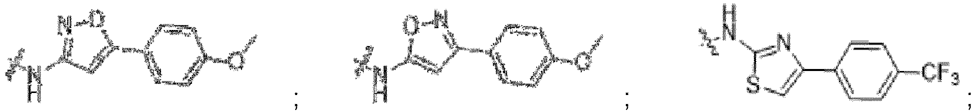

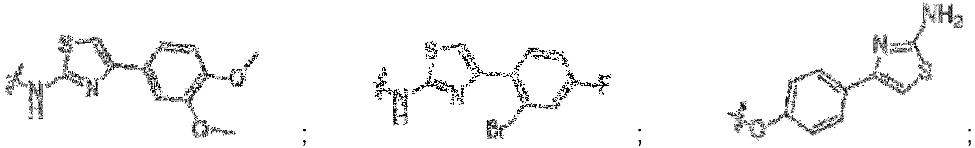

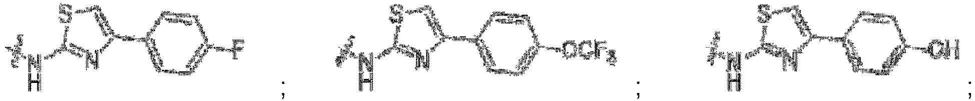

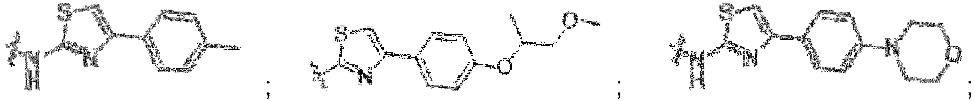

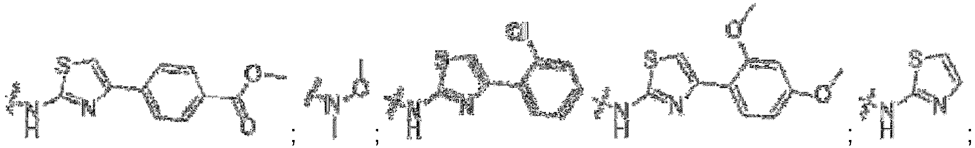

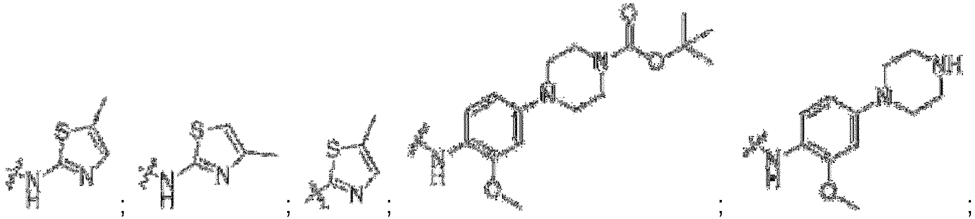

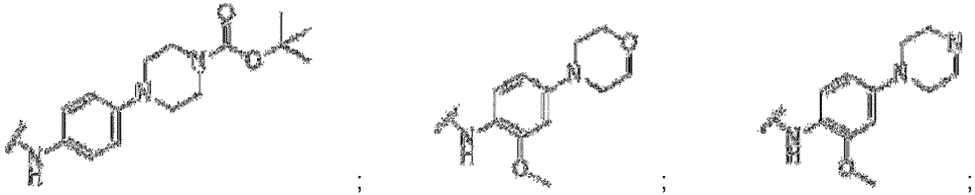

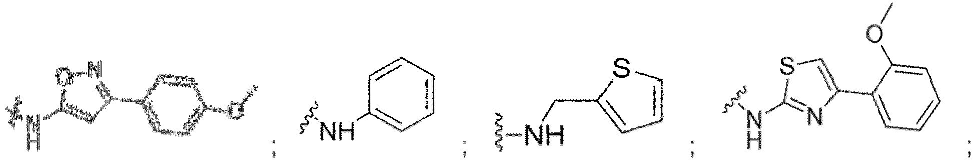

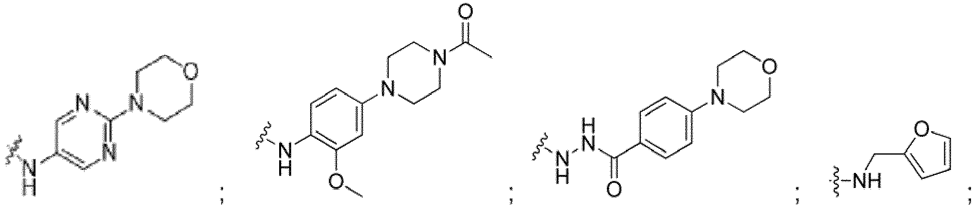

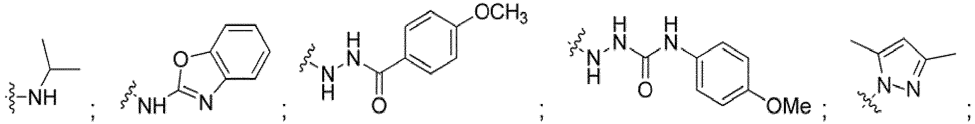

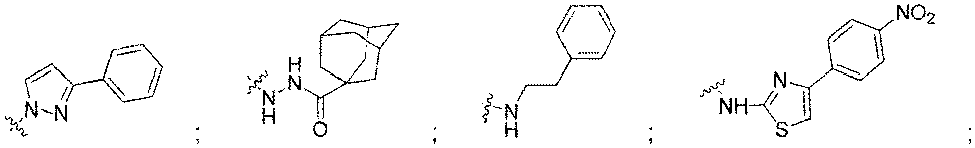

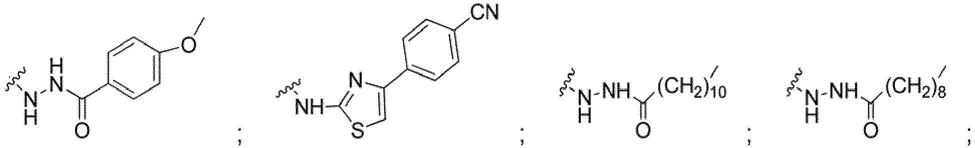

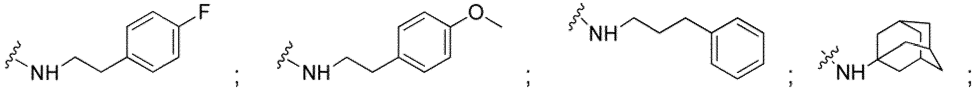

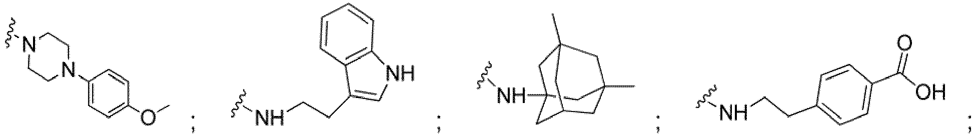

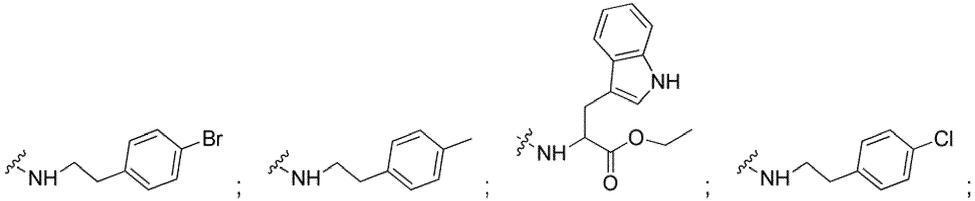

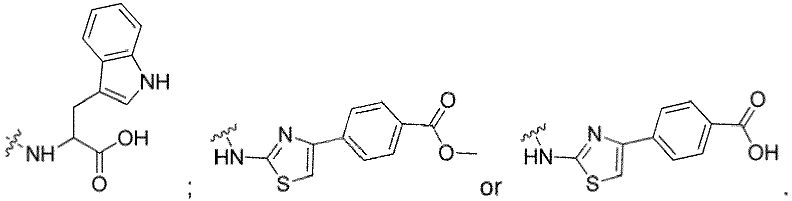

In the present invention, the term "alkyl" refers to an aliphatic hydrocarbon group, which may be straight or branched, containing 1 to 5 carbon atoms in the chain. Preferred alkyl groups contain 1 to 3 carbon atoms in the chain. More preferred alkyl groups contain about 1, 2 or 3 carbon atoms in the chain. Branched means that one or more lower alkyl groups such as methyl, ethyl or propyl are attached to the linear alkyl chain. "Lower alkyl" means a group having from 1 to 5 carbon atoms in the chain, which may be straight or branched. "Alkyl" may be unsubstituted or optionally substituted by one or more substituents, which may be the same or different.

In the present invention, "halo" or "halogen" means a fluorine atom, a chlorine atom, a bromine atom or an iodine atom.

When the groups disclosed herein are expressed as "substitution", these groups may be substituted with any suitable substituent or substituents. Illustrative examples of the substituent include those found in the exemplary compounds and embodiments disclosed herein, and halogens (chloro, iodo, bromo or fluoro group), alkyl, hydroxyl, alkoxy, alkoxyalkyl, amino, alkylamino, carboxy, nitro, ano, thiol, thioether, imine, imide, amidine, guanidine, enamine, aminocarbonyl, acylamino, phosphonato, phosphine, thiocarbonyl, sulfonyl, sulfone, sulfonamide, ketone, aldehyde, ester, urea, urethane, oxime, hydroxylamine, alkoxyamine, aralkoxyamine, N-oxide, hydrazine, hydrazide, hydrazone, azide, isocyanate, isothiocyanate, cyanate, thiocyanate, etc.

In one embodiment of the present invention, wherein R1 is indolylmethyl unsubstituted or substituted with one or more substituents selected from the group consisting of hydroxy, substituted or unsubstituted phenyl, mercapto and C1~C5 straight or branched alkyl substituted or unsubstituted with one or more substituents selected from the group consisting of C1-C5 alkylthio; or C1~C5 alkyl, hydroxy, hydroxycarbonyl and formyl,
wherein R2 is benzenesulfonyl unsubstituted or substituted with one or more substituents selected from the group consisting of C1~C5 straight or branched alkyl, nitro group, amine group, halo, -CF₃, C1~C5 alkylcarbonylamino, -COOH, C1~C5 alkoxy, phenyl, -OCH₂Ph, hydroxy, -COOCH₂Ph, C1~C5 alkyloxycarbonyl, -CONHCH₃ and nitrile,

Wherein R3 may be

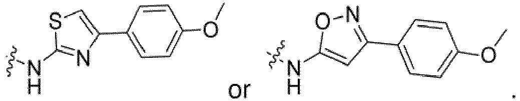

Preferably, wherein R1 is an unsubstituted C1~C5 straight or branched alkyl; or unsubstituted indolylmethyl,
wherein R2 is benzenesulfonyl unsubstituted or substituted with C1~C5 straight or branched alkyl,
wherein R3 may be

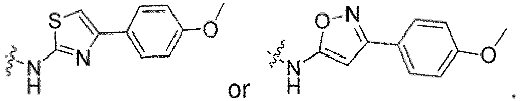

In one embodiment of the present invention, wherein R1 may be preferably hydrogen or cyclohexane, 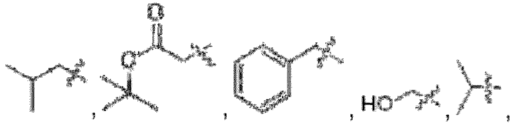 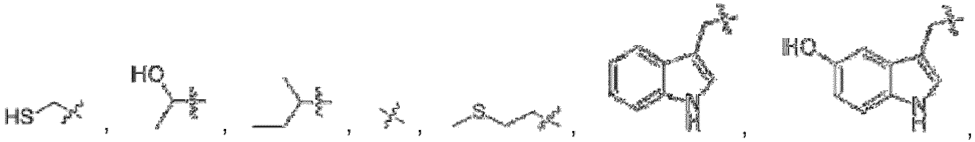 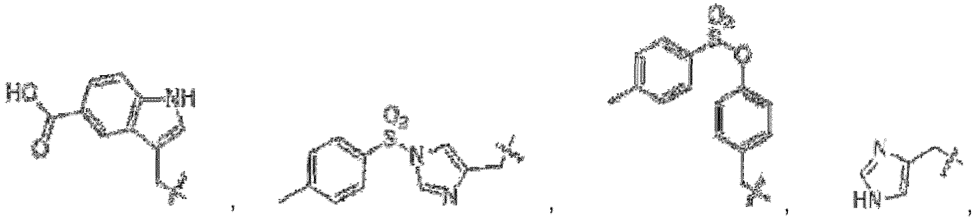 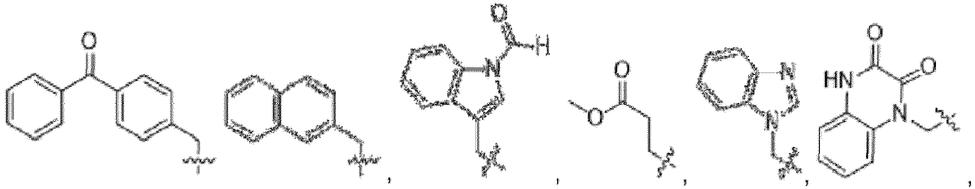 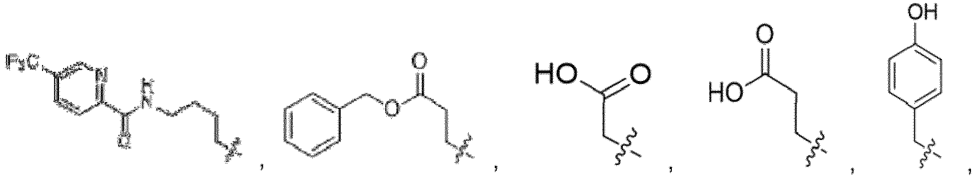 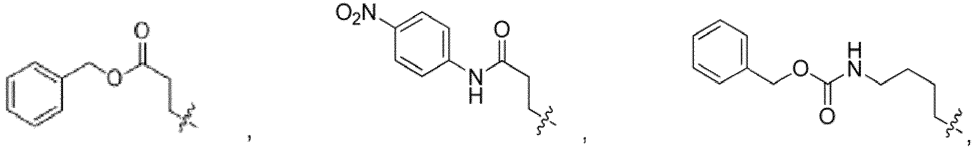 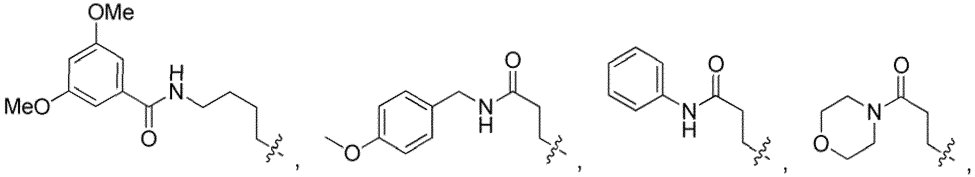 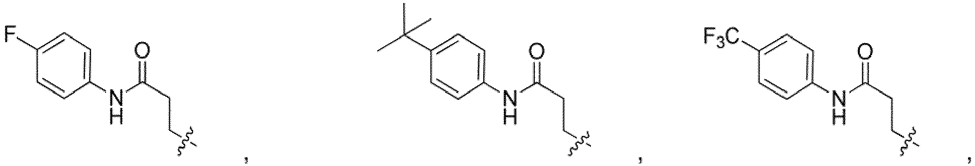 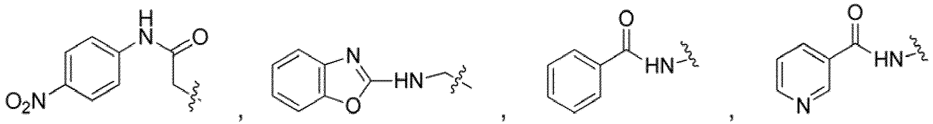 or

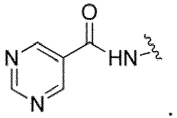

In another embodiment of the present invention, wherein R1 may be most preferably

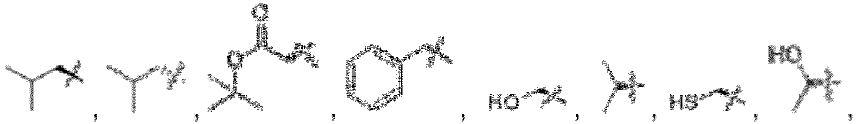

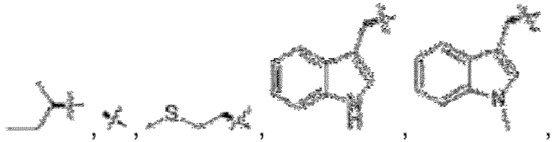

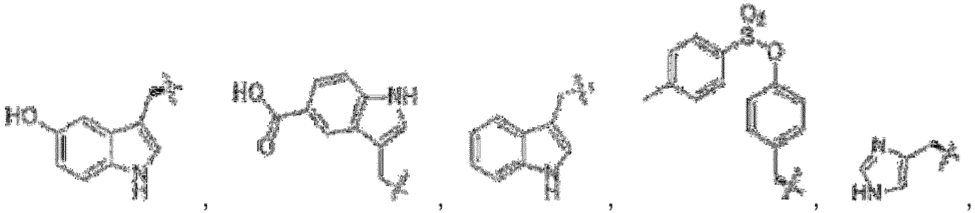

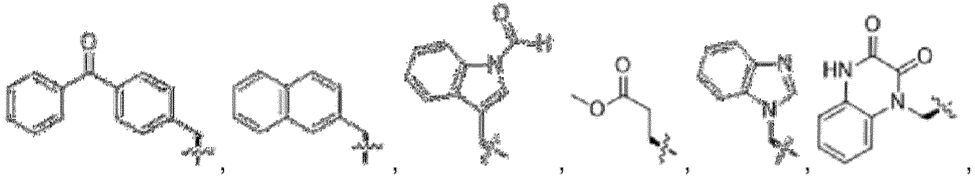

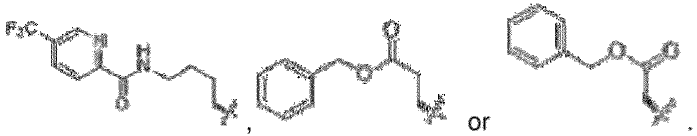

In another embodiment of the present invention, wherein R2 may be preferably hydrogen,

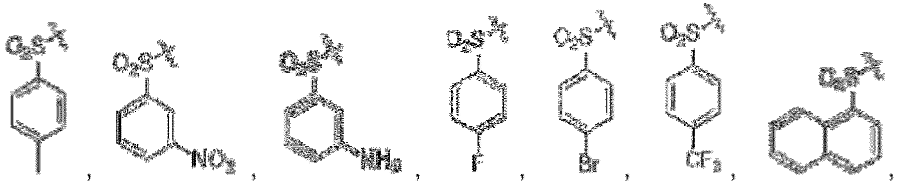

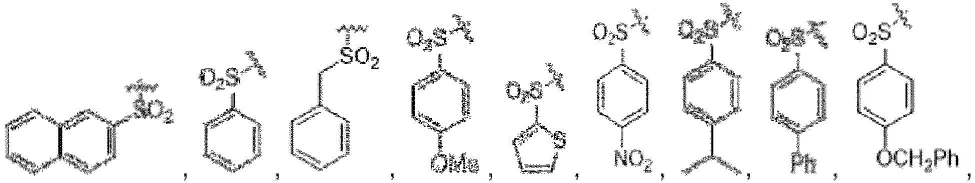

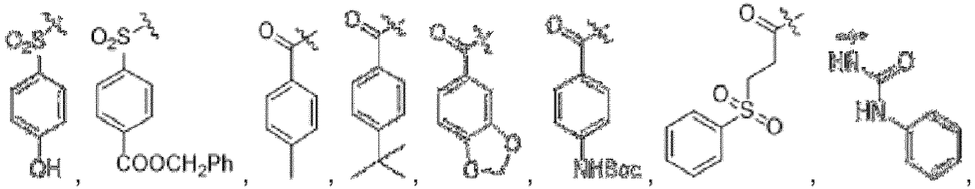

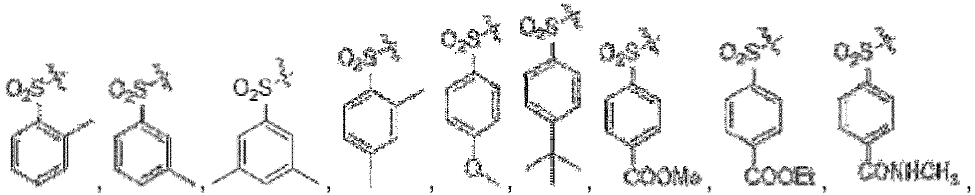

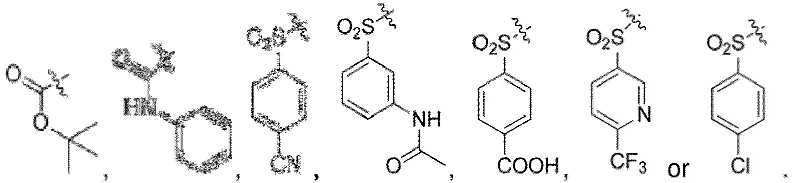

In another embodiment of the present invention, wherein R3 may be preferably

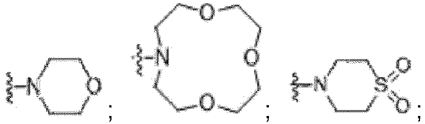

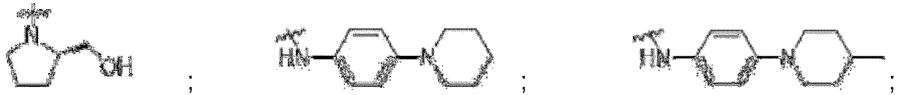

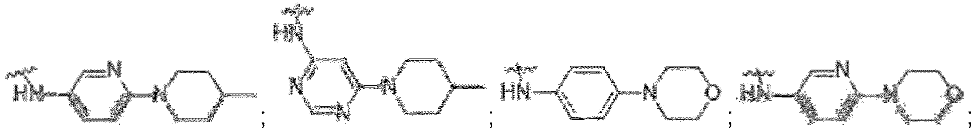

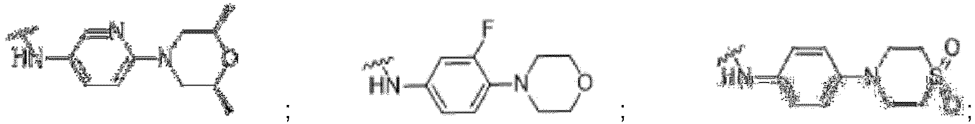

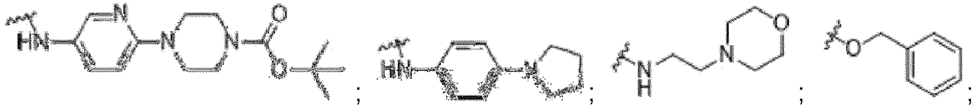

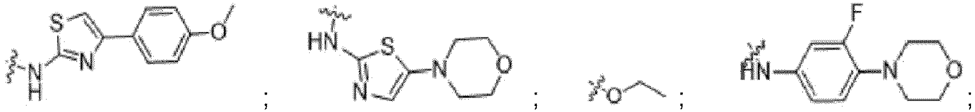

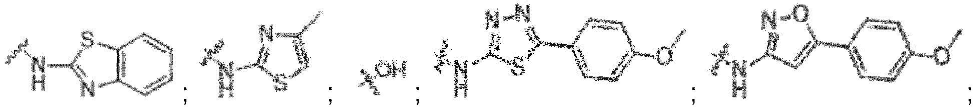

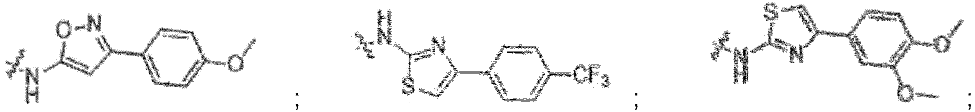

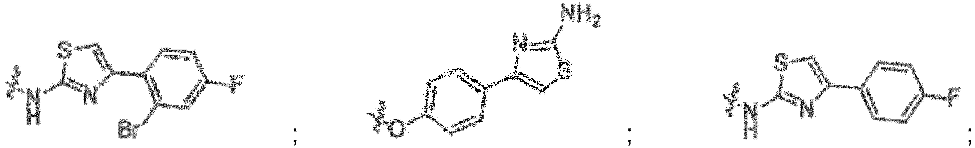

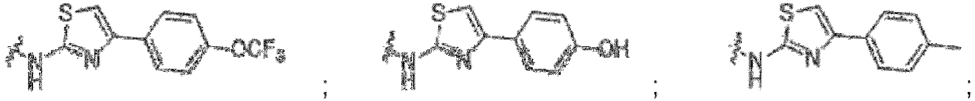

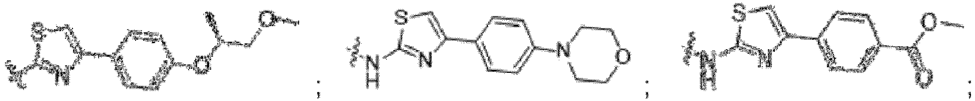

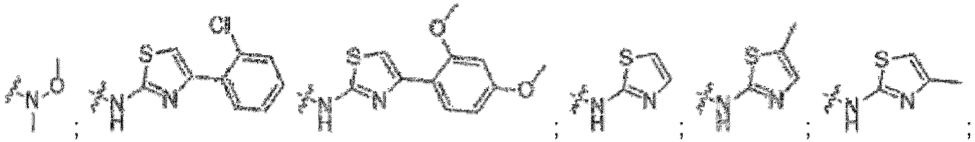

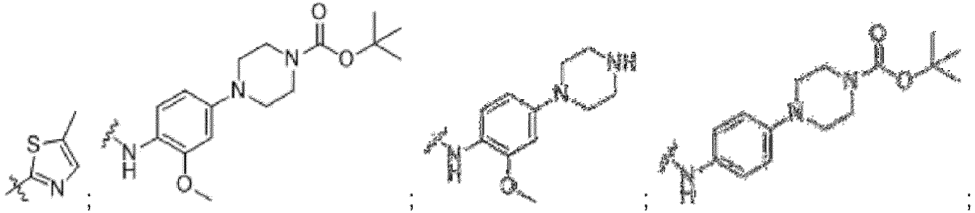

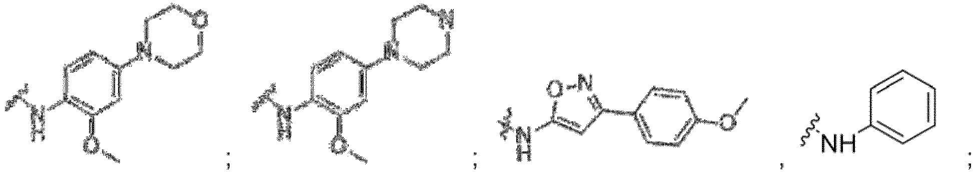

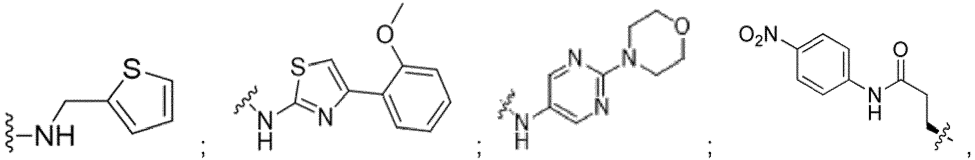

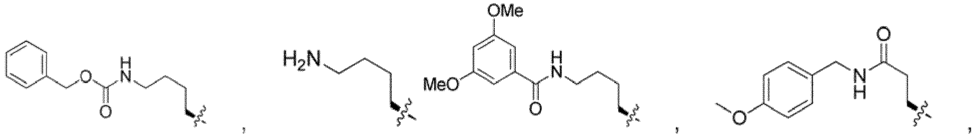

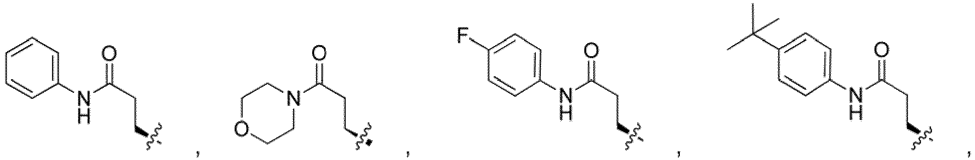

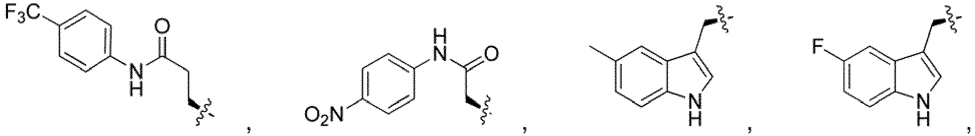

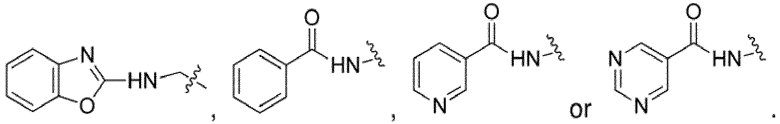

In addition, in the present invention, wherein the compound of Formula 1 may be
(*S*)-4-methyl-*N*-(4-methyl-1-morpholino-1-oxopentan-2-yl)benzenesulfonamide,
(*R*)-4-methyl-*N*-(4-methyl-1-morpholino-1-oxopentan-2-yl)benzenesulfonamide,
(*S*)-4-methyl-*N*-(4-methyl-1-oxo-1-(1,4,7-trioxa-10-azacyclododecan-10-yl)pentan-2-yl)benzenesulfonamide,
(*R*)-4-methyl-*N*-(4-methyl-1-oxo-1-(1,4,7-trioxa-10-azacyclododecan-10-yl)-Pentan-2-yl)benzenesulfonamide,
(*S*)-*N*-(1-(1,1-dioxidothiomorpholino)-4-methyl-1-oxopentan-2-yl)-4-methylbenzenesulfonamide,
(*N*-((*S*)-1-((*R*)-2-(hydroxymethyl)pyrrolidin-1-yl)-4-methyl-1-oxopentan-2-yl)-4-methylbenzenesulfonamide,
(*S*)-4-methyl-2-(4-methylphenylsulfonamido)-*N*-(4-(piperidin-1-yl)phenyl)pentanamide,
(*S*)-4-methyl-2-(4-methylphenylsulfonamido)-*N*-(4-(4-methylpiperidin-1-yl)phenyl) pentanamide,
(*S*)-4-Methyl-2-(4-methylphenylsulfonamido)-*N*-(6-(4-methylpiperidin-1-yl)pyridin-3-yl)pentanamide,
(*S*)-4-methyl-2-(4-methylphenylsulfonamido)-*N*-(6-(4-methylpiperidin-1-yl)pyrimidin-4-yl)pentanamide,
(*S*)-4-methyl-2-(4-methylphenylsulfonamido)-*N*-(4-morpholinophenyl)pentanamide,
(*S*)-4-Methyl-2-(4-methylphenylsulfonamido)-*N*-(6-morpholinopyridin-3-yl)pentanamide,
(*S*)-*N*(6-((2*S*,6*R*)-2,6-dimethylmorpholino)pyridin-3-yl)-4-methyl-2-(4-methylphenylsulfonamido)pentanamide,
(*S*)-*N*-(3-fluoro-4-morpholinophenyl)-4-methyl-2-(4-methylphenylsulfonamido)pentanamide,
(*S*)-*N*-(4-(1,1 -dioxidothiomorpholino)phenyl)-4-methyl-2-(4-methylphenylsulfonamido)pentanamide,
(*S*)*-tert*-butyl 4-(5-(4-methyl-2-(4-methylphenylsulfonamido)pentanamido) pyridin-2-yl)piperazine-1-carboxylate,
(*S*)-4-methyl-2-(4-methylphenylsulfonamido)-*N*-(4-(pyrrolidin-1-yl)phenyl)pentanamide,
(*S*)-4-methyl-2-(4-methylphenylsulfonamido)-*N*-(2-morpholinoethyl)pentanamide,
(*S*)-*N*-(4-methyl-1-morpholino-1-oxopentan-2-yl)-3-nitrobenzenesulfonamide,
(*S*)-3-amino-*N*-(4-methyl-1-morpholino-1-oxopentan-2-yl)benzenesulfonamide,
(*S*)-2-(4-fluorophenylsulfonamido)-4-methyl-*N*-(4-morpholinophenyl)pentanamide,
(*S*)-2-(4-bromophenylsulfonamido)-4-methyl-*N*-(4-morpholinophenyl)pentanamide,
(*S*)-4-methyl-*N*-(4-morpholinophenyl)-2-(4-(trifluoromethyl)phenylsulfonamido)pentanamide,
(*S*)-4-methyl-*N*-(4-morpholinophenyl)-2-(3-nitrophenylsulfonamido)pentanamide,
(*S*)-4-methyl-*N*-(4-morpholinophenyl)-2-(naphthalene-1-sulfonamido)pentanamide,
(*S*)-4-methyl-*N*-(4-morpholinophenyl)-2-(naphthalene-2-sulfonamido)pentanamide,
(*S*)-4-methyl-*N*-(4-morpholinophenyl)-2-(phenylsulfonamido)pentanamide,
(*S*)-Benzyl 4-methyl-2-(3-(phenylsulfonyl)propanamido)pentanoate,
(*S*)-2-(4-methoxyphenylsulfonamido)-4-methyl-*N*-(4-morpholinophenyl)pentanamide,
(*S*)-4-methyl-*N*-(4-morpholinophenyl)-2-(thiophene-2-sulfonamido)pentanamide,
(*S*)-4-methyl-*N*-(4-morpholinophenyl)-2-(4-nitrophenylsulfonamido)pentanamide,
(*S*)-2-(4-isopropylphenylsulfonamido)-4-methyl-*N*-(4-morpholinophenyl)pentanamide,
(*S*)-2-(biphenyl-4-ylsulfonamido)-4-methyl-*N*-(4-morpholinophenyl)pentanamide,
(*S*)-4-(benzyloxy)-*N*-(4-methyl-1-oxo-1-(1,4,7-trioxa-10-azacyclododecan-10-yl)pentan-2-yl)benzenesulfonamide,
(*S*)-4-hydroxy-*N*-(4-methyl-1-oxo-1-(1,4,7-trioxa-10-azacyclododecan-10-yl)pentan-2-yl)benzenesulfonamide,
(S)-benzyl 4-(*N*-(4-methyl-1-oxo-1-(1 ,4,7-trioxa-10-azacyclododecan-10-yl)pentan-2-yl)sulfamoyl)benzoate,
(*S*)*-tert*-butyl 3-(4-methylphenylsulfonamido)-4-morpholino-4-oxobutanoate,
(*S*)-4-methyl-*N*-(1-morpholino-1-oxo-3-phenylpropan-2-yl)benzenesulfonamide,
(*S*)-*N*-(3-hydroxy-1-morpholino-1-oxopropan-2-yl)-4-methylbenzenesulfonamide,
(*S*)-4-methyl-*N*-(3-methyl-1-morpholino-1-oxobutan-2-yl)benzenesulfonamide,
(*R*)-*N*-(3-mercapto-1-morpholino-1-oxopropan-2-yl)-4-methylbenzenesulfonamide,
(2*S*,3*S*)-3-hydroxy-2-(4-methylphenylsulfonamido)-*N*-(4-morpholinophenyl)butanamide,
(2*S*,3*S*)-3-methyl-2-(4-methylphenylsulfonamido)-*N*-(4-morpholinophenyl)pentanamide,
(*S*)-2-(4-methylphenylsulfonamido)-*N*-(4-morpholinophenyl)propanamide,
(*S*)-2-(4-methylphenylsulfonamido)-4-(methylthio)-*N*-(4-morpholinophenyl)butanamide,
(*S*)-3-(1*H*-indol-2-yl)-2-(4-methylphenylsulfonamido)-*N*-(4-morpholinophenyl) propanamide,
(*S*)-3-(1-methyl-1*H*-indol-3-yl)-2-(4-methylphenylsulfonamido)-*N*-(4-morpholinophenyl) propanamide,
(*S*)-2-(4-methylphenylsulfonamido)-*N*-(4-morpholinophenyl)-3-phenylpropanamide,
(S)-benzyl 4-methyl-2-(4-methylphenylsulfonamido)pentanoate,
(*S*)-benzyl 4-methyl-2-(4-methylbenzamido)pentanoate,
(*S*)-benzyl 2-(4-*tert*-butylbenzamido)-4-methylpentanoate,
(*S*)-benzyl 2-(benzo[d][1,3]dioxole-5-carboxamido)-4-methylpentanoate,
(*S*)-benzyl 2-(4-(tert-butoxycarbonylamino)benzamido)-4-methylpentanoate,
(*S*)-benzyl 4-methyl-2-(3-(phenylsulfonyl)propanamido)pentanoate,
(*S*)-*N*-(4-(4-methoxyphenyl)thiazol-2-yl)-4-methyl-2-(4-methylphenylsulfonamido)pentanamide,
(*S*)-1-(4-methyl-1-morpholino-1-oxopentan-2-yl)-3-phenylurea,
(*S*)-4-*tert*-butyl-*N*-(4-methyl-1-morpholino-1-oxopentan-2-yl)benzamide,
(*S*)-*N*-(4-methyl-1-morpholino-1-oxopentan-2-yl)-3-(phenylsulfonyl)propanamide,
(*S*)-4-*tert*-butyl-*N*-(4-methyl-1-(4-morpholinophenylamino)-1-oxopentan-2-yl)benzamide,
(*S*)-*N*-(4-methyl-1-(4-morpholinophenylamino)-1-oxopentan-2-yl)benzo[d][1,3]dioxole-5-carboxamide,
(*S*)-4-methyl-*N*-(4-morpholinophenyl)-2-(3-(phenylsulfonyl)propanamido)pentanamide,
(*S*)-2-(4-bromophenylsulfonamido)-3-(1*H*-indol-3-yl)-*N*-(4-(4-methoxyphenyl)thiazol-2-yl)propanamide,
(*S*)-3-(1*H*-indol-3-yl)-*N*-(4-(4-methoxyphenyl)thiazol-2-yl)-2-(4-methylphenylsulfonamido)propanamide,
(*S*)-3-(1*H*-indol-3-yl)-2-(2-methylphenylsulfonamido)-*N*-(4-morpholinophenyl)propanamide,
(*S*)-3-(1*H*-indol-3-yl)-2-(3-methylphenylsulfonamido)-*N*-(4-morpholinophenyl)propanamide,
(S)-2-(3,5-dimethylphenylsulfonamido)-3-(1H-indol-3-yl)-N-(4 morpholinophenyl)propanamide,
(*S*)-2-(4-Fluorophenylsulfonamido)-3-(1*H*-indol-3-yl)-*N*-(4-morpholinophenyl)propanamide,
(S)-3-(1*H*-indol-3-yl)-*N*-(4-morpholinophenyl)-2-(4-(trifluoromethyl)phenylsulfon-amido)propanamide,
(*S*)-3-(5-Hydroxy-1*H*-indol-3-yl)-2-(4-methylphenylsulfonamido)-*N*-(4-morpholinophenyl)propanamide,
(*S*)-*N*-(3-(1*H*-indol-3-yl)-1-morpholino-1-oxopropan-2-yl)-4-methylbenzenesulfonamide,
(*S*)-3-(*1*H-indol-3-yl)-2-(4-methylphenylsulfonamido)-*N*-(5-morpholinothiazol-2-yl)propan amide,
(*S*)-2-(2,4-dimethylphenylsulfonamido)-3-(1*H*-indol-3-yl)-*N*-(4-morpholinophenyl)propanamide,
3-(3-ethoxy-2-(4-methylphenylsulfonamido)-3-oxopropyl)-1*H*-indole-5-carboxylic acid,
(*S*)-2-(4-methylphenylsulfonamido)-*N*-(4-morpholinophenyl)-3-(1-tosyl-1*H-*imidazol-4-yl)propanamide,
(*S*)-*N*-(6-((2S,6R)-2,6-dimethylmorpholino)pyridin-3-yl)-3-(1*H*-indol-3-yl)-2-(4-methylphenylsulfonamido)propenamide,
(*R*)-3-(1*H*-indol-3-yl)-2-(4-methylphenylsulfonamido)-*N*-(4-morpholinophenyl)propanamide,
(S)-*N*-(3-fluoro-4-morpholinophenyl)-3-(1*H*-indol-3-yl)-2-(4-methylphenylsulfonamido)propanamide,
(*S*)-3-(1*H*-indol-3-yl)-*N*-(4-morpholinophenyl)-2-(4-nitrophenylsulfonamido)propanamide,
(*S*)-2-(4-bromophenylsulfonamido)-3-(1*H*-indol-3-yl)-*N*-(4-morpholinophenyl)propanamide,
(*S*)-3-(1*H*-indol-3-yl)-2-(4-methoxyphenylsulfonamido)-*N*-(4-morpholinophenyl)propanamide,
(*S*)-3-(1*H*-indol-3-yl)-2-(4-isopropylphenylsulfonamido)-*N*-(4 morpholinophenyl)propanamide,
(*S*)-3-(1*H*-indol-3-yl)-2-(4-isopropylphenylsulfonamido)-*N*-(4-(4-methoxyphenyl)thiazol-2-yl)propenamide,
(*S*)-3-(1*H*-indol-3-yl)-2-(4-methylphenylsulfonamido)-*N*-(4-methylthiazol-2-yl)propanamide,
(*S*)-*N*-(benzo[d]thiazol-2-yl)-3-(1*H*-indol-3-yl)-2-(4-methylphenylsulfonamido)propanamide,
(*S*)-2-(4-bromophenylsulfonamido)-3-(1*H*-indol-3-yl)propanoic acid,
(*S*)-4-methyl-2-(4-methylphenylsulfonamido)-*N*-(5-morpholinothiazol-2-yl)pentanamide,
(*S*)-*N*-(5-(4-methoxyphenyl)-1 ,3,4-thiadiazol-2-yl)-4-methyl-2-(4-methylphenylsulfonamido)pentanamide,
(*S*)-*N*-(5-(4-methoxyphenyl)isoxazol-3-yl)-4-methyl-2-(4-methylphenylsulfonamido)pentanamide,
(*S*)-*N*-(3-(4-methoxyphenyl)isoxazol-5-yl)-4-methyl-2-(4-methylphenylsulfonamido)pentanamide,
(*S*)-4-methyl-2-(4-methylphenylsulfonamido)-*N*-(4-(4-(trifluoromethyl)phenyl)thiazol-2-yl)pentanamide,
(*S*)-*N*-(4-(3,4-dimethoxyphenyl)thiazol-2-yl)-4-methyl-2-(4-methylphenylsulfonamido)pentanamide,
(*S*)-*N*-(4-(2-bromo-4-fluorophenyl)thiazol-2-yl)-4-methyl-2-(4-methylphenylsulfonamido)pentanamide,
(*S*)-4-(2-aminothiazol-4-yl)phenyl-4-methyl-2-(4-methylphenylsulfonamido)pentanoate,
(*S*)-*N*-(4-(4-fluorophenyl)thiazol-2-yl)-4-methyl-2-(4-methylphenylsulfonamido)pentanamide,
(*S*)-4-methyl-2-(4-methylphenylsulfonamido)-*N*-(4-(4-(trifluoromethoxy)phenyl)thiazol-2-yl)pentanamide,
(*S*)-*N*-(4-(4-hydroxyphenyl)thiazol-2-yl)-4-methyl-2-(4-methylphenylsulfonamido)pentanamide
(*S*)-4-methyl-2-(4-methylphenylsulfonamido)-*N*-(4-p-tolylthiazol-2-yl)pentanamide,
(*S*)-*N*-(4-(4-((*S*)-1-methoxypropan-2-yloxy)phenyl)thiazol-2-yl)-4-methyl-2-(4-methylphenylsulfonamido)pentanamide,
(*S*)-4-methyl-2-(4-methylphenylsulfonamido)-*N*-(4-(4-morpholinophenyl)thiazol-2-yl)pentanamide,
(S)-methyl-4-(2-(4-methyl-2-(4-methylphenylsulfonamido)pentanamido)thiazol-4-yl)benzoate,
(*S*)-4-(2-(4-methylphenylsulfonamido)-3-(4-morpholinophenylamino)-3-oxopropyl)phenyl 4-methylbenzenesulfonate,
(*S*)-2-(4-bromophenylsulfonamido)-3-(1*H*-imidazol-4-yl)-*N*-(4-(4-methoxyphenyl)thiazol-2-yl)propanamide,
(*S*)-*N*-methoxy-*N*,4-dimethyl-2-(4-methylphenylsulfonamido)pentanamide,
(*S*)-3-(1*H*-indol-3-yl)-*N*-methoxy-*N*-methyl-2-(4-methylphenylsulfonamido)propanamide,
(*S*)-*N*-(3-(1*H*-indol-3-yl)-1-oxo-1 -(1 ,4,7-trioxa-1 0-azacyclododecan-1 0-yl)propan-2-yl)-4-methylbenzenesulfonamide,
(*S*)-2-(4-*tert*-butylphenylsulfonamido)-3-(1*H*-indol-3-yl)-*N*-(4-morpholinophenyl)propan amide,
(S)-2-(4-bromophenylsulfonamido)-*N*-(4-(2-chlorophenyl)thiazol-2-yl)-3-(1*H-*indol-3-yl)propanamide,
(*S*)-2-(4-bromophenylsulfonamido)-*N*-(4-(2,4-dimethoxyphenyl)thiazol-2-yl)-3-(1*H*-indol-3-yl)propanamide,
(*S*)-2-(4-bromophenylsulfonamido)-3-(1*H*-indol-3-yl)-*N*-(thiazol-2-yl)propanamide,
(*S*)-2-(4-bromophenylsulfonamido)-3-(1*H*-indol-3-yl)-*N*-(5-methylthiazol-2-yl)propanamide,
(S)-3-(1*H*-indol-3-yl)-2-(4-methylphenylsulfonamido)-*N*-(4-methylthiazol-2-yl)propanamide,
(*S*)-3-(1*H*-indol-3-yl)-2-(4-methylphenylsulfonamido)-*N*-(5-methylthiazol-2-yl)propanamide,
(S)-methyl 4-(*N*-(3-(1*H*-indol-3-yl)-1-(4-morpholinophenylamino)-1-oxopropan-2-yl)sulfamoyl)benzoate,
(S)-ethyl 4-(*N*-(3-(1*H*-indol-3-yl)-1-(4-morpholinophenylamino)-1-oxopropan-2-yl)sulfamoyl)benzoate,
(*S*)-3-(4-benzoylphenyl)-2-(4-methylphenylsulfonamido)-*N*-(4-morpholinophenyl)propanamide,
(*S*)-4-(*N*-(3-(1*H*-indol-3-yl)-1-(4-morpholinophenylamino)-1-oxopropan-2-yl)sulfamoyl)-*N*-methylbenzamide,
(*S*)-2-(4-methylphenylsulfonamido)-*N*-(4-morpholinophenyl)-3-(naphthalen-2-yl)propanamide,
(*S*)*-tert*-butyl 3-(1*H*-indol-3-yl)-1-(4-morpholinophenylamino)-1-oxopropan-2-ylcarbamate,
(*S*)-3-(1*H*-indol-3-yl)-2-(4-methylphenylsulfonamido)propanoic acid,
(*S*)-*tert*-butyl-4-(4-(3-(1H-indol-3-yl)-2-(4-methylphenylsulfonamido)propanamido)-3-methoxyphenyl)piperazine-1-carboxylate,
(*S*)-3-(1*H*-indol-3-yl)-*N*-(2-methoxy-4-(piperazin-1-yl)phenyl)-2-(4-methylphenylsulfonamido)propanamide,
(*S*)-*tert*-butyl-4-(4-(3-(1*H*-indol-3-yl)-2-(4-methylphenylsulfonamido)propanamido)phenyl)piperazine-1-carboxylate,
(*S*)-3-(1*H*-indol-3-yl)-*N*-(2-methoxy-4-morpholinophenyl)-2-(4-methylphenylsulfonamido)propanamide,
(*S*)-3-(1*H*-indol-3-yl)-2-(4-methylphenylsulfonamido)-*N*-(4-(piperazin-1-yl)phenyl)propanamide,
(*S*)-3-(1-formyl-1*H*-indol-3-yl)-2-(4-methylphenylsulfonamido)-*N*-(4-morpholinophenyl)propanamide,
(*S*)-3-(1*H*-indol-3-yl)-*N*-(3-(4-methoxyphenyl)isoxazol-5-yl)-2-(4-methylphenylsulfonamido)propanamide,
(*S*)-benzyl 3-(1*H*-indol-3-yl)-2-(4-methylphenylsulfonamido)propanoate,
(*S*)-benzyl 3-(1*H*-indol-3-yl)-2-(4-methylbenzamido)propanoate,
(*S*)-*N*-(3-(1*H*-indol-3-yl)-1-(4-morpholinophenylamino)-1-oxopropan-2-yl)-4-methylbenzamide,
(*S*)-3-hydroxy-2-(4-methylphenylsulfonamido)-*N*-(4-morpholinophenyl)propanamide,
(*S*)-3-methyl-2-(4-methylphenylsulfonamido)-*N*-(4-morpholinophenyl)butanamide,
(*S*)-1-(3-(1*H*-indol-3-yl)-1-morpholino-1-oxopropan-2-yl)-3-phenylurea,
(*S*)-3-(1*H*-indol-3-yl)-2-(4-methylphenylsulfonamido)-*N*-(6-morpholinopyridin-3-yl)propanamide,
(*S*)-3-(1*H*-indol-3-yl)-2-(4-methylphenylsulfonamido)-*N*-(2-morpholinopyrimidin-5-yl)propanamide,
(*S*)-2-(4-cyanophenylsulfonamido)-3-(1*H*-indol-3-yl)-*N*-(4-morpholinophenyl)propanamide,
(*S*)-3-(1*H*-indol-3-yl)-*N*-(4-morpholinophenyl)-2-(6-(trifluoromethyl)pyridine-3-sulfonamido)propanamide,
(S)-methyl 4-(4-methylphenylsulfonamido)-5-(4-morpholinophenylamino)-5-oxopentanoate,
(*S*)-3-(1*H*-benzo[*d*]imidazol-1-yl)-2-(4-methylphenylsulfonamido)-*N*-(4-morpholinophenyl)propanamide,
(*R*)-3-(2,3-dioxo-3,4-dihydroquinoxalin-1(2H)-yl)-2-(4-methylphenylsulfonamido)-*N*-(4-morpholinophenyl)propanamide,
(*S*)-*N*-(5-(4-methylphenylsulfonamido)-6-(4-morpholinophenylamino)-6-oxohexyl)-5-(trifluoromethyl)picolinamide,
(*S*)-benzyl 4-(4-methylphenylsulfonamido)-5-(4-morpholinophenylamino)-5-oxopentanoate,
(*S*)-benzyl 3-(4-methylphenylsulfonamido)-4-(4-morpholinophenylamino)-4-oxobutanoate,
(*S*)-4-methyl-*N*-(4-methyl-1-(4-morpholinophenylamino)-1-oxopentan-2-yl)benzamide,
2-(4-methylphenylsulfonamido)-*N*-(4-morpholinophenyl)acetamide,
(S)-3-(4-methylphenylsulfonamido)-4-(4-morpholinophenylamino)-4-oxobutanoic acid,
(*S*)-4-(4-methylphenylsulfonamido)-5-(4-morpholinophenylamino)-5-oxopentanoic acid,
(*S*)-2-(4-methylphenylsulfonamido)-*N*-(4-morpholinophenyl)-3-phenylpropanamide,
(*S*)-3-(1*H*-indol-3-yl)-2-(4-methylphenylsulfonamido)-*N*-phenylpropanamide,
(*S*)-2-(4-bromophenylsulfonamido)-3-(1*H*-indol-3-yl)-*N*-phenylpropanamide,
(*S*)-3-(1*H*-indol-3-yl)-2-(4-methylphenylsulfonamido)-*N*-(thiophen-2-ylmethyl)propanamide,
(*S*)-2-(4-bromophenylsulfonamido)-3-(1*H*-indol-3-yl)-*N*-(thiophen-2-ylmethyl)propanamide,
(*S*)-2-(4-bromophenylsulfonamido)-3-(1*H*-indol-3-yl)-*N*-(4-(2-methoxyphenyl)thiazol-2-yl)propanamide,
(*S*)-*N*-(3-(*N*-(4-methyl-1-morpholino-1-oxopentan-2-yl)sulfamoyl)phenyl)acetamide,
(*S*)-Benzyl 4-methyl-2-(3-phenylureido)pentanoate,
4-Methyl-*N*-(2-morpholino-2-oxoethyl)benzenesulfonamide,
4-Methyl-*N*-(1-(morpholine-4-carbonyl)cyclohexyl)benzenesulfonamide,
(*S*)-4-(*N*-(4-methyl-1-oxo-1-(1,4,7-trioxa-10-azacyclododecan-10-yl)pentan-2-yl)sulfamoyl)benzoic acid,
(*S*)-2-(4-Hydroxyphenylsulfonamido)-3-(1*H*-indol-3-yl)-*N*-(4-morpholinophenyl)propenamide,
(*S*)-4-(*N*-(3-(1*H*-indol-3-yl)-1-(4-morpholinophenylamino)-1-oxopropan-2-yl)sulfamoyl)benzoic acid,
(*S*)-2-Amino-3-(1*H*-indol-3-yl)-N-(4-morpholinophenyl)propenamide,
(*S*)-*N*-(4-(4-Acetylpiperazin-1-yl)-2-methoxyphenyl)-3-(1*H*-indol-3-yl)-2-(4-methylphenylsulfonamido)propenamide,
(*S*)-*N*-(4-(4-Acetylpiperazin-1-yl)phenyl)-3-(1*H*-indol-3-yl)-2-(4-methylphenylsulfonamido)propenamide,
(*S*)-*N*-(3-(1*H*-indol-3-yl)-1-(2-(4-morpholinobenzoyl)hydrazinyl)-1-oxopropan-2-yl)-4-methylbenzenesulfonamide,
(*S*)-3-(1*H*-indol-3-yl)-2-(4-methylphenylsulfonamido)-*N*-(4-(4-morpholinophenyl)thiazol-2-yl)propanamide,
(*S*)-Methyl-4-(2-(3-(1*H*-indol-3-yl)-2-(4-methylphenylsulfonamido)propanamido)thiazol-4-yl)benzoate,
(*S*)-Benzyl-3-(1*H*-indol-3-yl)-2-(6-(trifluoromethyl)pyridine-3-sulfonamido)propanoate,
(*S*)-2-(4-Methylphenylsulfonamido)-*N*¹-(4-morpholinophenyl)-*N*⁵-(4-nitrophenyl)pentanediamide,
(S)-Benzyl-5-(4-methylphenylsulfonamido)-6-(4-morpholinophenylamino)-6-oxohexylcarbamate,
(*S*)-6-Amino-2-(4-methylphenylsulfonamido)-*N*-(4-morpholinophenyl)hexanamide,
(*S*)-3,5-Dimethoxy-*N*-(5-(4-methylphenylsulfonamido)-6-(4-morpholinophenylamino)-6-oxohexyl)benzamide,
(*S*)-*N*⁵-(4-Methoxybenzyl)-2-(4-methylphenylsulfonamido)-*N*¹-(4-morpholinophenyl)pentanediamide,
(*S*)-2-(4-Methylphenylsulfonamido)-*N*¹-(4-morpholinophenyl)-*N*⁵-phenylpentanediamide,
(*S*)-2-(4-Methylphenylsulfonamido)-5-morpholino-*N*-(4-morpholinophenyl)-5-oxopentanamide,
*S*)-*N*⁵-(4-fluorophenyl)-2-(4-methylphenylsulfonamido)-*N*¹-(4-morpholinophenyl)pentanediamide,
(*S*)-*N*⁵-(4-*tert*-butylphenyl)-2-(4-methylphenylsulfonamido)-*N*¹-(4-morpholinophenyl)pentanediamide,
(*S*)-2-(4-Methylphenylsulfonamido)-*N*¹-(4-morpholinophenyl)-*N*⁵-(4-(trifluoromethyl)phenyl)pentanediamide,
(*S*)-2-(4-Methylphenylsulfonamido)-*N*-(4-morpholinophenyl)-*N*⁴-(4-nitrophenyl)succinamide,
(*S*)-*N*-(furan-2-ylmethyl)-3-(1H-indol-3-yl)-2-(4-methylphenylsulfonamido)propanamide,
(*S*)-2-(4-Bromophenylsulfonamido)-*N*-(furan-2-ylmethyl)-3-(1*H*-indol-3-yl)propanamide,
(S)-3-(1*H*-indol-3-yl)-*N*-isopropyl-2-(4-methylphenylsulfonamido)propanamide,
(*S*)-2-(4-Bromophenylsulfonamido)-3-(1*H*-indol-3-yl)-*N*-isopropylpropanamide,
(*S*)-*N*-(3-(1*H*-indol-3-yl)-1-(2-(4-methoxybenzoyl)hydrazinyl)-1-oxopropan-2-yl)-4-methylbenzenesulfonamide,
(*S*)-2-(3-(1*H*-indol-3-yl)-2-(4-methylphenylsulfonamido)propanoyl)-*N*-(4-methoxyphenyl)hydrazinecarboxamide,
(*S*)-4-Bromo-*N*-(1-(3,5-dimethyl-1*H*-pyrazol-1-yl)-3-(1*H*-indol-3-yl)-1-oxopropan-2-yl)benzenesulfonamide,
(*S*)-*N*-(3-(1*H*-indol-3-yl)-1-oxo-1-(3-phenyl-1*H*-pyrazol-1-yl)propan-2-yl)-4-bromobenzenesulfonamide,
*N*-((*S*)-1-(2-((3*S*,5*S*,7*S*)-adamantane-1-carbonyl)hydrazinyl)-3-(1*H*-indol-3-yl)-1-oxopropan-2-yl)-4-methylbenzenesulfonamide,
(*S*)-2-(4-Bromophenylsulfonamido)-3-(1*H*-indol-3-yl)-*N*-(4-(p-tolyl)thiazol-2-yl)propanamide,
(S)-3-(1*H*-indol-3-yl)-2-(4-methylphenylsulfonamido)-*N*-phenethylpropanamide,
(S)-2-(4-Bromophenylsulfonamido)-*N*-(4-(4-fluorophenyl)thiazol-2-yl)-3-(1*H-*indol-3-yl)propanamide,
(*S*)-2-(4-Bromophenylsulfonamido)-3-(1*H*-indol-3-yl)-*N*-(4-(4-nitrophenyl)thiazol-2-yl)propanamide,
(*S*)-*N*-(3-(1*H*-indol-3-yl)-1-(2-(4-methoxybenzoyl)hydrazinyl)-1-oxopropan-2-yl)-4-methylbenzenesulfonamide,
(*S*)-2-(4-Bromophenylsulfonamido)-*N*-(4-(4-cyanophenyl)thiazol-2-yl)-3-(1*H-*indol-3-yl)propanamide,
(*S*)-2-(4-Bromophenylsulfonamido)-3-(1*H*-indol-3-yl)-*N*-(4-(4-(trifluoromethyl)phenyl)thiazol-2-yl)propanamide,
(*S*)-4-Bromo-*N*-(1-(2-dodecanoylhydrazinyl)-3-(1*H*-indol-3-yl)-1-oxopropan-2-yl)benzenesulfonamide,
(*S*)-4-Bromo-*N*-(1-(2-decanoylhydrazinyl)-3-(1*H*-indol-3-yl)-1-oxopropan-2-yl)benzenesulfonamide,
(*S*)-2-(4-Bromophenylsulfonamido)-3-(1*H*-indol-3-yl)-*N*-phenethylpropanamide,
*N*-((*S*)-1-(2-((3*S*,5*S*,7*S*)-adamantane-1-carbonyl)hydrazinyl)-3-(1*H*-indol-3-yl)-1-oxopropan-2-yl)-4-bromobenzenesulfonamide,
(*S*)-2-(4-Bromophenylsulfonamido)-*N*-(4-fluorophenethyl)-3-(1*H*-indol-3-yl)propanamide,
(*S*)-2-(4-Bromophenylsulfonamido)-3-(1*H*-indol-3-yl)-*N*-(4-methoxyphenethyl)propanamide,
(*S*)-2-(4-Bromophenylsulfonamido)-3-(1*H*-indol-3-yl)-*N*-(3-phenylpropyl)propanamide,
(*S*)-*N*-((3*R*,5*R*,7*R*)-adamantan-1-yl)-2-(4-bromophenylsulfonamido)-3-(1*H*-indol-3-yl)propanamide,
(*S*)-*N*-(3-(1*H*-indol-3-yl)-1-(4-(4-methoxyphenyl)piperazin-1-yl)-1-oxopropan-2-yl)-4-bromobenzenesulfonamide,
(*S*)-*N*-(3-(1*H*-indol-3-yl)-1-(4-(4-methoxyphenyl)piperazin-1-yl)-1-oxopropan-2-yl)-4-methylbenzenesulfonamide,
(*S*)-*N*-((3*R*,5*R*,7*R*)-adamantan-1-yl)-3-(1*H*-indol-3-yl)-2-(4-methylphenylsulfonamido)propanamide,
(*S*)-3-(1*H*-indol-3-yl)-*N*-(4-methoxyphenethyl)-2-(4-methylphenylsulfonamido)propanamide,
(S)-3-(1*H*-indol-3-yl)-2-(4-methylphenylsulfonamido)-*N*-(3-phenylpropyl)propanamide,
(*S*)-*N*-(4-fluorophenethyl)-3-(1*H*-indol-3-yl)-2-(4-methylphenylsulfonamido)propanamide,
(*S*)-3-(*1H*-indol-3-yl)-2-(4-methylphenylsulfonamido)-*N*-(4-(4-(trifluoromethyl)phenyl)thiazol-2-yl)propanamide,
(*S*)-*N*-(2-(1*H*-indol-3-yl)ethyl)-2-(4-bromophenylsulfonamido)-3-(1*H*-indol-3-yl)propanamide,
(*S*)-*N*-(3-(1*H*-indol-3-yl)-1-((4-(4-methoxyphenyl)thiazol-2-yl)amino)-1-oxopropan-2-yl)-4-methylbenzamide,
(S)-2-(4-Chlorophenylsulfonamido)-3-(1 *H*-indol-3-yl)-*N*-(4-(4-methoxyphenyl)thiazol-2-yl)propanamide,
(*S*)-3-(1*H*-indol-3-yl)-*N*-(4-(4-methoxyphenyl)thiazol-2-yl)-2-(4-(trifluoromethyl)phenylsulfonamido)propanamide,
(*S*)-2-(4-Fluorophenylsulfonamido)-3-(1*H*-indol-3-yl)-*N*-(4-(4-methoxyphenyl)thiazol-2-yl)propanamide,
(*S*)-2-(4-Bromophenylsulfonamido)-*N*-((1*r*,3*R*,5*S*,7*S*)-3,5-dimethyladamantan-1-yl)-3-(1 *H*-indol-3-yl)propanamide,
(*S*)-2-(4-Cyanophenylsulfonamido)-3-(1*H*-indol-3-yl)-*N*-(4-(4-methoxyphenyl)thiazol-2-yl)propanamide,
(S)-Benzyl 3-(1*H*-indol-3-yl)-2-(4-methylbenzamido)propanoate,
(*S*)-2-(4-Bromophenylsulfonamido)-3-(5-hydroxy-1*H*-indol-3-yl)-*N*-(4-(4-methoxyphenyl)thiazol-2-yl)propanamide,
(*S*)-2-(4-Bromophenylsulfonamido)-*N*-(4-(4-methoxyphenyl)thiazol-2-yl)-3-(5-methyl-1*H*-indol-3-yl)propenamide,
(*S*)-2-(4-Bromophenylsulfonamido)-3-(5-fluoro-1*H*-indol-3-yl)-*N*-(4-(4-methoxyphenyl)thiazol-2-yl)propanamide,
(*S*)-4-(2-(3-(1*H*-indol-3-yl)-2-(4-methylphenylsulfonamido)propanamido)ethyl)benzoic acid,
(*S*)-*N*-(4-bromophenethyl)-3-(1*H*-indol-3-yl)-2-(4-methylphenylsulfonamido)propanamide,
(*S*)-*N*-(2-(1*H*-indol-3-yl)ethyl)-3-(1*H*-indol-3-yl)-2-(4-methylphenylsulfonamido)propenamide,
(*S*)-3-(1*H*-indol-3-yl)-*N*-(4-methylphenethyl)-2-(4-methylphenylsulfonamido)propanamide,
Ethyl 2-((*S*)-3-(1*H*-indol-3-yl)-2-(4-methylphenylsulfonamido)propanamido)-3-(1*H*-indol-3-yl)propanoate,
(*S*)-*N*-(4-chlorophenethyl)-3-(1*H*-indol-3-yl)-2-(4-methylphenylsulfonamido)propanamide,
2-((*S*)-3-(1*H*-indol-3-yl)-2-(4-methylphenylsulfonamido)propanamido)-3-(1*H-*indol-3-yl)propanoic acid,
(*S*)-Methyl-4-(2-(3-(1*H*-indol-3-yl)-2-(4-methylphenylsulfonamido)propanamido)thiazol-4-yl)benzoate,
(S)-4-(2-(3-(1*H*-indol-3-yl)-2-(4-methylphenylsulfonamido)propanamido)thiazol-4-yl)benzoic acid,
(*S*)-3-(Benzo[*d*|oxazol-2-ylamino)-*N*-(4-(4-methoxyphenyl)thiazol-2-yl)-2-(4-methylphenylsulfonamido)propanamide,
(*S*)-*N*-(3-((4-(4-methoxyphenyl)thiazol-2-yl)amino)-2-(4-methylphenylsulfonamido)-3-oxopropyl)benzamide,
(*S*)-*N*-(3-((4-(4-methoxyphenyl)thiazol-2-yl)amino)-2-(4-methylphenylsulfonamido)-3-oxopropyl)nicotinamide or
(*S*)-*N*-(3-((4-(4-methoxyphenyl)thiazol-2-yl)amino)-2-(4-methylphenylsulfonamido)-3-oxopropyl)pyrimidine-5-carboxamide.

Structural formulas of the compounds listed above, the inhibitory effect of these compounds on AIMP2-DX2 expression, and the cytotoxicity to cancer cells are shown in Table 1 below.

**Table 1**

| No. | R1 | R2 | R3 | DX-Nanolucifer ase activity (40µM) | Cell Viability(% inhibition) (A549) |
|---|---|---|---|---|---|
| 1 | 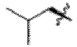 | 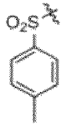 | 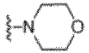 | 57.3±6.8 | 17.79 |
| 2 | 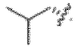 | 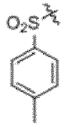 | 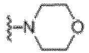 | 14.04±15.60 | 29.46 |
| 3 | 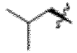 | 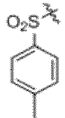 | 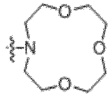 | 55.8±14.8 | 8.64 |
| 4 | 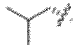 | 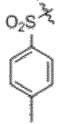 | 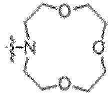 | 10.69±12.75 | 15.11 |
| 5 | 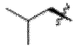 | 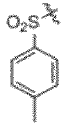 | 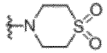 | 47.4±7.4 | 15.98 |
| 6 | 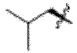 | 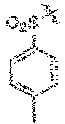 | 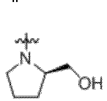 | 39.9±7.4 | 15.98 |
| 7 | 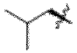 | 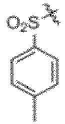 | 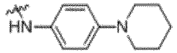 | 31.70±6.06 | 23.48 |
| 8 | 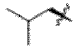 | 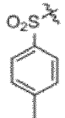 | 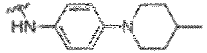 | 35.45±2.45 | 24.89 |
| 9 | 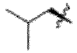 | 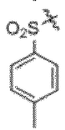 | 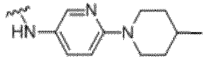 | 18.3 | 78.3 |
| 10 | 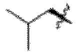 | 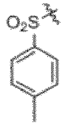 | 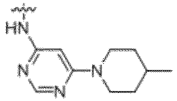 | 1.5 | 72.7 |
| 11 | 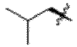 | 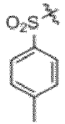 | 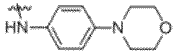 | 56.9±7.2 | 23.76 |
| 12 | 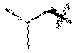 | 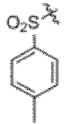 | 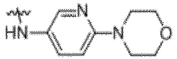 | 43.8±8.7 | 23.26 |
| 13 | 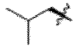 | 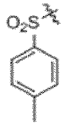 | 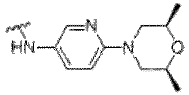 | 51.3±4.0 | 14.99 |
| 14 | 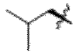 | 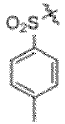 | 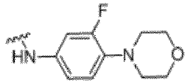 | 55.65±1.06 | 29.87±2.48 |
| 15 | 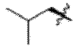 | 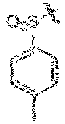 | 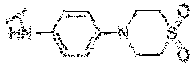 | 16.10±20.6 | 4.86±8.75 |
| 16 | 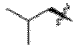 | 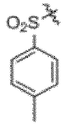 | 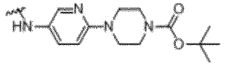 | 9.8 | 73.5 |
| 17 | 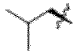 | 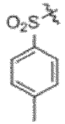 | 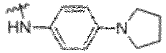 | 33.44±6.60 | 20.11 |
| 18 | 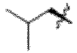 | 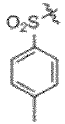 | 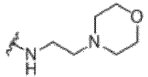 | 14.8 | 8.7 |
| 19 | 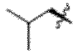 | 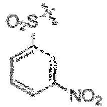 | 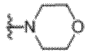 | 33.8±8.2 | 22.61 |
| 20 | 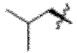 | 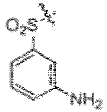 | 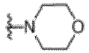 | 31.0±9.9 | 24.57 |
| 21 | 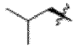 | 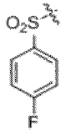 | 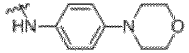 | 22.83±7.33 | 33.04 |
| 22 | 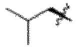 | 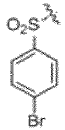 | 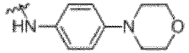 | 54.55±4.84 | 39.56±5.42 |
| 23 | 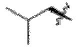 | 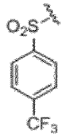 | 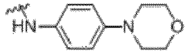 | 24.5±4.4 | 64.35 |
| 24 | 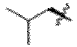 | 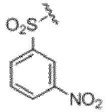 | 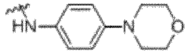 | 59.4±2.3 | 18.38±11.76 |
| 25 | 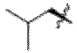 | 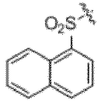 | 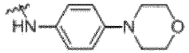 | 25.5±5.4 | 38.92±1.69 |
| 26 | 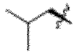 | 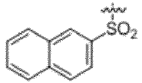 | 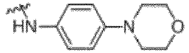 | 52.7±10.5 | 5.95±2.15 |
| 27 | 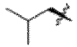 | 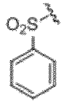 | 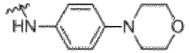 | 14.46±9.45 | 13.51±9.40 |
| 28 | 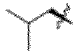 | 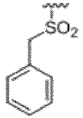 | 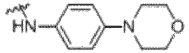 | 57.4±8.4 | 12.43±8.58 |
| 29 | 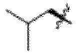 | 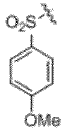 | 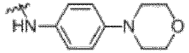 | 29.61±11.71 | 12.70±4.95 |
| 30 | 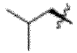 | 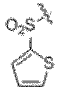 | 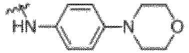 | 23.7±3.1 | 9.46±9.61 |
| 31 | 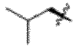 | 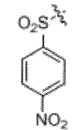 | 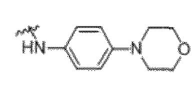 | 11.05±23.55 | 33.24±4.95 |
| 32 | 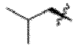 | 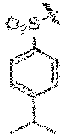 | 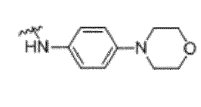 | 25.8±2.5 | 20.27±5.87 |
| 33 | 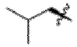 | 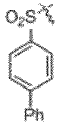 | 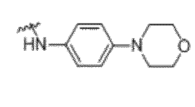 | -6.12±10.90 | 26.48±3.66 |
| 34 | 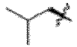 | 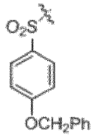 | 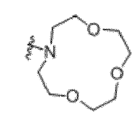 | 34.38±0.91 | 38.82±5.12 |
| 35 | 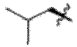 | 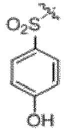 | 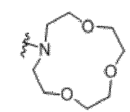 | 41.92±1.66 | -1.97±14.7 |
| 36 | 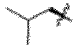 | 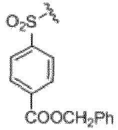 | 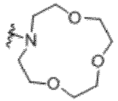 | 36.79±2.25 | 64.40±2.96 |
| 37 | 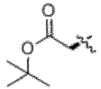 | 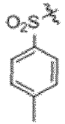 | 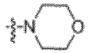 | 45.0±7.8 | 20.33 |
| 38 | 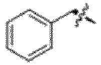 | 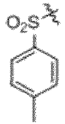 | 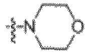 | 32.3±2.6 | 15.14±1.24 |
| 39 | 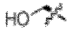 | 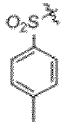 | 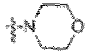 | 7.7±11.2 | -2.43±0.94 |
| 40 | 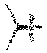 | 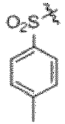 | 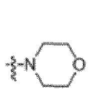 | 31.3±10.3 | -5.41+6.12 |
| 41 | 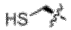 | 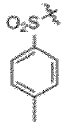 | 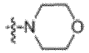 | 52.5±5.6 | 12.16±6.14 |
| 42 | 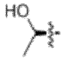 | 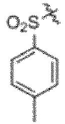 | 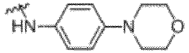 | -12.81±10.58 | 13.24±6.93 |
| 43 | 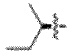 | 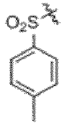 | 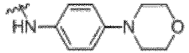 | 42.9±3.9 | 17.03±7.96 |
| 44 | 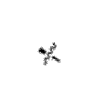 | 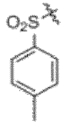 | 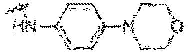 | 2.17±20.23 | 14.05±4.05 |
| 45 | 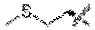 | 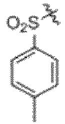 | 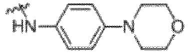 | 4.17±22.57 | 12.97±1.24 |
| 46 | 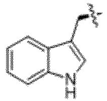 | 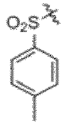 | 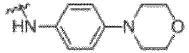 | 67.1±10.8 | 61.35±10.27 |
| 47 | 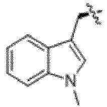 | 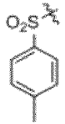 | 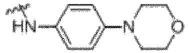 | 38.20±7.73 | 64.95±2.84 |
| 48 | 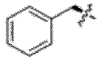 | 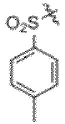 | 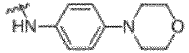 | 10.24±10.30 | 45.68±2.92 |
| 49 | 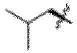 | 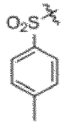 | 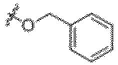 | 43.10±6.46 | 96.77±0.70 |
| 50 | 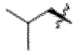 | 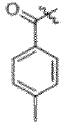 | 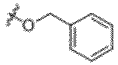 | 65.9±4.0 | 75.41±3.28 |
| 51 | 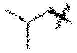 | 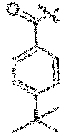 | 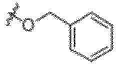 | 63.5±4.5 | 11.62±4.29 |
| 52 | 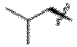 | 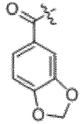 | 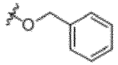 | -5.6±5.2 | 64.59±5.15 |
| 53 | 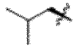 | 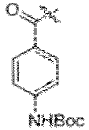 | 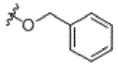 | 43.7±9.9 | 49.19±13.33 |
| 54 | 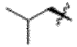 | 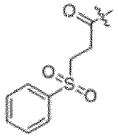 | 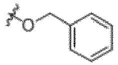 | 33.7±4.3 | 84.05±2.61 |
| 55 | 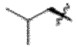 | 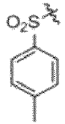 | 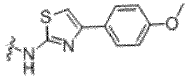 | 70.30±3.23 | 89.25±2.03 |
| 56 | 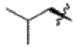 | 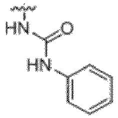 | 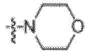 | 33.0±10.7 | 32.07 |
| 57 | 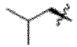 | 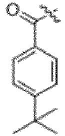 | 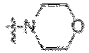 | 63.8±11.0 | -4.86±4.95 |
| 58 | 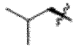 | 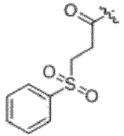 | 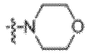 | 63.1±4.5 | -0.81±4.95 |
| 59 | 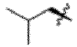 | 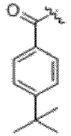 | 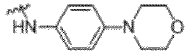 | 45.66±8.28 | 90.03±0.97 |
| 60 | 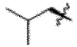 | 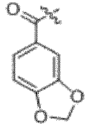 | 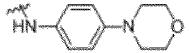 | 35.7±10.2 | 9.46±9.67 |
| 61 | 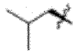 | 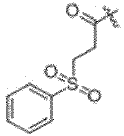 | 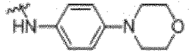 | 64.27±4.80 | -1.25±3.63 |
| 62 | 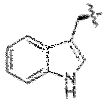 | 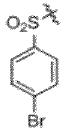 | 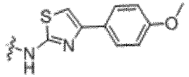 | 45.43±9.78 | 90.17±0.96 |
| 63 | 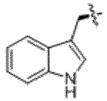 | 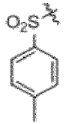 | 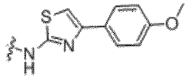 | 72.56±6.91 | 86.53±1.35 |
| 64 | 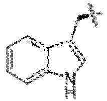 | 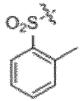 | 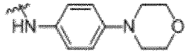 | 59.23±8.02 | 51.75±1.56 |
| 65 | 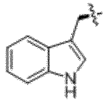 | 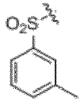 | 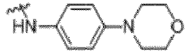 | 40.78±5.82 | 74.94±1.04 |
| 66 | 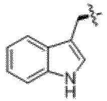 | 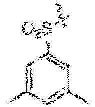 | 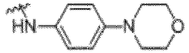 | 42.41±3.97 | 77.25±0.82 |
| 67 | 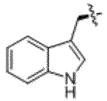 | 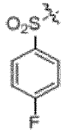 | 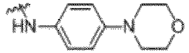 | 44.45±14.20 | 69.20±2.10 |
| 68 | 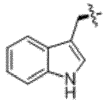 | 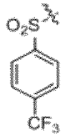 | 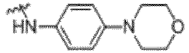 | 48.06±6.78 | 76.90±0.51 |
| 69 | 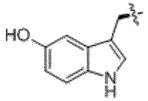 | 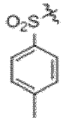 | 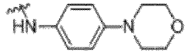 | 48.44±11.64 | 20.62±5.14 |
| 70 | 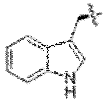 | 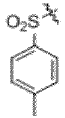 | 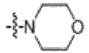 | 59.70±3.49 | 23.61±2.18 |
| 71 | 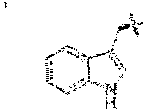 | 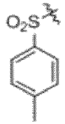 | 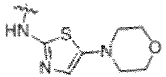 | 54.64±13.30 | 72.71±0.97 |
| 72 | 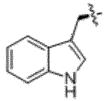 | 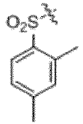 | 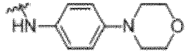 | 21.54±7.62 | 73.73±0.78 |
| 73 | 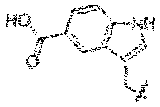 | 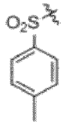 | 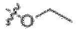 | 44.24±3.91 | 4.32±2.95 |
| 74 | 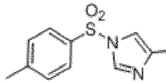 | 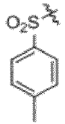 | 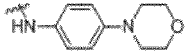 | 56.18±14.53 | 45.34±4.64 |
| 75 | 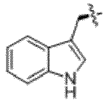 | 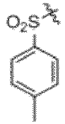 | 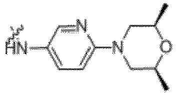 | 57.82±8.0 | 79.58±3.72 |
| 76 | 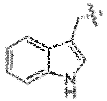 | 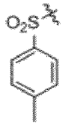 | 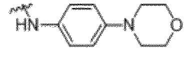 | 51.49±19.94 | 63.47±0.82 |
| 77 | 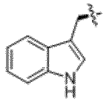 | 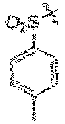 | 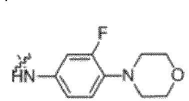 | 23.85±7.01 | 80.59±1.49 |
| 78 | 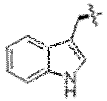 | 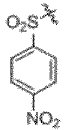 | 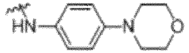 | 44.47±12.72 | 67.03±1.45 |
| 79 | 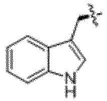 | 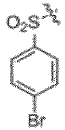 | 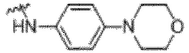 | 30.65±15.33 | 78.31±1.91 |
| 80 | 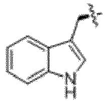 | 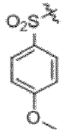 | 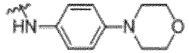 | 44.88±13.32 | 38.64±1.03 |
| 81 | 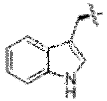 | 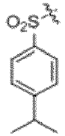 | 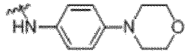 | 77.74±6.75 | 73.98±0.39 |
| 82 | 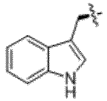 | 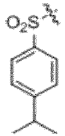 | 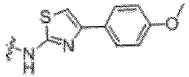 | 29.30±2.70 | 96.64±0.09 |
| 83 | 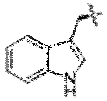 | 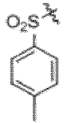 | 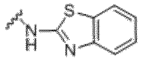 | 17.40±3.23 | 97.43±2.01 |
| 84 | 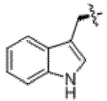 | 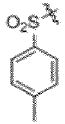 | 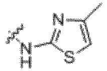 | -15.84±6.29 | 20.93±11.89 |
| 85 | 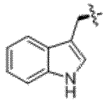 | 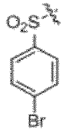 | 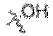 | 20.18±1.65 | 10.28±3.54 |
| 86 | 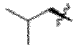 | 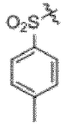 | 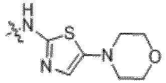 | 37.71±6.09 | 20.70±1.43 |
| 87 | 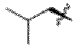 | 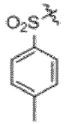 | 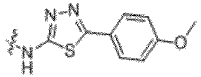 | 50.25±7.05 | 36.61±3.85 |
| 88 | 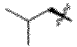 | 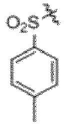 | 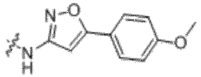 | 56.99+8.83 | 46.96±4.60 |
| 89 | 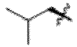 | 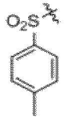 | 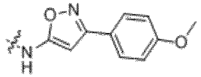 | 47.55±13.33 | 98.73±0.92 |
| 90 | 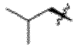 | 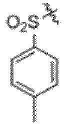 | 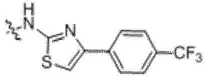 | 65.41±4.13 | 86.36±0.64 |
| 91 | 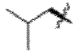 | 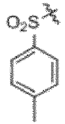 | 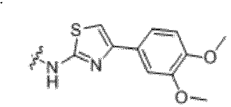 | 16.39±8.65 | 87.36±2.13 |
| 92 | 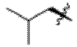 | 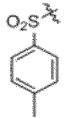 | 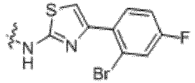 | 31.48±6.84 | 100.05±1.22 |
| 93 | 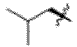 | 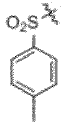 | 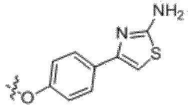 | 22.48±3.96 | 48.85±9.03 |
| 94 | 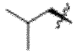 | 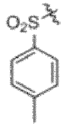 | 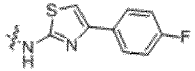 | 11.48±7.25 | 101.42±0.79 |
| 95 | 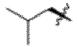 | 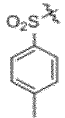 | 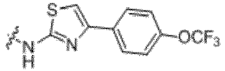 | 22.60±5.29 | 92.97±0.09 |
| 96 | 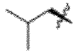 | 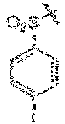 | 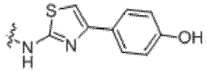 | 36.84±9.16 | 93.07±5.89 |
| 97 | 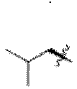 | 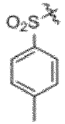 | 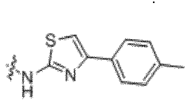 | 15.14±2.59 | 67.10±7.93 |
| 98 | 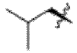 | 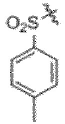 | 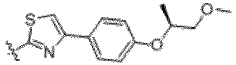 | nd | 76.55±1.52 |
| 99 | 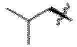 | 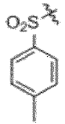 | 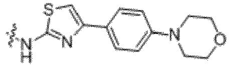 | nd | 73.11±3.83 |
| 100 | 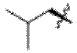 | 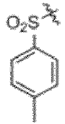 | 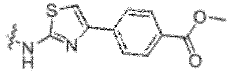 | nd | 79.24±1 |
| 101 | 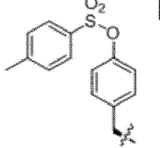 | 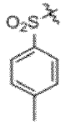 | 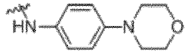 | 10.93±4.88 | 35.10±5.95 |
| 102 | 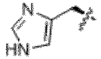 | 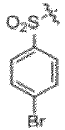 | 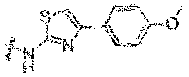 | 27.23±5.87 | 91.03±6.44 |
| 103 | 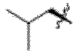 | 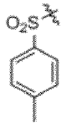 | 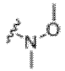 | 63.71±14.32 | -5.00+6.69 |
| 104 | 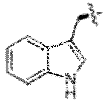 | 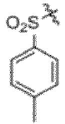 | 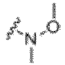 | 64.33±5.96 | 6.26±7.67 |
| 105 | 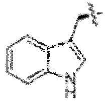 | 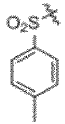 | 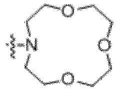 | 57.09±1.56 | 29.36±7.43 |
| 106 | 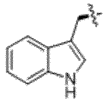 | 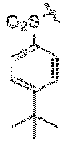 | 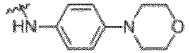 | 68.14±9.57 (10 µM) | 17.13±5.35 |
| 107 | 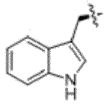 | 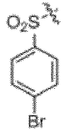 | 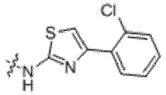 | 36.98±12.48 | 59.19±2.09 |
| 108 | 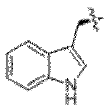 | 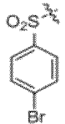 | 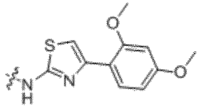 | -4.70±10.8 | 9.05±7.51 |
| 109 | 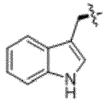 | 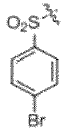 | 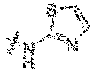 | 57.45±9.11 | 4.14±1.04 |
| 110 | 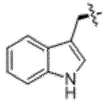 | 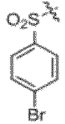 | 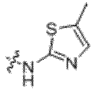 | 46.74±7.18 | 8.95±3.66 |
| 111 | 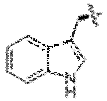 | 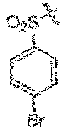 | 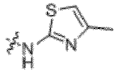 | 25.61±22.32 | 11.65±6.06 |
| 112 | 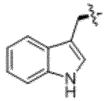 | 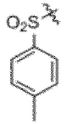 | 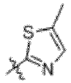 | 61.54±5.19 | 15.01±6.55 |
| 113 | 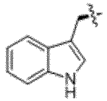 | 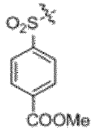 | 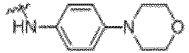 | 15.05±6.22 (10 µM) | 4.67±3.85 |
| 114 | 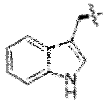 | 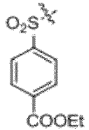 | 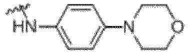 | 3.48±8.58 (10 µM) | 47.88±0.53 |
| 115 | 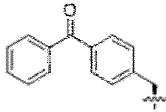 | 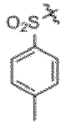 | 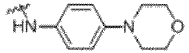 | 2.16±6.66 (10 µM) | 0.35±0.67 |
| 116 | 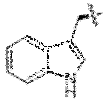 | 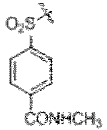 | 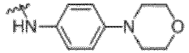 | -0.11±15.08 (10 µM) | 34.22±7.94 |
| 117 | 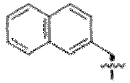 | 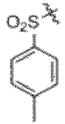 | 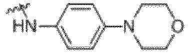 | -2.56±9.56 (10 µM) | 4.59±6.86 |
| 118 | 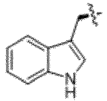 | 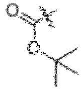 | 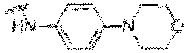 | 8.22±5.02 (10 µM) | 9.26±7.63 |
| 119 | 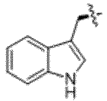 | 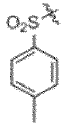 | OH | 14.92±10.18 (10 µM) | 1.59±8.71 |
| 120 | 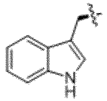 | 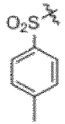 | 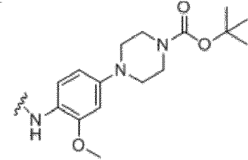 | 21.28±14.17 (10 µM) | 66.51±1.24 |
| 121 | 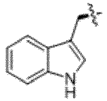 | 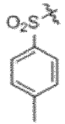 | 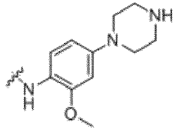 | 41.70±17.28 (10 µM) | 51.15±0.6 |
| 122 | 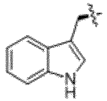 | 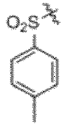 | 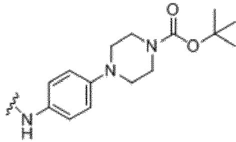 | 38.03±1.70 (10 µM) | 69.45±1.3 |
| 123 | 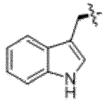 | 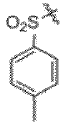 | 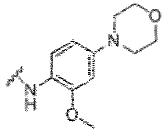 | 22.38±11.81 (10 µM) | 34.01±10.46 |
| 124 | 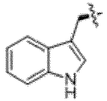 | 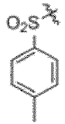 | 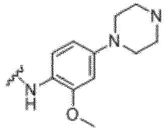 | 20.90±9.80 (10 µM) | 16.44±18.56 |
| 125 | 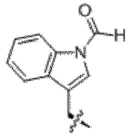 | 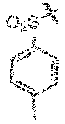 | 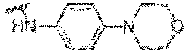 | nd | 49.76±2.98 |
| 126 | 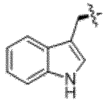 | 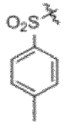 | 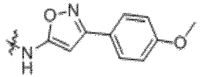 | nd | 85.53±0.66 |
| 127 | 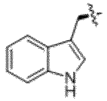 | 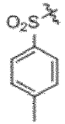 | 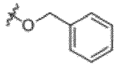 | 49.86±1.60 | 94.24±0.54 |
| 128 | 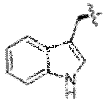 | 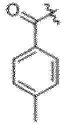 | 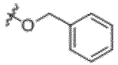 | 36.77±8.13 | 63.08±7.72 |
| 129 | 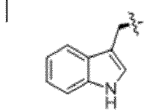 | 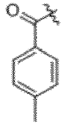 | 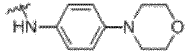 | 53.02±9.60 | 60.35±3.98 |
| 130 | 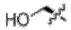 | 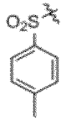 | 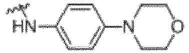 | -0.39±15.72 | 16.62±10.08 |
| 131 | 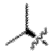 | 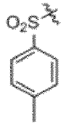 | 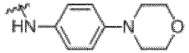 | 20.45±5.02 | 5.78±7.70 |
| 132 | 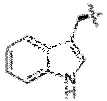 | 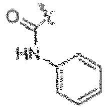 | 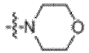 | | -11.18±5.31 |
| 133 | 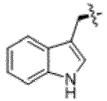 | 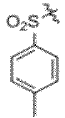 | 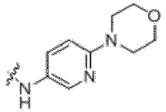 | | 6.18±6.05 |
| 134 | 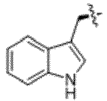 | 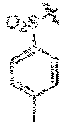 | 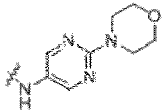 | | 33.86±2.97 |
| 135 | 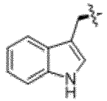 | 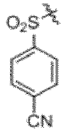 | 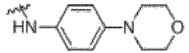 | | 55.02±1.06 |
| 136 | 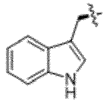 | 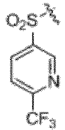 | 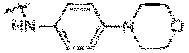 | | 87.12±2.3 |
| 137 | 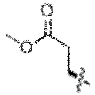 | 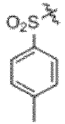 | 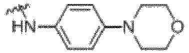 | | 17.75±5.15 |
| 138 | 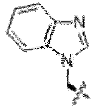 | 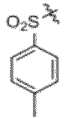 | 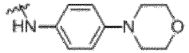 | | 14.63±1.98 |
| 139 | 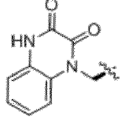 | 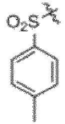 | 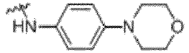 | | 9.99±0.24 |
| 140 | 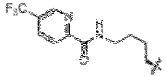 | 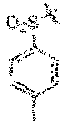 | 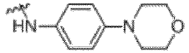 | | 86.68±1.25 |
| 141 | 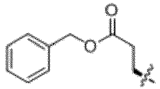 | 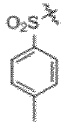 | 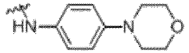 | | 40.15±6.15 |
| 142 | 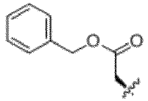 | 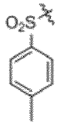 | 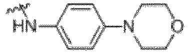 | | 15.9±4.84 |
| 143 | 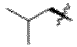 | 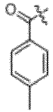 | 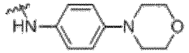 | 30.20±8.46 | |
| 144 | 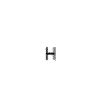 | 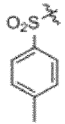 | 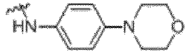 | 12.93±12.66 | |
| 145 | 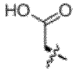 | 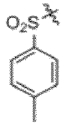 | 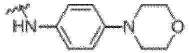 | -11.05±19.25 | |
| 146 | 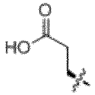 | 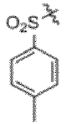 | 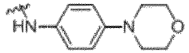 | 21.57±4.53 | |
| 147 | 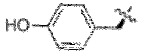 | 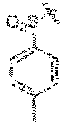 | 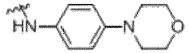 | -8.00±16.11 | |
| 148 | 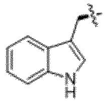 | 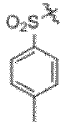 | 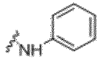 | -1.85±20.00 | |
| 149 | 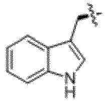 | 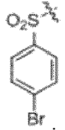 | 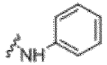 | 1.08±10.46 | |
| 150 | 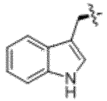 | 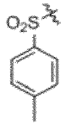 | 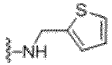 | -17.70±16.6 | |
| 151 | 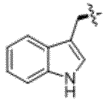 | 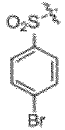 | 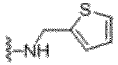 | 42.99±22.28 | |
| 152 | 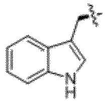 | 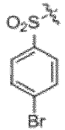 | 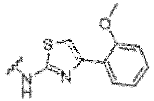 | 11.38±11.84 | |
| 153 | 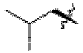 | 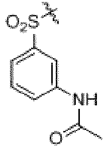 | 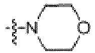 | 26.9±6.7 | 10.98 |
| 154 | 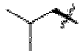 | 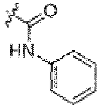 | 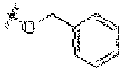 | 40.5±7.7 | 23.48 |
| 155 | H | 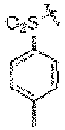 | 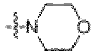 | 313±4.4 | 17.30±0.81 |
| 156 | Cyclohexane | 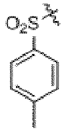 | 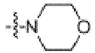 | -0.2±6.6 | 13.51±2.04 |
| 157 | 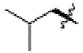 | 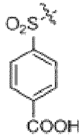 | 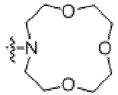 | 36.79±2.25 | 64.40±2.96 |
| 158 | 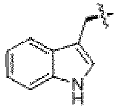 | 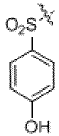 | 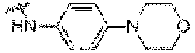 | -7.04±4.87 (10 µM) | 24.87±6.01 |
| 159 | 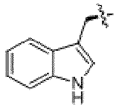 | 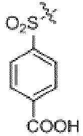 | 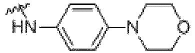 | 1.75±8.36 (10 µM) | 24.60±5.96 |
| 160 | 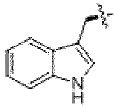 | H | 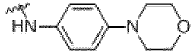 | 14.92±17.49 (10 µM) | 15.52±9.58 |
| 161 | 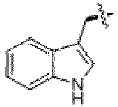 | 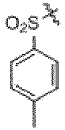 | 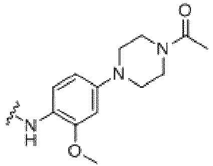 | 25.16±9.86 (10 µM) | 22.05±5.93 |
| 162 | 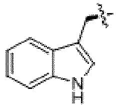 | 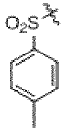 | 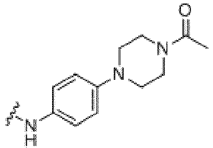 | 20.59±3.17 (10 µM) | 19.92±16.25 |
| 163 | 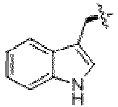 | 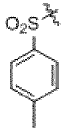 | 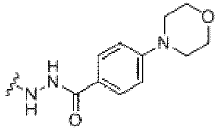 | nd | 18.63±9.48 |
| 164 | 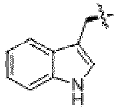 | 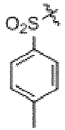 | 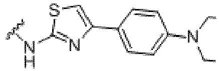 | nd | 88.05±0.8 |
| 165 | 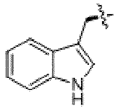 | 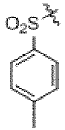 | 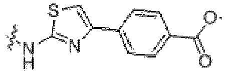 | nd | 78.22±0.97 |
| 166 | 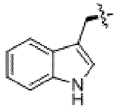 | 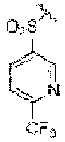 | 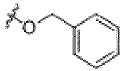 | nd | 97.48±1 |
| 167 | 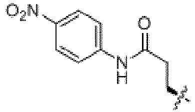 | 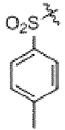 | 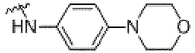 | nd | 85.33±1.28 |
| 168 | 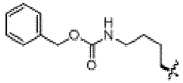 | 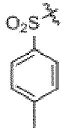 | 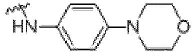 | nd | 84.11±1.25 |
| 169 | 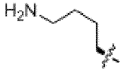 | 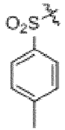 | 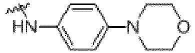 | nd | 3.97±3.91 |
| 170 | 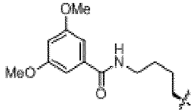 | 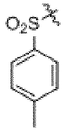 | 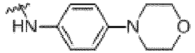 | nd | 31.41±4.96 |
| 171 | 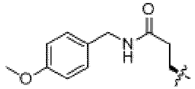 | 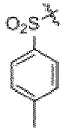 | 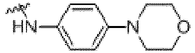 | nd | 40.19±4.29 |
| 172 | 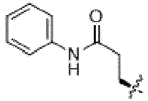 | 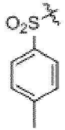 | 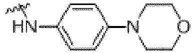 | nd | -2.14±5.1 |
| 173 | 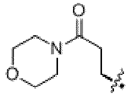 | 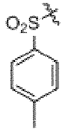 | 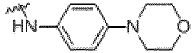 | nd | 0.1±5.51 |
| 174 | 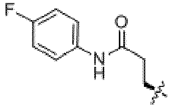 | 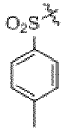 | 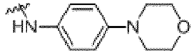 | nd | 10.3±2.26 |
| 175 | 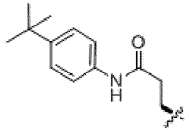 | 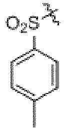 | 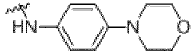 | nd | 28.4±4.52 |
| 176 | 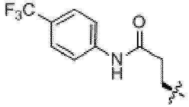 | 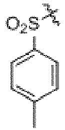 | 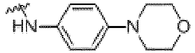 | nd | 58.84±1.48 |
| 177 | 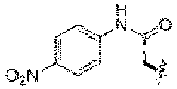 | 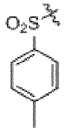 | 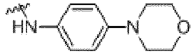 | nd | 53.91±0.92 |
| 178 | 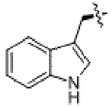 | 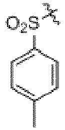 | 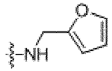 | 1.52±5.09 | 39.03+9.97 |
| 179 | 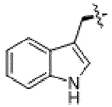 | 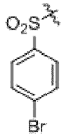 | 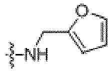 | -4.97±1.37 | 96.85±0.72 |
| 180 | 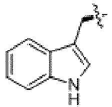 | 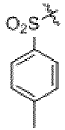 | 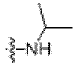 | 4.04±4.08 | 38.20±5.02 |
| 181 | 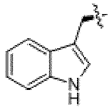 | 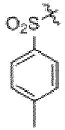 | 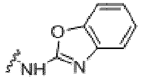 | 44.15±2.37 | 98.95±0.74 |
| 182 | 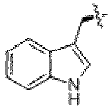 | 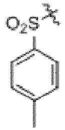 | 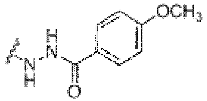 | 69.62±3.16 (10 µM) | 0.19±15.38 |
| 183 | 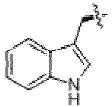 | 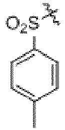 | 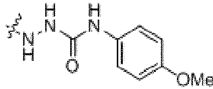 | 68.08±8.20 (10 µM) | 42.25±5.21 |
| 184 | 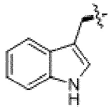 | 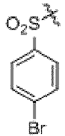 | 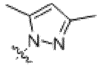 | 78.33±1.69 (10 µM) | -24.64±2.77 |
| 185 | 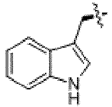 | 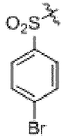 | 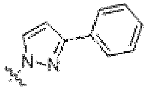 | 82.82±3.30 (10 µM) | 13.09±18.13 |
| 186 | 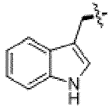 | 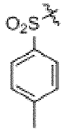 | 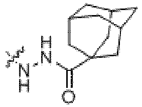 | 73.43±6.05 (10 µM) | 59.96±10.37 |
| 187 | 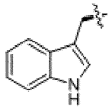 | 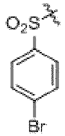 | 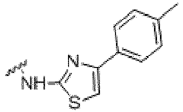 | 34.92±10.60 (10 µM) | 61.41±4.21 |
| 188 | 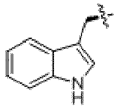 | 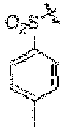 | 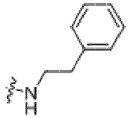 | 61.13±12.91 (10 µM) | 53.90±1.97 |
| 189 | 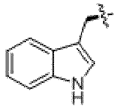 | 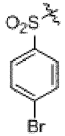 | 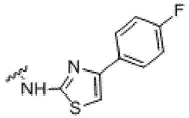 | 30.37±6.79 (1O µM) | 54.57±2.89 |
| 190 | 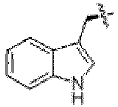 | 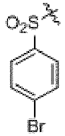 | 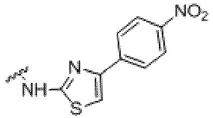 | 29.62±13.98 (10 µM) | 62.46±2.36 |
| 191 | 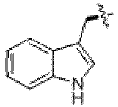 | 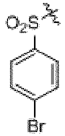 | 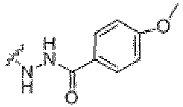 | 8.09±8.84 (10 µM) | 49.17±2.36 |
| 192 | 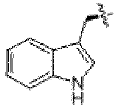 | 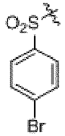 | 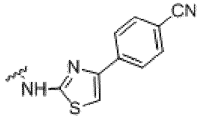 | 15.54±3.27 (10 µM) | 81.69±2.49 |
| 193 | 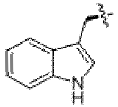 | 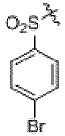 | 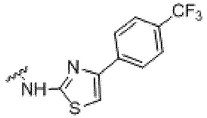 | 49:31±9.52 (10 µM) | 88.98±0.59 |
| 194 | 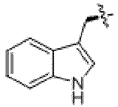 | 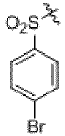 | 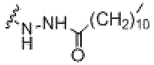 | 34.95±9.25 (10 µM) | 86.99±0.6 |
| 195 | 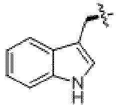 | 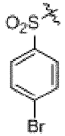 | 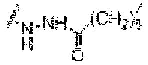 | 45.43±5.61 (10 µM) | 90.9±1.03 |
| 196 | 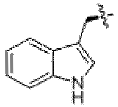 | 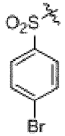 | 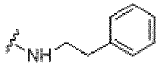 | 28.19±2.11 (10 µM) | 79.27±0.55 |
| 197 | 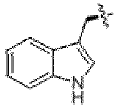 | 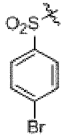 | 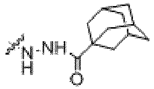 | 43.64±5.26 (10 µM) | 84.86±1.09 |
| 188 | 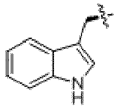 | 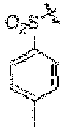 | 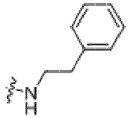 | 61.13±12.91 (10 µM) | 53.90±1.97 |
| 189 | 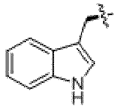 | 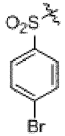 | 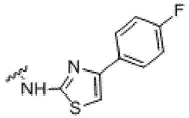 | 30.37±6.79 (10 µM) | 54.57±2.89 |
| 190 | 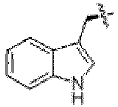 | 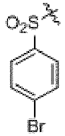 | 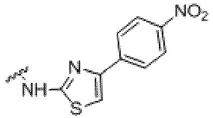 | 29.62±13.98 (10 µM) | 62.46±2.36 |
| 191 | 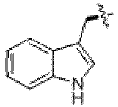 | 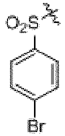 | 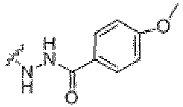 | 8.09±8.84 (10 µM) | 49.17±2.36 |
| 192 | 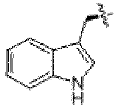 | 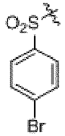 | 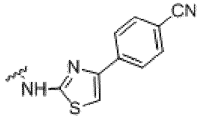 | 15.54±3.27 (10 µM) | 81.69±2.49 |
| 193 | 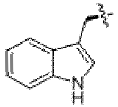 | 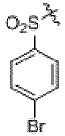 | 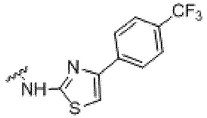 | 49:31±9.52 (10 µM) | 88.98±0.59 |
| 194 | 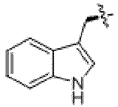 | 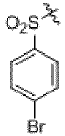 | 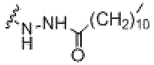 | 34.95±9.25 (10 µM) | 86.99±0.6 |
| 195 | 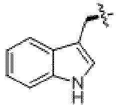 | 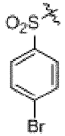 | 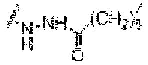 | 45.43±5.61 (10 µM) | 90.9±1.03 |
| 196 | 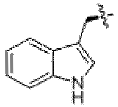 | 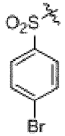 | 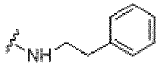 | 28.19±2.11 (10 µM) | 79.27±0.55 |
| 197 | 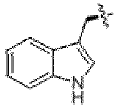 | 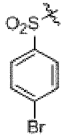 | 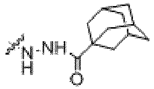 | 43.64±5.26 (10 µM) | 84.86±1.09 |
| 198 | 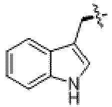 | 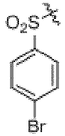 | 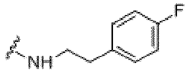 | 44.23±3.70 (10 µM) | 82.06±0.64 |
| 199 | 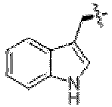 | 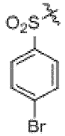 | 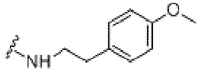 | 30.62±8.13 | 84.34±0.87 |
| 200 | 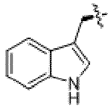 | 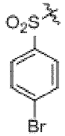 | 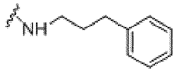 | 46.44±4.51 | 79.27±0.9 |
| 201 | 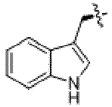 | 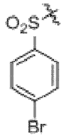 | 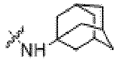 | 53.15±4.03 | 87.1±0.45 |
| 202 | 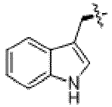 | 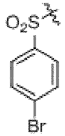 | 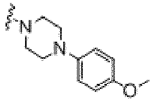 | 38.61±11.31 | 78.44±2.44 |
| 203 | 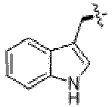 | 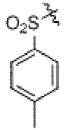 | 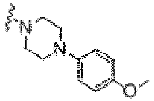 | 35.09±14.22 | 48.15±3.26 |
| 204 | 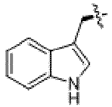 | 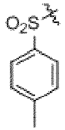 | 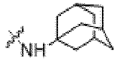 | nd | 78.9±2.62 |
| 205 | 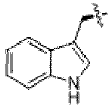 | 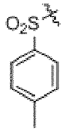 | 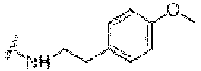 | nd | 65.64±5.37 |
| 206 | 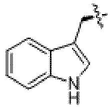 | 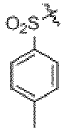 | 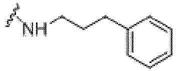 | | 67.48±2.35 |
| 207 | 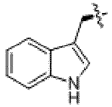 | 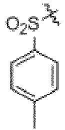 | 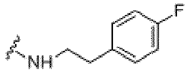 | | 64.02±2.15 |
| 208 | 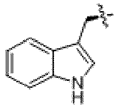 | 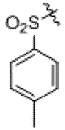 | 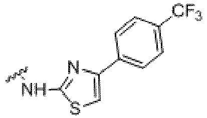 | | 82.89±1.06 |
| 209 | 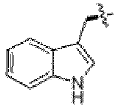 | 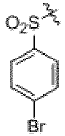 | 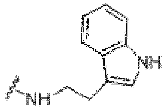 | | 89.27±0.77 |
| 210 | 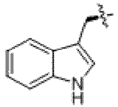 | 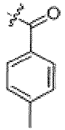 | 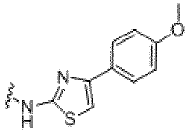 | | 40.83±8.13 |
| 211 | 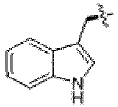 | 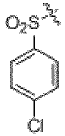 | 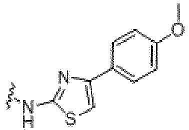 | | 83.3±1.04 |
| 212 | 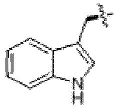 | 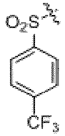 | 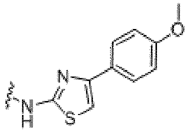 | | 84.25±0.99 |
| 213 | 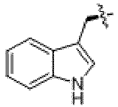 | 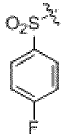 | 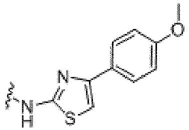 | | 83.29±1.04 |
| 214 | 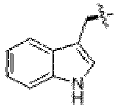 | 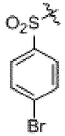 | 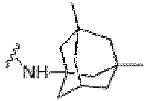 | | 86.81±0.26 |
| 215 | 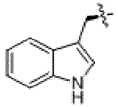 | 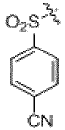 | 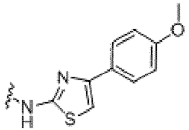 | | 80.78±1.18 |
| 216 | 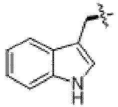 | 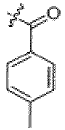 | 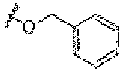 | | 69.96±2.46 |
| 217 | 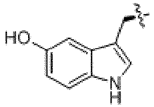 | 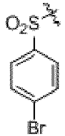 | 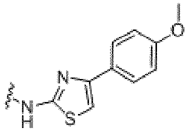 | 21.27±1.1 (10 µM) | 89.41±0.79 |
| 218 | 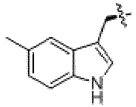 | 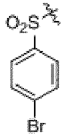 | 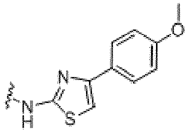 | 34.75±9.44 (10 µM) | 90.98±1 |
| 219 | 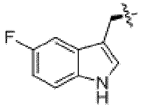 | 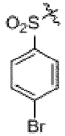 | 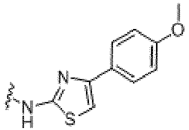 | 46.87±8.86 (10 µM) | 95.58±0.08 |
| 220 | 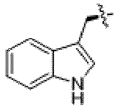 | 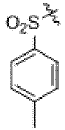 | 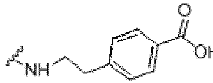 | 18.88±19.45 (10 µM) | -11.09±8.4 |
| 221 | 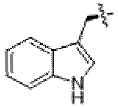 | 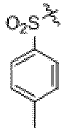 | 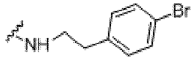 | 40.11±3.09 (10 µM) | 97.31±0.8 |
| 222 | 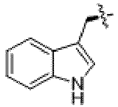 | 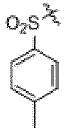 | 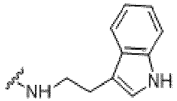 | 31.26±14.66 (10 µM) | 9105±1.38 |
| 223 | 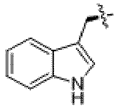 | 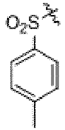 | 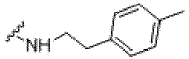 | 34.65±11.73 (10 µM) | 93.28±1.51 |
| 224 | 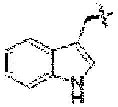 | 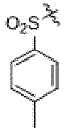 | 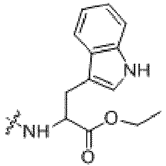 | 42.68±8.97 (10 µM) | 93.83±0.34 |
| 225 | 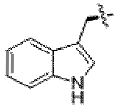 | 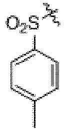 | 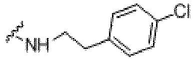 | 33.74±6.03 (10 µM) | 95.8±0.43 |
| 226 | 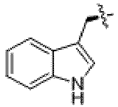 | 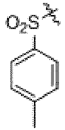 | 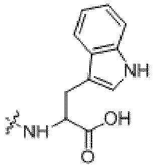 | 38.13±14.86 (10 | 1.11±7.23 |
| 227 | 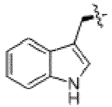 | 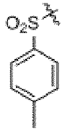 | 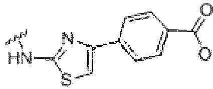 | | 84.63±0.51 |
| 228 | 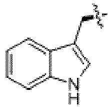 | 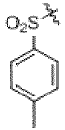 | 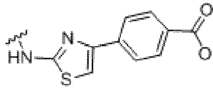 | | 48.75±5.65 |
| 229 | 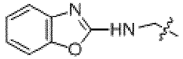 | 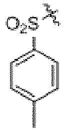 | 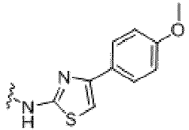 | | 74.91±2.05 |
| 230 | 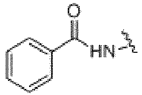 | 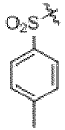 | 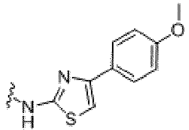 | | 71.56±1.67 |
| 231 | 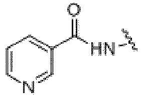 | 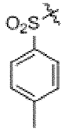 | 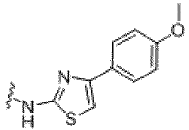 | | 47.63±5.89 |
| 232 | 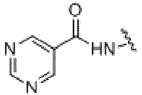 | 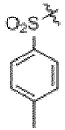 | 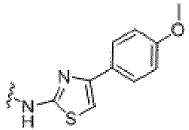 | | 40.98±2.65 |

The compound of the Formula 1 of the present invention may be used in the form of a pharmaceutically acceptable salt. As the salt, acid addition salts formed with various pharmaceutically or physiologically acceptable organic or inorganic acids are useful. As a suitable organic acid, for example, carboxylic acid, phosphonic acid, sulfonic acid, acetic acid, propionic acid, octanoic acid, decanoic acid, glycolic acid, lactic acid, fumaric acid, succinic acid, adipic acid, malic acid, tartaric acid, citric acid, glutamic acid, aspartic acid, maleic acid, benzoic acid, salicylic acid, phthalic acid, phenylacetic acid , benzenesulfonic acid, 2-naphthalenesulfonic acid, methylsulfuric acid, ethylsulfuric acid, dodecylsulfuric acid and the like can be used, and as a suitable inorganic acid, for example, hydrochloric acid, sulfuric acid or phosphoric acid can be used.

The compound of Formula 1 of the present invention may include all salts, hydrates and solvates, racemates, or stereoisomers that can be prepared by conventional methods as well as pharmaceutically acceptable salts.

According to an embodiment of the present invention, it was confirmed that the compounds exhibit the activity of inhibiting the expression of AIMP2-DX2. In particular, this activity was confirmed to be exerted by inhibiting the binding to HSP70, which contributes to the stabilization of the AIMP2-DX2 protein.

Accordingly, the compounds provided by the present invention may be characterized in that they exhibit the activity of inhibiting the expression of AIMP2-DX2.

The present invention also provides a method for preparing the compound of claim 1 or a pharmaceutically acceptable salt thereof, which is prepared by the following Reaction Formula:

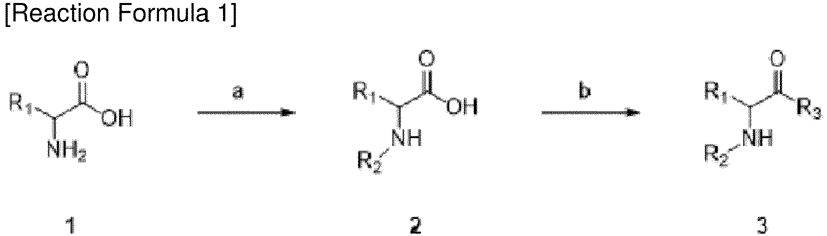

In the Reaction Formula 1,
R1, R2 and R3 are as defined above.

On the other hand, according to an embodiment of the present invention, it was confirmed that the compound of the Formula 1 or a pharmaceutically acceptable salt thereof exhibits excellent cytotoxicity to cancer cells by inhibiting the expression of AIMP2-DX2, and the effect of inhibiting the growth of the tumor was also very excellent in the *in vivo* tumor model.

Accordingly, the present invention provides a pharmaceutical composition for preventing or treating cancer comprising the compound of the Formula 1 or a pharmaceutically acceptable salt thereof as an active ingredient.

On the other hand, according to the prior reports, it has been confirmed that the expression of COX-2 is suppressed and the inflammatory response is alleviated when the expression of AIMP2-DX2 is inhibited (Korean Patent No. 1067816). Accordingly, the compounds of the present invention that inhibit the expression of AIMP2-DX2 may exhibit excellent activity against inflammatory diseases.

Accordingly, the present invention provides a pharmaceutical composition for preventing or treating inflammatory diseases comprising the compound of the Formula 1 or a pharmaceutically acceptable salt thereof as an active ingredient.

In the present invention, the inflammatory disease may be selected from the group consisting of inflammatory skin diseases, crohn's disease, ulcerative colitis, peritonitis, osteomyelitis, cellulitis, meningitis, encephalitis, pancreatitis, trauma-induced shock, bronchial asthma, allergic rhinitis, cystic fibrosis, stroke, acute bronchitis, chronic bronchitis, acute bronchiolitis, chronic bronchiolitis, osteoarthritis, gout, spondyloarthropathy, ankylosing spondylitis, Reiter's syndrome, psoriatic arthropathy, enteric spondylitis, juvenile arthropathy, juvenile ankylosing spondylitis, reactive arthropathy, infectious arthritis, post-infectious arthritis, gonococcal arthritis, tuberculous arthritis, viral arthritis, fungal arthritis, syphilitic arthritis, Lyme disease, arthritis associated with 'vasculitis syndrome', polyarteritis nodosa, hypersensitivity vasculitis, Lou Gehrig's granulomatosis, polymyalgia rheumatica, articular cell arteritis, calcium crystals deposited arthropathy, pseudo gout, non-articular rheumatism, bursitis, tendinitis, epicondylitis (tennis elbow), neuropathic joint disease (charco and joint), hemarthrosic, Henoch-Schönlein purpura, hypertrophic osteoarthropathy, multicentral reticulocytoma, surcoilosis, hemochromatosis, sickle cell disease and other hemoglobinopathy, hyperproteinemia, hypogammaglobulinemia, familial thalassemia, Behat's disease, systemic lupus erythematosus, relapsing fever, psoriasis , multiple sclerosis, sepsis, septic shock, multiple organ dysfunction syndrome, acute respiratory distress syndrome, chronic obstructive pulmonary disease, rheumatoid arthritis, acute lung injury and broncho-pulmonary dysplasia, but is not limited thereto.

The pharmaceutical composition according to the present invention may comprise the compound of the Formula 1 or a pharmaceutically acceptable salt thereof alone, or may additionally comprise one or more pharmaceutically acceptable carriers, excipients or diluents.

The pharmaceutically acceptable carrier may further include, for example, a carrier for oral administration or a carrier for parenteral administration. Carriers for oral administration may include lactose, starch, cellulose derivatives, magnesium stearate, stearic acid, and the like. In addition, the carrier for parenteral administration may include water, a suitable oil, saline, aqueous glucose and glycol, and the like, and may further include a stabilizing agent and a preservative. Suitable stabilizers include antioxidants such as sodium hydrogen sulfite, sodium sulfite or ascorbic acid. Suitable preservatives include benzalkonium chloride, methyl- or propyl-paraben and chlorobutanol. As other pharmaceutically acceptable carriers, those known in the art may be referred to.

The pharmaceutical composition of the present invention may be administered to mammals including humans by any method. For example, it may be administered orally or parenterally. The parenteral administration method is not limited thereto, but may be intravenous, Intramuscular, intraarterial, intramedullary, intrathecal, intracardiac, transdermal, subcutaneous, intraperitoneal, intranasal, enteral, topical, sublingual or rectal administration. For example, the pharmaceutical composition of the present invention may be prepared in an injectable form and administered by lightly pricking the skin with a 30-gauge thin injection needle, or by directly applying it to the skin.

The pharmaceutical composition of the present invention may be formulated as an agent for oral administration or parenteral administration according to the administration route as described above.

In the case of an agent for oral administration, the composition of the present invention may be formulated as a powder, granules, tablets, pills, dragees, capsules, liquids, gels, syrups, slurries, suspensions or the like, using a method known in the art. For example, oral preparations can be obtained by mixing the active ingredient with solid excipients, grinding them, adding suitable adjuvants, and processing them into a mixture of granules to obtain tablets or dragees. Examples of suitable excipients may include sugars including lactose, dextrose, sucrose, sorbitol, mannitol, xylitol, erythritol and maltitol and the like, starches including corn starch, wheat starch, rice starch and potato starch and the like, celluloses including cellulose, methyl cellulose, sodium carboxymethylcellulose and hydroxypropylmethyl-cellulose and the like, and fillers such as gelatin, polyvinylpyrrolidone, and the like. In addition, cross-linked polyvinylpyrrolidone, agar, alginic acid or sodium alginate may be added as a disintegrant if necessary. Furthermore, the pharmaceutical composition of the present invention may further include an anti-aggregating agent, a lubricant, a wetting agent, a flavoring agent, an emulsifying agent, and an antiseptic agent.

Agents for parenteral administration may be formulated in the form of injections or creams, lotion, external ointment, oil, moisturizer, gel, aerosol and nasal inhalant by methods known in the art. These formulations are described in formulary commonly known in all pharmaceutical chemistry.

The total effective amount of the pharmaceutical composition of the present invention may be administered to a patient as a single dose, and may be administered by a fractionated treatment protocol in which multiple doses are administered for a long period of time. The pharmaceutical composition of the present invention may vary the content of the active ingredient depending on the severity of the disease. Preferably, the total dose of the pharmaceutical composition of the present invention may be about 0.01 ug to 1,000 mg, most preferably 0.1 ug to 100 mg per 1 kg of the patient's body weight per day. However, since the dosage of the pharmaceutical composition of the present invention is determined in consideration of various factors such as the age, body weight, health status, sex, disease severity, diet and excretion rate etc. of the patient as well as the route of administration and the number of treatments, and the effective dosage for the patient is determined, considering this point, those of ordinary skill in the art will be able to determine an appropriate effective dosage according to the specific use of the pharmaceutical composition of the present invention as a therapeutic agent for neurodegenerative diseases. The pharmaceutical composition according to the present invention is not particularly limited in its formulation, administration route and administration method as long as the effect of the present invention is exhibited.

In the present invention, the 'treatment' refers to improving the symptoms of cancer or inflammatory disease comprehensively, and this may preferably include curing, substantially preventing, or ameliorating a condition of cancer or inflammatory disease, including, but not limited to, alleviating, curing or preventing one or most symptoms resulting from cancer or an inflammatory disease.

The present invention provides a use of the compound of the Formula 1 or a pharmaceutically acceptable salt thereof for preparing an agent for preventing or treating cancer.

The present invention provides a method for treating cancer comprising administering to a subject in need thereof an effective amount of a composition comprising the compound of the Formula 1 or a pharmaceutically acceptable salt thereof as an active ingredient.

The present invention provides a use of the compound of the Formula 1 or a pharmaceutically acceptable salt thereof for preparing an agent for preventing or treating inflammatory diseases.

The present invention provides a method for treating an inflammatory disease comprising administering to a subject in need thereof an effective amount of a composition comprising the compound of the Formula 1 or a pharmaceutically acceptable salt thereof as an active ingredient.

The 'effective amount' of the present invention refers to an amount showing effect of improvement, treatment, preventing, detecting, diagnosing of cancer or inflammatory disease or inhibiting or reducing cancer or inflammatory disease when administered to a subject, the 'subject' may be an animal, preferably an animal, including a mammal, particularly a human, and may be a cell, tissue, organ, or the like derived from an animal.

The subject may be a patient in need of the effect.

As used herein, the term "comprising" is used synonymously with "including" or "characterized by", in the composition or method according to the present invention, additional components or steps of the method not specifically mentioned are not excluded. In addition, the term "consisting of" means excluding additional elements, steps, or ingredients not specifically described. The term "essentially consisting of" means that, in the scope of a composition or method, it may include substances or steps that do not substantially affect its basic properties in addition to the substances or steps described.

### ADVANTAGEOUS EFFECT

The compound represented by Formula 1 according to the present invention is very effective in inhibiting the expression of AIMP2-DX2, so it can be very usefully used in the development of various diseases caused by AIMP2-DX2, in particular, a therapeutic agent for cancer.

### BRIEF DESCRIPTION OF THE DRAWINGS

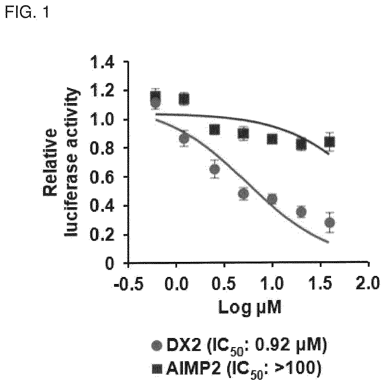

FIG. 1 is a result of calculating the IC50 for AIMP2-DX2 and AIMP2 by measuring the fluorescence after treating nanoruciferase-DX2 or -AIMP2-expressing A549 cells with compound No. 63 of the present invention for 4 hours at each concentration.

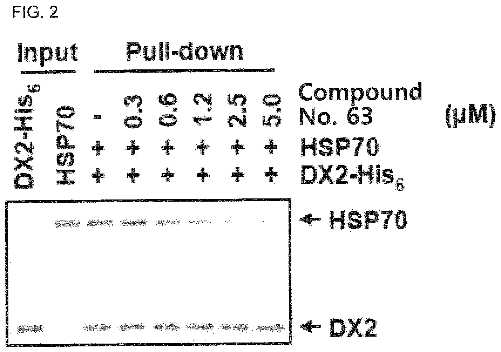

FIG. 2 is a result confirming through an in vitro pull-down assay that compound No. 63 of the present invention inhibits the binding of AIMP2-DX2 and HPS70. After mixing the purified AIMP2-DX2-His6 and HSP70 proteins and treating compound No. 63 for 4 hours at each concentration, AIMP2-DX2 was precipitated with Ni-IDA resin, and HSP70 precipitated with AIMP2-DX2 was visualized by SDS-PAGE and Coomassie staining.

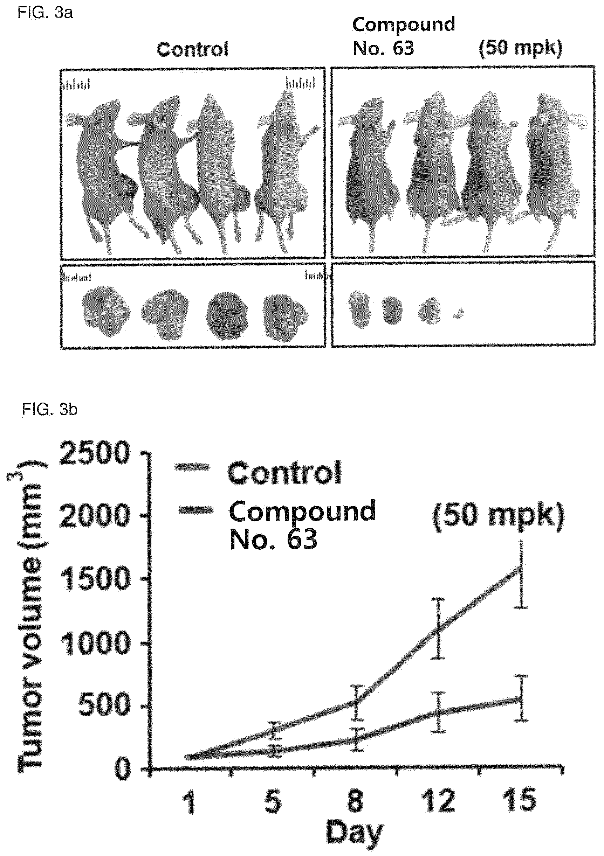

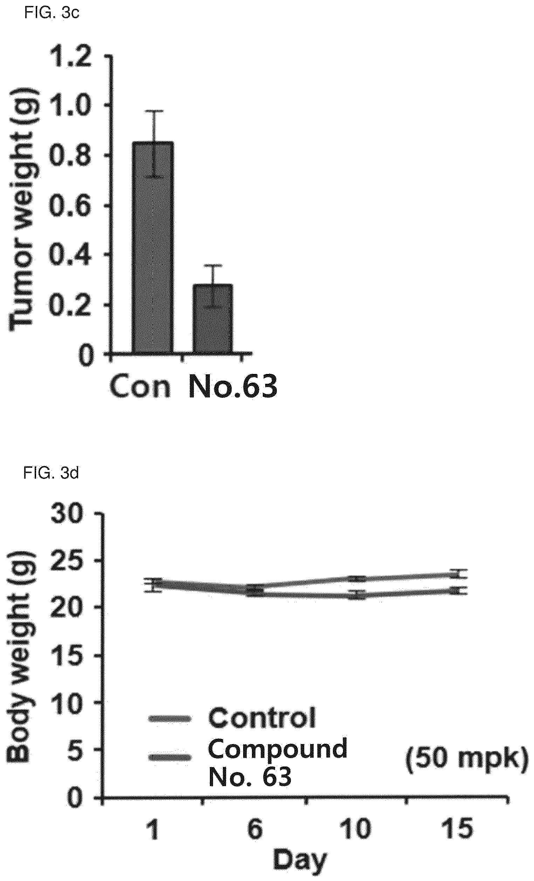

FIGs 3a to 3d are the results of observing the tumor growth while administering compound No. 63 (50 mg/kg) once every two days intraperitoneally for a total of 15 days to a mouse transplanted with H460 cells. (FIG. 3a: macroscopic observation of each mouse and tumor after the end of administration, FIG. 3b: tumor growth curve over time, FIG. 3c: the weight of each tumor obtained at the end of administration, FIG. 3d: change in body weight of animals during administration period).

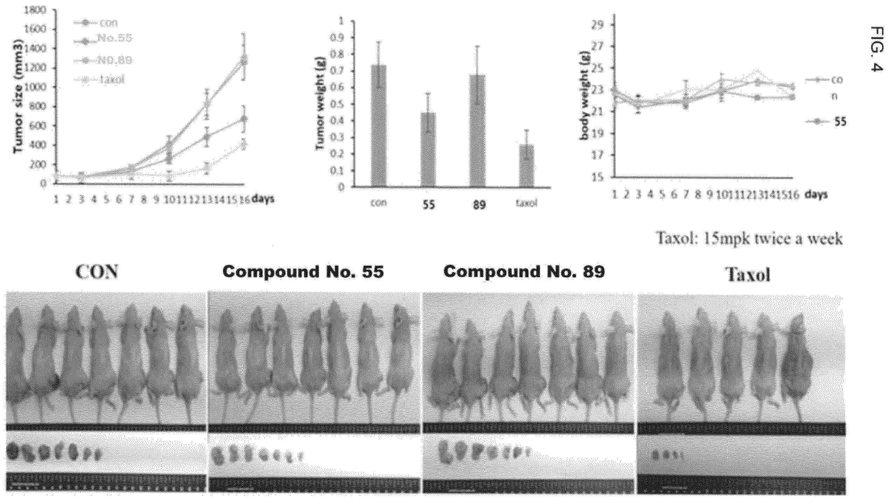

FIG. 4 is a result of testing the cancer cell growth inhibitory effect of compound No. 55 and compound No. 89 among the compounds of the Formula 1 according to the present invention and the tumor distribution of the drug in the H460 xenograft animal model. Taxol was used as a positive control.

### MODE FOR CARRYING OUT INVENTION

Hereinafter, the present invention will be described in detail.

However, the following examples are only illustrative of the present invention, and the content of the present invention is not limited to the following examples.

### Example 1: Preparation of compounds

In the present invention, the compound of Formula 1 was prepared according to the following Reaction Formula 1:

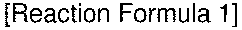

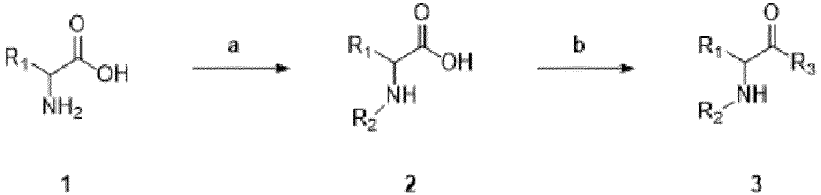

In the Reaction Formula, R1, R2 and R3 are as defined above.

Specifically, starting materials, acid (1) (1.0 eq.), 2M NaOH solution (1.0 eq.) were taken. The mixture was stirred at 0°C for several minutes until a clear solution was formed. To the above solution were added sulfonyl chloride (1.05 eq.), DIPEA (1.1 eq.) and acetone. The mixture was stirred at 0°C for 10 min and then at room temperature for 12 h. After completion of the reaction, the mixture was washed with ether to remove organic impurities. The combined organic layers were washed again with 2M NaOH. To the aqueous layer was added 2N HCI at 0°C until the pH was 2. Then the whole aqueous mixture was extracted with ethyl acetate, water and brine. The organic solvent was dried over MgSO₄ and evaporated in vacuo to obtain compound (2).

To 15 mL of DMF or DCM:THF = 1:1 was added the above compound (2) (2 eq.), amine (1.1 eq.), EDCI (1.1 eq.) and HOBt (1.1 eq.). The mixture was stirred at room temperature overnight, then extracted with ethyl acetate, water and brine. The organic layer was dried over MgSO₄ and evaporated in vacuo to obtain a crude product of compound (3), which was purified by column chromatography to obtain pure compound (3).

Specific examples, structures, and NMR analysis results of the compound of the Formula 1 prepared according to the above method are shown below in detail.

### (S)-4-Methyl-N-(4-methyl-1-morpholino-1-oxopentan-2-yl)benzenesulfonamide

¹H-NMR (400 MHz, CDCl₃) *δ* 7.70 (d, *J* = 8.40 Hz, 2H), 7.29 (d, *J* = 8.00 Hz, 2H), 5.74 (d, *J* = 9.60 Hz, 1H), 4.06 - 4.01 (m, 1H), 3.61 - 3.51 (m, 2H), 3.41 - 3.36 (m, 1H), 3.31 - 3.14 (m, 5H), 2.42 (s, 3H), 2.00 - 1.93 (m, 1H), 1.50 - 1.43 (m, 1H), 1.17 - 1.11 (m, 1H), 0.95 (d, *J* = 6.80 Hz, 3H), 0.92 (d, *J* = 6.80 Hz, 3H)

### (R)-4-methyl-N-(4-methyl-1-morpholino-1-oxopentan-2-yl)benzenesulfonamide

¹H-NMR (400 MHz, CDCl₃) *δ* 7.70 (d, *J* = 8.80 Hz, 2H), 7.29 (d, *J* = 8.40 Hz, 2H), 5.78 (d, *J* = 9.60 Hz, 1H), 4.08 - 4.02 (m, 1H), 3.59 - 3.42 (m, 2H), 3.41 - 3.32 (m, 1H), 3.31 - 3.15 (m, 5H), 2.41 (s, 3H), 1.99 - 1.92 (m, 1H), 1.50 - 1.42 (m, 1H), 1.19 - 1.12 (m, 1H), 0.95 (d, *J* = 6.80 Hz, 3H), 0.92 (d, *J* = 6.80 Hz, 3H)

### (S)-4-Methyl-N-(4-methyl-1-oxo-1-(1,4,7-trioxa-10-azacyclododecan-10-yl)pentan-2-yl)benzenesulfonamide

¹H-NMR (400 MHz, CDCl₃) *δ* 7.71 (d, *J* = 8.00 Hz, 2H), 7.27 (d, *J* = 8.00 Hz, 2H), 5.59 (d, *J* = 10.0 Hz, 1H), 4.25 - 4.19 (m, 1H), 3.78 - 3.71 (m, 2H), 3.69 - 3.37 (m, 11H), 3.22 - 3.12 (m, 3H), 2.40 (s, 3H), 1.98 - 1.91 (m, 1H), 1.49 - 1.33 (m, 2H), 0.91 (t, *J* = 6.00 Hz, 6H)

### (R)-4-methyl-N-(4-methyl-1-oxo-1-(1,4,7-trioxa-10-azacyclododecan-10-yl)-Pentan-2-yl)benzenesulfonamide

¹H-NMR (400 MHz, CDCl₃) *δ* 7.71 (d, *J* = 8.00 Hz, 2H), 7.27 (d, *J* = 8.40 Hz, 2H), 5.66 (d, *J* = 9.60 Hz, 1H), 4.26 - 4.20 (m, 1H), 3.79 - 3.67 (m, 2H), 3.66 - 3.38 (m, 11H), 3.23 - 3.12 (m, 3H), 2.40 (s, 3H), 1.97 - 1.90 (m, 1H), 1.49 - 1.33 (m, 2H), 0.92 (d, *J* = 4.20 Hz, 3H), 0.89 (d, *J* = 5.60 Hz, 3H)

### (S)-N-(1-(1,1-dioxidothiomorpholino)-4-methyl-1-oxopentan-2-yl)-4-methylbenzenesulfonamide

¹H-NMR (400 MHz, CDCl₃) *δ* 7.73 (d, *J* = 7.60 Hz, 2H), 7.34 (d, *J* = 7.60 Hz, 2H), 5.59 (d, *J* = 9.60 Hz, 1H), 4.40 (d, *J* = 14.0 Hz, 1H), 4.05 - 3.99 (m, 1H), 3.81 (d, *J* = 14.8 Hz, 1H), 3.66 (t, *J* = 12.6 Hz, 1H), 3.32 (t, *J* = 12.2 Hz, 1H), 2.88 (t, *J* = 11.2 Hz, 2H), 2.61 - 2.56 (m, 1H), 2.50 - 2.40 (m, 4H), 1.97 - 1.92 (m, 1H), 1.52 - 1.46 (m, 1H), 1.22 - 1.16 (m, 1H), 0.93 (d, *J* = 6.40 Hz, 3H), 0.85 (d, *J* = 6.40 Hz, 3H)

### N-((S)-1-((R)-2-(hydroxymethyl)pyrrolidin-1-yl)-4-methyl-1-oxopentan-2-yl)-4-methylbenzenesulfonamide

¹H-NMR (400 MHz, CDCl₃) *δ* 7.70 (d, *J* = 8.20 Hz, 2H), 7.28 (d, *J* = 7.60 Hz, 2H), 5.60 (d, *J* = 9.60 Hz, 1H), 4.05 (dd, *J* = 3.00, 7.40 Hz, 1H), 3.97 - 3.91 (m, 1H), 4.06 - 4.03 (m, 1H), 3.97 - 3.91 (m, 1H), 3.63 - 3.61 (m, 1H), 3.52 - 3.49 (m, 1H), 3.29 - 3.25 (m, 2H), 2.42 (s, 3H), 1.94 - 1.85 (m, 2H), 1.77 - 1.68 (m, 2H), 1.56 - 1.47 (m, 2H), 1.25 - 1.19 (m, 1H), 0.94 (d, *J* = 3.20 Hz, 3H), 0.93 (d, *J* = 4.00 Hz, 3H)

### (S)-4-Methyl-2-(4-methylphenylsulfonamido)-N-(4-(piperidin-1-yl)phenyl)pentanamide

¹H-NMR (400 MHz, CDCl₃) *δ* 7.85 (s, 1H), 7.76 (d, *J* = 8.00 Hz, 2H), 7.22 - 7.12 (m, 4H), 6.82 - 6.78 (m, 2H), 5.48 (s, 1H), 3.84 - 3.82 (m, 1H), 3.07 (t, *J* = 5.60 Hz, 4H), 2.32 (s, 3H), 1.71 - 1.46 (m, 8H), 0.85 (d, *J* = 6.00 Hz, 3H), 0.72 (d, *J* = 5.60 Hz, 3H)

### (S)-4-Methyl-2-(4-methylphenylsulfonamido)-N-(4-(4-methylpiperidin-1-yl)phenyl) pentanamide

¹H-NMR (400 MHz, CDCl₃) *δ* 7.97 (s, 1H), 7.76 (d, *J* = 8.00 Hz, 2H), 7.21 - 7.12 (m, 4H), 6.81 - 6.77 (m, 2H), 5.66 (d, *J* = 7.60 Hz, 1H), 3.88 - 3.83 (m, 1H), 3.56 - 3.53 (m, 2H), 2.65 - 2.58 (m, 2H), 2.31 (s, 3H), 1.73 - 1.70 (m, 2H), 1.62 - 1.56 (m, 2H), 1.50 - 1.45 (m, 2H), 1.37 - 1.25 (m, 2H), 0.97 (d, *J* = 6.40 Hz, 3H), 0.84 (d, *J* = 6.00 Hz, 3H), 0.71 (d, *J* = 5.60 Hz, 3H)

### (S)-4-Methyl-2-(4-methylphenylsulfonamido)-N-(6-(4-methylpiperidin-1-yl)pyridin-3-yl)pentanamide

¹H-NMR (400 MHz, CDCl₃) *δ* 8.21 (s, 1H), 7.98 (d, *J* = 2.80 Hz, 1H), 7.76 (d, *J* = 8.40 Hz, 2H), 7.48 (dd, *J* = 2.80, 8.80 Hz, 1H ), 7.22 (d, *J* = 8.00 Hz, 2H), 6.52 (d, *J* = 8.80 Hz, 1H), 5.85 (d, *J* = 8.00 Hz, 1H), 4.16 - 4.11 (m, 2H), 3.91 - 3.86 (m, 1H), 2.78 - 2.72 (m, 2H), 2.32 (s, 3H), 1.69 - 1.47 (m, 6H), 1.23 - 1.13 (m, 2H), 0.95 (d, *J* = 6.00 Hz, 3H), 0.83 (d, *J* = 6.40 Hz, 3H), 0.68 (d, *J* = 6.40 Hz, 3H)

### (S)-4-Methyl-2-(4-methylphenylsulfonamido)-N-(6-(4-methylpiperidin-1-yl)pyrimidin-4-yl)pentanamide

¹H-NMR (400 MHz, CDCl₃) *δ* 8.76 (s, 1H), 8.31 (s, 1H), 7.75 (d, *J* = 8.40 Hz, 2H), 7.22 (d, J= 8.40 Hz, 3H), 6.21 (s, 2H), 4.37 (s, 2H), 3.85 (s, 1H), 2.90 - 2.84 (m, 2H), 2.31 (s, 3H), 1.80 - 1.60 (m, 4H), 1.53 - 1.1.47 (m, 2H), 1.19 - 1.09 (m, 2H), 0.97 (d, *J* = 6.80 Hz, 3H), 0.82 (d, *J* = 6.80 Hz, 3H), 0.70 (d, *J* = 6.40 Hz, 3H)

### (S)-4-Methyl-2-(4-methylphenylsulfonamido)-N-(4-morpholinophenyl) pentanamide

¹H-NMR (400 MHz, CDCl₃) *δ* 7.95 (s, 1H), 7.76 (d, *J* = 8.00 Hz, 2H), 7.28 - 7.16 (m, 4H), 6.84 - 6.71 (m, 2H), 5.49 (d, *J* = 7.60 Hz, 1H), 3.84 (t, *J* = 4.80 Hz, 4H), 3.08 (t, *J* = 4.80 Hz, 4H), 2.33 (s, 3H), 1.60-1.58 (m, 2H), 1.50 - 1.46 (m, 1H), 0.84 (d, *J* = 6.00 Hz, 3H), 0.70 (d, *J* = 5.60 Hz, 3H)

### (S)-4-Methyl-2-(4-methylphenylsulfonamido)-N-(6-morpholinopyridin-3-yl)pentanamide

¹H-NMR (400 MHz, CDCl₃) *δ* 8.29 (s, 1H), 8.04 (d, *J* = 2.40 Hz, 1H), 7.77 (d, *J* = 8.40 Hz, 2H), 7.59 (dd, *J* = 2.80, 9.20 Hz, 1H ), 7.23 (d, *J* = 8.40 Hz, 2H), 6.52 (d, *J* = 8.80 Hz, 1H), 5.90 (d, *J* = 8.00 Hz, 1H), 3.92 - 3.86 (m, 1H), 3.80 (t, *J* = 5.00 Hz, 4H), 3.42 (t, *J* = 4.60 Hz, 4H), 2.34 (s, 3H), 1.60 - 1.48 (m, 3H), 0.83 (d, *J* = 6.00 Hz, 3H), 0.67 (d, *J* = 6.00 Hz, 3H)

### (S)-N-(6-((2S,6R)-2,6-dimethylmorpholino)pyridin-3-yl)-4-methyl-2-(4-methylphenylsulfonamido)pentanamide

¹H-NMR (400 MHz, CDCl₃) *δ* 8.03 (d, *J* = 2.40 Hz, 2H), 8.00 (s, 1H), 7.77 (d, *J* = 8.40 Hz, 2H), 7.61 (dd, *J* = 2.80, 9.20 Hz, 1H), 7.26 (d, *J* = 8.00 Hz, 2H), 6.53 (d, *J* = 9.20 Hz, 1H), 5.48 (d, *J* = 7.60 Hz, 1H), 4.13 (q, *J* = 7.20 Hz, 1H), 3.95 (dd, *J* = 2.00, 12.8 Hz, 2H), 3.87 - 3.82 (m, 1H), 3.72 - 3.68 (m, 2H), 2.49 - 2.44 (m, 2H), 2.35 (s, 3H), 1.63 - 1.58 (m, 2H), 1.52 - 1.47 (m, 1H), 1.25 (d, *J* = 8.00 Hz, 6H), 0.85 (d, *J* = 6.00 Hz, 3H), 0.69 (d, *J* = 6.40 Hz, 3H)

### (S)-N-(3-fluoro-4-morpholinophenyl)-4-methyl-2-(4-methylphenylsulfonamido)pentanamide

¹H-NMR (400 MHz, CDCl₃) *δ* 7.81 (s, 1H), 7.76 (d, *J* = 8.00 Hz, 2H), 7.29 (d, *J* = 8.00 Hz, 2H), 7.26 - 7.21 (m, 1H), 6.98 (d, *J* = 6.00 Hz, 1H), 6.85 (t, *J* = 9.00 Hz, 1H), 4.90 (d, *J* = 7.20 Hz, 1H), 3.86 (t, *J* = 4.60 Hz, 4H), 3.77 - 3.72 (m, 1H), 3.04 (t, *J* = 4.40 Hz, 4H), 2.38 (s, 3H), 1.67 - 1.43 (m, 3H), 0.86 (d, *J* = 6.40 Hz, 3H), 0.68 (d, *J* = 6.40 Hz, 3H)

### (S)-N-(4-(1,1-dioxidothiomorpholino)phenyl)-4-methyl-2-(4-methylphenylsulfonamido)pentanamide

¹H-NMR (400 MHz, CDCl₃) *δ* 8.07 (s, 1H), 7.78 (d, *J* = 8.40 Hz, 2H), 7.33 (d, *J* = 9.20 Hz, 2H), 7.28 (d, *J* = 8.40 Hz, 2H), 6.84 (d, *J* = 8.40 Hz, 2H), 5.31 (d, *J* = 7.20 Hz, 1H), 3.82 - 3.76 (m, 5H), 3.10 (t, *J* = 5.20 Hz, 4H), 2.37 (s, 3H), 1.67 - 1.45 (m, 3H), 0.84 (d, *J* = 6.40 Hz, 3H), 0.65 (d, *J* = 6.40 Hz, 3H)

### (S)-tert-butyl 4-(5-(4-methyl-2-(4-methylphenylsulfonamido)pentanamido) pyridin-2-yl)piperazine-1-carboxylate

¹H-NMR (400 MHz, CDCl₃) *δ* 8.15 (s, 1H), 8.02 (d, *J* = 2.80 Hz, 1H), 7.77 (d, *J* = 8.40 Hz, 2H), 7.6 (dd, *J* = 2.80, 9.20 Hz, 1H), 7.25 (d, *J* = 8.40 Hz, 2H), 6.55 (d, *J* = 9.20 Hz, 2H), 5.58 (d, *J* = 8.00 Hz, 1H), 3.89 - 3.84 (m, 1H), 3.53 - 3.43 (m, 8H), 2.33 (s, 3H), 1.62 - 1.59 (m, 2H), 1.52 - 1.48 (m, 10H), 0.85 (d, *J* = 5.60 Hz, 3H), 0.69 (d, *J* = 6.00 Hz, 3H)

### (S)-4-Methyl-2-(4-methylphenylsulfonamido)-N-(4-(pyrrolidin-1-yl)phenyl)pentanamide

¹H-NMR (400 MHz, CDCl₃) *δ* 7.76 (d, *J* = 8.00 Hz, 2H), 7.71 (s, 1H), 7.23 (d, *J* = 8.00 Hz, 2H), 7.08 - 7.04 (m, 2H), 6.43 - 6.39 (m, 2H), 5.47 (d, *J* = 7.60 Hz, 1H), 3.85 - 3.79 (m, 1H), 3.22 (t, *J* = 6.40 Hz, 4H), 2.43 (s, 3H), 2.03 - 1.96 (m, 4H), 1.64 - 1.47 (m, 3H), 0.86 (d, *J* = 6.40 Hz, 3H), 0.74 (d, *J* = 6.40 Hz, 3H)

### (S)-4-Methyl-2-(4-methylphenylsulfonamido)-N-(2-morpholinoethyl)pentanamide

¹H-NMR (400 MHz, CDCl₃) *δ* 7.76 - 7.73 (m, 2H), 7.29 (d, *J* = 8.00 Hz, 2H), 6.65 (t, *J* = 4.80 Hz, 1H), 5.50 (d, *J* = 7.20 Hz, 1H), 3.71 - 3.63 (m, 5H), 3.29 - 3.23 (m, 1H), 3.18 - 3.11 (m, 1H), 2.46 - 2.37 (m, 9H), 1.57 - 1.50 (m, 2H), 1.44 - 1.39 (m, 1H), 0.83 (d, *J* = 6.40 Hz, 3H), 0.67 (d, *J* = 6.00 Hz, 3H)

### (S)-N-(4-methyl-1-morpholino-1-oxopentan-2-yl)-3-nitrobenzenesulfonamide

¹H-NMR (400 MHz, CDCl₃) *δ* 8.62 (t, *J* = 2.00 Hz, 1H), 8.43 - 8.40 (m, 1H), 8.17 - 8.14 (m, 1H), 7.73 (t, *J* = 8.00 Hz, 1H), 5.82 (d, *J* = 10.0 Hz, 1H), 4.28 - 4.22 (m, 1H), 3.66 - 3.58 (m, 1H), 3.57 - 3.51 (m, 2H), 3.39 - 3.21 (m, 5H), 1.97 - 1.90 (m, 1H), 1.53 - 1.45 (m, 1H), 1.25 - 1.18 (m, 1H), 0.98 (d, *J* = 6.80 Hz, 3H), 0.94 (d, *J* = 6.40 Hz, 3H)

### (S)-3-Amino-N-(4-methyl-1-morpholino-1-oxopentan-2-yl)benzenesulfonamide

¹H-NMR (400 MHz, CDCl₃) *δ* 7.23 (t, *J* = 7.80 Hz, 1H), 7.14 - 7.09 (m, 2H), 6.80 (dd, *J* = 1.40, 7.80 Hz, 1H), 4.11 - 4.02 (m, 3H), 3.61 - 3.53 (m, 2H), 3.44 - 3.35 (m, 3H), 3.29 - 3.20 (m, 3H), 1.97 - 1.93 (m, 1H), 1.51 - 1.41 (m, 1H), 1.19 - 1.13 (m, 1H), 0.95 (d, *J* = 6.00 Hz, 3H), 0.91 (d, *J* = 6.80 Hz, 3H)

### (S)-2-(4-Fluorophenylsulfonamido)-4-methyl-N-(4-morpholinophenyl)pentanamide

¹H-NMR (400 MHz, CDCl₃) *δ* 7.98 (s, 1H), 7.91 - 7.87 (m, 2H), 7.22 - 7.19 (m, 2H), 7.12 - 7.07 (m, 2H), 6.80 (dd, *J* = 2.20, 6.60 Hz, 2H), 5.74 (d, *J* = 7.60 Hz, 1H), 3.85 (t, *J* = 5.00 Hz, 4H), 3.09 (t, *J* = 4.80 Hz, 4H), 1.63 - 1.57 (m, 2H), 1.53 - 1.48 (m, 1H), 0.86 (d, *J* = 6.40 Hz, 3H), 0.73 (d, *J* = 6.00 Hz, 3H)

### (S)-2-(4-Bromophenylsulfonamido)-4-methyl-N-(4-morpholinophenyl)pentanamide

¹H-NMR (400 MHz, CDCl₃) *δ* 7.72 (d, *J* = 8.40 Hz, 2H), 7.59 (d, *J* = 8.40 Hz, 2H), 7.45 (s, 1H), 7.18 (d, *J* = 9.20 Hz, 2H), 6.85 (d, *J* = 8.80 Hz, 2H), 5.19 (d, *J* = 8.00 Hz, 1H), 3.86 (t, *J* = 4.60 Hz, 4H), 3.82 - 3.77 (m, 1H), 3.12 (t, *J* = 5.00 Hz, 4H), 2.04 - 1.50 (m, 3H), 0.90 (d, *J* = 6.40 Hz, 3H), 0.8 (d, *J* = 5.60 Hz, 3H)

### (S)-4-Methyl-N-(4-morpholinophenyl)-2-(4-(trifluoromethyl)phenylsulfonamido) pentanamide

¹H-NMR (400 MHz, CDCl₃) *δ* 7.99 (d, *J* = 8.00 Hz, 2H), 7.68 (s, 1H), 7.66 (d, *J* = 4.00 Hz, 2H), 7.10 (d, *J* = 12.4 Hz, 2H), 6.79 (d, *J* = 12.0 Hz, 2H), 5.70 (d, *J* = 8.40 Hz, 1H), 3.92 - 3.98 (m, 1H), 3.85 (t, *J* = 4.80 Hz, 4H), 3.09 (t, *J* = 4.80 Hz, 4H), 1.67 - 1.54 (m, 3H), 0.89 (d, *J* = 6.00 Hz, 3H), 0.79 (d, *J* = 6.40 Hz, 3H)

### (S)-4-Methyl-N-(4-morpholinophenyl)-2-(3-nitrophenylsulfonamido) pentanamide

¹H-NMR (400 MHz, CDCl₃) *δ* 8.70 (s, 1H), 8.26 (d, *J* = 8.00 Hz, 1H), 8.0 (d, *J* = 8.20 Hz, 1H), 7.95 (s, 1H), 7.61 (t, *J* = 8.00 Hz, 1H), 7.12 (d, *J* = 8.80 Hz, 2H), 6.73 (d, *J* = 9.20 Hz, 2H)), 6.15 (d, *J* = 8.80 Hz, 1H), 4.07 - 4.03 (m, 1H), 3.85 (t, *J* = 4.60 Hz, 4H), 3.08 (t, *J* = 4.60 Hz, 4H), 1.72 - 1.67 (m, 1H), 1.60 - 1.56 (m, 2H), 0.90 (d, *J* = 6.40 Hz, 3H), 0.84 (d, *J* = 6.40 Hz, 3H)

### (S)-4-Methyl-N-(4-morpholinophenyl)-2-(naphthalene-1-sulfonamido)pentanamide

¹H-NMR (400 MHz, CDCl₃) *δ* 8.82 (d, *J* = 8.40 Hz, 1H), 8.38 (dd, *J* = 0.80, 7.60 Hz, 1H), 8.13 (s, 1H), 8.09 (d, *J* = 8.00 Hz, 1H), 7.99 (d, *J* = 8.00 Hz, 1H), 7.76 - 7.65 (m, 2H), 7.55 (t, *J* = 7.80 Hz, 1H), 7.09 - 7.06 (m, 2H), 6.79 - 6.76 (m, 2H), 6.07 (d, *J* = 7.20 Hz, 1H), 3.95 - 3.90 (m, 5H), 3.13 (t, *J* = 4.60 Hz, 4H), 1.65 - 1.61 (m, 1H), 1.51 - 1.44 (m, 2H), 0.76 (d, *J* = 6.00 Hz, 3H), 0.50 (d, *J* = 6.80 Hz, 3H)

### (S)-4-Methyl-N-(4-morpholinophenyl)-2-(naphthalene-2-sulfonamido)pentanamide

¹H-NMR (400 MHz, CDCl₃) *δ* 8.47 (s, 1H), 7.96 (s, 1H), 7.80 - 7.81 (m, 4H), 7.62 - 7.52 (m, 2H), 7.08 - 7.05 (m, 2H), 6.68 - 6.64 (m, 2H), 5.70 (d, *J* = 7.60 Hz, 1H), 3.97 - 3.91 (m, 1H), 3.83 (t, *J* = 4.80 Hz, 4H), 3.04 (t, *J* = 4.80 Hz, 4H), 1.63 - 1.48 (m, 3H), 0.81 (d, *J* = 6.40 Hz, 3H), 0.63 (d, *J* = 5.60 Hz, 3H)

### (S)-4-Methyl-N-(4-morpholinophenyl)-2-(phenylsulfonamido)pentanamide

¹H-NMR (400 MHz, CDCl₃) *δ* 7.89 (d, *J* = 7.20 Hz, 2H), 7.75 (s, 1H), 7.56 (t, *J* = 7.40 Hz, 1H), 7.49 (t, *J* = 7.40 Hz, 2H), 7.24 (d, *J* = 9.20 Hz, 2H), 6.82 (d, *J* = 9.20 Hz, 2H), 5.20 (d, *J* = 7.20 Hz, 2H)), 3.85 (t, *J* = 4.80 Hz, 4H), 3.81 - 3.77 (m, 1H), 3.10 (t, *J* = 4.80 Hz, 4H), 1.66 - 1.46 (m, 3H), 0.85 (d, *J* = 6.00 Hz, 3H), 0.69 (d, *J* = 6.00 Hz, 3H)

### (S)-Benzyl 4-methyl-2-(3-(phenylsulfonyl)propanamido)pentanoate

¹H-NMR (400 MHz, CDCl₃) *δ* 7.50 (s, 1H), 7.42 - 7.31 (m, 7H), 6.88 - 6.84 (m, 2H), 5.01 (d, *J* = 8.00 0Hz, 1H), 4.27 (d, *J* = 3.20 Hz, 2H), 3.86 - 3.81 (m, 5H), 3.12 (t, *J* = 4.80 Hz, 4H), 1.74 - 1.60 (m, 2H), 1.54 - 1.47 (m, 1H), 0.93 (d, *J* = 6.40 Hz, 3H), 0.90 (d, *J* = 6.40 Hz, 3H)

### (S)-2-(4-Methoxyphenylsulfonamido)-4-methyl-N-(4-morpholinophenyl)pentanamide

¹H-NMR (400 MHz, CDCl₃) *δ* 8.10 (s, 1H), 7.83 - 7.79 (m, 2H), 7.26 - 7.22 (m, 2H), 6.89 - 6.86 (m, 2H), 6.80-6.76 (m, 2H), 5.60 (d, *J* = 7.60 Hz, 1H), 3.87 - 3.81 (m, 5H), 3.76 (s, 3H), 3.08 (t, *J* = 5.40 Hz, 4H), 1.63 - 1.58 (m, 2H), 1.51 - 1.47 (m, 1H), 0.85 (d, *J* = 6.00 Hz, 3H), 0.70 (d, *J* = 5.60 Hz, 3H)

### (S)-4-Methyl-N-(4-morpholinophenyl)-2-(thiophene-2-sulfonamido)pentanamide

¹H-NMR (400 MHz, CDCl₃) *δ* 7.73 (s, 1H), 7.65 (dd, *J* = 1.20, 4.00 Hz, 1H), 7.58 (dd, *J* = 1.20, 4.80 Hz, 1H), 7.30 - 7.27 (m, 2H), 7.05 (dd, *J* = 3.60, 5.20 Hz, 1H), 6.85 - 6.81 (m, 2H), 5.34 (s, 1H), 3.91 - 3.84 (m, 5H), 3.11 (t, *J* = 5.00 Hz, 4H), 1.72 - 1.51 (m, 3H), 0.89 (d, *J* = 6.00 Hz, 3H), 0.79 (d, *J* = 6.00 Hz, 3H)

### (S)-4-Methyl-N-(4-morpholinophenyl)-2-(4-nitrophenylsulfonamido)pentanamide

¹H-NMR (400 MHz, CDCl₃) *δ* 8.23 - 8.21 (m, 2H), 8.03 - 8.00 (m, 2H), 7.12 (d, *J* = 8.80 Hz, 2H), 6.79 (d, *J* = 9.20 Hz, 2H), 5.49 (d, *J* = 8.80 Hz, 1H), 3.91 - 3.84 (m, 5H), 3.10 (t, *J* = 4.80 Hz, 4H), 1.76 - 1.70 (m, 1H), 1.61 - 1.58 (m, 3H), 0.93 (d, *J* = 6.80 Hz, 3H), 0.87 (d, *J* = 6.80 Hz, 3H)

### (S)-2-(4-Isopropylphenylsulfonamido)-4-methyl-N-(4-morpholinophenyl)pentanamide

¹H-NMR (400 MHz, CDCl₃) *δ* 7.81 - 7.78 (m, 3H), 7.31 (d, *J* = 8.40 Hz, 2H), 7.26 - 7.21 (m, 2H), 6.82 - 6.78 (m, 2H), 5.17 (d, *J* = 6.40 Hz, 1H), 3.85 (t, *J* = 4.00 Hz, 4H), 3.79 - 3.77 (m, 1H), 3.09 (t, *J* = 4.80 Hz, 4H), 2.94 - 2.87 (m, 1H), 1.68 - 1.44 (m, 3H), 1.19 (d, *J* = 6.80 Hz, 6H), 0.85 (d, *J* = 6.00 Hz, 3H), 0.67 (d, *J* = 6.00 Hz, 3H)

### (S)-2-(Biphenyl-4-ylsulfonamido)-4-methyl-N-(4-morpholinophenyl)pentanamide

¹H-NMR (400 MHz, CDCl₃) *δ* 7.94 (d, *J* = 8.20 Hz, 2H), 7.87 (brs, 1H), 7.59 - 7.64 (m, 2H), 7.37 - 7.49 (m, 5H), 7.15 - 7.20 (m, 2H), 6.72 (d, *J* = 9.00 Hz, 2H), 5.48 - 5.60 (m, 1H), 3.87 - 3.95 (m, 1H), 3.79 - 3.85 (m, 4H), 2.98 - 3.05 (m, 4H), 1.62 - 1.69 (m, 2H), 1.51 - 1.60 (m, 1H), 0.88 (d, *J* = 5.80 Hz, 3H), 0.76 (d, *J* = 5.80 Hz, 3H)

### (S)-4-(Benzyloxy)-N-(4-methyl-1-oxo-1-(1,4,7-trioxa-10-azacyclododecan-10-yl)pentan-2-yl)benzenesulfonamide

¹H-NMR (400 MHz, CDCl₃) *δ* 7.76 - 7.73 (m, 2H), 7.41 - 7.32 (m, 5H), 7.03 - 6.99 (m, 2H), 5.55 (d, *J* = 10.0 Hz, 1H), 5.11 (s, 2H), 4.22 - 4.17 (m, 1H), 3.78 - 3.39 (m, 13H), 3.22 - 3.06 (m, 3H), 1.97 - 1.90 (m, 1H), 1.48 - 1.33 (m, 2H), 0.92 (d, *J* = 4.00 Hz, 3H), 0.89 (d, *J* = 4.80 Hz, 3H)

### (S)-4-Hydroxy-N-(4-methyl-1-oxo-1-(1,4,7-trioxa-10-azacyclododecan-10-yl)pentan-2-yl)benzenesulfonamide

¹H-NMR (400 MHz, CDCl₃) *δ* 8.20 (s, 1H), 7.65 (d, *J* = 8.80 Hz, 2H), 6.83 (d, *J* = 8.80 Hz, 2H), 5.65 (d, *J* = 9.60 Hz, 1H), 4.31 - 4.26 (m, 1H), 3.84 - 3.19 (m, 16H), 1.99 - 1.93 (m, 1H), 1.53 - 1.52 (m, 2H), 0.93 (t, *J* = 6.20 Hz, 6H)

### (S)-Benzyl 4-(N-(4-methyl-1-oxo-1-(1,4,7-trioxa-10-azacyclododecan-10-yl)pentan-2-yl)sulfamoyl)benzoate

¹H-NMR (400 MHz, CDCl₃) *δ* 8.19 - 8.16 (m, 2H), 7.92 - 7.89 (m, 2H), 7.45-7.34 (m, 5H), 5.76 (d, *J* = 9.20 Hz, 1H), 5.37 (s, 2H), 4.34 - 4.28 (m, 1H), 3.82 - 3.76 (m, 1H), 3.68 - 3.63 (m, 1H), 3.58 - 3.07 (m, 14H), 1.97 - 1.90 (m, 1H), 1.50 - 1.34 (m, 2H), 0.94 (d, *J* = 6.40 Hz, 3H), 0.91 (d, *J* = 6.80 Hz, 3H)

### (S)-tert-Butyl 3-(4-methylphenylsulfonamido)-4-morpholino-4-oxobutanoate

¹H-NMR (400 MHz, CDCl₃) *δ* 7.72 (d, *J* = 8.40 Hz, 2H), 7.31 (d, *J* = 7.60 Hz, 2H), 5.89 (d, *J* = 9.60 Hz, 1H), 4.55 - 4.498 (m, 1H), 3.62 - 3.28 (m, 8H), 2.55 - 2.40 (m, 4H), 1.41 (m, 9H)

### (S)-4-Methyl-N-(1-morpholino-1-oxo-3-phenylpropan-2-yl)benzenesulfonamide

¹H-NMR (400 MHz, CDCl₃) *δ* 7.68 (d, *J* = 4.80 Hz, 2H), 7.23 - 7.29 (m, 5H), 7.11 - 7.16 (m, 2H), 5.88 (d, *J* = 9.60 Hz, 1H), 4.29 - 4.35 (m, 1H), 3.22 - 3.34 (m, 3H), 3.05 - 3.14 (m, 2H), 2.90 - 2.98 (m, 2H), 2.74 - 2.81 (m, 1H), 2.55 - 2.61 (m, 2H), 2.40 (s, 3H)

### (S)-N-(3-hydroxy-1-morpholino-1-oxopropan-2-yl)-4-methylbenzenesulfonamide

¹H-NMR (400 MHz, CDCl₃) *δ* 7.74 (d, *J* = 8.40 Hz, 2H), 7.32 (d, *J* = 8.4 Hz, 2H), 6.00 (d, *J* = 8.80 Hz, 1H), 4.19 - 4.24 (m, 1H), 3.52 - 3.68 (m, 4H), 3.34 - 3.49 (m, 6H), 2.82 (q, *J* = 4.30 Hz, 1H), 2.44 (s, 3H)

### (S)-4-Methyl-N-(3-methyl-1-morpholino-1-oxobutan-2-yl)benzenesulfonamide

¹H-NMR (400 MHz, CDCl₃) *δ* 7.69 (d, *J* = 8.40 Hz, 2H), 7.29 (d, *J* = 8.40 Hz, 2H), 5.66 (d, *J* = 9.20 Hz, 1H), 3.81 (q, *J* = 4.80 Hz, 1H), 3.51 - 3.58 (m, 2H), 3.24 - 3.35 (m, 5H), 3.10-3.17 (m, 1H), 2.42 (s, 3H), 1.76 - 1.84 (m, 1H), 1.02 (d, *J* = 6.80 Hz, 3H), 0.88 (d, *J* = 6.80 Hz, 3H)

### (R)-N-(3-mercapto-1-morpholino-1-oxopropan-2-yl)-4-methylbenzenesulfonamide

¹H-NMR (400 MHz, CDCl₃) *δ* 7.76 (d, *J* = 8.00 Hz, 2H), 7.33 (d, *J* = 8.00 Hz, 2H), 5.95 (d, *J* = 9.60 Hz, 1H), 4.48 - 4.53 (m, 1H), 3.54 - 3.63 (m, 2H), 3.30 - 3.45 (m, 6H), 2.95 (dd, *J* = 7.00, 13.8 Hz, 1H), 2.84 (dd, *J* = 6.20, 14.2 Hz, 1H), 2.44 (s, 3H)

### (2S, 3S)-3-Hydroxy-2-(4-methylphenylsulfonamido)-N-(4-morpholinophenyl)butanamide

¹H-NMR (400 MHz, OMSO-*d₆*) *δ* 8.44 (s, 1H), 7.77 (d, *J* = 7.20 Hz, 2H), 7.28 - 7.30 (m, 3H), 6.82 (d, *J* = 8.80 Hz, 2H), 5.95 (d, *J* = 7.20 Hz, 1H), 4.38 - 4.40 (m, 1H), 3.84 (t, *J* = 4.60 Hz, 4H), 3.77 (dd, *J* = 2.20, 6.60 Hz, 1H), 3.08 - 3.10 (m, 5H), 2.39 (s, 3H), 0.96 (d, *J* = 6.40 Hz, 3H)

### (2S,3S)-3-Methyl-2-(4-methylphenylsulfonamido)-N-(4-morpholinophenyl)pentanamide

¹H-NMR (400 MHz, CDCl₃) *δ* 9.57 (s, 1H), 7.84 (d, *J* = 7.60 Hz, 1H), 7.61 (d, *J* = 8.40 Hz, 2H), 7.18 (d, *J* = 8.00 Hz, 2H), 7.11 (d, *J* = 9.20 Hz, 2H), 6.80 (d, *J* = 8.80 Hz, 2H), 3.71 (t, *J* = 4.80 Hz, 4H), 3.57 (t, *J* = 7.60 Hz, 1H), 3.00 (t, *J* = 4.60 Hz, 4H), 2.20 (s, 3H), 1.50 - 1.63 (m, 2H), 1.08 - 1.12 (m, 1H), 0.76 - 0.79 (m, 6H)

### (S)-2-(4-Methylphenylsulfonamido)-N-(4-morpholinophenyl)propanamide

¹H-NMR (400 MHz, OMSO-*d₆*) *δ* 9.63 (s, 1H), 8.00 (s, 1H) 7.66 (d, *J* = 7.60 Hz, 2H), 7.23 - 7.29 (m, 4H), 6.84 (d, *J* = 8.80 Hz, 2H), 3.87 (q, *J* = 6.80 Hz, 1H), 3.71 (t, *J* = 4.60 Hz, 4H), 3.01 (t, *J* = 4.40 Hz, 4H), 2.29 (s, 3H), 1.12 (d, *J* = 7.20 Hz, 3H)

### (S)-2-(4-Methylphenylsulfonamido)-4-(methylthio)-N-(4-morpholinophenyl)butanamide

¹H-NMR (400 MHz, OMSO-*d₆*) *δ* 10.06 (s, 1H), 8.42 (s, 1H), 8.03 (d, *J* = 8.00 Hz, 2H), 7.63 (d, *J* = 8.40 Hz, 2H), 7.59 (d, *J* = 9.20 Hz, 2H), 7.21 (d, *J* = 8.80 Hz, 2H), 4.26 - 4.29 (m, 1H), 4.09 (t, *J* = 4.40 Hz, 4H), 3.76 (s, 3H), 3.39 (t, *J* = 4.60 Hz, 4H), 2.74 - 2.87 (m, 2H), 2.33 (s, 3H), 2.05 - 2.25 (m, 2H)

### (S)-3-(1H-indol-2-yl)-2-(4-methylphenylsulfonamido)-N-(4-morpholinophenyl) propanamide

¹H-NMR (400 MHz, DMSO-*d₆*) *δ* 10.75 (s, 1H), 9.64 (s, 1H), 8.05 (s, 1H), 7.50 (d, *J* = 8.00 Hz, 2H), 7.39 (d, *J* = 7.60 Hz, 1H), 7.27 (d, *J* = 8.40 Hz, 1H), 7.18 (d, *J* = 9.20 Hz, 2H), 7.09 (d, *J* = 8.40 Hz, 2H), 7.06 (d, *J* = 2.00 Hz, 1H), 7.02 (t, *J* = 7.40 Hz, 1H), 6.91 (t, *J* = 7.20 Hz, 1H), 6.81 (d, *J* = 8.80 Hz, 2H), 3.99 - 4.06 (m, 1H), 3.71 (t, *J* = 4.80 Hz, 4H), 2.99 - 3.05 (m, 5H), 2.79 - 2.85 (m, 1H), 2.18 (s, 3H)

### (S)-3-(1-Methyl-1H-indol-3-yl)-2-(4-methylphenylsulfonamido)-N-(4-morpholinophenyl) propanamide

¹H-NMR (400 MHz, CDCl₃) *δ* 8.04 (s, 1H), 7.48 (d, *J* = 8.20 Hz, 2H), 7.17 - 7.31 (m, 5H), 7.06 (d, *J* = 8.20 Hz, 2H), 7.00 (dt, *J* = 1.10, 7.20 Hz, 1H), 6.82 (d, *J* = 9.00 Hz, 2H), 6.76 (s, 1H), 5.17 (d, *J* = 6.20 Hz, 1H), 4.01 (q, *J* = 6.20 Hz, 1H), 3.82 - 3.87 (m, 3H), 3.67 (s, 2H), 3.17 (dd, *J* = 2.93, 6.46 Hz, 1H), 3.07 - 3.12 (m, 2H), 2.34 (s, 3H)

### (S)-2-(4-Methylphenylsulfonamido)-N-(4-morpholinophenyl)-3-phenylpropanamide

¹H-NMR (400 MHz, OMSO-*d₆*) *δ* 9.64 (s, 1H), 8.11 (s, 1H), 7.48 (d, *J* = 7.60 Hz, 2H), 7.12 - 7.20 (m, 9H), 6.81 (d, *J* = 8.40 Hz, 2H), 4.03 - 4.05 (m, 1H), 3.71 (t, *J* = 4.40 Hz, 4H), 3.01 (t, *J* = 4.40 Hz, 4H), 2.85 - 2.90 (m, 1H), 2.70 - 2.73 (m, 1H), 2.24 (s, 3H)

### (S)-Benzyl4-methyl-2-(4-methylphenylsulfonamido)pentanoate

¹H-NMR (400 MHz, CDCl₃) *δ* 7.66 - 7.73 (m, 2H), 7.30 - 7.38 (m, 3H), 7.15 - 7.24 (m, 4H), 5.20 (d, *J* = 10.1 Hz, 1H), 4.79 - 4.89 (m, 2H), 3.98 (ddd, *J* = 6.20, 8.30, 10.0 Hz, 1H), 2.39 (s, 3H), 1.72 - 1.85 (m, 1H), 1.43 - 1.55 (m, 2H), 0.87 (dd, *J* = 6.60, 7.80 Hz, 6H)

### (S)-Benzyl4-methyl-2-(4-methylbenzamido)pentanoate

¹H-NMR (400 MHz, CDCl₃) *δ* 7.69 (d, *J* = 8.00 Hz, 2H), 7.39 - 7.32 (m, 5H), 7.24 (d, *J* = 8.40 Hz, 2H), 6.48 (d, *J* = 7.60 Hz, 1H), 5.20 (d, *J* = 1.20 Hz, 2H), 4.93 - 4.87 (m, 1H), 2.40 (s, 3H), 1.80 - 1.62 (m, 3H), 0.97 (d, *J* = 6.00 Hz, 3H), 0.95 (d, *J* = 6.00 Hz, 3H)

### (S)-Benzyl2-(4-tert-butylbenzamido)-4-methylpentanoate

¹H-NMR (400 MHz, CDCl₃) *δ* 7.74 - 7.72 (m, 2H), 7.52 - 7.44 (m, 2H), 7.37 - 7.31 (m, 5H), 6.49 (d, *J* = 8.40 Hz, 1H), 5.19 (s, 2H), 4.94 - 4.88 (m, 1H), 1.78 - 1.63 (m, 3H), 1.33 (s, 9H), 0.97 (d, *J* = 6.00 Hz, 3H), 0.94 (d, *J* = 5.60 Hz, 3H)

### (S)-Benzyl 2-(benzo[d][1,3]dioxole-5-carboxamido)-4-methylpentanoate

¹H-NMR (400 MHz, CDCl₃) *δ* 7.71 (dd, *J* = 2.00, 8.00 Hz, 1H), 7.51 (d, *J* = 2.00 Hz, 1H), 7.38 - 7.33 (m, 5H), 6.86 (d, *J* = 8.40 Hz, 1H), 6.06 (s, 2H), 5.16 (d, *J* = 1.20 Hz, 2H), 3.56 - 3.52 (m, 1H), 1.78 - 1.73 (m, 1H), 1.63 - 1.56 (m, 1H), 1.48 - 1.41 (m, 1H), 0.93 (d, *J* = 6.40 Hz, 3H), 0.91 (d, *J* = 6.80 Hz, 3H)

### (S)-Benzyl2-(4-(tert-butoxycarbonylamino)benzamido)-4-methylpentanoate

¹H-NMR (400 MHz, CDCl₃) *δ* 7.75 - 7.72 (m, 2H), 7.43 (d, *J* = 8.40 Hz, 2H), 7.39 - 7.31 (m, 5H), 6.61 (s, 1H), 6.43 (d, *J* = 8.40 Hz, 1H), 5.19 (d, *J* = 2.00 Hz, 2H), 4.92 - 4.86 (m, 1H), 1.78 - 1.62 (m, 3H), 0.97 (d, *J* = 6.00 Hz, 3H), 0.95 (d, *J* = 6.40 Hz, 3H)

### (S)-Benzyl 4-methyl-2-(3-(phenylsulfonyl)propanamido)pentanoate

¹H-NMR (400 MHz, CDCl₃) *δ* 7.91 - 7.89 (m, 2H), 7.68 - 7.54 (m, 3H), 7.38 - 7.31 (m, 5H), 5.97 (d, *J* = 8.00 Hz, 1H), 5.14 (s, 2H), 4.61 - 4.56 (m, 1H), 3.46 - 3.40 (m, 2H), 2.71 - 2.66 (m, 2H), 1.64 - 1.50 (m, 3H), 0.90 (d, *J* = 5.60 Hz, 6H)

### (S)-N-(4-(4-methoxyphenyl)thiazol-2-yl)-4-methyl-2-(4-methylphenylsulfonamido)pentanamide

¹H-NMR (400 MHz, CDCl₃) *δ* 9.54 (brs, 1H), 7.75 (t, *J* = 7.60 Hz, 4H), 7.26 (d, *J* = 8.20 Hz, 2H), 6.99 (d, *J* = 0.70 Hz, 1H), 6.95 (d, *J* = 8.20 Hz, 2H), 5.19 (d, *J* = 7.00 Hz, 1H), 3.91 (d, *J* = 5.00 Hz, 1H), 3.84 (d, *J* = 0.70 Hz, 3H), 2.32 (s, 3H), 1.53 - 1.63 (m, 2H), 1.43 - 1.51 (m, 1H), 0.84 (d, *J* = 5.80 Hz, 3H), 0.68 (d, *J* = 5.80 Hz, 3H)

### (S)-1-(4-Methyl-1-morpholino-1-oxopentan-2-yl)-3-phenylurea

¹H-NMR (400 MHz, CDCl₃) *δ* 7.80 (s, 1H), 7.26 - 7.17 (m, 4H), 6.94 - 6.90 (m, 1H), 6.72 (d, *J* = 8.80 Hz, 1H), 5.03 - 4.97 (m, 1H), 3.81 - 3.57 (m, 8H), 1.84 - 1.77 (m, 1H), 1.61 - 1.43 (m, 2H), 0.90 (d, *J* = 3.60 Hz, 3H), 0.87 (d, *J* = 7.20 Hz, 3H)

### (S)-4-ferf-Butyl-N-(4-methyl-1-morpholino-1-oxopentan-2-yl)benzamide

¹H-NMR (400 MHz, OMSO-*d₆*) *δ* 8.40 (s, 1H), 7.81 (d, *J* = 8.00 Hz, 2H), 7.45 (d, *J* = 8.80 Hz, 2H), 4.82 - 4.73 (m, 1H), 3.53 - 3.42 (m, 8H), 1.74 - 1.50 (m, 3H), 1.28 (s, 9H), 0.89 (d, *J* = 6.80 Hz, 6H)

### (S)-N-(4--morpholino-1-oxopentan-2-yl)-3-(phenylsulfonyl)propanamide

¹H-NMR (400 MHz, DMSO-*d₆*) *δ* 8.30 (d, *J* = 8.80 Hz, 1H), 7.88 - 7.86 (m, 2H), 7.75 (t, *J* = 8.00 Hz, 1H), 7.65 (d, *J* = 8.00 Hz, 2H), 4.66 - 4.61 (m, 1H), 3.52 - 3.25 (m, 10H), 2.46 - 2.42 (m, 2H), 1.52 - 1.46 (m, 1H), 1.38 - 1.33 (m, 2H), 0.82 (d, *J* = 6.60 Hz, 6H)

### (S)-4-tert-Butyl-N-(4-methyl-1-(4-morpholinophenylamino)-1-oxopentan-2-yl)benzamide

¹H-NMR (400 MHz, MeOD) *δ* 7.81(d, *J* = 8.00 Hz, 2H), 7.51 (t, *J* = 8.80 Hz, 2H), 7.45 (d, *J* = 8.80 Hz, 2H), 6.94 (d, *J* = 8.80 Hz, 2H), 4.85 - 4.81 (m, 1H), 3.82 (t, *J* = 5.00 Hz, 3H), 3.10 (t, *J* = 4.80 Hz, 3H), 1.82 - 1.62 (m, 3H), 1.28 (s, 9H), 1.03 (d, *J* = 6.60 Hz, 6H)

### (S)-N-(4-Methyl-1-(4-morpholinophenylamino)-1-oxopentan-2-yl)benzo[d][1,3]dioxole-5-carboxamide

¹H-NMR (400 MHz, OMSO-*d₆*) *δ* 9.84 (s, 1H), 8.35 (d, *J* = 7.60 Hz, 1H), 7.52 - 7.44 (m, 4H), 6.97 (d, *J* = 8.40 Hz, 1H), 6.87 (d, *J* = 9.20 Hz, 2H), 6.08 (d, *J* = 2.40 Hz, 2H), 4.60 - 4.54 (m, 1H), 3.71 (d, *J* = 4.80 Hz, 4H), 3.02 (t, *J* = 4.80 Hz, 4H), 1.74 - 1.50 (m, 3H), 0.92 (d, *J* = 6.80 Hz, 3H), 0.89 (d, *J* = 6.40 Hz, 3H)

### (S)-4-Methyl-N-(4-morpholinophenyl)-2-(3-(phenylsulfonyl)propanamido)pentanamide

¹H-NMR (400 MHz, CDCl₃) *δ* 8.69 - 8.63 (m, 1H), 7.87 (d, *J* = 7.20 Hz, 2H), 7.71 - 7.32 (m, 5H), 7.18 - 6.60 (m, 3H), 4.59 - 4.55 (m, 1H), 3.82 (t, *J* = 5.00 Hz, 3H), 3.54 - 3.48 (m, 2H), 3.06 (t, *J* = 4.80 Hz, 3H), 2.75 - 2.73 (m, 2H), 1.85 - 1.56 (m, 3H), 0.89 (d, *J* = 6.2 Hz, 6H)

### (S)-2-(4-Bromophenylsulfonamido)-3-(1H-indol-3-yl)-N-(4-(4-methoxyphenyl)thiazol-2-yl)propanamide

¹H-NMR (500 MHz, DMSO-*d₆*) *δ* 12.44 (s, 1H), 10.78 (s, 1H), 8.51 (brs, 1H), 7.82 - 7.80 (d, *J* = 8.50 Hz, 2H), 7.47 - 7.37 (m, 6H), 7.28 - 7.26 (d, *J* = 8.00 Hz), 7.08 - 6.90 (m, 5H), 4.32 (brs, 1H), 3.79 (s, 3H), 3.33 (s, 2H), 3.11 - 3.07 (dd, *J* = 14.5, 6.00 Hz, 1H), 2.90 - 2.85 (dd, *J* = 14.0, 8.50 Hz, 1H)

### (S)-3-(1H-indol-3-yl)-N-(4-(4-methoxyphenyl)thiazol-2-yl)-2-(4-methylphenylsulfonamido)propanamide

¹H-NMR (500 MHz, DMSO-*d₆*) *δ* 12.3 (s, 1H), 10.7 (s, 1H), 8.24 - 8.22 (d, *J* = 8.50 Hz, 1H), 7.95 - 7.93 (d, *J* = 8.50 Hz, 1H), 7.50 - 7.48 (d, *J* = 8.50 Hz, 2H), 7.15 - 7.09 (m, 4H), 6.97 - 6.95 (d, *J* = 8.00 Hz, 1H), 6.74 - 6.58 (m, 6H), 4.01 - 3.96 (q, *J* = 7.50 Hz, 1H), 3.47 (s, 3H), 2.79 - 2.74 (dd, *J* = 14.2, 7.00 Hz, 1H), 2.56 - 2.51 (dd, *J* = 14.5, 8.00 Hz, 1H), 1.86 (s, 3H)

### (S)-3-(1H-indol-3-yl)-2-(2-methylphenylsulfonamido)-N-(4-morpholinophenyl)propanamide

¹H-NMR (400 MHz, CDCl₃) *δ* 8.27 (s, 1H), 7.86 - 7.83 (m, 2H), 7.33 - 7.29 (m, 3H), 7.20 - 7.14 (m, 4H), 7.01 - 6.95 (m, 2H), 6.91 (d, *J* = 2.40 Hz, 4H), 6.80 - 6.78 (m, 2H), 5.38 (d, *J* = 5.60 Hz, 1H), 4.00 - 3.95 (m, 1H), 3.83 (t, *J* = 4.60 Hz, 4H), 3.27 - 3.14 (m, 2H), 3.07 (t, *J* = 5.00 Hz, 4H), 2.23 (s, 3H)

### (S)-3-(1H-indol-3-yl)-2-(3-methylphenylsulfonamido)-N-(4-morpholinophenyl)propanamide

¹H-NMR (400 MHz, OMSO-*d₆*) *δ* 10.76 (s, 1H), 9.70 (s, 1H), 8.11 (s, 1H), 7.45 - 7.39 (m, 3H), 7.28 - 7.19 (m, 5H), 7.06 - 7.00 (m, 2H), 6.93 - 6.89 (m, 1H), 6.81 (d, *J* = 8.80 Hz, 2H), 4.09 - 4.07 (m, 1H), 3.71 (t, *J* = 4.80 Hz, 4H), 3.05 - 2.99 (m, 5H), 2.85 - 2.79 (m, 1H), 2.14 (s, 3H)

### (S)-2-(3,5-Dimethylphenylsulfonamido)-3-(1H-indol-3-yl)-N-(4 morpholinophenyl)propanamide

¹H-NMR (400 MHz, OMSO-*d₆*) *δ* 10.77 (s, 1H), 9.71 (s, 1H), 8.04 (s, 1H), 7.42 (d, *J* = 8.00 Hz, 1H), 7.28 - 7.22 (m, 5H), 7.07 - 7.00 (m, 3H), 6.92 (t, *J* = 7.40 Hz, 1H), 6.82 (d, *J* = 9.20 Hz, 2H), 4.11 - 4.07 (m, 1H), 3.71 (t, *J* = 4.80 Hz, 4H), 3.05 - 2.99 (m, 5H), 2.85 - 2.80 (m, 1H), 2.12 (s, 6H)

### (S)-2-(4-Fluorophenylsulfonamido)-3-(1H-indol-3-yl)-N-(4-morpholinophenyl)propanamide

¹H-NMR (400 MHz, OMSO-*d₆*) *δ* 10.77 (d, *J* = 2.00 Hz, 1H), 9.75 (s, 1H), 8.26 (s, 1H), 7.62 - 7.59 (m, 2H), 7.46 (d, *J* = 8.00 Hz, 1H), 7.28 - 7.22 (m, 3H), 7.09 - 7.01 (m, 4H), 6.93 (t, *J* = 7.40 Hz, 1H), 6.85 - 6.81 (m, 2H), 4.10 (s, 1H), 3.72 (t, *J* = 4.80 Hz, 4H), 3.07 - 3.01 (m, 5H), 2.89 - 2.83 (m, 1H)

### (S)-3-(1H-indol-3-yl)-N-(4-morpholinophenyl)-2-(4-(trifluoromethyl)phenylsulfon-amido)propanamide

¹H-NMR (400 MHz, OMSO-*d₆*) *δ* 10.77 (d, *J* = 1.60 Hz, 1H), 9.79 (s, 1H), 8.55 (s, 1H), 7.72 (d, *J* = 8.80 Hz, 2H), 7.57 (d, *J* = 8.40 Hz, 2H), 4.47 (d, *J* = 8.00 Hz, 1H), 7.25 (d, *J* = 7.60 Hz, 1H), 7.19 (d, *J* = 9.20 Hz, 2H), 7.11 (d, *J* = 2.40 Hz, 1H), 7.02 (t, *J* = 7.60 Hz, 1H), 6.92 (t, *J* = 7.40 Hz, 1H), 6.81 (d, *J* = 9.20 Hz, 2H), 4.14 (s, 1 H), 3.72 (t, *J* = 4.60 Hz, 4H), 3.08 - 2.99 (m, 5H), 2.92 - 2.86 (m, 1H)

### (S)-3-(5-Hydroxy-1H-indol-3-yl)-2-(4-methylphenylsulfonamido)-N-(4-morpholinophenyl)propanamide

¹H-NMR (400 MHz, OMSO-*d₆*) *δ* 10.45 (d, *J* = 2.00 Hz, 1H), 9.63 (s, 1H), 8.54 (s, 1H), 8.02 (s, 1H), 7.53 (d, *J* = 8.00 Hz, 2H), 7.18 (d, *J* = 9.20 Hz, 2H), 7.11 (d, *J* = 8.20 Hz, 2H), 7.07 (d, *J* = 8.40 Hz, 1H), 6.97 (d, *J* = 2.40 Hz, 1H), 6.81 (d, *J* = 8.00 Hz, 2H), 6.77 (d, *J* = 2.00 Hz, 1H), 6.57 (dd, *J* = 2.00, 8.40 Hz, 1H), 4.09 - 4.06 (s, 1H), 3.72 (t, *J* = 4.80 Hz, 4H), 3.01 (t, *J* = 5.00 Hz, 4H), 2.96 - 2.91 (m, 1H), 2.76 - 2.70 (m, 1H), 2.23 (s, 3H)

### (S)-N-(3-(1H-indol-3-yl)-1-morpholino-1-oxopropan-2-yl)-4-methylbenzenesulfonamide

¹H-NMR (400 MHz, CDCl₃) *δ* 8.19 (brs, 1H), 7.67 - 7.71 (m, 2H), 7.48 (d, *J* = 7.83 Hz, 1H), 7.34 (d, *J* = 8.22 Hz, 1H), 7.25 (d, *J* = 7.83 Hz, 2H), 7.18 (dt, *J* = 1.17, 7.63 Hz, 1H), 7.07 - 7.12 (m, 1H), 7.06 (d, *J* = 1.96 Hz, 1H), 5.92 (d, *J* = 9.78 Hz, 1H), 4.42 (dt, *J* = 5.28, 9.88 Hz, 1H), 3.19 - 3.34 (m, 4H), 3.08 - 3.15 (m, 1H), 2.86 - 2.98 (m, 2H), 2.75 (m, 1H), 2.62 - 2.70 (m, 1H), 2.50 - 2.56 (m, 1H), 2.39 (s, 3H), 2.19 (m, 1H).

### (S)-3-(1H-indol-3-yl)-2-(4-methylphenylsulfonamido)-N-(5-morpholinothiazol-2-yl)propan amide

¹H-NMR (400 MHz, CDCl₃) *δ* 8.20 - 8.24 (m, 1H), 7.54 (d, *J* = 8.22 Hz, 2H), 7.20 (d, *J* = 8.22 Hz, 2H), 7.01 - 7.10 (m, 3H), 6.91 (t, *J* = 7.43 Hz, 1H), 6.86 (d, *J* = 2.35 Hz, 1H), 6.48 (s, 1H), 6.17 - 6.23 (m, 1H), 4.27 (d, *J* = 6.65 Hz, 1H), 3.78 (t, *J* = 4.70 Hz, 4H), 3.16 (d, *J* = 6.65 Hz, 2H), 2.87 - 2.99 (m, 4H), 2.25 (s, 3H)

### (S)-2-(2,4-Dimethylphenylsulfonamido)-3-(1H-indol-3-yl)-N-(4-morpholinophenyl)propanamide

¹H-NMR (400 MHz, DMSO-*d₆*) *δ* 10.75 (s, 1H), 9.58 (s, 1H), 7.98 (s, 1H), 7.54 (d, *J* = 8.00 Hz, 1H), 7.38 (d, *J* = 8.00 Hz, 1H), 7.28 (d, *J* = 8.40 Hz, 1H), 7.19 (d, *J* = 9.20 Hz, 2H), 7.08 (d, *J* = 2.40 Hz, 1H), 7.02 (t, *J* = 7.40 Hz, 1H), 6.90 - 6.81 (m, 5H), 4.05 - 4.03 (m, 1H), 3.72 (t, *J* = 4.60 Hz, 4H), 3.04 - 3.00 (m, 5H), 2.90 - 2.85 (m, 1H), 2.39 (s, 3H), 2.15 (s, 3H)

### 3-(3-Ethoxy-2-(4-methylphenylsulfonamido)-3-oxopropyl)-1H-indole-5-carboxylic acid

¹H-NMR (400 MHz, CDCl₃) *δ* 12.37 (s, 1H), 11.17 (s, 1H), 8.37 (d, *J* = 8.40 Hz, 1H), 8.01 (s, 1H), 7.68 (dd, *J* = 1.40, 8.60 Hz, 1H), 7.48 (d, *J* = 8.00 Hz, 1H), 7.35 (d, *J* = 8.40 Hz, 1H), 7.20 - 7.08 (m, 3H), 3.96 - 3.90 (m, 1H), 3.08 - 3.06 (m, 1H), 2.94 - 2.88 (m, 1H), 2.31 (s, 1H), 0.86 (t, *J* = 6.60 Hz, 3H)

### (S)-2-(4-Methylphenylsulfonamido)-N-(4-morpholinophenyl)-3-(1-tosyl-1H-imidazol-4-yl)propanamide

¹H-NMR (400 MHz, CDCl₃) *δ* 8.62 (s, 1H), 7.84 (s, 1H), 7.71 (t, *J* = 8.40 Hz, 4H), 7.29 - 7.23 (m, 6H), 6.98 (s, 1H), 6.89 (d, *J* = 6.40 Hz, 1H), 6.83 (dt, *J* = 8.80 Hz, 2H), 3.99 - 3.97 (m, 1H), 3.85 (t, *J* = 4.80 Hz, 4H), 3.10 (t, *J* = 4.80 Hz, 4H), 3.01 (dd, *J* = 5.60, 15.2 Hz, 1H), 2.61 (dd, *J* = 4.80, 14.8 Hz, 1H), 2.40 (d, *J* = 8.40 Hz, 6H)

### (S)-N-(6-((25,6R)-2,6-dimethylmorpholino)pyridin-3-yl)-3-(1H-indol-3-yl)-2-(4-methylphenylsulfonamido)propanamide

¹H-NMR (400 MHz, CDCl₃) *δ* 8.14 (brs, 1H), 8.00 (d, *J* = 2.35 Hz, 1H), 7.86 (s, 1H), 7.62 (dd, *J* = 2.74, 9.00 Hz, 1H), 7.49 - 7.53 (m, 2H), 7.33 (dd, *J* = 3.91, 7.83 Hz, 2H), 7.20 (t, *J* = 7.63 Hz, 1H), 7.08 - 7.12 (m, 2H), 7.02 (t, *J* = 7.63 Hz, 1H), 6.96 (d, *J* = 1.96 Hz, 1H), 6.57 (d, *J* = 9.00 Hz, 1H), 5.11 (d, *J* = 6.26 Hz, 1H), 4.05 (q, *J* = 6.52 Hz, 1H), 3.97 (d, *J* = 11.35 Hz, 2H), 3.67 - 3.77 (m, 2H), 3.12 - 3.28 (m, 2H), 2.45 - 2.53 (m, 2H), 2.36 (s, 3H), 1.27 (d, *J* = 6.26 Hz, 6H)

### (R)-3-(1H-indol-3-yl)-2-(4-methylphenylsulfonamido)-N-(4-morpholinophenyl)propanamide

¹H-NMR (400 MHz, CDCl₃) *δ* 8.80 (brs, 1H), 8.27 (s, 1H), 7.97 (s, 2H), 7.45 - 7.48 (m, 2H), 7.32 (d, *J* = 7.83 Hz, 1H), 7.29 (d, *J* = 3.52 Hz, 1H), 7.22 (s, 1H), 7.13 (t, *J* = 7.43 Hz, 1H), 7.00 (d, *J* = 8.22 Hz, 2H), 6.94 (s, 1H), 6.78 - 6.81 (m, *J* = 9.00 Hz, 2H), 5.57 (d, *J* = 6.65 Hz, 1H), 4.05 (d, *J* = 6.65 Hz, 1H), 3.80 - 3.86 (m, 4H), 3.19 (dd, *J* = 6.46, 13.50 Hz, 2H), 3.05 - 3.10 (m, 4H), 2.28 (s, 3H)

### (S)-N-(3-fluoro-4-morpholinophenyl)-3-(1H-indol-3-yl)-2-(4-methylphenylsulfonamido)propanamide

¹H-NMR (400 MHz, OMSO-*d₆*) *δ* 10.78 (s, 1H), 9.90 (s, 1H), 8.16 (brs, 1H), 7.51 (d, *J* = 8.22 Hz, 2H), 7.40 (d, *J* = 7.83 Hz, 1H), 7.29 (d, *J* = 7.83 Hz, 1H), 7.22 (dd, *J* = 2.15, 15.1 Hz, 1H), 7.09 (d, *J* = 8.61 Hz, 3H), 7.04 (t, *J* = 7.63 Hz, 1H), 6.89 - 6.99 (m, 3H), 4.06 (brs, 1H), 3.70 - 3.74 (m, 4H), 3.04 (dd, *J* = 6.85, 14.3 Hz, 1H), 2.90 - 2.94 (m, 4H), 2.85 (dd, *J* = 7.40, 14.4 Hz, 1H), 2.21 (s, 3H)

### (S)-3-(1H-indol-3-yl)-N-(4-morpholinophenyl)-2-(4-nitrophenylsulfonamido)propanamide

¹H-NMR (400 MHz, OMSO-*d₆*) *δ* 10.71 (brs, 1H), 9.90 (s, 1H), 8.66 (brs, 1H), 7.84 - 7.90 (m, 2H), 7.56 - 7.61 (m, 2H), 7.48 (d, *J* = 7.83 Hz, 1H), 7.29 - 7.35 (m, *J=* 9.00 Hz, 2H), 7.12 (d, *J* = 7.83 Hz, 1H), 7.05 - 7.08 (m, 1H), 6.87 - 6.96 (m, 2H), 6.81 - 6.86 (m, 2H), 4.13 (dd, *J* = 5.28, 9.19 Hz, 1H), 3.70 - 3.75 (m, 4H), 2.98 - 3.05 (m, 5H), 2.88 (dd, *J* = 9.78, 14.5 Hz, 1H)

### (S)-2-(4-Bromophenylsulfonamido)-3-(1H-indol-3-yl)-N-(4-morpholinophenyl)propanamide

¹H-NMR (400 MHz, DMSO-*d₆*) *δ* 10.77 (brs, 1H), 9.77 (s, 1H), 8.35 (brs, 1H), 7.40 - 7.48 (m, 5H), 7.28 (d, *J* = 8.22 Hz, 1H), 7.19 - 7.24 (m, 2H), 7.07 - 7.11 (m, 1H), 7.04 (t, *J* = 7.43 Hz, 1H), 6.91 - 6.96 (m, 1H), 6.81 - 6.86 (m, 2H), 4.10 (t, *J* = 7.24 Hz, 1H), 3.69 - 3.75 (m, 4H), 3.00 - 3.08 (m, 5H), 2.86 (dd, *J* = 8.41, 14.28 Hz, 1H)

### (S)-3-(1H-indol-3-yl)-2-(4-methoxyphenylsulfonamido)-N-(4-morpholinophenyl)propanamide

¹H-NMR (400 MHz, OMSO-*d₆*) *δ* 10.78 (brs, 1H), 9.67 (s, 1H), 7.99 (brs, 1H), 7.54 (d, *J* = 8.61 Hz, 2H), 7.41 (d, *J* = 7.83 Hz, 1H), 7.28 (d, *J* = 8.22 Hz, 1H), 7.22 (d, *J* = 9.00 Hz, 2H), 7.07 - 7.10 (m, 1H), 7.03 (t, *J* = 7.63 Hz, 1H), 6.90 - 6.95 (m, 1H), 6.81 (t, *J* = 9.59 Hz, 4H), 4.06 (brs, 1H), 3.70 - 3.74 (m, 4H), 3.69 (s, 3H), 2.98 - 3.07 (m, 5H), 2.80 - 2.90 (m, 1H)

### (S)-3-(1H-indol-3-yl)-2-(4-isopropylphenylsulfonamido)-N-(4 morpholinophenyl)propanamide

¹H-NMR (400 MHz, CDCl₃) *δ* 8.09 (brs, 1H), 8.03 (s, 1H), 7.81 (s, 1H), 7.58 - 7.62 (m, 2H), 7.33 - 7.38 (m, 2H), 7.18 - 7.25 (m, 5H), 7.05 (t, *J* = 7.43 Hz, 1H), 6.97 (d, *J* = 2.35 Hz, 1H), 6.80 - 6.85 (m, *J* = 9.00 Hz, 2H), 5.11 (d, *J* = 6.26 Hz, 1H), 4.08 (d, *J* = 6.26 Hz, 1H), 3.83 - 3.89 (m, 4H), 3.34 (dd, *J* = 6.06, 14.28 Hz, 1H), 3.09 - 3.16 (m, 5H), 1.25 (dd, *J* = 2.35, 7.04 Hz, 6H)

### (S)-3-(1H-indol-3-yl)-2-(4-isopropylphenylsulfonamido)-N-(4-(4-methoxyphenyl)thiazol-2-yl)propanamide

¹H-NMR (400 MHz, CDCl₃) *δ*7.74 (d, *J* = 8.40 Hz, 2H), 7.67 (d, *J* = 7.60 Hz, 2H), 7.21 (t, *J* = 6.80 Hz, 4H), 7.04 (s, 1H), 3.98 - 3.95 (m, 1H), 2.37 (s, 1H), 2.26 (m, 3H), 1.64 - 1.49 (m, 3H), 0.85 (d, *J* = 6.40 Hz, 3H), 0.72 (d, *J* = 6.40 Hz, 3H)

### (S)-3-(1H-indol-3-yl)-2-(4-methylphenylsulfonamido)-N-(4-methylthiazol-2-yl)propanamide

¹ H-NMR (500 MHz, DMSO-*d₆*) *δ* 12.13 (s, 1H), 10.83 (s, 1H), 8.26 - 8.24 (d, *J* = 8.50 Hz, 1H), 7.45 - 7.44 (d, *J* = 8.00 Hz, 2H), 7.39 - 7.38 (d, J= 7.50 Hz, 1H), 7.29 - 7.27 (d, *J* = 8.00 Hz, 1H), 7.06 - 7.02 (m, 4H), 6.91 - 6.89 (m, 1H), 6.72 (s, 1H), 4.27 - 4.22 (m, 1H), 3.07 - 3.03 (dd, *J* = 14.2, 7.50 Hz, 1H), 2.86 - 2.81 (dd, *J* = 14.2, 8.00 Hz, 1H), 2.24 (s, 3H), 2.22 (s, 3H)

### (S)-N-(benzo[d]thiazol-2-yl)-3-(1H-indol-3-yl)-2-(4-methylphenylsulfonamido)propanamide

¹H-NMR (500 MHz, DMSO-*d₆*) *δ* 12.4 (s, 1H), 10.8 (s, 1H), 8.32 (m, 1H), 7.91 (m, 1H), 7.72 (m, 1H), 7.47 - 7.44 (m, 6H), 7.03 - 6.91 (m, 5H), 4.34 (brs, 1H), 3.11 (m, 1H), 2.91 (m, 1H), 2.08 (s, 3H)

### (S)-2-(4-Bromophenylsulfonamido)-3-(1H-indol-3-yl)propanoic acid

¹H-NMR (500 MHz, DMSO-*d₆*) *δ* 12.73 (brs, 1H), 10.80 (s, 1H), 8.41 - 8.39 (d, *J* = 8.50 Hz, 1H), 7.51 - 7.49 (d, *J* = 8.50 Hz, 2H), 7.42 - 7.40 (d, *J* = 8.00 Hz, 2H), 7.32 - 7.29 (t, *J* = 8.00 Hz, 2H), 7.08 - 7.03 (m, 2H), 6.94 - 6.91 (t, *J* = 7.50 Hz, 1H), 3.94 - 3.89 (m, 1H), 3.10 - 3.08 (dd, *J* = 14.5, 6.00 Hz, 1H), 2.89 - 2.84 (dd, *J* = 14.0, 8.50 Hz, 1H)

### (S)-4-Methyl-2-(4-methylphenylsulfonamido)-N-(5-morpholinothiazol-2-yl)pentanamide

¹H-NMR (400 MHz, CDCl₃) *δ* 7.71 (d, *J* = 8.00 Hz, 2H), 7.16 (d, *J* = 8.00 Hz, 2H), 6.95 (s, 1H), 6.35 (d, *J* = 10.0 Hz, 1H), 4.14 - 4.06 (m, 1H), 3.86 (t, *J* = 4.80 Hz, 4H), 3.13 - 3.07 (m, 4H), 2.29 (s, 3H), 1.75 - 1.70(m, 1H), 1.60 - 1.53 (m, 1H), 1.46 - 1.41 (m, 1H), 0.81 (d, *J* = 6.80 Hz, 6H)

### (S)-N-(5-(4-methoxyphenyl)-1,3,4-thiadiazol-2-yl)-4-methyl-2-(4-methylphenylsulfonamido)pentanamide

¹H-NMR (400 MHz, CDCl₃) *δ* 13.52 (s, 1H), 8.14 (d, *J* = 8.80 Hz, 1H), 7.92 - 7.88 (m, 2H), 7.68 (d, *J* = 8.40 Hz, 2H), 7.18 (d, *J* = 8.40 Hz, 2H), 7.07 - 7.04 (m, 2H), 4.36 - 4.29 (m, 1H), 3.91 (s, 3H), 2.21 (s, 3H), 1.84 - 1.79 (m, 1H), 1.72 - 1.64 (m, 1H), 1.59 - 1.54 (m, 1H), 0.89 (d, *J* = 6.40 Hz, 3H), 0.86 (d, *J* = 6.80 Hz, 1H)

### (S)-N-(5-(4-methoxyphenyl)isoxazol-3-yl)-4-methyl-2-(4-methylphenylsulfonamido)pentanamide

¹H-NMR (400 MHz, CDCl₃) *δ* 10.55 (s, 1H), 7.80 - 7.76 (m, 4H), 7.19 - 7.15 (m, 3H), 6.99 (d, *J =* 9.20 Hz, 2H), 6.43 (d, *J =* 9.20 Hz, 1H), 4.22 - 4.16 (m, 1H), 3.86 (s, 3H), 2.21 (s, 3H), 1.88 - 1.83 (m, 1H), 1.72 - 1.59 (m, 2H), 0.91 (d, *J* = 6.80 Hz, 3H), 0.88 (d, *J* = 6.40 Hz, 3H).

### (S)-N-(3-(4-methoxyphenyl)isoxazol-5-yl)-4-methyl-2-(4-methylphenylsulfonamido)pentanamide

¹H-NMR (400 MHz, CDCl₃) *δ* 9.22 (s, 1H), 7.78 (d, *J* = 8.40 Hz, 2H), 7.74 - 7.71 (m, 2H), 7.30 (d, *J* = 8.00 Hz, 2H), 6.98 - 6.94 (m, 2H), 6.56 (s, 1H), 5.40 (d, *J* = 7.20 Hz, 1H), 3.97 - 3.92 (m, 1H), 3.85 (s, 3H), 2.35 (s, 3H), 1.64 - 1.47 (m, 3H), 0.83 (d, *J* = 6.40 Hz, 3H), 0.66 (d, *J* = 5.60 Hz, 1H)

### (S)-4-Methyl-2-(4-methylphenylsulfonamido)-N-(4-(4-(trifluoromethyl)phenyl)thiazol-2-yl)pentanamide

¹H-NMR (400 MHz, CDCl₃) *δ* 9.69 (s, 1H), 7.91 (d, *J* = 8.40 Hz, 2H), 7.79 (d, *J* = 8.40 Hz, 2H), 7.65 (d, *J =* 8.40 Hz, 2H), 7.29 - 7.26 (m, 2H), 7.23 (s, 1H), 5.26 (d, *J* = 6.80 Hz, 1H), 3.97 - 3.92 (m, 1H), 2.33 (s, 3H), 1.67 - 1.45 (m, 3H), 0.83 (d, *J* = 6.40 Hz, 3H), 0.65 (d, *J* = 6.40 Hz, 3H)

### (S)-N-(4-(3,4-dimethoxyphenyl)thiazol-2-yl)-4-methyl-2-(4-methylphenylsulfonamido)pentanamide

¹H-NMR (400 MHz, CDCl₃) *δ* 9.74 (s, 1H), 7.76 (d, *J =* 7.60 Hz, 2H), 7.38 - 7.34 (m, 2H), 7.26 (d, *J* = 6.80 Hz, 2H), 7.01 (s, 1H), 6.90 (d, *J* = 8.00 Hz, 1H), 5.40 (d, *J* = 6.80 Hz, 1H), 4.19 - 4.10 (m, 1H), 3.95 (s, 3H), 3.90 (s, 3H), 2.32 (s, 3H), 1.60 - 1.42 (m, 3H), 0.80 (d, *J* = 6.00 Hz, 3H), 0.63 (d, *J =* 5.60 Hz, 3H)

### (S)-N-(4-(2-bromo-4-fluorophenyl)thiazol-2-yl)-4-methyl-2-(4-methylphenylsulfonamido)pentanamide

¹H-NMR (400 MHz, CDCl₃) *δ* 9.62 (s, 1H), 7.78 - 7.75 (m, 4H), 7.26 (s, 1H), 7.10 - 7.05 (m, 3H), 5.31 (d, *J =* 7.60 Hz, 1H), 3.95 - 3.92 (m, 1H), 2.32 (s, 3H), 1.64 - 1.46 (m, 3H), 0.82 (d, *J* = 6.40 Hz, 3H), 0.66 (d, *J =* 6.00 Hz, 3H).

### (S)-4-(2-Aminothiazol-4-yl)phenyl 4-methyl-2-(4-methylphenylsulfonamido)pentanoate

¹H-NMR (400 MHz, CDCl₃) *δ* 7.79 (d, *J =* 7.60 Hz, 2H), 7.67 (d, *J* = 7.60 Hz, 2H), 7.29 (d, *J* = 7.20 Hz, 2H), 6.68 (d, *J* = 7.60 Hz, 2H), 6.65 (s, 1H), 5.53 (d, *J =* 9.20 Hz, 1H), 5.27 (s, 2H), 4.16 - 4.11 (m, 1H), 2.41 (m, 3H), 1.90 - 1.87 (m, 1H), 1.63 - 1.60 (m, 1H), 1.27 - 1.24 (m, 1H), 0.94 (s, 6H)

### (S)-N-(4-(4-fluorophenyl)thiazol-2-yl)-4-methyl-2-(4-methylphenylsulfonamido)pentanamide

¹H-NMR (400 MHz, CDCl₃) *δ* 9.58 (s, 1H), 7.79 - 7.76 (m, 3H), 7.28 - 7.26 (m, 2H), 7.11 - 7.06 (m, 2H), 5.20 (d, *J =* 6.80 Hz, 2H), 5.27 (s, 2H), 3.93 - 3.91 (m, 1H), 2.41 (m, 3H), 1.66 - 1.46 (m, 3H), 0.83 (d, *J* = 6.40 Hz, 3H), 0.65 (d, *J* = 6.40 Hz, 3H)

### (S)-4-Methyl-2-(4-methylphenylsulfonamido)-N-(4-(4-(trifluoromethoxy)phenyl)thiazol-2-yl)pentanamide

¹H-NMR (400 MHz, CDCl₃) *δ* 9.60 (s, 1H), 7.83 (d, *J* = 8.80 Hz, 2H), 7.79 (d, *J* = 8.00 Hz, 2H), 7.29 - 7.23 (m, 4H), 7.13 (s, 1H), 5.21 (d, *J* = 6.80 Hz, 1H), 3.93 (s, 1H), 2.33 (m, 3H), 1.65 - 1.48 (m, 3H), 0.83 (d, *J* = 6.40 Hz, 3H), 0.64 (d, *J* = 6.00 Hz, 3H)

### (S)-N-(4-(4-hydroxyphenyl)thiazol-2-yl)-4-methyl-2-(4-methylphenylsulfonamido)pentanamide

¹H-NMR (400 MHz, CDCl₃) *δ* 7.72 (d, *J* = 8.00 Hz, 2H), 7.64 (d, *J =* 8.80 Hz, 2H), 7.20 (d, *J =* 8.00 Hz, 2H), 6.93 (s, 1H), 6.88 (d, *J =* 8.40 Hz, 2H), 3.97 - 3.93 (m, 1H), 2.25 (s, 3H), 1.65 - 1.51 (m, 3H), 0.88 (d, *J* = 6.40 Hz, 3H), 0.75 (d, *J* = 6.00 Hz, 3H)

### (S)-4-Methyl-2-(4-methylphenylsulfonamido)-N-(4-p-tolylthiazol-2-yl)pentanamide

¹H-NMR (400 MHz, CDCl₃) *δ* 7.74 (d, *J =* 8.40 Hz, 2H), 7.67 (d, *J =* 7.60 Hz, 2H), 7.21 (t, *J* = 6.80 Hz, 4H), 7.04 (s, 1H), 3.98 - 3.95 (m, 1H), 2.37 (s, 1H), 2.26 (m, 3H), 1.64 - 1.49 (m, 3H), 0.85 (d, *J* = 6.40 Hz, 3H), 0.72 (d, *J* = 6.40 Hz, 3H)

### (S)-N-(4-(4-((S)-1-methoxypropan-2-yloxy)phenyl)thiazol-2-yl)-4-methyl-2-(4-methylphenylsulfonamido)pentanamide

¹H-NMR (400 MHz, CDCl₃) *δ* 9.97 (s, 1H), 7.74 (d, *J* = 8.40 Hz, 2H), 7.69 (d, *J* = 8.80 Hz, 2H), 7.23 (d, *J* = 8.00 Hz, 2H), 6.97 (s, 1H), 6.95 (d, *J* = 9.20 Hz, 2H), 5.71 (d, *J* = 7.60 Hz, 1H), 4.58 - 4.54 (m, 1H), 4.00 - 3.94 (m, 1H), 3.60 - 3.46 (m, 2H), 3.40 (s, 3H), 2.28 (s, 3H), 1.56 - 1.40 (m, 3H), 1.31 (d, *J =* 6.00 Hz, 3H), 0.78 (d, *J =* 6.00 Hz, 3H), 0.66 (d, *J* = 5.60 Hz, 3H)

### (S)-4-Methyl-2-(4-methylphenylsulfonamido)-N-(4-(4-morpholinophenyl)thiazol-2-yl)pentanamide

¹H-NMR (400 MHz, CDCl₃) *δ* 8.28 (dd, *J* = 9.00, 11.4 Hz, 4H), 7.19 (d, *J* = 8.40 Hz, 2H), 6.95 (t, *J* = 4.60 Hz, 3H), 4.13 - 3.95 (m, 1H), 3.89 (t, *J* = 4.80 Hz, 3H), 3.21 (t, *J* = 5.00 Hz, 3H), 2.25 (s, 3H), 1.67 - 1.64 (m, 1H), 1.55 - 1.51 (m, 2H), 0.88 (d, *J* = 6.80 Hz, 3H), 0.75 (d, *J =* 6.40 Hz, 3H)

### (S)-Methyl 4-(2-(4-methyl-2-(4-methylphenylsulfonamido)pentanamido)thiazol-4-yl)benzoate

¹H-NMR (400 MHz, CDCl₃) *δ* 10.05 (s, 1H), 8.18 (d, *J =* 8.00 Hz, 2H), 7.93 (d, *J* = 8.40 Hz, 2H), 7.76 (d, *J* = 8.00 Hz, 2H), 7.26 (s, 1H), 7.20 (d, *J* = 8.00 Hz, 2H), 5.63 (d, *J* = 8.00 Hz, 1H), 4.12 - 4.09 (m, 1H), 3.99 (s, 3H), 2.24 (s, 3H), 1.65 - 1.52 (m, 1H), 0.86 (d, *J* = 5.60 Hz, 3H), 0.74 (d, *J* = 5.60 Hz, 3H)

### (S)-4-(2-(4-Methylphenylsulfonamido)-3-(4-morpholinophenylamino)-3-oxopropyl)phenyl 4-methylbenzenesulfonate

¹H-NMR (400 MHz, DMSO-*d₆*) *δ* 9.62 (s, 1H), 9.18 (s, 1H), 8.05 (s, 1H), 7.49 (d, *J* = 8.00 Hz, 2H), 7.15 (d, *J* = 8.40 Hz, 4H), 6.93 (d, *J* = 8.40 Hz, 2H), 6.83 (d, *J* = 9.20 Hz, 2H), 6.58 (d, *J* = 8.00 Hz, 2H), 3.95 (m, 1H), 3.72 (t, *J* = 4.60 Hz, 4H), 3.02 (t, *J* = 4.60 Hz, 4H), 2.78 - 2.58 (m, 2H), 2.25 (s, 3H)

### (S)-2-(4-Bromophenylsulfonamido)-3-(1H-imidazol-4-yl)-N-(4-(4-methoxyphenyl)thiazol-2-yl)propanamide

¹H-NMR (500 MHz, OMSO-*d₆*) 7.80-7.71 (d, *J* = 9.00 Hz, 2H), 7.59 (s, 4H), 7.45 - 7.41 (d, *J* = 15.0 Hz 2H), 6.98 - 6.96 (d, *J* = 11.5 Hz, 2H), 6.75 (brs, 1H), 4.31 - 4.28 (t, *J* = 7.50 Hz, 1H), 3.77 (s, 3H), 2.92 - 2.90 (dd, *J₁* = 14.0, 7.00 Hz, 1H), 2.77 - 2.73 (dd, *J* = 14.0, 7.50 Hz, 1H)

### (S)-N-methoxy-N,4-dimethyl-2-(4-methylphenylsulfonamido)pentanamide

¹H-NMR (400 MHz, CDCl₃) *δ* 7.72 (d, *J* = 8.00 Hz, 2H), 7.27 (d, *J* = 8.40 Hz, 2H), 5.40 (d, *J* = 10.4 Hz, 1H), 4.28 (t, *J* = 10.4 Hz, 1H), 3.56 (s, 3H), 2.92 (s, 3H), 2.40 (m, 3H), 1.92 - 1.89 (m, 1H), 1.45 - 1.39 (m, 3H), 1.31 - 1.24 (m, 1H), 0.91 (d, *J* = 6.40 Hz, 6H)

### (S)-3-(1H-indol-3-yl)-N-methoxy-N-methyl-2-(4-methylphenylsulfonamido)propanamide

¹H-NMR (400 MHz, CDCl₃) *δ* 8.00 (s, 1H), 7.54 (d, *J* = 8.40 Hz, 2H), 7.44 (d, *J* = 7.60 Hz, 1H), 7.31 (d, *J* = 8.00 Hz, 1H), 7.16 (t, *J* = 7.40 Hz, 1H), 7.11 - 7.03 (m, 4H), 5.46 (d, *J* = 9.60 Hz, 1H), 4.61 - 4.55 (m, 1H), 3.45 (s, 3H), 3.17 - 3.12 (m, 1H), 3.05 - 3.00 (m, 1H), 2.95 (s, 3H), 2.34 (m, 3H)

### (S)-N-(3-(1H-indol-3-yl)-1-oxo-1-(1,4,7-trioxa-10-azacyclododecan-10-yl)propan-2-yl)-4-methylbenzenesulfonamide

¹H-NMR (400 MHz, CDCl₃) *δ* 8.25 (s, 1H), 7.60 (d, *J* = 8.40 Hz, 2H), 7.47 (d, *J* = 8.00 Hz, 1H), 7.29 - 7.26 (m, 1H), 7.14 - 7.02 (m, 5H), 5.86 (d, *J* = 9.60 Hz, 1H), 4.66 - 4.60 (m, 1H), 3.67 - 3.63 (m, 1H), 3.58 - 3.36 (m, 13H), 3.25 - 2.97 (m, 4H)

### (S)-2-(4-tert-Butylphenyisulfonamido)-3-(1H-indol-3-yl)-N-(4-morpholinophenyl)propan amide

¹H-NMR (400 MHz, CD₃OD) *δ* 7.94 (s, 1H), 7.58 - 7.62 (m, 2H), 7.39 (d, *J* = 7.83 Hz, 1H), 7.30 - 7.34 (m, 2H), 7.25 (d, *J* = 7.83 Hz, 1H), 6.99 - 7.04 (m, 4H), 6.90 (s, 1H), 6.77 (d, *J* = 9.00 Hz, 2H), 4.06 (s, 1H), 3.75 - 3.79 (m, 4H), 3.12 - 3.20 (m, 1H), 2.98 - 3.05 (m, 5H), 1.18 (s, 9H)

### (S)-2-(4-Bromophenylsulfonamido)-N-(4-(2-chlorophenyl)thiazol-2-yl)-3-(1H-indol-3-yl)propanamide

¹H-NMR (500 MHz, OMSO-*d₆*) *δ* 2.89 - 2.93 (dd, *J* = 14.67, 8.80 Hz, 1H), 3.10 - 3.14 (dd, *J* = 14.18, 6.35 Hz, 1H), 4.34 (brs, 1H), 6.91 - 6.94 (t, *J* = 7.33 Hz, 1H), 7.04 - 7.07 (t, *J* = 7.82 Hz, 1H), 7.11 (s, 1H), 7.28 - 7.30 (d, *J* = 8.31 Hz, 1H), 7.37 - 7.45 (m, 6H), 7.47 - 7.49 (d, *J* = 7.82 Hz, 1H), 7.55 - 7.57 (d, *J* = 7.82 Hz, 1H), 7.60 (s, 1H), 7.81 - 7.83 (m, 1H), 8.55 (brs, 1H), 10.80 (brs, 1H), 12.53 (brs, 1H)

### (S)-2-(4-Bromophenylsulfonamido)-N-(4-(2,4-dimethoxyphenyl)thiazol-2-yl)-3-(1H-indol-3-yl)propanamide

¹H-NMR (500 MHz, DMSO-*d₆*) *δ* 2.85 - 2.90 (dd, *J* = 14.18, 8.80 Hz, 1H), 3.08 - 3.12 (dd, *J* = 14.67, 6.35 Hz, 1H), 3.80 (s, 3H), 3.90 (s, 3H), 4.32 - 4.34 (d, *J* = 6.35 Hz, 1H), 6.61 - 6.63 (d, *J* = 8.80 Hz, 1H), 6.66 (s, 1H), 6.90 - 6.93 (t, *J* = 7.33 Hz, 1H), 7.03 - 7.06 (t, *J* = 7.33 Hz, 1H), 7.08 (s, 1H), 7.26 - 7.28 (d, *J* = 7.82 Hz, 1H), 7.36 - 7.40 (m, 4H), 7.47 - 7.48 (m, 2H), 7.96 - 7.98 (d, *J* = 8.80 Hz, 1H), 8.51 - 8.52 (d, *J* = 6.84 Hz, 1H), 10.78 (s, 1H), 12.39 (s, 1H)

### (S)-2-(4-Bromophenylsulfonamido)-3-(1H-indol-3-yl)-N-(thiazol-2-yl)propanamide

¹H-NM R (500 MHz, DMSO-*d₆*) *δ* 12.34 (s, 1H), 10.80 (s, 1H), 8.53 (s, 1H), 7.48 - 7.45 (m, 2H), 7.40 - 7.36 (m, 4H), 7.30 - 7.27 (m, 1H), 7.23 - 7.20 (m, 1H), 7.10 - 7.02 (m, 2H), 6.95 - 6.90 (m, 1H), 4.33 (brs, 1H), 3.12 - 3.07 (m, 1H), 2.92 - 2.86 (m, 1H)

### (S)-2-(4-Bromophenylsulfonamido)-3-(1H-indol-3-yl)-N-(5-methylthiazol-2-yl)propanamide

¹H-NMR (500 MHz, DMSO-*d₆*) *δ* 12.10 (s, 1H), 10.80 (s, 1H), 8.50 - 8.45 (d, *J* = 8.00 Hz, 1H), 7.46 - 7.45 (d, *J* = 7.50 Hz, 1H), 7.41 - 7.36 (m, 4H), 7.29 - 7.27 (d, *J* = 8.00 Hz, 1H), 7.11 - 7.03 (m, 3H), 6.93 - 6.90 (t, *J* = 7.00 Hz, 1H), 4.32 - 4.27 (m, 1H), 3.08 - 3.04 (dd, *J* = 14.20, 6.50 Hz, 1H), 2.89 - 2.85 (dd, *J* = 14.20, 8.50 Hz, 1H), 2.52 (s, 3H)

### (S)-3-(1H-indol-3-yl)-2-(4-methylphenylsulfonamido)-N-(4-methylthiazol-2-yl)propanamide

¹H-NMR (500 MHz, DMSO-*d₆*) *δ* 12.2 (s, 1H), 10.8 (s, 1H), 8.53 - 8.52 (d, *J* = 8.50 Hz, 1H), 7.45 - 7.44 (d, *J* = 8.00 Hz, 1H), 7.39 (m, 4H), 7.28 - 7.27 (d, *J* = 8.00 Hz, 1H), 7.07 - 7.02 (m, 2H), 6.92 - 6.90 (t, *J* = 7.00 Hz, 1H), 6.75 (s, 1H), 4.29 - 4.25 (m, 1H), 3.09 - 3.05 (dd, *J* = 14.2, 6.00 Hz, 1H), 2.89 - 2.85 (dd, *J* = 14.7, 8.00 Hz, 1H), 2.52 (s, 3H)

### (S)-3-(1H-indol-3-yl)-2-(4-methylphenylsulfonamido)-N-(5-methylthiazol-2-yl)propanamide

¹H-NMR (500 MHz, OMSO-*d₆*) *δ* 12.01 (s, 1H), 10.79 (s, 1H), 8.25 (d, *J* = 8.00 Hz, 1H), 7.46 - 7.44 (m, 2H), 7.40 - 7.39 (d, *J* = 7.50 Hz, 1H), 7.29 - 7.27 (d, *J* = 8.00 Hz, 1H), 7.10 - 7.02 (m, 5H), 6.93 - 6.90 (m, 1H), 4.28 - 4.26 (m, 1H), 3.04 - 3.03 (dd, *J* = 14.2, 7.00 Hz, 1H), 2.86 - 2.84 (dd, *J* = 14.2, 7.00 Hz, 1H), 2.32 (s, 3H), 2.22 (s, 3H)

### (S)-Methyl 4-(N-(3-(1H-indol-3-yl)-1-(4-morpholinophenylamino)-1-oxopropan-2-yl)sulfamoyl)benzoate

¹H-NMR (400 MHz, DMSO-*d₆*) *δ* 10.74 (s, 1H), 9.77 (s, 1H), 8.44 (s, 1H), 7.74 (d, *J* = 8.40 Hz, 2H), 7.62 (d, *J* = 8.00 Hz, 2H), 7.44 (d, *J =* 7.20 Hz, 1H), 7.22 (t, *J* = 8.40 Hz, 3H), 7.08 (d, *J* = 2.00 Hz, 1H), 6.99 (d, *J* = 7.40 Hz, 1H), 6.90 (t, *J* = 7.20 Hz, 1H), 6.81 (d, *J* = 9.20 Hz, 2H), 4.13 - 4.09 (m, 1H), 3.72 (t, *J* = 4.60 Hz, 4H), 3.05 - 3.00 (m, 5H), 2.89-2.83 (m, 1H)

### (S)-Ethyl 4-(N-(3-(1H-indol-3-yl)-1-(4-morpholinophenylamino)-1-oxopropan-2-yl)sulfamoyl)benzoate

¹H-NMR (400 MHz, OMSO-*d₆*) *δ* 10.74 (s, 1H), 9.76 (s, 1H), 7.73 (d, *J* = 8.80 Hz, 2H), 7.62 (d, *J* = 8.40 Hz, 2H), 7.44 (d, *J* = 7.60 Hz, 1H), 7.23 - 7.20 (m, 3H), 7.09 (d, *J* = 2.40 Hz, 1H), 7.00 - 6.81 (m, 2H), 6.81 (d, *J* = 9.20 Hz, 2H), 4.31 (d, *J* = 7.10 Hz, 2H), 4.12 - 4.09 (m, 1H), 3.72 (t, *J* = 4.60 Hz, 4H), 3.05 - 3.00 (m, 5H), 2.89 - 2.83 (m, 1H), 1.34 (t, *J* = 7.00 Hz, 3H)

### (S)-3-(4-Benzoylphenyl)-2-(4-methylphenylsulfonamido)-N-(4-morpholinophenyl)propanamide

¹H-NMR (400 MHz, OMSO-*d₆*) *δ* 9.75 (s, 1H), 7.68 (d, *J* = 7.20 Hz, 3H), 7.57 (t, *J* = 5.80 Hz, 4H), 7.47 (d, *J* = 8.00 Hz, 2H), 7.33 (d, *J* = 8.00 Hz, 2H), 7.20 (d, *J =* 8.80 Hz, 2H), 7.12 (d, *J* = 7.60 Hz, 2H), 6.85 (d, *J* = 8.80 Hz, 2H), 4.31 (d, *J* = 7.10 Hz, 2H), 4.12 - 4.09 (m, 1H), 3.72 (t, *J* = 4.40 Hz, 4H), 3.04 - 2.95 (m, 5H), 2.20 (s, 3H)

### (S)-4-(N-(3-(1H-indol-3-yl)-1-(4-morpholinophenylamino)-1-oxopropan-2-yl)sulfamoyl)-N-methylbenzamide

¹H-NMR (400 MHz, DMSO-*d₆*) *δ* 10.75 (s, 1H), 9.72 (s, 1H), 8.44 (d, *J* = 4.00 Hz, 1H), 8.22 (s, 1H), 7.75 (d, *J* = 8.40 Hz, 2H), 7.67 (d, *J* = 8.00 Hz, 2H), 7.42 (d, *J* = 7.60 Hz, 1H), 7.26 (d, *J* = 8.00 Hz, 1H), 7.18 (d, *J =* 9.20 Hz, 2H), 7.07 (d, *J* = 1.60 Hz, 1H), 7.01 (t, *J* = 7.60 Hz, 1H), 6.92 (t, *J* = 7.60 Hz, 1H), 6.79 (d, *J* = 8.80 Hz, 2H), 4.15 - 4.11 (m, 1H), 3.72 (t, *J* = 4.80 Hz, 4H), 3.08 - 2.99 (m, 5H), 2.88 - 2.82 (m, 3H), 2.78 (d, *J* = 4.40 Hz, 3H)

### (S)-2-(4-Methylphenylsulfonamido)-N-(4-morpholinophenyl)-3-(naphthalen-2-yl)propanamide

¹H-NMR (400 MHz, DMSO-*d₆*) *δ* 9.64 (s, 1H), 8.27 (s, 1H), 7.89 - 7.84 (m, 2H), 7.75 (t, *J* = 4.60 Hz, 1H), 7.50 - 7.46 (m, 4H), 7.33 (d, *J* = 4.20 Hz, 2H), 7.17 (d, *J* = 8.80 Hz, 2H), 7.09 (d, *J* = 8.40 Hz, 2H), 7.82 (d, *J* = 9.20 Hz, 2H), 4.17 - 4.14 (m, 1H), 3.72 (t, *J* = 4.60 Hz, 4H), 3.40 - 3.34 (m, 1H), 3.18 - 3.13 (m, 1H), 3.02 (t, *J* = 5.00 Hz, 4H), 2.24 (s, 3H)

### (S)-tert-Butyl 3-(1H-indot-3-yl)-1-(4-morpholinophenylamino)-1-oxopropan-2-ylcarbamate

¹H-NMR (400 MHz, OMSO-*d₆*) *δ* 10.80 (s, 1H), 9.80 (s, 1H), 7.64 (d, *J* = 8.00 Hz, 1H), 7.45 (d, *J* = 9.20 Hz, 2H), 7.32 (d, *J* = 8.40 Hz, 1H), 7.15 (s, 1H), 7.05 (t, *J* = 7.40 Hz, 1H), 6.97 (t, *J* = 7.20 Hz, 2H), 6.90 - 6.84 (m, 3H), 4.34 - 4.33 (m, 1H), 3.73 (t, *J* = 4.80 Hz, 4H), 3.13 - 2.95 (m, 6H), 1.32 (s, 9H)

### (S)-3-(1H-indol-3-yl)-2-(4-methylphenylsulfonamido)propanoic acid

¹H-NMR (400 MHz, OMSO-d₆) *δ* 10.77 (s, 1H), 8.12 (d, *J* = 8.00 Hz, 1H), 7.45 (d, *J* = 4.00 Hz, 2H), 7.25 (t, *J* = 12.00 Hz, 2H), 7.12 (d, *J* = 16.40 Hz, 2H), 6.96 - 7.01 (m, 2H), 6.88 (t, *J* = 11.80 Hz, 1H), 3.86 (m, 1H), 3.03 (m, 1H), 2.83 (m, 1H), 2.30 (s, 3H)

### (S)-tert-Butyl 4-(4-(3-(1H-indol-3-yl)-2-(4-methylphenylsulfonamido)propanamido)-3-methoxyphenyl)piperazine-1-carboxylate

¹H-NMR (400 MHz, CDCl₃) *δ* 8.42 (s, 1H), 8.36 (s, 1H), 8.11 (d, *J* = 8.80 Hz, 1H), 7.48 (d, *J* = 8.00 Hz, 2H), 7.33 (d, *J* = 8.00 Hz, 1H), 7.28 (d, *J* = 8.40 Hz, 1H), 7.14 (t, *J* = 7.60 Hz, 1H), 7.02 - 6.96 (m, 2H), 6.92 (d, *J* = 2.40 Hz, 1H), 6.45 (dd, *J* = 2.40, 9.20 Hz, 1H), 6.41 (d, *J* = 2.40 Hz, 1H), 5.25 (d, *J* = 6.40 Hz, 1H), 4.14 - 4.05 (m, 1H),3.57 (t, *J* = 5.20 Hz, 4H), 3.20 (d, *J* = 6.80 Hz, 2H), 3.05 (t, *J* = 4.80 Hz, 4H), 2.28 (s, 6H), 1.48 (s, 9H)

### (S)-3-(1H-indol-3-yl)-N-(2-methoxy-4-(piperazin-1-yl)phenyl)-2-(4-methylphenylsulfonamido)propanamide

¹H-NMR (400MHz, OMSO-*d₆*) *δ* 10.69 (s, 1H), 8.97 (s, 1H), 7.50 (d, *J* = 9.20 Hz, 1H), 7.40 (d, *J* = 7.60 Hz, 2H), 7.35 (d, *J* = 8.00 Hz, 1H), 7.26 (d, *J* = 7.60 Hz, 1H), 7.08 (d, *J* = 2.00 Hz, 1H), 7.03 - 7.00 (m, 3H), 6.90 (d, *J* = 7.40 Hz, 1H), 6.56 (d, *J* = 2.40 Hz, 1H), 6.38 (dd, *J* = 2.60, 8.60 Hz, 1H), 4.12 - 4.09 (m, 2H), 3.77 (s, 3H), 3.10 - 3.05 (m, 1H), 3.01 (t, *J* = 4.80 Hz, 4H), 2.85 - 2.79 (m, 5H), 2.24 (s, 3H)

### (S)-tert-Butyl 4-(4-(3-(1H-indol-3-yl)-2-(4-methylphenylsulfonamido)propanamido)phenyl)piperazine-1-carboxylate

¹H-NMR (400 MHz, CDCl₃) *δ* 8.28 (s, 1H), 8.02 (s, 1H), 7.46 (d, *J* = 8.40 Hz, 2H), 7.30 (d, *J =* 8.80 Hz, 2H), 7.24 (d, *J =* 7.20 Hz, 2H), 7.16 (t, *J =* 7.20 Hz, 1H), 7.04 (d, *J* = 8.00 Hz, 2H), 6.98 (t, *J* = 7.40 Hz, 1H), 6.93 (d, *J* = 2.40 Hz, 1H), 6.82 (d, *J* = 9.20 Hz, 2H), 5.19 (d, *J =* 5.60 Hz, 1H), 4.01 - 4.00 (m, 1H), 3.56 (t, *J =* 5.20 Hz, 4H), 3.18 (d, *J* = 6.00 Hz, 2H), 3.05 (t, *J* = 4.80 Hz, 4H), 2.31 (s, 3H), 1.48 (s, 9H)

### (S)-3-(1H-indol-3-yl)-N-(2-methoxy-4-morpholinophenyl)-2-(4-methylphenylsulfonamido)propanamide

¹H-NMR (400 MHz, CDCl₃) *δ* 8.37 (s, 1H), 8.13 (d, *J =* 8.80 Hz, 1H), 8.02 (s, 1H), 7.53 (d, *J* = 8.00 Hz, 2H), 7.36 (d, *J* = 8.00 Hz, 1H), 7.32 (d, *J* = 8.40 Hz, 1H), 7.18 (t, *J* = 7.60 Hz, 1H), 7.08 (d, *J* = 8.40 Hz, 2H), 7.02 (t, *J* = 7.00 Hz, 1H), 6.96 (d, *J* = 2.40 Hz, 1H), 6.47 (dd, *J* = 2.40, 8.80 Hz, 1H), 6.41 (d, *J* = 2.40 Hz, 1H), 5.08 (d, *J =* 6.40 Hz, 1H), 4.14 - 4.05 (m, 1H), 3.86 (t, *J* = 4.60 Hz, 4H), 3.73 (s, 3H), 3.29 - 3.14 (m, 2H), 3.11 (t, *J* = 4.80 Hz, 4H), 2.32 (s, 3H)

### (S)-3-(1H-indol-3-yl)-2-(4-methylphenylsulfonamido)-N-(4-(piperazin-1-yl)phenyl)propanamide

¹H-NMR (400 MHz, DMSO-*d₆*) *δ* 10.76 (s, 1H), 9.63 (s, 1H), 7.51(d, *J* = 8.40 Hz, 2H), 7.40 (d, *J* = 7.60 Hz, 1H), 7.28 (d, *J* = 8.40 Hz, 1H), 7.17 (d, *J* = 8.80 Hz, 2H), 7.10 - 7.01 (m, 4H), 6.92 (t, *J* = 7.20 Hz, 1H), 6.79 (d, *J* = 9.20 Hz, 2H), 4.08 - 4.05 (m, 2H), 3.05 - 3.00 (m, 1H), 2.95 (t, *J* = 4.80 Hz, 4H), 2.85 - 2.79 (m, 5H), 2.23 (s, 3H)

### (S)-3-(1-Formyl-1H-indol-3-yl)-2-(4-methylphenylsulfonamido)-N-(4-morpholinophenyl)propanamide

¹H-NMR (400 MHz, DMSO-*d₆*) *δ* 9.86 (s, 1H), 9.14 (s, 1H), 8.23 (s, 1H), 8.15 (s, 1H), 7.60 - 7.43 (m, 2H), 7.37 - 7.28 (m, 6H), 6.95 (d, *J* = 8.00 Hz, 2H), 6.88 (d, *J* = 8.40 Hz, 2H), 4.13 (m, 2H), 3.73 (t, *J* = 4.60 Hz, 3H), 3.05 - 2.96 (m, 5H), 2.89 - 2.83 (m, 1H), 2.20 (s, 3H)

### (S)-3-(1H-indol-3-yl)-N-(3-(4-methoxyphenyl)isoxazol-5-yl)-2-(4-methylphenylsulfonamido)propanamide

¹H-NMR (400 MHz, CDCl₃) *δ* 9.42 (s, 1H), 8.28 (d, *J* = 1.60 Hz, 1H), 7.73 - 7.70 (m, 2H), 7.42 (d, *J* = 8.40 Hz, 2H), 7.22 (t, *J =* 7.60 Hz, 1H), 7.12 (t, *J* = 7.60 Hz, 1H), 6.97 - 6.90 (m, 6H), 6.65 (s, 1H), 5.39 (d, *J* = 6.00 Hz, 1H), 4.15 - 4.09 (m, 1H), 3.83 (s, 3H), 3.26 - 3.08 (m, 2H), 2.25 (s, 3H)

### (S)-Benzyl 3-(1H-indol-3-yl)-2-(4-methylphenylsulfonamido)propanoate

¹H-NMR (400 MHz, CDCl₃) *δ* 8.06 (brs, 1H), 7.57 (d, *J* = 8.20 Hz, 2H), 7.42 (d, *J* = 8.20 Hz, 1H), 7.25 - 7.33 (m, 4H), 7.08 - 7.18 (m, 3H), 6.99 - 7.07 (m, 3H), 6.84 (d, *J* = 2.30 Hz, 1H), 5.24 (d, *J* = 9.00 Hz, 1H), 4.74 - 4.83 (m, 2H), 4.29 (td, *J* = 5.60, 9.00 Hz, 1H), 3.17 - 3.27 (m, 2H), 2.34 (s, 3H)

### (S)-Benzyl 3-(1H-indol-3-yl)-2-(4-methylbenzamido)propanoate

¹H-NMR (400 MHz, CDCl₃) *δ* 7.95 (brs, 1H), 7.52 - 7.60 (m, 3H), 7.33 - 7.38 (m, 3H), 7.27 - 7.32 (m, 2H), 7.16 - 7.21 (m, 3H), 7.06 (t, *J* = 7.43 Hz, 1H), 6.75 (d, *J* = 2.35 Hz, 1H), 6.64 (d, *J =* 7.83 Hz, 1H), 5.17 - 5.23 (m, 1H), 5.14 (d, *J* = 7.04 Hz, 2H), 3.44 (d, *J* = 5.09 Hz, 2H), 2.37 (s, 3H)

### (S)-N-(3-(1H-indol-3-yl)-1-(4-morpholinophenylamino)-1-oxopropan-2-yl)-4-methylbenzamide

¹H-NMR (400 MHz, CDCl₃) *δ* 8.10 (brs, 1H), 7.84 (d, *J* = 7.83 Hz, 1H), 7.64 (d, *J* = 8.22 Hz, 2H), 7.38 (d, *J* = 8.22 Hz, 1H), 7.15 - 7.24 (m, 5H), 7.14 (d, *J* = 2.35 Hz, 1H), 7.03 (d, *J* = 7.43 Hz, 1H), 6.78 - 6.82 (m, 2H), 5.06 (dt, *J* = 5.09, 7.83 Hz, 1H), 3.82 - 3.86 (m, 4H), 3.57 (dd, *J* = 5.09, 14.48, 4H)

### (S)-3-Hydroxy-2-(4-methylphenylsulfonamido)-N-(4-morpholinophenyl)propanamide

¹H-NMR (400 MHz, CDCl₃) *δ* 8.39 (s, 1H), 7.79 (d, *J* = 8.40 Hz, 2H), 7.33-7.38 (m, 4H), 6.87 (d, *J* = 9.20 Hz, 2H), 5.69 (s, 1H), 4.11 (d, *J* = 10.40 Hz, 1H), 3.85 - 3.87 (m, 5H), 3.49 - 3.50 (m, 1H), 3.36 - 3.39 (m, 1H), 3.12 (t, *J* = 5.00 Hz, 4H), 2.43 (s, 3H), 2.30 (s, 1H)

### (S)-3-Methyl-2-(4-methylphenylsulfonamido)-N-(4-morpholinophenyl)butanamide

¹H-NMR (400 MHz, OMSO-d₆) *δ* 9.56 (s, 1H), 7.81 (s, 1H), 7.62 (d, *J* = 8.00 Hz, 2H), 7.20 (d, *J* = 8.40 Hz, 2H), 7.13 (d, *J =* 8.80 Hz, 2H), 6.81 (d, *J* = 8.80 Hz, 2H), 3.71 (t, *J* = 4.80 Hz, 4H), 3.53 (d, *J* = 7.60 Hz, 1H), 3.00 (t, *J* = 4.60 Hz, 4H), 1.81 - 1.86 (m, 1H), 0.82 (q, *J* = 6.10 Hz, 6H)

### (S)-1-(3-(1H-indol-3-yl)-1-morpholino-1-oxopropan-2-yl)-3-phenylurea

¹H-NMR (400 MHz, OMSO-*d₆*) *δ* 10.90 (s, 1H), 8.72 (s, 1H), 7.53 (d, *J* = 7.60 Hz, 1H), 7.36 (t, *J* = 8.20 Hz, 3H), 7.22 - 7.17 (m, 3H), 7.06 (d, *J* = 3.40 Hz, 1H), 7.00 - 6.89 (m, 2H), 6.59 (d, *J* = 8.00 Hz, 1H), 4.96 (d, *J* = 6.00 Hz, 1H), 3.45 - 3.42 (m, 2H), 3.26 - 3.04 (m, 8H)

### (S)-3-(1H-indol-3-yL)-2-(4-methylphenyisulfonamido)-N-(6-morpholinopyridin-3-yl)propanamide

¹H-NMR (400 MHz, DMSO-*d₆*) *δ* 10.78 (s, 1H), 9.71 (s, 1H), 8.12 (s, 1H), 8.00 (d, *J* = 2.40 Hz, 1H), 7.53 (d, *J* = 8.00 Hz, 2H), 7.45 (dd, *J* = 2.40, 9.20 Hz, 1H), 7.40 (d, *J* = 8.00 Hz, 1H), 7.29 (d, *J* = 7.60 Hz, 1H), 7.12 (d, *J* = 7.60 Hz, 2H), 7.08 (d, *J* = 2.00 Hz, 1H), 7.04 (t, *J* = 7.40 Hz, 1H), 6.92 (t, *J* = 7.40 Hz, 1H), 6.74 (d, *J* = 9.20 Hz, 1H), 4.06 (s, 1H), 3.68 (t, *J* = 4.80 Hz, 4H), 3.34 - 3.33 (m, 4H), 3.07 - 3.01 (m, 1H), 2.88 - 2.82 (m, 1H)

### (S)-3-(1H-indol-3-yl)-2-(4-methylphenylsulfonamido)-N-(2-morpholinopyrimidin-5-yl)propanamide

¹H-NMR (400 MHz, DMSO-*d₆*) *δ* 10.80 (s, 1H), 9.75 (s, 1H), 8.19 (s, 1H), 8.15 (s, 2H), 7.54 (d, *J* = 8.40 Hz, 2H), 7.40 (d, *J* = 8.00 Hz, 1H), 7.30 (d, *J* = 8.00 Hz, 1H), 7.15 (d, *J* = 8.40 Hz, 1H), 7.09 (s, 1H), 7.04 (t, *J* = 7.40 Hz, 1H), 6.93 (t, *J* = 7.40 Hz, 1H), 4.04 (m, 1H), 3.62 - 3.61 (m, 8H), 3.08 - 3.02 (m, 1H), 2.91 - 2.85 (m, 1H), 2.23 (s, 3H)

### (S)-2-(4-Cyanophenylsulfonamido)-3-(1H-indol-3-yl)-N-(4-morpholinophenyl)propanamide

¹H-NMR (400 MHz, DMSO-*d₆*) *δ* 10.72 (s, 1H), 9.85 (s, 1H), 8.57 (s, 1H), 7.57 (s, 4H), 7.48 (d, *J* = 7.60 Hz, 1H), 7.29 - 7.23 (m, 3H), 7.07 - 7.01 (m, 2H), 6.93 (t, *J* = 7.20 Hz, 1H), 6.86 (d, *J* = 8.80 Hz, 2H), 4.12 (s, 1H), 3.73 (s, 4H), 3.04 - 3.00 (m, 3H), 2.90 - 2.84 (m, 1H)

### (S)-3-(1H-indol-3-yl)-N-(4-morpholinophenyl)-2-(6-(trifluoromethyl)pyridine-3-sulfonamido)propanamide

¹H-NMR (400 MHz, DMSO-*d₆*) *δ* 10.76 (s, 1H), 9.92 (s, 1H), 8.87 (s, 1H), 8.81 (s, 1H), 8.0 (d, *J* = 8.40 Hz, 1H), 7.60 (d, *J* = 8.00 Hz, 1H), 7.52 (d, *J* = 7.60 Hz, 1H), 7.22 - 7.19 (m, 3H), 7.12 (d, *J* = 1.60 Hz, 1H), 7.01 (d, *J* = 7.40 Hz, 1H), 6.92 (d, *J* = 7.40 Hz, 1H), 6.82 (d, *J* = 9.20 Hz, 2H), 4.21 (m, 1H), 3.73 (t, *J=* 4.40 Hz, 4H), 3.08 - 2.90 (m, 6H)

### (S)-Methyl4-(4-methylphenylsulfonamido)-5-(4-morpholinophenylamino)-5-oxopentanoate

¹H-NMR (400 MHz, CDCl₃) *δ* 8.16 (s, 1H), 7.74 (d, *J* = 8.40 Hz, 2H), 7.27 - 7.25 (m, 4H), 6.83 (d, *J* = 8.80 Hz, 2H), 6.21 (d, *J* = 7.20 Hz, 1H), 3.9 - 3.84 (m, 5H), 3.69 (s, 3H), 3.10 (t, *J* = 4.60 Hz, 4H), 2.58 - 2.50 (m, 1H), 2.36 (s, 3H), 2.32 - 2.25 (m, 1H), 2.05 - 1.93 (m, 2H)

### (S)-3-(1H-benzo[d]imidazol-1-yl)-2-(4-methylphenyisulfonamido)-N-(4-morpholinophenyl)propanamide

¹H-NMR (400 MHz, CDCl₃) *δ* 8.97 (s, 1H), 7.68 (s, 1H), 7.57 (d, *J* = 7.60 Hz, 2H), 7.38 (d, *J* = 8.00 Hz, 1H), 7.27 (d, *J* = 8.00 Hz, 1H), 7.12 - 7.00 (m, 6H), 6.72 (d, *J* = 8.80 Hz, 2H), 4.49 (s, 2H), 4.44 - 4.43 (m, 1H), 3.80 (t, *J* = 4.60 Hz, 4H), 3.03 (t, *J* = 4.60 Hz, 4H), 2.24 (s, 3H)

### (R)-3-(2,3-dioxo-3,4-dihydroquinoxalin-1(2H)-yl)-2-(4-methylphenylsulfonamido)-N-(4-morpholinophenyl)propanamide

¹H-NMR (400 MHz, CDCl₃) *δ* 7.48 (d, *J* = 8.80 Hz, 2H), 7.43 (d, *J* = 7.60 Hz, 2H), 7.37 (d, *J=* 8.00 Hz, 1H), 7.32 (s, 1H), 7.27 - 7.20 (m, 2H), 7.07 (d, *J=* 7.20 Hz, 1H), 6.91 (t, *J* = 6.60 Hz, 4H), 4.75 - 4.68 (m, 1H), 4.32 (d, *J* = 12.4 Hz, 2H), 3.87 (s, 4H), 3.14 (s, 4H), 2.26 (s, 3H)

### (S)-N-(5-(4-methylphenylsulfonamido)-6-(4-morpholinophenylamino)-6-oxohexyl)-5-(trifluoromethyl)picolinamide

¹H-NMR (400 MHz, CDCl₃) *δ* 8.82 (s, 1H), 8.35 (d, *J* = 8.00 Hz, 1H), 8.22 (s, 1H), 8.13 (d, *J* = 8.00 Hz, 1H), 7.71 (d, *J* = 8.00 Hz, 2H), 7.28 - 7.25 (m, 2H), 7.19 (d, *J* = 8.40 Hz, 2H), 6.81 (d, *J* = 8.80 Hz, 2H), 3.85 (t, *J* = 4.80 Hz, 4H), 3.80 - 3.76 (m, 1H), 3.45 - 3.39 (m, 2H), 3.09 (t, *J* = 4.60 Hz, 4H), 2.31 (s, 3H), 1.87 - 1.73 (m, 2H), 1.60 - 1.55 (m, 2H), 1.33- 1.30 (m, 2H)

### (S)-Benzyl 4-(4-methylphenylsulfonamido)-5-(4-morpholinophenylamino)-5-oxopentanoate

¹H-NMR (400 MHz, CDCl₃) *δ* 8.25 (s, 1H), 7.71 (d, *J* = 8.00 Hz, 2H), 7.36 - 7.30 (m, 5H), 7.21 (d, *J* = 8.80 Hz, 2H), 7.16 (d, *J* = 8.00 Hz, 2H), 6.77 (d, *J* = 8.80 Hz, 2H), 6.30 (d, *J* = 7.60 Hz, 1H), 5.12 - 5.04 (m, 2H), 3.95 - 3.90 (m, 1H), 3.83 (t, *J* = 4.60 Hz, 4H), 3.06 (t, *J* = 4.80 Hz, 4H), 2.55 - 2.48 (m, 1H), 2.36 - 2.30 (m, 1H), 2.28 (s, 3H), 2.06 - 2.03 (m, 1H), 1.96- 1.93 (m, 1H)

### (S)-Benzyl 3-(4-methylphenylsulfonamido)-4-(4-morpholinophenylamino)-4-oxobutanoate

¹H-NMR (400 MHz, CDCl₃) *δ* 8.39 (s, 1H), 7.75 (d, *J* = 8.00 Hz, 2H), 7.33 - 7.23 (m, 9H), 6.81 (d, *J* = 8.80 Hz, 2H), 6.25 (d, *J* = 8.80 Hz, 1H), 5.09 - 5.00 (m, 2H), 4.22 - 4.21 (m, 1H), 3.84 (t, *J* = 4.80 Hz, 4H), 3.08 (t, *J* = 4.80 Hz, 4H), 3.03 (dd, *J* = 4.00, 17.2 Hz, 1H), 2.41 (dd, *J* = 6.20, 17.4 Hz, 1H), 2.36 (s, 3H)

### (S)-4-Methyl-N-(4-methyl-1-(4-morpholinophenylamino)-1-oxopentan-2-yl)benzamide

¹H-NMR (400 MHz, CDCl₃)*δ* 8.73 (s, 1H), 7.69 (d, *J* = 8.00 Hz, 2H), 7.42 (d, *J* = 8.00 Hz, 2H), 7.22 (d, *J* = 8.80 Hz, 2H), 6.84 - 6.80 (m, 3H) 4.90 - 4.86 (m, 1H), 3.84 (t, *J* = 4.80 Hz, 4H), 3.08 (t, *J* = 4.80 Hz, 4H), 2.39 (s, 3H), 1.90 - 1.88 (m, 1H), 1.86 - 1.71 (m, 2H), 0.97 (d, *J* = 6.40 Hz, 3H), 0.90 (d, *J* = 6.40 Hz, 3H)

### 2-(4-Methylphenyisulfonamido)-N-(4-morpholinophenyl)acetamide

¹H-NMR (400 MHz, CDCl₃)*δ* 7.96 (s, 1H), 7.76 (d, *J* = 8.40 Hz, 2H), 7.35 - 7.32 (m, 4H), 6.85 (d, *J* = 8.80 Hz, 2H), 5.31 (t, *J* = 6.80 Hz, 1H), 3.85 (t, *J* = 4.80 Hz, 4H), 3.69 (d, *J* = 6.40 Hz, 2H), 3.11 (t, *J* = 4.80 Hz, 4H), 2.42 (s, 3H)

### (S)-3-(4-Methylphenylsulfonamido)-4-(4-morpholinophenylamino)-4-oxobutanoic acid

¹H-NMR (400 MHz, DMSO-*d₆*) *δ* 9.77 (s, 1H), 7.65 - 7.20 (m, 4H), 7.21 (d, *J* = 4.00 Hz, 2H), 6.82 (d, *J* = 8.80 Hz, 2H), 4.17 (t, *J* = 7.00 Hz, 1H), 3.72 (t, *J* = 4.40 Hz, 4H), 3.02 (t, *J* = 4.60 Hz, 4H), 2.57 (dd, *J* = 16.2, 6.60 Hz, 1H), 2.35 (dd, *J* = 16.0, 7.60 Hz, 1H), 2.25 (s, 3H)

### (S)-4-(4-Methylphenylsulfonamido)-5-(4-morpholinophenylamino)-5-oxopentanoic acid

¹H-NMR (400 MHz, DMSO-*d₆*) *δ* 9.67 (s, 1H), 7.65 (d, *J* = 8.40 Hz, 2H), 7.25 (d, *J* = 7.60 Hz, 2H), 7.19 (d, *J* = 8.80 Hz, 2H), 6.84 (d, *J* = 8.40 Hz, 2H), 3.83 - 3.79 (m, 1H), 3.72 (t, *J* = 4.60 Hz, 4H), 3.02 (t, *J* = 4.80 Hz, 4H), 2.26 (s, 3H), 2.20 - 2.11 (m, 2H), 1.80 - 1.70 (m, 2H)

### (S)-3-(4-hydroxyphenyl)-2-(4-methylphenylsulfonamido)-N-(4-morpholinophenyl)propanamide

¹H-NMR (400 MHz, DMSO-*d₆*) *δ* 9.61 (s, 1H), 9.18 (s, 1H), 8.05 (s, 1H), 7.49 (d, *J* = 8.00 Hz, 2H), 7.15 (d, *J* = 8.40 Hz, 4H), 6.93 (d, *J* = 7.60 Hz, 2H), 6.82 (d, *J* = 9.20 Hz, 2H), 6.57 (d, *J* = 8.80 Hz, 2H), 3.98 - 3.85 (m, 1H), 3.72 (t, *J* = 4.60 Hz, 4H), 3.01 (t, *J* = 4.60 Hz, 4H), 2.78 - 2.73 (m, 1H), 2.67 - 2.59 (m, 1H), 2.33 (s, 3H)

### (S)-3-(1H-indol-3-yl)-2-(4-methylphenylsulfonamido)-N-phenylpropanamide

¹H-NMR (400 MHz, CDCl₃) *δ* 8.14 (s, 1H), 8.10 (s, 1H), 7.45 (d, *J* = 8.40 Hz, 2H), 7.34 (d, *J* = 7.60 Hz, 2H), 7.30 - 7.24 (m, 4H), 7.17 - 7.11 (m, 2H), 7.09 - 6.92 (m, 3H), 6.91 (s, 1H), 5.19 (d, *J* = 5.60 Hz, 1H), 4.05 - 4.01 (m, 1H), 3.20 - 3.17 (m, 2H), 2.29 (s, 3H)

### (S)-2-(4-Bromophenylsulfonamido)-3-(1H-indol-3-yl)-N-phenylpropanamide)

¹H NMR (400 MHz, DMSO-*d₆*) *δ* 10.78 (s, 1H), 9.98 (s, 1H), 8.40 (s, 1H), 7.40 - 7.39 (m, 5H), 7.36 (d, *J* = 8.40 Hz, 2H), 7.30 - 7.23 (m, 3H), 7.10 (s, 1H), 7.06 - 7.01 (m, 2H), 6.93 (t, *J* = 7.20 Hz, 1H), 4.16 - 4.13 (m, 1H), 3.05 (dd, *J* = 14.2, 6.00 Hz, 1H), 2.87 (dd, *J* = 14.7, 8.00 Hz, 1H)

### (S)-3-(1H-indol-3-yl)-2-(4-methylphenylsulfonamido)-N-(thiophen-2-ylmethyl)propanamide

¹H-NMR (400 MHz, CDCl₃) *δ* 8.20 (s, 1H), 7.41 (d, *J* = 8.40 Hz, 2H), 7.28 - 7.24 (m, 2H), 7.16-7.11 (m, 2H), 7.01 -6.88 (m, 3H), 6.87 (s, 1H), 6.81 (s, 1H), 6.74 (s, 1H), 5.13 (d, *J* = 6.00 Hz, 1H), 4.48-4.45 (m, 2H), 3.94-3.91 (m, 1H), 3.11 -3.09 (m, 2H), 2.31 (s, 3H)

### (S)-2-(4-Bromophenylsulfonamido)-3-(1H-indol-3-yl)-N-(thiophen-2-ylmethyl)propanamide

¹H-NMR (400 MHz, DMSO-*d₆*) *δ* 10.73 (s, 1H), 8.62 (t, *J* = 6.00 Hz, 1H), 8.22 (s, 1H), 7.42 - 7.33 (m, 6H), 7.27 (d, *J* = 8.40 Hz, 1H), 7.05 - 7.01 (m, 2H), 6.94 - 6.88 (m, 2H), 6.86 (d, *J* = 2.40 Hz, 1H), 4.30 - 4.26 (m, 2H), 3.99 - 3.95 (m, 1H), 2.99 (dd, *J* = 14.2, 6.00 Hz, 1H), 2.80 (dd, *J* = 14.7, 8.00 Hz, 1H)

### (S)-2-(4-Bromophenylsulfonamido)-3-(1H-indol-3-yl)-N-(4-(2-methoxyphenyl)thiazol-2-yl)propanamide

¹H-NMR (400 MHz, DMSO-*d₆*) *δ* 12.39 (s, 1H), 10.76 (s, 1H), 8.48 (s, 1H), 7.64 (s, 1H), 7.47 (d, *J* = 7.60 Hz, 1H), 7.41 - 7.11 (m, 6H), 7.08 - 7.01 (m, 5H), 6.91(t, *J* = 7.60 Hz, 1H), 4.34 - 4.30 (m, 1H), 3.91 (s, 3H), 3.12 - 3.07 (m, 1H), 2.91 - 2.85 (m, 1H)

### (S)-N-(3-(N-(4-methy)-1-morpholino-1-oxopentan-2-yl)sulfamoyl)phenyl)acetamide

¹H-NMR (400 MHz, CDCl₃)*δ* 8.47 (s, 1H), 8.25 (d, *J* = 8.80 Hz, 1H), 7.76 (t, *J* = 1.80 Hz, 1H), 7.54 (d, *J* = 7.60 Hz, 1H), 7.45 (t, *J* = 8.00 Hz, 1H), 6.69 (d, *J* = 9.60 Hz, 1H), 4.15 - 4.08 (m, 1H), 3.59 - 3.49 (m, 2H), 3.42 - 3.18 (m, 6H), 2.16 (s, 3H), 1.99 -1.94 (m, 1H), 1.52 - 1.45 (m, 1H), 1.18 - 1.11 (m, 1H), 0.95 (d, *J* = 6.80 Hz, 3H), 0.91 (d, *J* = 6.80 Hz, 3H)

### (S)-Benzyl 4-methyl-2-(3-phenylureido)pentanoate

¹H-NMR (400 MHz, CDCl₃)*δ* 7.38 (s, 1H), 7.34 - 7.01 (m, 8H), 6.99 - 6.97 (m, 1H), 5.83 (d, *J* = 8.00 Hz, 1H), 5.19 - 5.07 (m, 2H), 4.65 - 4.60 (m, 1H), 1.71 - 1.57 (m, 2H), 1.51 - 1.46 (m, 1H), 0.91 (d, *J* = 6.40 Hz, 3H), 0.88 (d, *J* = 6.40 Hz, 3H)

### 4-Methyl-N-(2-morpholino-2-oxoethyl)benzenesulfonamide

¹H-NMR (400 MHz, CDCl₃)*δ* 7.76 (d, *J* = 8.00 Hz, 2H), 7.30 (d, *J* = 8.00 Hz, 2H), 6.20 (t, *J* = 4.60 Hz, 1H), 3.79 (d, *J* = 4.80 Hz, 2H), 3.58 - 3.62 (m, 4H), 3.51 (t, *J* = 4.40 Hz, 2H), 3.32 (t, *J* = 4.60 Hz, 2H), 2.4 (s, 3H)

### 4-Methyl-N-(1-(morpholine-4-carbonyl)cyclohexyl)benzenesulfonamide

¹H-NMR (400 MHz, CDCl₃)*δ* 7.79 (d, *J* = 8.00 Hz, 2H), 7.29 (d, *J* = 8.00 Hz, 2H), 5.33 (s, 1H), 3.74 - 3.78 (m, 8H), 2.43 (s, 3H), 1.74 - 1.91 (m, 4H), 1.17 - 1.40 (m, 6H).

### (S)-4-(N-(4-methyl-1-oxo-1-(1,4,7-trioxa-10-azacyclododecan-10-yl)pentan-2-yl)sulfamoyl)benzoic acid

¹H-NMR (400 MHz, CDCl₃)*δ* 8.20 (d, *J* = 8.40 Hz, 2H), 7.94 (d, *J* = 8.40 Hz, 2H), 6.49 (d, *J* = 9.20 Hz, 1H), 4.46 - 4.41 (m, 1H), 3.84 - 3.78 (m, 2H), 3.65 - 3.25 (m, 14H), 1.93 - 1.89 (m, 1H), 1.56 - 1.36 (m, 2H), 0.91 (t, *J* = 7.00 Hz, 6H)

### (S)-2-(4-Hydroxyphenyisulfonamido)-3-(1H-indol-3-yl)-N-(4-morpholinophenyl)propanamide

¹H-NMR (400 MHz, DMSO-*d₆*) *δ* 10.78 (s, 1H), 10.18 (s, 1H), 9.64 (s, 1H), 7.84 (d, *J* = 8.40 Hz, 1H), 7.50 (d, *J* = 8.80 Hz, 2H), 7.39 (d, *J* = 7.60 Hz, 1H), 7.29 (d, *J* = 7.60 Hz, 1H), 7.22 (d, *J* = 9.20 Hz, 2H), 7.06 (d, *J* = 2.40 Hz, 1H), 7.03 (d, *J* = 7.20 Hz, 1H), 6.93 (t, *J* = 7.40 Hz, 1H), 6.82 (d, *J* = 9.20 Hz, 2H), 6.70 (d, *J* = 8.40 Hz, 2H), 4.08 - 4.02 (m, 1H), 3.72 (t, *J* = 4.80 Hz, 4H), 3.07 - 3.00 (m, 5H), 2.84 - 2.79 (m, 1H)

### (S)-4-(N-(3-(1H-indol-3-yl)-1-(4-morpholinophenylamino)-1-oxopropan-2-yl)sulfamoyl)benzoic acid

¹H-NMR (400 MHz, DMSO-*d₆*) *δ* 10.77 (s, 1H), 9.83 (s, 1H), 8.43 (d, *J* = 9.20 Hz, 1H), 7.76 (d, *J* = 8.00 Hz, 2H), 7.63 (d, *J* = 8.40 Hz, 2H), 7.44 (d, *J* = 7.60 Hz, 1H), 7.26 - 7.22 (m, 3H), 7.09 (d, *J* = 2.04 Hz, 1H), 7.00 (t, *J* = 7.20 Hz, 1H), 6.91 (t, *J* = 7.60 Hz, 3H), 4.14 - 4.12 (m, 1H), 3.76 (t, *J* = 4.60 Hz, 4H), 3.07 - 3.03 (m, 5H), 2.90 - 2.86 (m, 1H)

### (S)-2-Amino-3-(1H-indol-3-yl)-N-(4-morpholinophenyl)propanamide

¹H-NMR (400 MHz, CDCl₃)*δ* 9.27 (s, 1H), 8.21 (s, 1H), 7.70 (d, *J* = 8.00 Hz, 1H), 7.51 - 7.47 (m, 2H), 7.37 (d, *J* = 8.40 Hz, 1H), 7.22 - 7.11 (m, 2H), 7.08 (d, *J* = 2.40 Hz, 1H), 6.89 - 6.86 (m, 2H), 3.86 - 3.82 (m, 5H), 3.49 - 3.45 (m, 1H), 3.11 (t, *J* = 4.80 Hz, 4H), 3.04 - 2.99 (m, 1H), 1.81 (s, 2H)

### (S)-N-(4-(4-Acetylpiperazin-1-yl)-2-methoxyphenyl)-3-(1H-indol-3-yl)-2-(4-methylphenylsulfonamido)propanamide

¹H-NMR (400 MHz, CDCl₃)*δ* 8.54 (s, 1H), 8.49 (s, 1H), 8.12 (d, *J* = 8.40 Hz, 1H), 7.46 (d, *J=* 8.40 Hz, 2H), 7.32 - 7.26 (m, 2H), 7.13 (t, *J* = 7.60 Hz, 1H), 7.00 - 6.95 (m, 3H), 6.92 (d, *J* = 2.40 Hz, 1H), 6.45 - 6.40 (m, 2H), 5.37 (d, *J* = 5.60 Hz, 1H), 4.09 - 4.05 (m, 1H), 3.75 (t, *J=* 5.40 Hz, 2H), 3.71 (s, 3H), 3.59 (t, *J* = 5.00 Hz, 2H), 3.21 - 3.04 (m, 6H), 2.28 (s, 3H), 2.13 (s, 3H)

### (S)-N-(4-(4-Acetylpiperazin-1-yl)phenyl)-3-(1H-indol-3-yl)-2-(4-methylphenylsulfonamido)propanamide

¹H-NMR (400 MHz, CDCl₃)*δ* 8.31 (s, 1H), 8.11 (s, 1H), 8.47 (d, *J* = 8.40 Hz, 2H), 7.31 - 7.25 (m, 5H), 7.16 (t, *J* = 7.60 Hz, 1H), 7.04 (d, *J* = 8.40 Hz, 2H), 6.99 (t, *J* = 7.60 Hz, 1H), 6.94 (d, *J* = 2.40 Hz, 1H), 6.82 (d, *J* = 9.20 Hz, 2H), 5.22 (d, *J* = 6.00 Hz, 1H), 4.02 - 4.00 (m, 1H), 3.74 (t, *J* = 5.00 Hz, 2H), 3.59 (t, *J* = 5.00 Hz, 2H), 3.18 (d, *J* = 6.40 Hz, 2H), 3.10 (t, *J* = 5.00 Hz, 2H), 3.05 (t, *J* = 5.00 Hz, 2H), 2.31 (s, 3H), 2.12 (s, 3H)

### (S)-N-(3-(1H-indol-3-yl)-1-(2-(4-morpholinobenzoyl)hydrazinyl)-1-oxopropan-2-yl)-4-methylbenzenesulfonamide

¹H-NMR (400 MHz, DMSO-*d₆*) *δ* 10.71 (s, 1H), 10.17 (s, 2H), 7.99 (s, 1H), 7.82 (d, *J* = 8.80 Hz, 2H), 7.37 (d, *J* = 8.00 Hz, 1H), 7.33 (d, *J* = 8.40 Hz, 2H), 7.26 (d, *J* = 7.26 Hz, 1H), 7.14 (s, 1H), 7.04- 6.98 (m, 5H), 6.90 (t, *J* = 7.40 Hz, 1H),4.07 (s, 1H), 3.74 (t, *J* = 4.40 Hz, 4H), 3.24 (t, *J* = 4.80 Hz, 4H), 3.11 - 3.06 (m, 1H), 2.80 - 2.74 (m, 1H), 2.21 (s, 3H)

### (S)-3-(1H-indol-3-yl)-2-(4-methylphenylsulfonamido)-N-(4-(4-morpholinophenyl)thiazol-2-yl)propanamide

¹H-NMR (400 MHz, DMSO-*d₆*) *δ* 12.24 (s, 1H), 10.76 (s, 1H), 8.21 (s, 1H), 7.73 (d, *J* = 8.80 Hz, 2H), 7.46 (d, *J* = 8.00 Hz, 2H), 7.41 (d, *J* = 8.00 Hz, 1H), 7.35 (s, 1H), 7.27 (d, *J* = 8.40 Hz, 1H), 7.05 - 6.97 (m, 6H), 6.91 (t, *J* = 7.60 Hz, 1H), 4.29 (s, 1H), 3.74 (t, *J* = 4.60 Hz, 4H), 3.15 (t, *J* = 4.60 Hz, 4H), 3.10 - 3.05 (m, 1H), 2.88 - 2.82 (m, 1H), 2.18 (s, 3H)

### (S)-Methyl4-(2-(3-(1H-indol-3-yl)-2-(4-methylphenylsulfonamido)propanamido)thiazol-4-yl)benzoate

¹H-NMR (400 MHz, DMSO-*d₆*) *δ* 12.39 (s, 1H), 10.77 (s, 1H), 8.25 (d, *J* = 8.00 Hz, 1H), 8.02 (s, 4H), 7.81 (s, 1H), 7.46 (d, *J* = 8.40 Hz, 2H), 7.42 (d, *J* = 8.00 Hz, 1H), 7.27 (d, *J* = 8.40 Hz, 1H), 7.06 - 7.01 (m, 4H), 6.91 (t, *J* = 7.60 Hz, 1H), 4.32 - 4.30 (s, 1H), 3.86 (s, 3H), 3.12 - 3.06 (m, 1H), 2.90 - 2.84 (m, 1H), 2.17 (s, 3H)

### (S)-Benzyl 3-(1H-indol-3-yl)-2-(6-(trifluoromethyl)pyridine-3-sulfonamido)propanoate

¹H-NMR (400 MHz, CDCl₃)*δ* 8.85 (d, *J* = 1.60 Hz, 1H), 7.91 (s, 1H), 7.83 (dd, *J* = 1.60, 8.40 Hz, 1H), 7.42 (d, *J* = 8.40 Hz, 1H), 7.36 - 7.33 (m, 4H), 7.26 - 7.15 (m, 5H), 7.08 - 7.04 (m, 1H), 6.84 (d, *J* = 2.40 Hz, 1H), 5.40 (d, *J* = 8.80 Hz, 1H), 5.03 (s, 2H), 4.43 - 4.38 (m, 1H), 3.33-3.28 (m, 1H), 3.16 - 3.10 (m, 1H)

### (S)-2-(4-Methylphenylsulfonamido)-N¹-(4-morpholinophenyl)-N⁵-(4-nitrophenyl)pentanediamide

¹H-NMR (400 MHz, DMSO-*d₆*) *δ* 10.57 (s, 1H), 9.71 (s, 1H), 8.21 (d, *J* = 8.80 Hz, 2H), 8.08 (s, 1H), 7.81 (d, *J* = 9.20 Hz, 2H), 7.65 (d, *J* = 8.40 Hz, 2H), 7.22 - 7.18 (m, 4H), 6.84 (d, *J* = 8.80 Hz, 2H), 3.86 (t, *J* = 6.80 Hz, 1H), 3.72 (t, *J* = 4.60 Hz, 4H), 3.02 (t, *J* = 4.80 Hz, 4H), 2.46 - 2.37 (m, 2H), 2.21 (s, 3H), 1.93 - 1.79 (m, 2H)

### (S)-Benzyl 5-(4-methylphenylsulfonamido)-6-(4-morpholinophenylamino)-6-oxohexylcarbamate

¹H-NMR (400 MHz, CD₃OD)*δ* 7.71 (d, *J* = 8.00 Hz, 2H), 7.32 - 7.25 (m, 5H), 7.22 (d, *J* = 8.40 Hz, 2H), 7.15 (d, *J* = 8.40 Hz, 2H), 6.86 (d, *J* = 9.20 Hz, 2H), 5.06 (s, 2H), 3.82 - 3.78 (m, 5H), 3.07 - 3.03 (m, 6H), 2.26 (s, 3H), 1.69 - 1.59 (m, 2H), 1.48 - 1.28 (m, 4H)

### (S)-6-Amino-2-(4-methylphenylsulfonamido)-N-(4-morpholinophenyl)hexanamide

¹H-NMR (400 MHz, DMSO-*d₆*) *δ* 9.65 (s, 1H), 7.65 (d, *J* = 8.40 Hz, 2H), 7.25 -7.19 (m, 4H), 6.83 (d, *J* = 8.80 Hz, 2H), 3.77 - 3.71 (m, 5H), 3.02 (t, *J* = 4.80 Hz, 4H), 2.42 (t, *J* = 6.40 Hz, 2H), 2.26 (s, 3H), 1.52 - 1.44 (m, 2H), 1.24 - 1.12 (m, 4H)

### (S)-3,5-Dimethoxy-N-(5-(4-methylphenylsulfonamido)-6-(4-morpholinophenylamino)-6-oxohexyl)benzamide

¹H-NMR (400 MHz, CDCl₃)*δ* 8.60 (s, 1H), 7.68 (d, *J* = 8.00 Hz, 2H), 7.25 (d, *J* = 8.80 Hz, 2H), 7.14 (d, *J* = 8.40 Hz, 2H), 6.98 (d, *J* = 2.40 Hz, 2H), 6.77 (d, *J* = 9.20 Hz, 2H), 6.86 (t, *J* = 6.00 Hz, 1H), 6.58 (s, 1H), 6.35 (d, *J* = 7.20 Hz, 1H), 3.83 (t, *J* = 4.60 Hz, 4H), 3.78 - 3.75 (m, 7H), 3.39 - 3.32 (m, 2H), 3.06 (t, *J* = 4.80 Hz, 4H), 2.26 (s, 3H), 1.85 - 1.71 (m, 2H), 1.52 - 1.47 (m, 2H), 1.30 - 1.23 (m, 2H)

### (S)-N⁵-(4-Methoxybenzyl)-2-(4-methylphenylsulfonamido)-N¹-(4-morpholinophenyl)pentanediamide

¹H-NMR (400 MHz, DMSO-*d₆*) *δ* 9.60 (s, 1H), 8.25 (t, *J* = 5.80 Hz, 1H), 7.95 (s, 1H), 7.64 (d, *J* = 8.40 Hz, 2H), 7.23 (d, *J* = 8.40 Hz, 2H), 7.19 (d, *J* = 9.20 Hz, 2H), 7.14 (d, *J* = 8.80 Hz, 2H), 6.84 (t, *J* = 8.80 Hz, 4H), 4.15 (d, *J* = 6.00 Hz, 2H), 3.82 - 3.71 (m, 8H), 3.02 (t, *J* = 4.80 Hz, 4H), 2.26 (s, 3H), 2.17 - 2.05 (m, 2H), 1.82 - 1.75 (m, 2H)

### (S)-2-(4-Methylphenyisulfonamido)-N¹-(4-morpholinophenyl)-N⁵-phenylpentanediamide

¹H-NMR (400 MHz, DMSO-*d₆*) *δ* 9.89 (s, 1H), 9.64 (s, 1H), 7.98 (s, 1H), 7.66 (d, *J* = 8.00 Hz, 2H), 7.56 (d, *J* = 8.40 Hz, 2H), 7.28 - 7.19 (m, 6H), 7.01 (t, *J* = 7.40 Hz, 1H), 6.84 (d, *J* = 8.80 Hz, 2H), 3.86 (t, *J* = 6.80 Hz, 1H), 3.72 (t, *J* = 4.60 Hz, 4H), 3.02 (t, *J* = 4.60 Hz, 4H), 2.41 - 2.26 (m, 2H), 2.23 (s, 3H), 1.94 - 1.80 (m, 2H)

### (S)-2-(4-Methylphenyisulfonamido)-5-morpholino-N-(4-morpholinophenyl)-5-oxopentanamide

¹H-NMR (400 MHz, CDCl₃)*δ* 8.75 (s, 1H), 7.74 (d, *J* = 8.40 Hz, 2H), 7.34 (d, *J* = 8.80 Hz, 2H), 7.28 (d, *J* = 8.00 Hz, 2H), 7.11 (d, *J* = 6.00 Hz, 1H), 8.65 (d, *J* = 8.80 Hz, 2H), 3.86 (t, *J* = 4.60 Hz, 1H), 3.78 - 3.65 (m, 6H), 3.59 - 3.55 (m, 1H), 3.45 - 3.33 (m, 2H), 3.10 (t, *J* = 5.00 Hz, 4H), 2.64 - 2.63 (m, 2H), 2.38 (s, 3H), 2.23 - 2.18 (m, 2H), 2.07 - 2.02 (m, 2H)

### (S)-N⁵-(4-fluorophenyl)-2-(4-methylphenylsulfonamido)-N¹-(4-morpholinophenyl)pentanediamide

¹H-NMR (400 MHz, DMSO-*d₆*) *δ* 9.99 (s, 1H), 9.68 (s, 1H), 8.04 (s, 1H), 7.65 (d, *J* = 8.00 Hz, 2H), 7.58 - 7.55 (m, 2H), 7.21 (t, *J* = 8.00 Hz, 4H), 7.12 (t, *J* = 8.80 Hz, 2H), 6.84 (d, *J* = 8.80 Hz, 2H), 3.85 (s, 1H), 3.72 (s, 4H), 3.02 (s, 4H), 2.38 - 2.26 (m, 2H), 2.22 (s, 3H), 1.89 - 1.81 (m, 2H)

### (S)-N⁵-(4-tert-butylphenyl)-2-(4-methylphenylsulfonamido)-N¹-(4-morpholinophenyl)pentanediamide

¹H-NMR (400 MHz, DMSO-*d₆*) *δ* 9.86 (s, 1H), 9.69 (s, 1H), 8.05 (s, 1H), 7.65 (d, *J* = 8.40 Hz, 2H), 7.47 (d, *J* = 8.80 Hz, 2H), 7.29 (d, *J* = 8.80 Hz, 2H), 7.22 - 7.19 (m, 4H), 6.84 (d, *J* = 8.80 Hz, 2H), 3.84 (t, *J* = 6.80 Hz, 1H), 3.73 (t, *J* = 4.60 Hz, 4H), 3.02 (t, *J* = 4.80 Hz, 4H), 2.40 - 2.25 (m, 2H), 2.22 (s, 3H), 1.92 - 1.77 (m, 2H), 1.24 (s, 9H)

### (S)-2-(4-Methylphenylsulfonamido)-N¹-(4-morpholinophenyl)-N⁵-(4-(trifluoromethyl)phenyl)pentanediamide

¹H-NMR (400 MHz, DMSO-*d₆*) *δ* 10.30 (s, 1H), 9.69 (s, 1H), 8.05 (s, 1H), 7.77 (d, *J* = 8.80 Hz, 2H), 7.65 (d, *J* = 8.00 Hz, 4H), 7.22 - 7.19 (m, 4H), 6.84 (d, *J* = 8.80 Hz, 2H), 3.86 (s, 1H), 3.72 (s, 4H), 3.02 (s, 4H), 2.44 - 2.33 (m, 2H), 2.21 (s, 3H), 1.93 - 1.82 (m, 2H)

### (S)-2-(4-Methylphenylsulfonamido)-N¹-(4-morpholinophenyl)-N⁴-(4-nitrophenyl)succinamide

¹H-NMR (400 MHz, DMSO-*d₆*) *δ* 10.55 (s, 1H), 9.80 (s, 1H), 8.21 (d, *J* = 8.80 Hz, 2H), 8.18 (s, 1H), 7.75 (d, *J* = 9.20 Hz, 2H), 7.65 (d, *J* = 8.40 Hz, 2H), 7.25 (d, *J* = 8.80 Hz, 2H), 7.18 (d, *J* = 8.40 Hz, 2H), 6.84 (d, *J* = 8.80 Hz, 2H), 4.35 (t, *J* = 6.80 Hz, 1H), 3.72 (t, *J* = 4.60 Hz, 4H), 3.02 (t, *J* = 4.80 Hz, 4H), 2.78 (dd, *J* = 6.60, 15.40 Hz, 1H), 2.62 (dd, *J* = 7.80, 15.40 Hz, 1H), 2.21 (s, 3H)

### (S)-N-(furan-2-ylmethyl)-3-(1H-indol-3-yl)-2-(4-methylphenylsulfonamido)propanamide

¹H-NMR (500 MHz, CDCl₃)*δ* 8.10 (s, 1H), 7.45 - 7.43 (d, *J* = 10.00 Hz, 2H), 7.32 (s, 1H), 7.31 - 7.30 (d, *J* = 5.00 Hz, 1H), 7.26 - 7.25 (d, *J* = 5.00 Hz, 1H), 7.18 - 7.15 (t, *J* = 7.50 Hz, 1H), 7.06 - 7.05 (d, *J* = 5.00 Hz, 2H), 7.01 - 6.98 (t, *J* = 7.50 Hz, 1H), 6.83 (s, 1H), 6.67 (s, 1H), 6.30 (s, 1H), 6.15 (s, 1H), 4.97-4.96 (d, *J* = 0.94 Hz, 1H), 4.40-4.31 (m, 2H), 3.93 - 3.90 (m, 1H), 3.15 - 3.07 (m, 2H), 2.34 (s, 3H)

### (S)-2-(4-Bromophenylsulfonamido)-N-(furan-2-ylmethyl)-3-(1H-indol-3-yl)propanamide

¹H-NMR (500 MHz, CDCl₃)*δ* 8.64 (s, 1H), 7.98 (s, 3H), 7.32 - 7.22 (m, 6H), 7.18 - 7.15 (t, *J* = 7.50 Hz, 1H), 6.88 (s, 1H), 6.29 (s, 1H), 6.15 (s, 1H), 5.69 (s, 1H), 4.33 (m, 2H), 3.95 - 3.91 (m, 1H), 3.28 - 3.24 (m, 1H), 3.05 - 3.00 (m, 1H)

### (S)-3-(1H-indol-3-yl)-N-isopropyl-2-(4-methylphenylsulfonamido)propanamide

¹H-NMR (500 MHz, CDCl₃)*δ* 8.27 (s, 1H), 7.47 - 7.45 (d, *J* = 10.00 Hz, 2H), 7.33 - 7.31 (d, *J* = 10.00 Hz, 2H), 7.19 - 7.16 (t, *J* = 7.50 Hz, 1H), 7.07 - 7.05 (d, *J* = 10.00 Hz, 2H), 7.02 - 6.99 (t, *J* = 7.50 Hz, 1H), 6.92 (s, 1H), 6.02 - 6.00 (d, *J* = 10.00 Hz, 1H), 5.09 - 5.07 (d, *J* = 10.00 Hz, 1H), 3.97 - 3.90 (m, 1H), 3.86 - 3.82 (m, 1H), 3.15 - 3.07 (m, 2H), 2.33 (s, 3H)

### (S)-2-(4-Bromophenylsulfonamido)-3-(1H-indol-3-y))-N-isopropylpropanamide

¹H-NMR (500 MHz, MeOD)*δ* 10.23 (s, 1H), 7.97-7.95 (d, *J* = 5.00 Hz, 1H), 7.50 - 7.48 (d, *J* = 10.00 Hz, 2H), 7.42 - 7.41 (d, *J* = 5.00 Hz, 1H), 7.27 - 7.25 (d, *J* = 10.00 Hz, 1H), 7.06 - 7.02 (m, 4H), 6.94 - 6.90 (m, 2H), 6.86 - 6.84 (d, *J* = 10.00 Hz, 1H), 6.80 - 6.76 (t, *J* = 10.00 Hz, 1H), 4.66 (s, 1H), 4.15 - 4.12 (t, *J* = 7.50 Hz, 1H), 3.22 - 3.18 (m, 1H), 3.03-2.99 (m, 1H), 2.11 (s, 3H)

### (S)-N-(3-(1H-indol-3-yl)-1-(2-(4-methoxybenzoyl)hydrazinyl)-1-oxopropan-2-yl)-4-methylbenzenesulfonamide

¹H-NMR (500 MHz, CDCl₃)*δ* 7.86 - 7.64 (d, *J=* 8.50 Hz, 2H), 7.31 - 7.26 (m, 4H), 7.11 - 7.08 (t, *J* = 7.50 Hz, 1H), 7.04 (s, 1H), 6.95 - 6.91 (m, 3H), 6.89 - 6.87 (d, *J* = 8.00 Hz, 2H), 4.10 - 4.07 (m, 1H), 3.85 (s, 3H), 3.35 - 3.31 (m, 1H), 3.04 - 3.00 (m, 1H), 2.26 (s, 3H)

### (S)-2-(3-(1H-indol-3-yl)-2-(4-methylphenylsulfonamido)propanoyl)-N-(4-methoxyphenyl)hydrazinecarboxamide

¹H-NMR (500 MHz, MeOD)*δ* 7.39 - 7.36 (t, *J* = 7.00 Hz, 4H), 7.28 - 7.26 (d, *J* = 8.00 Hz, 1H), 7.22 - 7.20 (d, *J* = 7.50 Hz, 1H), 7.08 - 7.05 (t, *J* = 7.50 Hz, 1H), 7.01 (s, 1H), 6.98 (s, 1H), 6.97 (s, 1H), 6.89 - 6.84 (m, 3H), 3.89 - 3.86 (m, 1H), 3.76 (s, 3H), 3.25 - 3.21 (m, 1H), 2.93 - 2.89 (m, 1H), 2.30 (s, 3H)

### (S)-4-Bromo-N-(1-(3,5-dimethyl-1H-pyrazol-1-yl)-3-(1H-indol-3-yl)-1-oxopropan-2-yl)benzenesulfonamide

¹H-NMR (500 MHz, DMSO-*d₆*)*δ* 10.76 (s, 1H), 8.65 (s, 1H), 7.66 - 7.65 (d, *J* = 9.50 Hz, 1H), 7.30 - 7.27 (t, *J* = 7.50 Hz, 3H), 7.16 - 7.15 (d, *J* = 8.00 Hz, 2H), 7.10 (s, 1H), 7.07 - 7.04 (t, *J* = 7.50 Hz, 1H), 6.96 - 6.93 (t, *J* = 7.50 Hz, 1H), 6.24 (s, 2H), 5.39 - 5.37 (d, *J* = 8.50 Hz, 1H), 3.20 - 3.16 (d, *J* = 14.00, 3.00 Hz, 1H), 2.89 - 2.84 (d, *J* = 14.00, 10.50 Hz, 1H), 2.92 (s, 3H), 2.27 (s, 3H)

### (S)-N-(3-(1H-indol-3-yl)-1-oxo-1-(3-phenyl-1H-pyrazol-1-yl)propan-2-yl)-4-bromobenzenesulfonamide

¹H-NMR (500 MHz, DMSO-d₆)*δ* 10.77 (s, 1H), 8.86 (s, 1H), 8.48 (s, 1H), 8.00 - 7.98 (d, *J* = 7.50 Hz, 2H), 7.69 - 7.68 (d, *J* = 7.50 Hz, 1H), 7.58 - 7.55 (t, *J* = 7.00 Hz, 2H), 7.52 - 7.49 (t, *J* = 7.50 Hz, 1H), 7.29 - 7.23 (m, 4H), 7.17 (s, 1H), 7.15 (s, 1H), 7.11 - 7.04 (m, 2H), 6.98 - 6.92 (m, 1H), 5.50 - 5.49 (d, *J* = 7.00 Hz, 1H), 3.28 - 3.25 (d, *J* = 14.00, 4.00 Hz, 1H), 2.97 - 2.92 (d, *J* = 14.00, 10.00 Hz, 1H)

### N((S)-1-(2-((3S,5S,7S)-adamantane-1-carbonyl)hydrazinyl)-3-(1H-indol-3-yl)-1-oxopropan-2-yl)-4-methylbenzenesulfonamide

¹H-NMR (500 MHz, MeOD)*δ* 7.33 - 7.32 (d, *J* = 8.00 Hz, 1H), 7.25 - 7.20 (t, *J* = 7.50 Hz, 3H), 7.06 - 7.03 (t, *J* = 7.50 Hz, 1H), 7.00 (s, 1H), 6.91 - 6.88 (t, *J* = 8.00 Hz, 1H), 6.85 - 6.83 (d, J = 8.00 Hz, 2H), 4.04 - 4.01 (d, *J* = 9.50, 4.50 Hz, 1H), 3.29 - 3.25 (d, *J =* 15.00, 5.00 Hz, 1H), 2.94 - 2.89 (d, *J* = 15.00, 10.00 Hz, 1H), 2.24 (s, 3H), 2.02 - 2.01 (d, *J* = 6.00 Hz, 3H), 1.95 (s, 6H), 1.80 - 1.74 (t, *J* = 12.50 Hz, 6H)

### (S)-2-(4-Bromophenylsulfonamido)-3-(1H-indol-3-yl)-N-(4-(p-tolyl)thiazol-2-yl)propanamide

¹H-NMR (500 MHz, DMSO-*d₆*)*δ* 12.47 (s, 1H), 10.79 (s, 1H), 8.53 - 8.52 (d, *J* = 7.50 Hz, 1H), 7.78 - 7.77 (d, *J* = 8.00 Hz, 2H), 7.55 (s, 1H), 7.48 - 7.47 (d, *J* = 7.50 Hz, 1H), 7.44 - 7.37 (m, 4H), 7.29 - 7.27 (d, *J=* 8.00 Hz, 1H), 7.25 - 7.23 (d, *J* = 8.50 Hz, 2H), 7.09 (d, *J* = 2.50 Hz, 1H), 7.06 - 7.03 (t, *J* = 7.50 Hz, 1H), 6.94 - 6.91 (t, *J* = 7.50 Hz, 1H), 4.35 - 4.31 (m, 1H), 3.12 - 3.08 (d, *J* = 14.50, 6.50 Hz, 1H), 2.91 - 2.86 (d, *J* = 15.00, 10.00 Hz, 1H), 2.33 (s, 3H)

### (S)-3-(1H-indol-3-yl)-2-(4-methylphenylsulfonamido)-N-phenethylpropanamide

¹H-NMR (500 MHz, CDCl₃)*δ* 8.27 (s, 1H), 8.02 (s, 1H), 7.47 - 7.46 (d, *J* = 10.00 Hz, 2H), 7.32 - 7.31 (d, *J* = 5.00 Hz, 2H), 7.28 - 7.25 (d, *J* = 7.50 Hz, 2H), 7.23 - 7.22 (d, *J* = 5.00 Hz, 1H), 7.20 - 7.17 (t, *J* = 7.50 Hz, 1H), 7.08 - 7.01 (m, 5H), 6.84 (s, 1H), 6.37 - 6.35 (t, *J* = 5.00 Hz, 1H), 5.08 - 5.07 (d, *J* = 5.00 Hz, 1H), 3.43 - 3.35 (m, 2H), 3.10 - 3.07 (t, *J* = 7.50 Hz, 2H), 2.70 - 2.59 (m, 2H), 2.35 (s, 3H)

### (S)-2-(4-Bromophenylsulfonamido)-N-(4-(4-fluorophenyl)thiazol-2-yl)-3-(1H-indol-3-yl)propanamide

¹H-NMR (500 MHz, MeOD)*δ* 7.91 - 7.88 (m, 2H), 7.45 - 7.42 (m, 3H), 7.32 (s, 1H), 7.30 - 7.27 (m, 3H), 7.14 - 7.10 (t, *J* = 10.00 Hz, 2H), 7.09 - 7.06 (t, *J* = 7.50 Hz, 1H), 7.02 (s, 1H), 6.97 - 6.94 (t, *J* = 7.50 Hz, 1H), 4.31 - 4.28 (m, 1H), 3.28 - 3.24 (m, 1H), 3.08-3.03 (m, 1H)

### (S)-2-(4-Bromophenylsulfonamido)-3-(1H-indol-3-yl)-N-(4-(4-nitrophenyl)thiazol-2-yl)propanamide

¹H-NMR (500 MHz, MeOD)*δ* 8.28 - 8.26 (d, *J* = 10.00 Hz, 2H), 8.12 - 8.10 (d, *J =* 10.00 Hz, 2H), 7.68 (s, 1H), 7.47 - 7.44 (m, 3H), 7.31 - 7.27 (m, 3H), 7.09 - 7.06 (t, *J* = 7.50 Hz, 1H), 6.97 - 6.94 (t, *J* = 7.50 Hz, 1H), 4.31 - 4.28 (m, 1H), 3.29 - 3.25 (m, 1H), 3.10 - 3.05 (m, 1H)

### (S)-N-(3-(1H-indol-3-yl)-1-(2-(4-methoxybenzoyl)hydrazinyl)-1-oxopropan-2-yl)-4-methylbenzenesulfonamide

¹H-NMR (500 MHz, MeOD)*δ* 7.89 - 7.88 (d, *J* = 8.50 Hz, 2H), 7.43 - 7.41 (d, *J* = 8.80 Hz, 1H), 7.28 - 7.26 (d, *J* = 8.00 Hz, 1H), 7.22 - 7.21 (d, *J* = 8.50 Hz, 2H), 7.16 - 7.14 (d, *J* = 8.00 Hz, 2H), 7.10 - 7.07 (t, *J* = 7.00 Hz, 1H), 7.03 - 7.01 (d, *J* = 11.00 Hz, 3H), 6.96 - 6.93 (t, *J* = 7.00 Hz, 1H), 4.18 - 4.15 (d, *J* = 10.00, 5.00 Hz, 1H), 3.87 (s, 3H), 3.35 - 3.32 (m, 1H), 3.00 - 2.95 (d, *J* = 10.50, 4.00 Hz, 1H)

### (S)-2-(4-Bromophenylsulfonamido)-N-(4-(4-cyanophenyl)thiazol-2-yl)-3-(1H-indol-3-yl)propanamide

¹H-NMR (500 MHz, DMSO-*d₆*) *δ* 2.88 - 2.93 (dd, *J* = 14.18, 8.80 Hz, 1H), 3.09-3.13 (dd, *J* = 14.18, 6.35 Hz, 1H), 4.34 - 4.35 (d, *J* = 6.35 Hz, 1H), 6.91 - 6.94 (t, *J* = 7.33 Hz, 1H), 7.02 - 7.05 (t, *J* = 7.82 Hz, 1H), 7.09 - 7.10 (d, *J* = 1.95 Hz, 1H), 7.27 - 7.28 (d, *J* = 8.31 Hz, 1H), 7.37 - 7.42 (m, 4H), 7.47 - 7.48 (d, *J* = 7.82 Hz, 1H), 7.88 - 7.91 (m, 3H), 8.05 - 8.06 (d, *J* = 8.31 Hz, 2H), 8.55 - 8.57 (d, *J* = 7.33 Hz, 1H), 10.80 (s, 1H), 12.55 (brs, 1H)

### (S)-2-(4-Bromophenylsulfonamido)-3-(1H-indol-3-yl)-N-(4-(4-(trifluoromethyl)phenyl)thiazol-2-yl)propanamide

¹H-NMR (500 MHz, DMSO-*d₆*) *δ* 2.88 - 2.92 (dd, *J* = 14.18, 8.80 Hz, 1H), 3.09 - 3.13 (dd, *J* = 14.18, 6.35 Hz, 1H), 4.33 - 4.35 (m, 1H), 6.90 - 6.93 (t, *J* = 7.33 Hz, 1H), 7.02 - 7.05 (t, *J* = 7.33 Hz, 1H), 7.09 (d, *J* = 1.95 Hz, 1H), 7.27 - 7.28 (d, *J* = 8.31 Hz, 1H), 7.37 - 7.42 (q, *J* = 8.31, 8.80, 7.82 Hz, 4H), 7.47 - 7.48 (d, *J* = 7.82 Hz, 1H), 7.78 - 7.80 (d, *J* = 8.31 Hz, 2H), 7.85 (s, 1H), 8.08 - 8.10 (d, *J* = 8.31 Hz, 2H), 8.52 - 8.54 (d, *J* = 7.33 Hz, 1H), 10.78 (s, 1H), 12.54 (brs, 1H).

### (S)-4-Bromo-N-(1-(2-dodecanoylhydrazinyl)-3-(1H-indol-3-yl)-1-oxopropan-2-yl)benzenesulfonamide

¹H-NMR (500 MHz, CDCl₃)*δ* 0.86 - 0.89 (t, *J* = 6.84 Hz, 3H), 1.26 (s, 16H), 1.51-1.54 (t, *J* = 6.35 Hz, 2H), 2.30 - 2.37 (m, 2H), 3.13 - 3.17 (dd, *J* = 14.67, 7.82 Hz, 1H), 3.22 - 3.26 (dd, *J* = 14.67, 5.86 Hz, 1H), 4.36 - 4.38 (t, *J* = 6.84 Hz, 1H), 7.02 (s, 1H), 7.06 - 7.09 (t, *J* = 7.33 Hz, 1H), 7.18 - 7.21 (t, *J* = 7.33 Hz, 1H), 7.32 - 7.34 (d, *J* = 8.31 Hz, 1H), 7.38 - 7.39 (d, *J* = 8.31 Hz, 2H), 7.42 - 7.44 (d, *J* = 7.82 Hz, 1H), 7.47 - 7.48 (d, *J* = 8.31 Hz, 2H), 8.34 (brs, 1H)

### (S)-4-Bromo-N-(1-(2-decanoylhydrazinyl)-3-(1H-indol-3-yl)-1-oxopropan-2-yl)benzenesulfonamide

¹H-NMR (500 MHz, CDCl₃)*δ* 0.87 - 0.89 (t, *J* = 6.35 Hz, 3H), 1.28 (s, 12H), 1.50-1.54 (m, 2H), 2.28 - 2.39 (m, 2H), 3.13 - 3.23 (dd, *J* = 14.67, 7.33 Hz, 1H), 3.23 - 3.27 (dd, *J* = 14.67, 5.38 Hz, 1H), 4.36 - 4.39 (t, *J* = 6.84 Hz, 1H), 7.02 (s, 1H), 7.06 - 7.09 (t, *J* = 7.82 Hz, 1H), 7.19 - 7.22 (t, *J* = 7.33 Hz, 1H), 7.33 - 7.34 (d, *J* = 8.31 Hz, 1H), 7.39-7.40 (d, *J* = 8.31 Hz, 2H), 7.42 - 7.44 (d, *J* = 7.82 Hz, 1H), 7.47 - 7.48 (d, *J* = 8.31 Hz, 2H), 8.33 (brs. 1H)

### (S)-2-(4-Bromophenylsulfonamido)-3-(1H-indol-3-yl)-N-phenethylpropanamide

¹H-NMR (500 MHz, CDCl₃)*δ* 2.60 - 2.73 (m, 2H), 2.93 - 2.98 (dd, *J* = 14.18, 7.82 Hz, 1H), 3.17 - 3.21 (dd, J = 14.67, 5.38 Hz, 1H), 3.33 - 3.40 (dq, *J* = 13.69, 6.84x3 Hz, 1H), 3.43 - 3.49 (dq, *J* = 13.20, 6.84x2, 6.35 Hz, 1H), 3.83 - 3.87 (q, *J* = 6.35x2, 6.84 Hz, 1H), 5.26 - 5.27 (d, J = 6.35 Hz, 1H), 6.36 - 6.38 (t, *J* = 5.38 Hz, 1H), 6.82 (s, 1H), 7.00 - 7.03 (t, *J* = 7.33 Hz, 1H), 7.06 - 7.08 (d, *J* = 7.82 Hz, 2H), 7.19 - 7.31 (m, 10H), 8.16 (brs, 1H)

### N((S)-1-(2-((3S,5S,7S)-adamantane-1-carbonyl)hydrazinyl)-3-(1H-indol-3-yl)-1-oxopropan-2-yl)-4-bromobenzenesulfonamide

¹H-NMR (500 MHz, CDCl₃)*δ* 1.66 - 1.75 (m, 6H), 1.89 (s, 6H), 2.03 (s, 3H), 3.03-3.07 (dd, J = 14.67, 9.29 Hz, 1H), 3.26 - 3.30 (dd, *J* = 15.16, 4.89 Hz, 1H), 4.27 (brs, 1H), 6.54 (brs, 1H), 6.96 - 7.01 (m, 2H), 7.13 - 7.18 (m, 3H), 7.24 - 7.32 (m, 4H), 8.32 (brs, 1H), 8.49 (brs, 1H), 9.66 (brs, 1H)

### (S)-2-(4-Bromophenylsulfonamido)-N-(4-fluorophenethyl)-3-(1H-indol-3-yl)propanamide

¹H-NMR (500 MHz, CDCl₃)*δ* 2.59 - 2.71 (m, 2H), 2.92 - 3.00 (m, 1H), 3.18 - 3.22 (dd, *J* = 14.67, 5.86 Hz, 1H), 3.31 - 3.37 (m, 1H), 3.43 - 3.49 (m, 1H), 3.83 - 3.87 (m, 1H), 5.19 - 5.22 (m, 1H), 6.41 - 6.43 (m, 1H), 6.86 - 7.04 (m, 5H), 7.20 - 7.35 (m, 7H), 8.17-7.20 (m, 1H)

### (S)-2-(4-Bromophenylsulfonamido)-3-(1H-indol-3-yl)-N-(4-methoxyphenethyl)propanamide

¹H-NMR (500 MHz, CDCl₃)*δ* 2.55 - 2.67 (m, 2H), 2.95 - 3.00 (m, 1H), 3.17 - 3.21 (m, 1H), 3.32 - 3.35 (m, 1H), 3.41 - 3.45 (m, 1H), 3.77 - 3.78 (d, *J* = 4.40 Hz, 3H), 3.86 (m, 1H), 5.21 - 5.23 (t, *J* = 4.40 Hz, 1H), 6.34 - 6.35 (d, *J* = 4.40 Hz, 1H), 6.80 - 6.82 (m, 3H), 6.98 - 7.05 (m, 3H), 7.20 - 7.33 (m, 7H), 8.17 (brs, 1H)

### (S)-2-(4-Bromophenylsulfonamido)-3-(1H-indol-3-yl)-N-(3-phenylpropyl)propanamide

¹H-NMR (500 MHz, CDCl₃)*δ* 1.69 - 1.74 (m, 2H), 2.53 - 2.56 (t, 6.84 Hz, 2H), 2.95 - 2.99 (dd, *J* = 14.67, 6.84 Hz, 1H), 3.13 - 3.19 (m, 1H), 3.22 - 3.26 (dd, *J* = 13.20, 5.86 Hz, 2H), 3.83 - 3.86 (m, 1H), 5.20 - 5.21 (d, *J* = 3.91 Hz, 1H), 6.37 (brs, 1H), 6.90 (s, 1H), 7.00 - 7.03 (m, 1H), 7.11 - 7.12 (d, *J* = 7.33 Hz, 2H), 7.17 - 7.31 (m, 10H), 8.15 (brs, 1H)

### (S)-N-((3R,5R,7R)-adamantan-1-yl)-2-(4-bromophenylsulfonamido)-3-(1H-indol-3-yl)propanamide

¹H-NMR (500 MHz, CDCl₃)*δ* 1.58 - 1.64 (m, 6H), 1.74 - 1.80 (m, 6H), 2.01 (s, 3H), 3.05 - 3.09 (dd, *J* = 14.67, 7.33 Hz, 1H), 3.15 - 3.20 (dd, *J* = 14.67, 6.84 Hz, 1H), 3.77 - 3.81 (q, *J* = 7.33, 6.84, 6.35 Hz, 1H), 5.35 - 5.36 (d, *J* = 6.35 Hz, 1H), 5.65 (s, 1H), 6.96 (d, *J* = 1.95 Hz, 1H), 7.04 - 7.07 (t, *J* = 7.82 Hz, 1H), 7.20 - 7.23 (t, *J* = 7.82 Hz, 1H), 7.33 - 7.39 (m, 6H), 8.21 (brs, 1H)

### (S)-N-(3-(1H-indol-3-yl)-1-(4-(4-methoxyphenyl)piperazin-1-yl)-1-oxopropan-2-yl)-4-bromobenzenesulfonamide

¹H-NMR (500 MHz, CDCl₃)*δ* 1.77 - 1.80 (t, *J* = 8.31 Hz, 1H), 2.34 - 2.41 (m, 2H), 2.62 - 2.66 (m, 1H), 2.78 - 2.81 (m, 1H), 2.92 - 2.97 (m, 1H), 3.10 - 3.16 (m, 2H), 3.19-3.23 (m, 1H), 3.38 - 3.42 (m, 1H), 3.76 (s, 3H), 4.48 - 4.53 (m, 1H), 6.02 - 6.04 (d, *J* = 9.78 Hz, 1H), 6.68 - 6.66 (m, 2H), 6.77 - 6.80 (m, 2H), 7.04 (d, *J* = 2.44 Hz, 1H), 7.11-7.14 (t, *J* = 7.82 Hz, 1H), 7.17 - 7.20 (t, *J* = 7.33 Hz, 1H), 7.30 - 7.31 (d, *J* = 8.31 Hz, 1H), 7.50 - 7.54 (m, 3H), 7.62 - 7.64 (d, *J* = 8.31 Hz, 2H), 8.11 (brs, 1H)

### (S)-N-(3-(1H-indol-3-yl)-1-(4-(4-methoxyphenyl)piperazin-1-yl)-1-oxopropan-2-yl)-4-methylbenzenesulfonamide

¹H-NMR (500 MHz, CDCl₃)*δ* 1.65 - 1.69 (m, 1H), 2.21 - 2.24 (m, 1H), 2.28 - 2.37 (m, 4H), 2.53 - 2.56 (m, 1H), 2.66 - 2.69 (m, 1H), 2.81 - 2.84 (m, 1H), 3.04 - 3.15 (m, 2H), 3.21 - 3.25 (dd, *J* = 13.69, 4.89 Hz, 1H), 3.32 - 3.35 (m, 1H), 3.75 (s, 3H), 4.45 - 4.50 (td, *J* = 5.38x2, 4.40 Hz, 1H), 5.91 - 5.93 (d, *J* = 9.78 Hz, 1H), 6.59 - 6.61 (d, *J* = 9.29 Hz, 2H), 6.77 - 6.79 (d, *J* = 9.29 Hz, 2H), 7.05 - 7.06 (d, *J* = 2.44 Hz, 1H), 7.10 - 7.13 (t, *J* = 7.82 Hz, 1H), 7.16 - 7.19 (t, *J* = 7.33 Hz, 1H), 7.20 - 7.22 (d, *J* = 8.31 Hz, 2H), 7.29 - 7.31 (d, *J* = 7.82 Hz, 1H), 7.51 - 7.53 (d, *J* = 7.82 Hz, 1H), 7.68 - 7.70 (d, *J* = 8.31 Hz, 2H, 8.14 (brs, 1H)

### (S)-N-((3R,5R,7R)-adamantan-1-yl)-3-(1H-indol-3-yl)-2-(4-methylphenylsulfonamido)propanamide

¹H-NMR (500 MHz, CDCl₃)*δ* 1.57 - 1.64 (m, 6H), 1.74 - 1.80 (m, 6H), 2.00 (brs, 3H), 2.34 (s, 3H), 3.04 - 3.08 (m, 1H), 3.12 - 3.16 (m, 1H), 3.78 - 3.82 (q, *J* = 6.84, 6.35x2 Hz, 1H), 5.13 - 5.14 (d, *J* = 6.35 Hz, 1H), 5.75 (s, 1H), 6.94 - 6.95 (d, *J* = 1.46 Hz, 1H), 7.01 - 7.04 (t, *J* = 7.33 Hz, 1H), 7.08 - 7.09 (d, *J* = 7.82 Hz, 2H), 7.15 - 7.19 (m, 1H), 7.32 - 7.36 (t, *J* = 8.80 Hz, 2H), 7.48 - 7.50 (d, *J* = 8.31 Hz, 2H), 8.27 (brs, 1H)

### (S)-3-(1H-indol-3-yl)-N-(4-methoxyphenethyl)-2-(4-methylphenylsulfonamido)propanamide

¹H-NMR (500 MHz, CDCl₃)*δ* 2.33 (s, 3H), 2.50 - 2.63 (m, 2H), 3.01 - 3.10 (m, 2H), 3.30 - 3.40 (m, 2H), 3.77 (s, 3H), 3.85 - 3.89 (q, *J* = 6.35 Hz, 1H), 5.03 - 5.04 (d, *J* = 5.86 Hz, 1H), 6.36 - 6.38 (t, 5.38 Hz, 1H), 6.77 - 6.81 (m, 3H), 6.93 - 6.95 (d, *J* = 8.80 Hz, 2H), 6.98 - 7.01 (t, *J* = 7.33 Hz, 1H), 7.04 - 7.06 (d, *J* = 7.82 Hz, 2H), 7.15 - 7.18 (t, *J* = 7.82 Hz, 1H), 7.26 - 7.31 (m, 2H), 7.43 - 7.45 (d, *J* = 8.31 Hz, 2H), 8.22 (brs, 1H)

### (S)-3-(1H-indol-3-yl)-2-(4-methylphenylsulfonamido)-N-(3-phenylpropyl)propanamide

¹H-NMR (500 MHz, DMSO-*d₆*) *δ* 1.40 - 1.46 (m, 2H), 2.23 (s, 3H), 2.34 - 2.37 (t, *J* = 7.33 Hz, 2H), 2.75 - 2.83 (m, 3H), 2.94 - 2.99 (dd, *J* = 14.18, 6.84 Hz, 1H), 3.89 - 3.92 (t, *J* = 6.84 Hz, 1H), 6.90 - 6.93 (t, *J* = 7.33 Hz, 1H), 7.01 - 7.04 (t, *J* = 7.33 Hz, 1H), 7.07 - 7.17 (m, 6H), 7.24 - 7.28 (m, 3H), 7.34 - 7.36 (d, *J* = 7.82 Hz, 1H), 7.48 - 7.49 (d, *J* = 7.82 Hz, 2H), 7.85 - 7.87 (t, *J* = 5.38 Hz, 1H), 7.93 (brs, 1H), 10.77 (s, 1H)

### (S)-N-(4-fluorophenethyl)-3-(1H-indol-3-yl)-2-(4-methylphenylsulfonamido)propanamide

¹H-NMR (500 MHz, CD₃OD) *δ* 2.28 (s, 3H), 2.49 - 2.54 (m, 2H), 2.80 - 2.84 (dd, J = 14.18, 7.82 Hz, 1H), 3.07 - 3.14 (m, 2H), 3.23 - 3.27 (m, 1H), 3.86 - 3.89 (t, *J* = 7.82 Hz, 1H), 6.92 - 7.06 (m, 9H), 7.26 - 7.27 (d, *J* = 7.82 Hz, 1H), 7.31 - 7.33 (d, *J* = 7.82 Hz, 1H), 7.40 - 7.42 (d, *J* = 7.82 Hz, 2H)

### (S)-3-(1H-indol-3-yl)-2-(4-methylphenylsulfonamido)-N-(4-(4-(trifluoromethyl)phenyl)thiazol-2-yl)propanamide

¹H-NMR (500 MHz, DMSO-*d₆*) *δ* 2.15 (s, 3H), 2.85 - 2.90 (dd, J= 14.67, 7.82 Hz, 1H), 3.07 - 3.11 (dd, *J* = 14.67, 6.84 Hz, 1H), 4.28 - 4.31 (t, *J* = 7.33 Hz, 1H), 6.89 - 6.92 (t, *J* = 7.33 Hz, 1H), 7.01 - 7.06 (m, 4H), 7.26 - 7.28 (d, *J* = 8.31 Hz, 1H), 7.40 - 7.42 (d, *J* = 7.82 Hz, 1H), 7.45 - 7.47 (d, *J* = 8.31 Hz, 2H), 7.78 - 7.80 (m, 3H), 8.06 - 8.08 (d, *J* = 8.31 Hz, 2H), 8.27 (brs, 1H), 10.78 (s, 1H), 12.39 (brs, 1H)

### (S)-N-(2-(1H-indol-3-yl)ethyl)-2-(4-bromophenylsulfonamido)-3-(1H-indol-3-yl)propanamide

¹H-NMR (500 MHz, CD₃OD) *δ* 2.72 - 2.84 (m, 3H), 3.14 - 3.18 (dd, *J* = 14.67, 5.38 Hz, 1H), 3.27 - 3.30 (m, 1H), 3.38 - 3.42 (m, 1H), 3.92 - 3.95 (dd, 9.29, 5.38 Hz, 1H), 6.93 - 6.96 (m, 2H), 6.99 - 7.03 (m, 2H), 7.07 - 7.11 (m, 2H), 7.22 - 7.24 (m, 2H), 7.27 - 7.29 (m, 3H), 7.32 - 7.36 (dd, *J* = 7.82, 4.40 Hz, 2H), 7.52 - 7.53 (d, *J* = 7.82 Hz, 1H)

### (S)-N-(3-(1H-indol-3-yl)-1-((4-(4-methoxyphenyl)thiazol-2-yl)amino)-1-oxopropan-2-yl)-4-methylbenzamide

¹H-NMR (500 MHz, DMSO-*d₆*) *δ* 2.33 (s, 3H), 3.23 - 3.34 (m, 2H), 3.78 (s, 3H), 4.95 - 4.99 (m, 1H), 6.97 - 7.00 (m, 3H), 7.04 - 7.07 (t, *J* = 7.82 Hz, 1H), 7.24 - 7.26 (d, *J* = 7.82 Hz, 2H), 7.30 - 7.32 (m, 2H), 7.45 (s, 1H), 7.73 - 7.75 (d, *J* = 7.82 Hz, 2H), 7.83 - 7.84 (d, *J* = 8.80 Hz, 3H), 8.62 - 8.63 (d, *J* = 7.82 Hz, 1H), 10.84 (s, 1H), 12.64 (s, 1H)

### (S)-2-(4-Chlorophenylsulfonamido)-3-(1H-indol-3-yl)-N-(4-(4-methoxyphenyl)thiazol-2-yl)propanamide

¹H-NMR (500 MHz, DMSO-*d₆*) *δ* 2.86 - 2.91 (dd, *J* = 14.18, 8.80 Hz, 1H), 3.08 - 3.12 (dd, *J* = 14.67, 6.35 Hz, 1H), 3.78 (s, 3H), 4.33 (brs, 1H), 6.90 - 6.93 (t, *J* = 7.33 Hz, 1H), 6.97 - 6.99 (d, *J* = 8.80 Hz, 2H), 7.02 - 7.05 (t, *J* = 7.33 Hz, 1H), 7.08 (d, *J* = 1.95 Hz, 1H), 7.22 - 7.24 (d, *J* = 8.80 Hz, 2H), 7.26 - 7.27 (d, *J* = 8.31 Hz, 1H), 7.43 (s, 1H), 7.46 - 7.48 (d, *J* = 8.80 Hz, 3H), 7.79 - 7.81 (d, *J* = 8.80 Hz, 2H), 8.50 (brs, 1H), 10.78 (s, 1H), 12.44 (brs, 1H)

### (S)-3-(1H-indol-3-yl)-N-(4-(4-methoxyphenyl)thiazol-2-yl)-2-(4-(trifluoromethyl)phenylsulfonamido)propanamide

¹H-NMR (500 MHz, DMSO-*d₆*) *δ* 2.88 - 2.93 (dd, *J* = 14.67, 9.29 Hz, 1H), 3.09-3.13 (dd, *J* = 14.18, 5.18 Hz, 1H), 3.78 (s, 3H), 4.37 (brs, 1H), 6.88 - 6.91 (t, *J* = 7.33 Hz, 1H), 6.97 - 7.02 (m, 3H), 7.10 (d, *J* = 1.95 Hz, 1H), 7.22 - 7.24 (d, *J* = 8.31 Hz, 1H), 7.42 (s, 1H), 7.48 - 7.53 (m, 3H), 7.65 - 7.67 (d, *J* = 7.82 Hz, 2H), 7.79 - 7.80 (d, *J* = 8.31 Hz, 2H), 8.70 (brs, 1H), 10.77 (s, 1H), 12.46 (brs, 1H)

### (S)-2-(4-Fluorophenylsulfonamido)-3-(1H-indol-3-yl)-N-(4-(4-methoxyphenyl)thiazol-2-yl)propanamide

¹H-NMR (500 MHz, CD₃OD) *δ* 2.87 - 2.92 (dd, *J* = 14.67, 8.80, Hz, 1H), 3.08 - 3.12 (dd, *J* = 14.18, 6.35 Hz, 1H), 3.76 (s, 3H), 4.28 - 4.31 (dd, *J* = 8.31, 6.84 Hz, 1H), 6.89 - 6.92 (m, 1H), 6.96 - 7.06 (m, 4H), 7.08 (s, 1H), 7.26 - 7.28 (m, 1H), 7.39 (s, 1H), 7.46 - 7.47 (d, *J* = 7.82 Hz, 1H), 7.54 - 7.58 (m, 2H), 7.78 - 7.81 (m, 2H), 10.82 (brs, 1H)

### (S)-2-(4-Bromophenylsulfonamido)-N-((1r,3R,5S,7S)-3,5-dimethyladamantan-1-yl)-3-(1H-indol-3-yl)propanamide

¹H-NMR (500 MHz, CDCl₃)*δ* 0.80 (s, 6H), 1.08 (s, 2H), 1.22 - 1.43 (m, 8H), 1.59 (s, 2H), 2.07 (s, 1H), 3.03 - 3.07 (dd, *J* = 14.67, 7.33 Hz, 1H), 3.16 - 3.20 (dd, *J* = 14.67, 6.84 Hz, 1H), 3.77 - 3.80 (m, 1H), 5.36 - 5.37 (d, *J* = 6.35 Hz, 1H), 5.70 (s, 1H), 6.97 (s, 1H), 7.03 - 7.06 (t, *J* = 7.33 Hz, 1H), 7.20 - 7.23 (t, *J* = 7.33 Hz, 1H), 7.32 - 7.38 (m, 4H), 7.63 - 7.64 (d, *J* = 8.80 Hz, 1H), 7.77 - 7.79 (d, *J* = 8.31 Hz, 1H), 8.20 (s, 1H)

### (S)-2-(4-Cyanophenylsulfonamido)-3-(1H-indol-3-yl)-N-(4-(4-methoxyphenyl)thiazol-2-yl)propanamide

¹H-NMR (500 MHz, DMSO-*d₆*) *δ* 2.85 - 2.90 (dd, *J* = 14.67, 9.78 Hz, 1H), 3.05-3.09 (dd, *J* = 14.18, 5.38 Hz, 1H), 3.78 (s, 3H), 4.34 (brs, 1H), 6.89 - 6.92 (t, *J* = 7.82 Hz, 1H), 6.98 - 7.04 (m, 3H), 7.07 - 7.08 (d, *J* = 1.95 Hz, 1H), 7.22 - 7.23 (d, *J* = 8.31 Hz, 1H), 7.46 - 7.52 (m, 6H), 7.82 - 7.83 (d, *J =* 8.80 Hz, 2H), 8.74 (brs, 1H), 10.72 - 10.72 (d, *J* = 1.46 Hz, 1H), 12.58 (brs, 1H)

### (S)-Benzyl3-(1H-indol-3-yl)-2-(4-methylbenzamido)propanoate

¹H-NMR (500 MHz, CDCl₃)*δ* 3.36 - 3.43 (m, 2H), 5.03 - 5.10 (m, 2H), 5.15 - 5.18 (dt, *J* = 7.82, 5.38x2 Hz, 1H), 6.69 - 6.70 (d, *J* = 2.44 Hz, 1H), 6.76 - 6.78 (d, *J* = 7.82 Hz, 1H), 7.01 - 7.04 (t, *J* = 7.33 Hz, 1H), 7.07 - 7.08 (d, *J* = 8.31 Hz, 2H), 7.10 - 7.13 (t, *J* = 7.33 Hz, 1H), 7.21 - 7.31 (m, 6H), 7.50 - 7.51 (d, *J =* 7.82 Hz, 1H), 7.53 - 7.54 (d, *J =* 8.31 Hz, 2H), 8.64 (brs, 1H)

### (S)-2-(4-Bromophenylsulfonamido)-3-(5-hydroxy-1H-indol-3-yl)-N-(4-(4-methoxyphenyl)thiazol-2-yl)propanamide

¹H-NMR (500 MHz, OMSO-*d₆*) *δ* 2.79 - 2.83 (dd, *J =* 14.18, 8.31 Hz, 1H), 2.99-3.03 (dd, *J =* 14.18, 6.84 Hz, 1H), 3.77 (s, 3H), 4.31 - 4.34 (q, *J =* 8.31, 7.82, 7.33 Hz, 1H), 6.58 - 6.60 (dd, *J =* 8.80, 2.44 Hz, 1H), 6.86 (d, *J* = 1.95 Hz, 1H), 6.97 - 6.99 (m, 3H), 7.06 - 7.07 (d, *J* = 8.80 Hz, 1H), 7.40 - 7.46 (m, 4H), 7.77 - 7.84 (m, 2H), 8.46 - 8.48 (d, *J* = 8.31 Hz, 1H), 8.61 (brs, 1H), 10.47 - 10.48 (d, *J =* 1.95 Hz, 1H), 12.4 (s, 1H)

### (S)-2-(4-Bromophenylsulfonamido)-N-(4-(4-methoxyphenyl)thiazol-2-yl)-3-(5-methyl-1H-indol-3-yl)propanamide

¹H-NMR (500 MHz, DMSO-*d₆*) *δ* 2.33 (s, 3H), 2.82 - 2.86 (dd, *J* = 14.18, 8.31 Hz, 1H), 3.04 - 3.09 (dd, *J =* 14.67, 6.84 Hz, 1H), 3.77 (s, 3H), 4.29 (brs, 1H), 6.84 - 6.86 (d, *J =* 8.31 Hz, 1H), 6.97 - 7.02 (m, 3H), 7.14 - 7.16 (m, 2H), 7.41 - 7.45 (m, 5H), 7.79 - 7.81 (d, *J* = 8.31 Hz, 2H), 8.47 (brs, 1H), 10.64 (s, 1H), 12.40 (brs, 1H)

### (S)-2-(4-Bromophenyisulfonamido)-3-(5-fluoro-1H-indol-3-yl)-N-(4-(4-methoxyphenyl)thiazol-2-yl)propanamide

¹H-NMR (500 MHz, DMSO-*d₆*) *δ* 2.97 - 3.02 (dd, *J* = 14.18, 8.80 Hz, 1H), 3.17-3.22 (dd, *J* = 14.18, 5.86 Hz, 1H), 3.81 (s, 3H), 4.24 - 4.27 (dd, *J =* 8.80, 6.35 Hz, 1H), 4.62 (s, 1H), 6.81 - 6.85 (td, *J* = 9.29×2, 2.44 Hz, 1H), 6.92 - 6.94 (d, *J =* 8.80 Hz, 2H), 7.08 (s, 1H), 7.12 - 7.14 (dd, *J* = 10.27, 1.95 Hz, 1H), 7.17 (s, 1H), 7.21 - 7.22 (dd, *J* = 8.80, 4.40 Hz, 1H), 7.27 - 7.29 (d, *J* = 8.31 Hz, 2H), 7.41 - 7.43 (d, *J* = 8.31 Hz, 2H), 7.77 - 7.79 (d, *J* = 8.31 Hz, 2H)

### (S)-4-(2-(3-(1H-indol-3-yl)-2-(4-methylphenylsulfonamido)propanamido)ethyl)benzoic acid

¹H-NMR (500 MHz, OMSO-*d₆*) *δ* 2.28 (s, 3H), 2.54 - 2.64 (m, 2H), 2.80 - 2.85 (dd, *J* = 14.18, 7.82 Hz, 1H), 3.09 - 3.20 (m, 2H), 3.26 - 3.30 (dd, *J* = 13.20, 6.35 Hz, 1H), 3.86 - 3.89 (dd, *J* = 7.82, 6.35 Hz, 1H), 6.90 - 6.93 (t, *J =* 7.82 Hz, 1H), 6.95 (s, 1H), 7.00-7.02 (d, *J* = 8.31 Hz, 2H), 7.04 - 7.07 (t, *J* = 6.84 Hz, 1H), 7.14 - 7.15 (d, *J* = 8.31 Hz, 2H), 7.26 - 7.27 (d, *J* = 7.82 Hz, 1H), 7.31 - 7.32 (d, *J* = 7.82 Hz, 1H), 7.40 - 7.41 (d, *J* = 8.31 Hz, 2H), 7.90 - 7.91 (d, *J* = 7.82 Hz, 3H), 10.22 (s, 1H)

### (S)-N-(4-bromophenethyl)-3-(1H-indol-3-yl)-2-(4-methylphenylsulfonamido)propanamide

¹H-NMR (500 MHz, DMSO-*d₆*) *δ* 2.27 (s, 3H), 2.36 - 2.43 (m, 2H), 2.69 - 2.73 (dd, *J* = 14.18, 7.82 Hz, 1H), 2.88 - 2.98 (m, 2H), 3.03 - 3.08 (dt, *J* = 13.20, 6.84, 6.35 Hz, 1H), 3.82 - 3.85 (t, *J* = 6.84 Hz, 1H), 6.89 - 6.92 (t, *J* = 7.33 Hz, 1H), 6.97 - 6.99 (d, 8.31 Hz, 2H), 7.01 - 7.04 (t, *J* = 7.33 Hz, 2H), 7.12 - 7.14 (d, *J* = 8.31 Hz, 2H), 7.28 - 7.32 (dd, *J* = 11.24, 8.31 Hz, 2H), 7.37 - 7.39 (d, *J =* 7.33 Hz, 2H), 7.44 - 7.46 (d, *J =* 7.82 Hz, 2H), 7.91 - 7.93 (t, *J =* 5.38 Hz, 2H), 10.75 (brs, 1H)

### (S)-N-(2-(1H-indol-3-yl)ethyl)-3-(1H-indol-3-yl}-2-(4-methylphenylsulfonamido)propanamide

¹H-NMR (500Mz, CD₃OD) *δ* 2.24 (s, 3H), 2.64 - 2.76 (m, 2H), 2.82 - 2.86 (dd, *J* = 14.67, 8.31 Hz, 1H), 3.11 - 3.15 (dd, *J* = 14.67, 5.86 Hz, 1H), 3.20 - 3.26 (m, 1H), 3.30-3.36 (m, 1H), 3.89 - 3.91 (dd, *J* = 8.31, 6.35 Hz, 1H), 6.91 - 7.01 (m, 6H), 7.04 - 7.10 (m, 2H), 7.25 - 7.27 (d, *J* = 7.82 Hz, 1H), 7.32 - 7.35 (m, 2H), 7.36 - 7.38 (d, *J* = 8.31 Hz, 2H), 7.48 - 7.49 (d, *J* = 7.82 Hz, 1H)

### (S)-3-(1H-indol-3-yl)-N-(4-methylphenethyl)-2-(4-methylphenylsulfonamido)propanamide

¹H-NMR (500 MHz, CD₃OD) *δ* 2.27 - 2.27 (d, *J* = 4.89 Hz, 6H), 2.43 - 2.54 (m, 2H), 2.80 - 2.85 (dd, *J* = 14.18, 8.13 Hz, 1H), 3.07 - 3.14 (m, 2H), 3.21 - 3.26 (m, 1H), 3.86-3.89 (t, *J* = 6.84 Hz, 1H), 6.91 - 7.07 (m, 9H), 7.25 - 7.27 (d, *J* = 7.82 Hz, 1H), 7.32 - 7.33 (d, *J* = 7.82 Hz, 1H), 7.40 - 7.41 (d, *J* = 7.82 Hz, 2H)

### Ethyl 2-((S)-3-(1H-indol-3-yl)-2-(4-methylphenylsulfonamido)propanamido)-3-(1H-indol-3-yl)propanoate

¹H-NMR (500 MHz, CD₃OD) *δ* 1.07 - 1.10 (t, *J =* 7.33 Hz, 3H), 2.18 (s, 3H), 2.80-2.85 (dd, *J* = 14.67, 8.80 Hz, 1H), 3.06 - 3.18 (m, 3H), 3.97 - 4.05 (m, 3H), 4.53 - 4.55 (t, *J* = 6.84 Hz, 1H), 6.82 - 6.84 (d, *J* = 7.82 Hz, 2H), 6.90 - 6.94 (m, 1H), 6.96 (s, 1H), 7.00 - 7.06 (m, 3H), 7.10 - 7.13 (td, *J* = 7.82,7.33 Hz, 1H), 7.24 - 7.26 (d, *J* = 7.82 Hz, 1H), 7.29 - 7.30 (d, *J* = 8.31 Hz, 2H), 7.34 - 7.36 (dt, *J* = 8.31×2 Hz, 2H), 7.45 - 7.46 (d, *J* = 7.82 Hz, 1H)

### (S)-N-(4-chlorophenethyl)-3-(1H-indol-3-yl)-2-(4-methylphenylsulfonamido)propanamide

¹H-NMR (500 MHz, CD₃OD) *δ* 2.29 (s, 3H), 2.46 - 2.57 (m, 2H), 2.80 - 2.84 (dd, *J* = 14.18, 7.82 Hz, 1H), 3.07 - 3.14 (m, 2H), 3.24 - 3.29 (m, 1H), 3.86 - 3.89 (t, *J =* 6.35 Hz, 1H), 6.91 - 6.96 (m, 2H), 7.02 - 7.08 (m, 5H), 7.19 - 7.21 (d, *J =* 8.31 Hz, 2H), 7.26 - 7.28 (d, *J* = 7.82 Hz, 1H), 7.31 - 7.33 (d, *J* = 7.82 Hz, 1H), 7.41 - 7.42 (d, *J* = 7.82 Hz, 2H)

### 2-((S)-3-(1H-indol-3-yl)-2-(4-methylphenylsulfonamido)propanamido)-3-(1H-indol-3-yl)propanoic acid

¹H-NMR (500 MHz, CD₃OD) *δ* 2.18 (s, 3H), 2.57 - 2.62 (dd, *J* = 14.67, 10.27, Hz, 1H), 3.12 - 3.21 (dd, *J* = 14.67, 3.42 Hz, 1H), 3.21 - 3.25 (dd, *J* = 14.67, 6.35 Hz, 1H), 3.37 - 3.41 (dd, *J* = 14.67, 4.89 Hz, 1H), 3.87 - 3.89 (dd, *J* = 9.78, 3.91 Hz, 1H), 4.51-4.53 (t, *J* = 5.86 Hz, 1H), 6.72 - 6.74 (d, *J* = 8.31 Hz, 2H), 6.84 (s, 1H), 6.86 - 6.89 (t, *J* = 7.82 Hz, 1H), 6.97 - 7.00 (t, *J* = 7.82 Hz, 1H), 7.02 - 7.09 (m, 2H), 7.13 - 7.15 (m, 3H), 7.20 - 7.21 (d, *J* = 7.82 Hz, 1H), 7.29 - 7.32 (dd, *J* = 7.33, 6.84 Hz, 2H), 7.59 - 7.61 (d, *J* = 7.82 Hz, 1H)

### (S)-Methyl 4-(2-(3-(1H-indol-3-yl)-2-(4-methylphenylsulfonamido)propanamido)thiazol-4-yl)benzoate

¹H-NMR (500 MHz, DMSO-*d₆*) *δ* 2.16 (s, 3H), 2.49 - 2.50 (dd, *J* = 14.18, 7.82, 6.35 Hz, 1H), 3.07 - 3.11 (dd, *J* = 14.18, 7.33, 6.84 Hz, 1H), 3.85 (s, 3H), 4.30 (brs, 1H), 6.89 - 6.92 (t, *J* = 7.33 Hz, 1H), 7.01 - 7.07 (m, 4H), 7.26 - 7.28 (d, *J* = 7.82 Hz, 1H), 7.41-7.42 (d, *J* = 7.82 Hz, 1H), 7.45 - 7.47 (d, *J* = 8.31 Hz, 2H), 7.80 (s, 1H), 8.01 (s, 4H), 8.27 (brs, 1H), 10.79 (s, 1H), 12.41 (brs, 1H)

### (S)-4-(2-(3-(1H-indol-3-yl)-2-(4-methylphenylsulfonamido)propanamido)thiazol-4-yl)benzoic acid

¹H-NMR (500 MHz, DMSO-*d₆*) *δ* 2.18 (s, 3H), 2.87 - 2.91 (dd. *J* = 14.18, 7.82 Hz, 1H), 3.09 - 3.13 (dd, *J* = 14.18, 6.84 Hz, 1H), 4.31 - 4.36 (q, *J =* 7.82x3 Hz, 1H), 6.91 - 6.94 (t, *J* = 7.33 Hz, 1H), 7.03 - 7.09 (m, 4H), 7.28 - 7.30 (d, *J =* 7.82 Hz, 1H), 7.43 - 7.44 (d, *J =* 7.82 Hz, 1H), 7.47 - 7.49 (d, *J =* 7.82 Hz, 2H), 7.80 (s, 1H), 7.99 - 8.03 (m, 4H), 8.28 - 8.30 (d, *J* = 8.80 Hz, 1H), 10.81 (s, 1H), 12.43 (s, 1H), 12.98 (brs, 1H)

### (S)-3-(Benzo[d]oxazol-2-ylamino)-N-(4-(4-methoxyphenyl)thiazol-2-yl)-2-(4-methylphenylsulfonamido)propanamide

¹H-NMR (500 MHz, DMSO-*d₆*) *δ* 2.13 (s, 3H), 3.51 - 3.55 (m, 2H), 3.79 (s, 3H), 4.35 - 4.36 (d, *J* = 5.38 Hz, 1H), 6.98 - 7.01 (m, 3H), 7.11 - 7.15 (m, 3H), 7.22 - 7.24 (d, *J* = 7.82 Hz, 1H), 7.30 - 7.32 (d, *J* = 7.82 Hz, 1H), 7.44 (s, 1H), 7.65 - 7.66 (d, *J* = 8.31 Hz, 2H), 7.80 - 7.82 (d, *J* = 8.31 Hz, 2H), 7.99 - 8.01 (t, *J* = 6.35 Hz, 1H), 8.20 - 8.21 (d, *J* = 5.86 Hz, 1H), 12.33 (brs, 1H)

### (S)-N-(3-((4-(4-methoxyphenyl)thiazol-2-yl)amino)-2-(4-methylphenylsulfonamido)-3-oxopropyl)benzamide

¹H-NMR (500 MHz, CD₃OD) *δ* 2.20 (s, 3H), 3.63 - 3.67 (m, 1H), 3.73 - 3.75 (m, 1H), 3.88 (s, 3H), 4.33 - 4.35 (t, *J* = 6.35 Hz, 1H), 6.99 - 7.01 (d, *J* = 8.80 Hz, 2H), 7.17-7.18 (d, *J* = 7.82 Hz, 2H), 7.24 (s, 1H), 7.47 - 7.50 (m, 2H), 7.57 - 7.60 (m, 1H), 7.74-7.78 (dd, *J* = 14.18, 7.82 Hz, 4H), 7.85 - 7.86 (d, *J* = 8.31 Hz, 2H)

### (S)-N-(3-((4-(4-methoxyphenyl)thiazol-2-yl)amino)-2-(4-methylphenylsulfonamido)-3-oxopropyl)nicotinamide

¹H-NMR (500 MHz, DMSO-*d₆*) *δ* 2.14 (s, 3H), 3.48 - 3.53 (dt, *J =* 13.20, 6.84, 6.35 Hz, 1H), 3.56 - 3.63 (m, 1H), 3.78 (s, 3H), 4.23 - 4.26 (t, *J* = 6.84 Hz, 1H), 6.97 - 6.99 (d, *J* = 8.80 Hz, 2H), 7.14 - 7.16 (d, *J* = 8.31 Hz, 2H), 7.42 (s, 1H), 7.45 - 7.48 (dd, *J* = 7.82, 4.89, 2.93 Hz, 1H), 7.64 - 7.66 (d, *J* = 8.31 Hz, 2H), 7.79 - 7.80 (d, *J* = 8.80 Hz, 2H), 8.01 - 8.03 (dt, *J* = 7.82, 1.95x2 Hz, 1H), 8.11 (brs, 1H), 8.65 - 8.68 (m, 2H), 8.84 - 8.85 (d, *J* = 1.46 Hz, 1H), 12.28 (brs, 1H)

### (S)-N-(3-((4-(4-methoxyphenyl)thiazol-2-yl)amino)-2-(4-methylphenylsulfonamido)-3-oxopropyl)pyrimidine-5-carboxamide

¹H-NMR (500 MHz, DMSO-*d₆*) *δ* 2.16 (s, 3H), 3.49 - 3.53 (dt, *J* = 13.69, 6.84 × 2 Hz, 1H), 3.57 - 3.63 (dt, *J =* 13.20, 6.35x2 Hz, 1H), 3.79 (s, 3H), 4.23 - 4.26 (m, 1H), 6.98 - 7.00 (d, *J =* 8.80 Hz, 2H), 7.17 - 7.19 (d, *J =* 8.31 Hz, 2H), 7.45 (s, 1H), 7.65 - 7.67 (d, *J* = 8.31 Hz, 2H), 7.79 - 7.81 (d, *J* = 8.80 Hz, 2H), 8.18 - 8.19 (d, *J* = 7.82 Hz, 1H), 8.87-8.89 (t, *J =* 5.86 Hz, 1H), 9.00 (s, 2H), 9.31 (s, 1H), 12.31 (brs, 1H)

### Example 2: DX2-Nanoluciferase activity (40uM) and Cell viability(% inhibition)

After cloning DX2 into a plasmid expressing nanoluciferase, the plasmid was transfected into A549 cells. Transfected cells were seeded 1×10⁴ per well in a 96-well plate and stabilized for 12 hours. The compound was treated for 4 hours, and then the expression change of DX2 could be observed through luciferase assay. Cell viability was measured by the MTT method after 72 hours of treatment with each concentration of the compound in A549 cells.

The results are shown in Table 1 above.

### Example 3: AIMP2-DX2 and AIMP2 inhibition evaluation (nanoluciferase assay)

Compound No. 63 was selected from among the compounds showing excellent effects in Table 1, and the following experiment was performed.

In order to calculate the IC50 of compound No. 63, A549 cells expressing nanoluciferase-DX2 or -AIMP2 were treated with compound No. 63 at each concentration for 4 hours, and then fluorescence was measured.

The results for this are shown in FIG. 1.

As shown in FIG. 1, it was confirmed that the inhibition rate for AIMP2-DX2 and AIMP2 of compound No.63 shows 100-fold or more selectivity for AIMP2-DX2 (DX2 IC50 = 0.92 µM and AIMP2 IC50 >100 µM).

### Example 4: AIMP2-DX2 inhibition mechanism study

As previously reported, AIMP2-DX2 is stabilized by binding to HSP70. Therefore, the present inventors checked whether the compound No. 63, which showed the best AIMP2-DX2 inhibitory ability, can inhibit the binding of AIMP2-DX2 and HSP70. Specifically, an in vitro pull-down assay was performed in the presence of compound No. 63 using purified AIMP2-DX2 and HSP70 protein.

The results for this are shown in FIG. 2.

As shown in FIG. 2, it was confirmed that compound No. 63 inhibited the binding of AIMP2-DX2 and HSP70 in a concentration-dependent manner.

### Example 5: In vivo anti-cancer efficacy evaluation

Next, the anti-cancer efficacy of compound No. 63 was evaluated in a mouse model transplanted with H460 cells.

Specifically, the compound No. 63 was intraperitoneally administered to mice transplanted with H460 cells at 50 mg/kg, once every two days for a total of 15 days.

The results are shown in FIGs 3a to 3d.

As shown in FIGs 3a to 3d, compound No. 63 significantly inhibited the growth of tumors, and it was confirmed that there was no change in the body weight of the animals during the administration period.

### Exampl 6: Inhibitory effect of compound No. 55 and compound No. 89 on cancer growth in vivo

Additionally, compounds No. 55 and No. 88 were selected from Table 1, and an *in vivo* cancer growth inhibitory effect experiment was performed.

H460 cells (2×10⁷) were subcutaneously implanted in 7-week-old BALB/cAnCr female rats, and then Compound No. 55 or Compound No. 89 (50 mg/kg) and Taxol (10 mg/kg) were administered intraperitoneally once every 2 days. The drug was administered for 16 days, and the size of the tumor and the weight of the animal were measured during administration. After 16 days, the animals were sacrificed and the size and weight of the removed tumors were measured.

The results for this are shown in FIG. 4.

At the treated concentration, compound No. 55 showed about 50% cancer growth inhibitory effect during the treatment period, and it is judged that there is no side effect of the compound as there is no change in the body weight of the animal during the treatment period.

### INDUSTRIAL APPLICABILITY

The compound represented by Formula 1 according to the present invention is very effective in inhibiting the expression of AIMP2-DX2, so it can be very usefully used in the development of various diseases caused by AIMP2-DX2, in particular, a therapeutic agent for cancer.

## Claims

1. A compound represented by the following formula 1 or a pharmaceutically acceptable salt thereof: 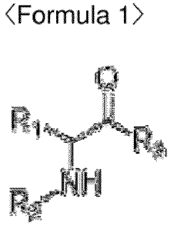 wherein Formula 1,
R1 is hydrogen; C1~C5 straight or branched alkyl substituted or unsubstituted with one or more substituents selected from the group consisting of hydroxy, substituted or unsubstituted phenyl, mercapto, and C1~C5 alkylthio; Indolylmethyl unsubstituted or substituted with one or more substituents selected from the group consisting of C1~C5 alkyl, hydroxy, hydroxycarbonyl and formyl; C1~C5 carboxymethyl; C7~C10 hydroxyphenylalkyl; substituted or unsubstituted C3~C8 cycloalkyl; substituted or unsubstituted C1~C5 aminoalkyl; 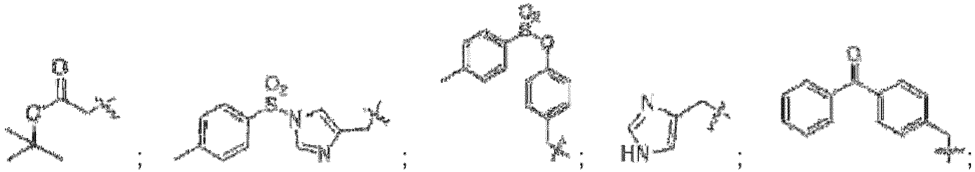 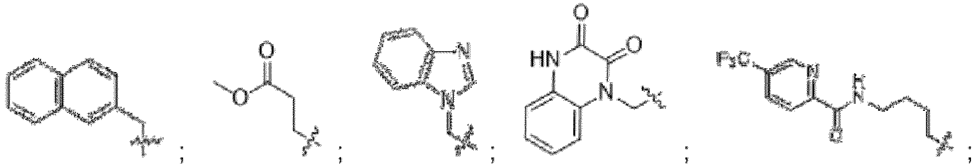 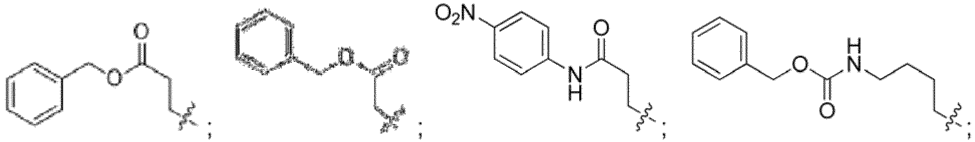 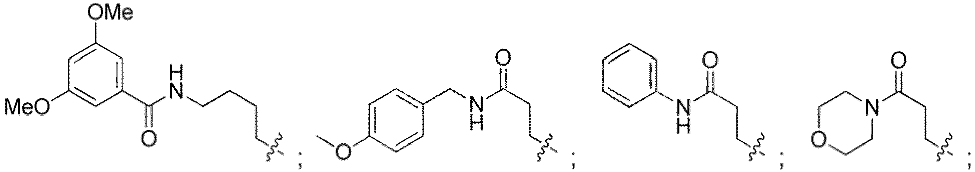 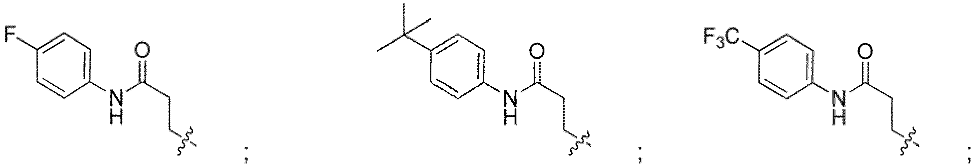 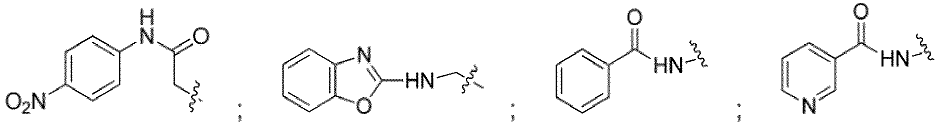 or 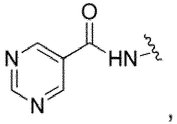
R2 is hydrogen; benzenesulfonyl unsubstituted or substituted with one or more substituents selected from the group consisting of C1~C5 straight or branched alkyl, nitro group, amine group, halo, -CF₃, C1~C5 alkylcarbonylamino, -COOH, C1~C5 alkoxy, phenyl, -OCH₂Ph, hydroxy, -GOOGH₂Ph, C1~C5 alkyloxycarbonyl, -CONHCH₃ and nitrile; naphthalenesulfonyl; benzylsulfonyl; benzoyl unsubstituted or substituted with one or more substituents selected from the group consisting of C1~C5 straight or branched alkyl and C1~C5 straight or branched alkylamine groups; thiophenesulfonyl; 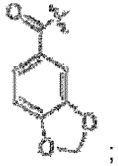 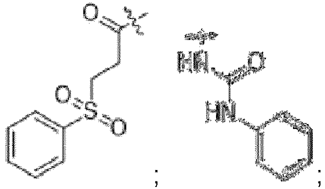 C1~C5 alkyloxycarbonyl; or phenylaminocarbonyl,
R3 is

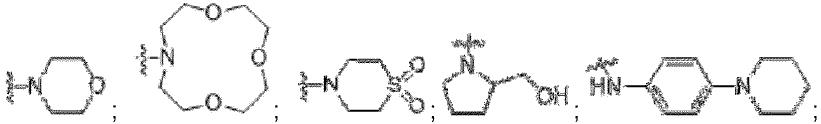

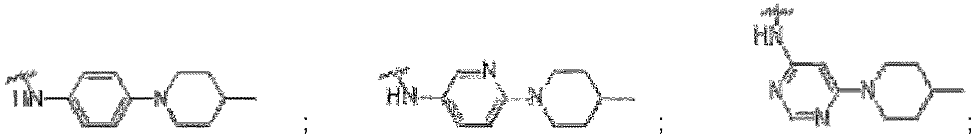

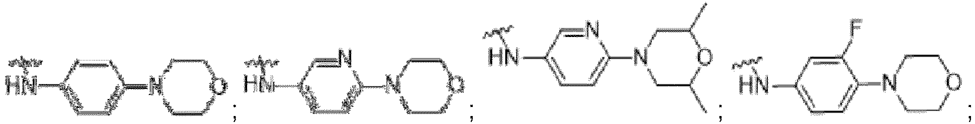

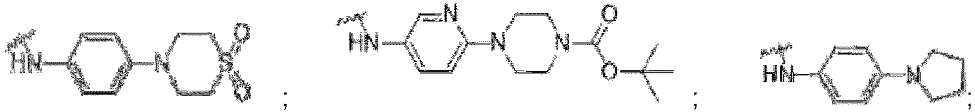

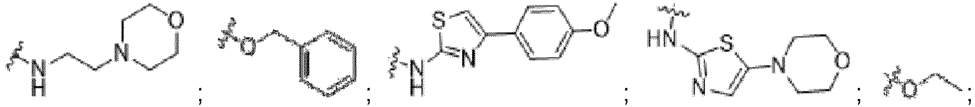

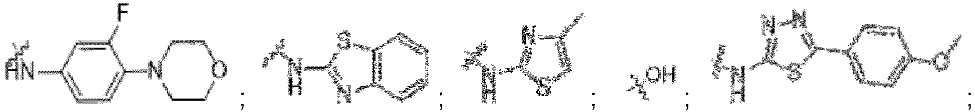

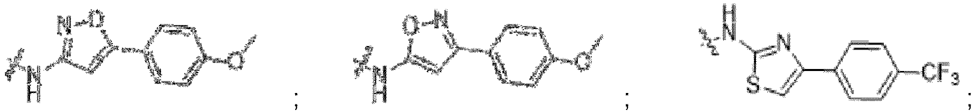

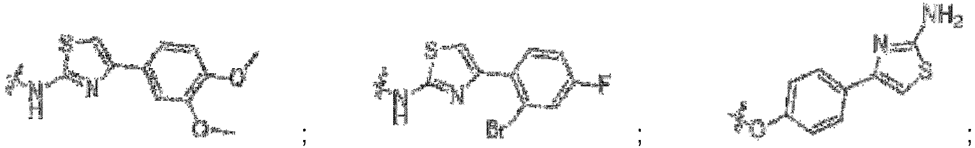

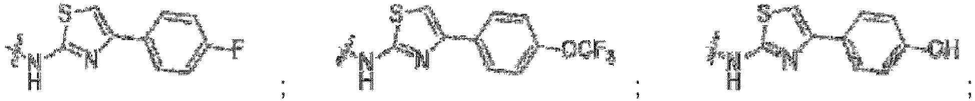

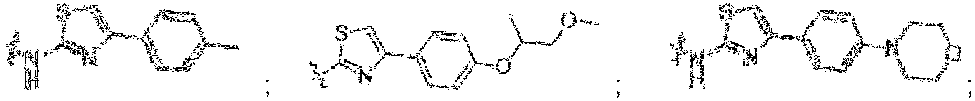

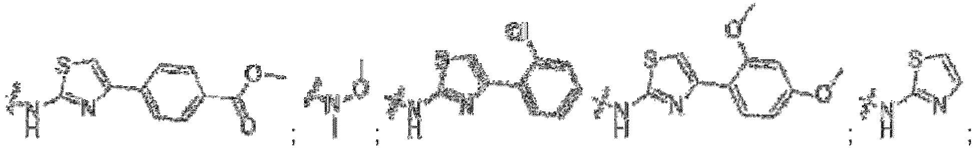

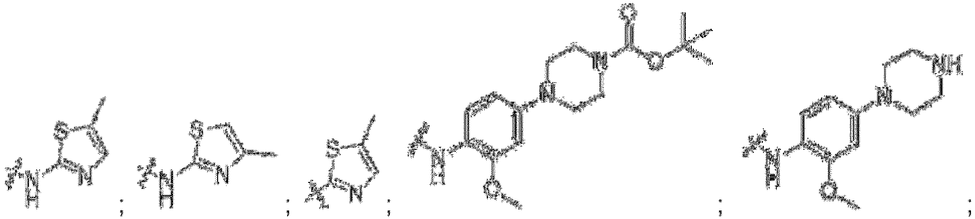

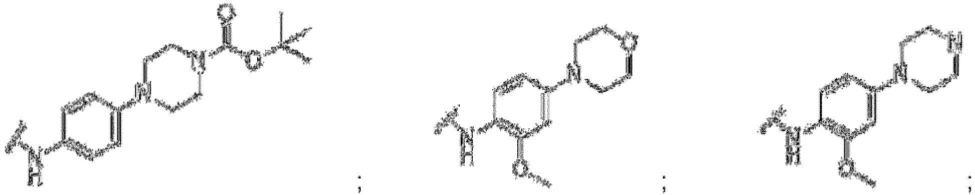

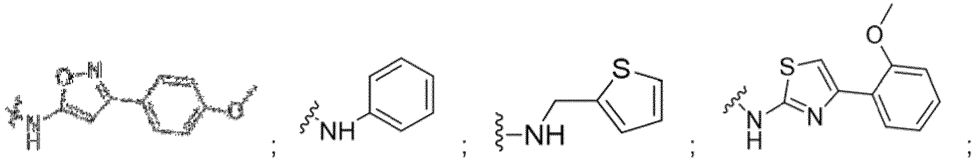

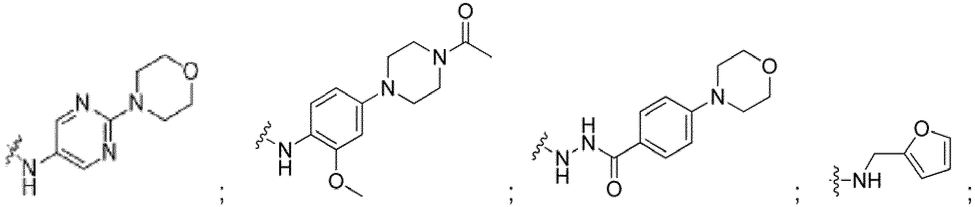

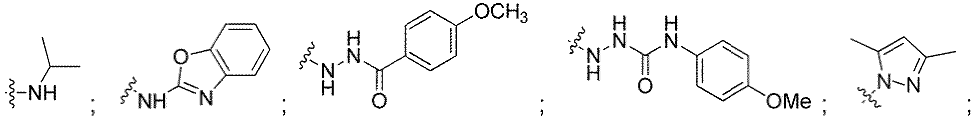

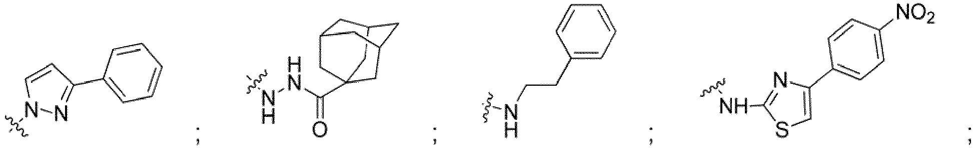

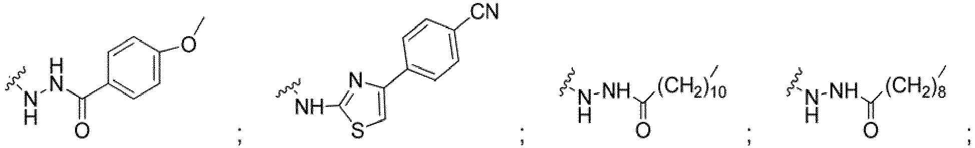

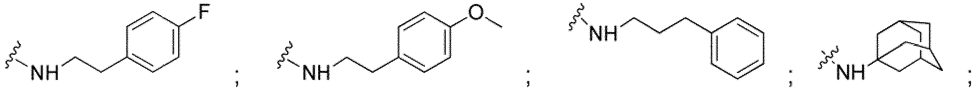

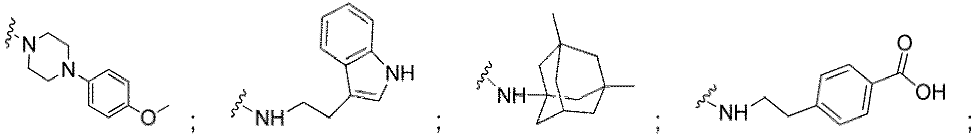

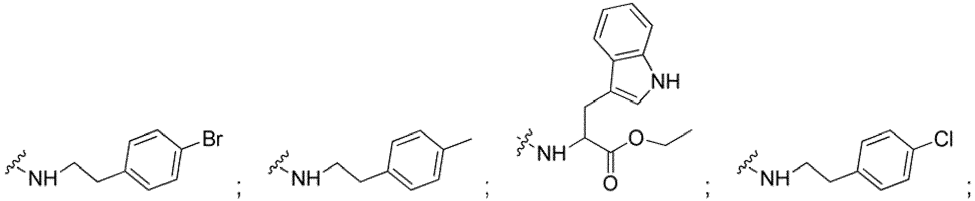

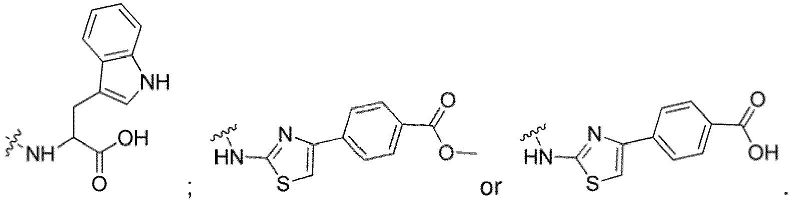

2. The compound according to claim 1 or a pharmaceutically acceptable salt thereof,
wherein R1 is indolylmethyl unsubstituted or substituted with one or more substituents selected from the group consisting of hydroxy, substituted or unsubstituted phenyl, mercapto and C1~C5 straight or branched alkyl substituted or unsubstituted with one or more substituents selected from the group consisting of C1~C5 alkylthio; or C1~C5 alkyl, hydroxy, hydroxycarbonyl and formyl,
wherein R2 is benzenesulfonyl unsubstituted or substituted with one or more substituents selected from the group consisting of C1~C5 straight or branched alkyl, nitro group, amine group, halo, -CF₃, C1~C5 alkylcarbonylamino, -COOH, C1~C5 alkoxy, phenyl, -OCH₂Ph, hydroxy, -COOCH₂Ph, C1~C5 alkyloxycarbonyl, -CONHCH₃ and nitrile,
Wherein R3 is

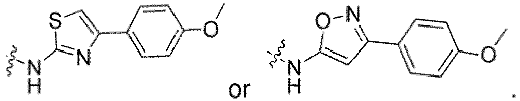

3. The compound according to claim 1 or a pharmaceutically acceptable salt thereof,
wherein R1 is hydrogen, cyclohexane,

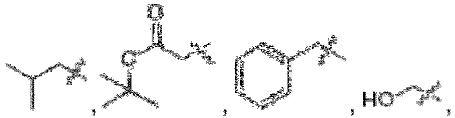

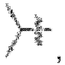

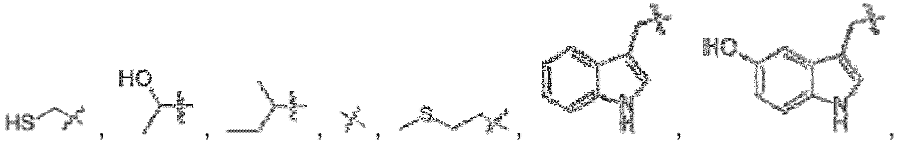

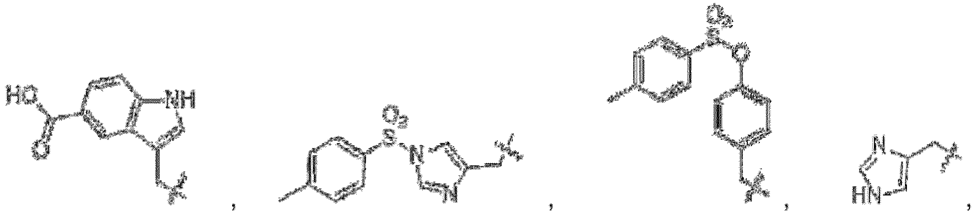

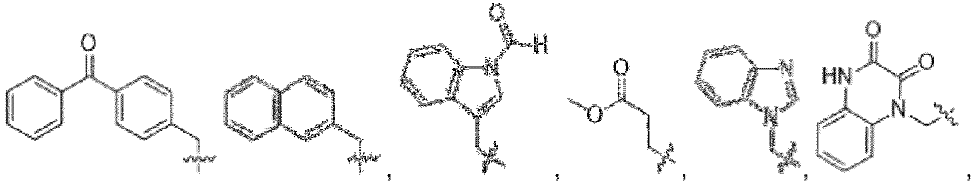

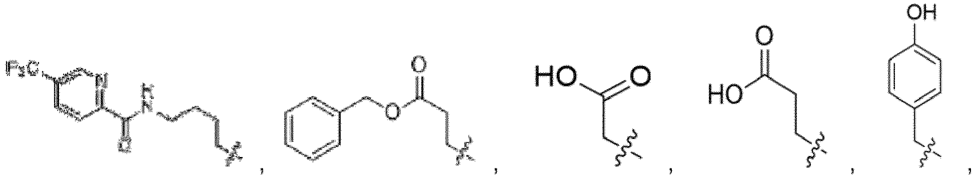

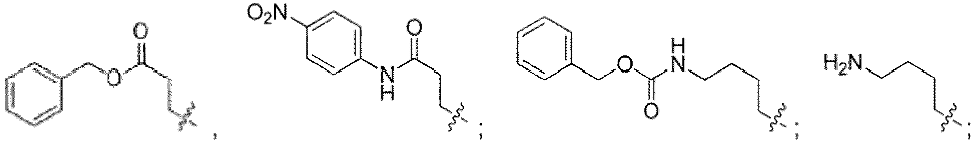

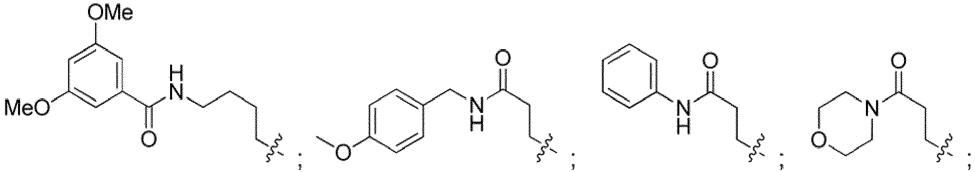

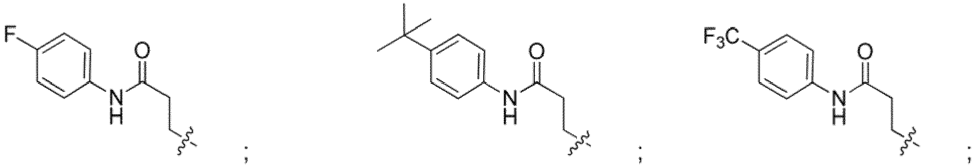

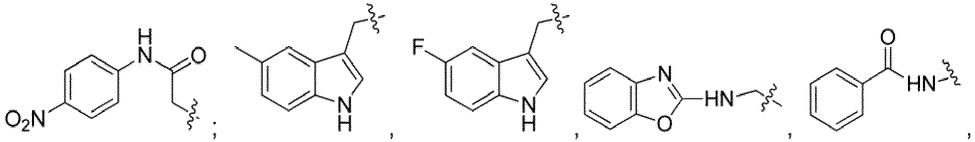

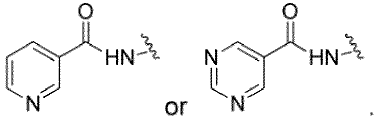

4. The compound according to claim 1 or a pharmaceutically acceptable salt thereof,
wherein R2 is hydrogen,

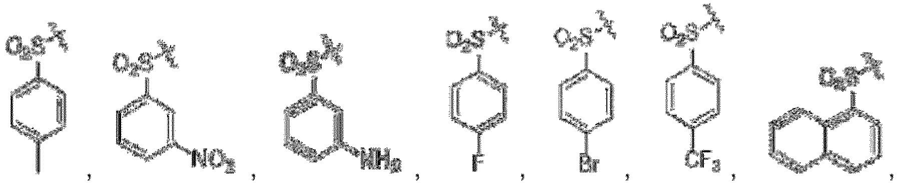

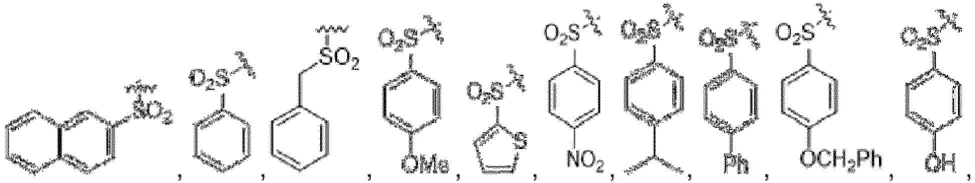

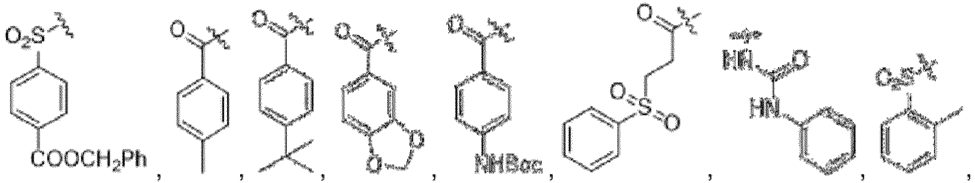

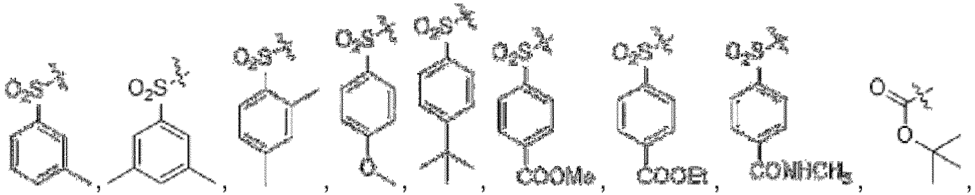

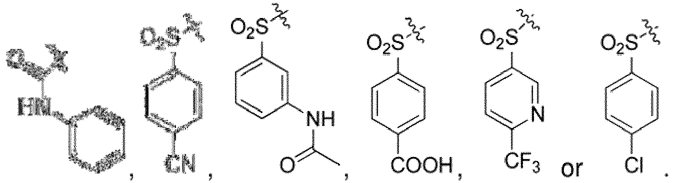

5. The compound according to claim 1 or a pharmaceutically acceptable salt thereof,
wherein the compound of Formula 1 is
(*S*)-4-methyl-*N*-(4-methyl-1-morpholino-1-oxopentan-2-yl)benzenesulfonamide,
(*R*)-4-methyl-*N*-(4-methyl-1-morpholino-1-oxopentan-2-yl)benzenesulfonamide,
(*S*)-4-methyl-*N*-(4-methyl-1-oxo-1 -(1 ,4,7-trioxa-1 0-azacyclododecan-1 0-yl)pentan-2-yl)benzenesulfonamide,
(*R*)-4-methyl-*N*-(4-methyl-1-oxo-1-(1 ,4,7-trioxa-10-azacyclododecan-10-yl)-Pentan-2-yl)benzenesulfonamide,
(*S*)-*N*-(1-(1,1-dioxidothiomorpholino)-4-methyl-1-oxopentan-2-yl)-4-methylbenzenesulfonamide,
(*N*-((*S*)-1-((*R*)-2-(hydroxymethyl)pyrrolidin-1-yl)-4-methyl-1-oxopentan-2-yl)-4-methylbenzenesulfonamide,
(*S*)-4-methyl-2-(4-methylphenylsulfonamido)-*N*-(4-(piperidin-1-yl)phenyl)pentanamide,
(*S*)-4-methyl-2-(4-methylphenylsulfonamido)-*N*-(4-(4-methylpiperidin-1-yl)phenyl) pentanamide,
(*S*)-4-Methyl-2-(4-methylphenylsulfonamido)-*N*-(6-(4-methylpiperidin-1-yl)pyridin-3-yl)pentanamide,
(*S*)-4-methyl-2-(4-methylphenylsulfonamido)-*N*-(6-(4-methylpiperidin-1-yl)pyrimidin-4-yl)pentanamide,
(*S*)-4-methyl-2-(4-methylphenylsulfonamido)-*N*-(4-morpholinophenyl)pentanamide,
(*S*)-4-Methyl-2-(4-methylphenylsulfonamido)-*N*-(6-morpholinopyridin-3-yl)pentanamide,
(*S*)-*N*-(6-((2S,6*R*)-2,6-dimethylmorpholino)pyridin-3-yl)-4-methyl-2-(4-methylphenylsulfonamido)pentanamide,
(*S*)-*N*-(3-fluoro-4-morpholinophenyl)-4-methyl-2-(4-methylphenylsulfonamido)pentanamide,
(*S*)-*N*-(4-(1,1 -dioxidothiomorpholino)phenyl)-4-methyl-2-(4-methylphenylsulfonamido)pentanamide,
(*S*)*-tert-*butyl 4-(5-(4-methyl-2-(4-methylphenylsulfonamido)pentanamido) pyridin-2-yl)piperazine-1-carboxylate,
(*S*)-4-methyl-2-(4-methylphenylsulfonamido)-*N*-(4-(pyrrolidin-1-yl)phenyl)pentanamide,
(*S*)-4-methyl-2-(4-methylphenylsulfonamido)-*N*-(2-morpholinoethyl)pentanamide,
(*S*)-*N*-(4-methyl-1-morpholino-1-oxopentan-2-yl)-3-nitrobenzenesulfonamide,
(*S*)-3-amino-*N*-(4-methyl-1-morpholino-1-oxopentan-2-yl)benzenesulfonamide,
(*S*)-2-(4-fluorophenylsulfonamido)-4-methyl-*N*-(4-morpholinophenyl)pentanamide,
(*S*)-2-(4-bromophenylsulfonamido)-4-methyl-*N*-(4-morpholinophenyl)pentanamide,
(*S*)-4-methyl-*N*-(4-morpholinophenyl)-2-(4-(trifluoromethyl)phenylsulfonamido)pentanamide,
(*S*)-4-methyl-*N*-(4-morpholinophenyl)-2-(3-nitrophenylsulfonamido)pentanamide,
(*S*)-4-methyl-*N*-(4-morpholinophenyl)-2-(naphthalene-1-sulfonamido)pentanamide,
(*S*)-4-methyl-*N*-(4-morpholinophenyl)-2-(naphthalene-2-sulfonamido)pentanamide,
(*S*)-4-methyl-*N-*(4-morpholinophenyl)-2-(phenylsulfonamido)pentanamide,
(S)-Benzyl 4-methyl-2-(3-(phenylsulfonyl)propanamido)pentanoate,
(*S*)-2-(4-methoxyphenylsulfonamido)-4-methyl-*N*-(4-morpholinophenyl)pentanamide,
(*S*)-4-methyl-*N*-(4-morpholinophenyl)-2-(thiophene-2-sulfonamido)pentanamide,
(*S*)-4-methyl-*N*-(4-morpholinophenyl)-2-(4-nitrophenylsulfonamido)pentanamide,
(*S*)-2-(4-isopropylphenylsulfonamido)-4-methyl-*N*-(4-morpholinophenyl)pentanamide,
(*S*)-2-(biphenyl-4-ylsulfonamido)-4-methyl-*N*-(4-morpholinophenyl)pentanamide, (*S*)-4-(benzyloxy)-*N*-(4-methyl-1-oxo-1-(1,4,7-trioxa-10-azacyclododecan-10-yl)pentan-2-yl)benzenesulfonamide,
(*S*)-4-hydroxy-*N*-(4-methyl-1-oxo-1-(1,4,7-trioxa-10-azacyclododecan-10-yl)pentan-2-yl)benzenesulfonamide,
(S)-benzyl 4-(*N*-(4-methyl-1-oxo-1-(1,4,7-trioxa-10-azacyclododecan-10-yl)pentan-2-yl)sulfamoyl)benzoate,
(*S*)*-*tert*-butyl* 3-(4-methylphenylsulfonamido)-4-morpholino-4-oxobutanoate,
(*S*)-4-methyl-*N*-(1-morpholino-1-oxo-3-phenylpropan-2-yl)benzenesulfonamide,
(*S*)-*N*-(3-hydroxy-1-morpholino-1-oxopropan-2-yl)-4-methylbenzenesulfonamide,
(*S*)-4-methyl-*N*-(3-methyl-1-morpholino-1-oxobutan-2-yl)benzenesulfonamide,
(*R*)-*N*-(3-mercapto-1-morpholino-1-oxopropan-2-yl)-4-methylbenzenesulfonamide,
(2*S*,3*S*)-3-hydroxy-2-(4-methylphenylsulfonamido)-*N*-(4-morpholinophenyl)butanamide,
(2*S*,3*S*)-3-methyl-2-(4-methylphenylsulfonamido)-*N*-(4-morpholinophenyl)pentanamide,
(*S*)-2-(4-methylphenylsulfonamido)-*N*-(4-morpholinophenyl)propanamide,
(*S*)-2-(4-methylphenylsulfonamido)-4-(methylthio)-*N*-(4-morpholinophenyl)butanamide,
(*S*)-3-(1*H*-indol-2-yl)-2-(4-methylphenylsulfonamido)-*N*-(4-morpholinophenyl) propanamide,
(*S*)-3-(1-methyl-1*H*-indol-3-yl)-2-(4-methylphenylsulfonamido)-*N*-(4-morpholinophenyl) propanamide,
(*S*)-2-(4-methylphenylsulfonamido)-*N*-(4-morpholinophenyl)-3-phenylpropanamide,
(*S*)-benzyl 4-methyl-2-(4-methylphenylsulfonamido)pentanoate,
(*S*)-benzyl 4-methyl-2-(4-methylbenzamido)pentanoate,
(*S*)-benzyl 2-(4-tert-butylbenzamido)-4-methylpentanoate,
(*S*)-benzyl 2-(benzo[*d*][1,3]dioxole-5-carboxamido)-4-methylpentanoate,
(*S*)-benzyl 2-(4-(*tert*-butoxycarbonylamino)benzamido)-4-methylpentanoate,
(*S*)-benzyl 4-methyl-2-(3-(phenylsulfonyl)propanamido)pentanoate,
(*S*)-*N*-(4-(4-methoxyphenyl)thiazol-2-yl)-4-methyl-2-(4-methylphenylsulfonamido)pentanamide,
(*S*)-1-(4-methyl-1-morpholino-1-oxopentan-2-yl)-3-phenylurea,
(*S*)-4-*tert*-butyl-*N*-(4-methyl-1-morpholino-1-oxopentan-2-yl)benzamide,
(*S*)-*N*-(4-methyl-1-morpholino-1-oxopentan-2-yl)-3-(phenylsulfonyl)propanamide,
(*S*)-4-*tert*-butyl-*N*-(4-methyl-1-(4-morpholinophenylamino)-1-oxopentan-2-yl)benzamide,
(*S*)-*N*-(4-methyl-1-(4-morphotinophenylamino)-1-oxopentan-2-yl)benzo[d][1,3]dioxole-5-carboxamide,
(*S*)-4-methyl-*N*-(4-morpholinophenyl)-2-(3-(phenylsulfonyl)propanamido)pentanamide,
(S)-2-(4-bromophenylsulfonamido)-3-(1*H*-indol-3-yl)-*N*-(4-(4-methoxyphenyl)thiazol-2-yl)propanamide,
(*S*)-3-(1*H*-indol-3-yl)-*N*-(4-(4-methoxyphenyl)thiazol-2-yl)-2-(4-methylphenylsulfonamido)propanamide,
(*S*)-3-(1*H*-indol-3-yl)-2-(2-methylphenylsulfonamido)-*N*-(4-morpholinophenyl)propanamide,
(*S*)-3-(1*H*-indol-3-yl)-2-(3-methylphenylsulfonamido)-*N*-(4-morpholinophenyl)propanamide,
(S)-2-(3,5-dimethylphenylsulfonamido)-3-(1H-indol-3-yl)-N-(4 morpholinophenyl)propanamide,
(*S*)-2-(4-Fluorophenylsulfonamido)-3-(1*H*-indol-3-yi)-*N*-(4-morpholinophenyl)propanamide,
(S)-3-(1*H*-indol-3-yl)-*N*-(4-morpholinophenyl)-2-(4-(trifluoromethyl)phenylsulfon-amido)propanamide,
(*S*)-3-(5-Hydroxy-1*H*-indol-3-yl)-2-(4-methylphenylsulfonamido)-*N*-(4-morpholinophenyl)propanamide,
(*S*)-*N*-(3-(1*H*-indol-3-yL)-1-morpholino-1-oxopropan-2-yl)-4-methylbenzenesulfonamide,
(*S*)-3-(1*H*-indol-3-yl)-2-(4-methylphenylsulfonamido)-*N*-(5-morpholinothiazol-2-yl)propan amide,
(*S*)-2-(2,4-dimethylphenylsulfonamido)-3-(1*H*-indol-3-yl)-*N*-(4-morpholinophenyl)propanamide,
3-(3-ethoxy-2-(4-methylphenylsulfonamido)-3-oxopropyl)-1*H*-indole-5-carboxylic acid,
(*S*)-2-(4-methylphenylsulfonamido)-*N*-(4-morpholinophenyl)-3-(1-tosyl-1*H-*imidazol-4-yl)propanamide,
(*S*)-*N*-(6-((2S,6R)-2,6-dimethylmorpholino)pyridin-3-yl)-3-(1*H*-indol-3-yl)-2-(4-methylphenylsulfonamido)propenamide,
(*R*)-3-(1*H*-indol-3-yl)-2-(4-methylphenylsulfonamido)-*N*-(4-morpholinophenyl)propanamide,
(*S*)-*N*-(3-fluoro-4-morpholinophenyl)-3-(1*H*-indol-3-yl)-2-(4-methylphenylsulfonamido)propanamide,
(*S*)-3-(1*H*-indol-3-yl)-*N*-(4-morpholinophenyl)-2-(4-nitrophenylsulfonamido)propanamide,
(*S*)-2-(4-bromophenylsulfonamido)-3-(1*H*-indol-3-yl)-*N*-(4-morpholinophenyl)propanamide,
(*S*)-3-(1*H*-indol-3-yl)-2-(4-methoxyphenylsulfonamido)-*N*-(4-morpholinophenyl)propanamide,
(*S*)-3-(1*H*-indol-3-yl)-2-(4-isopropylphenylsulfonamido)-*N*-(4 morpholinophenyl)propanamide,
(*S*)-3-(1H-indol-3-yl)-2-(4-isopropylphenylsulfonamido)-*N*-(4-(4-methoxyphenyl)thiazol-2-yl)propenamide,
(*S*)-3-(1*H*-indol-3-yl)-2-(4-methylphenylsulfonamido)-*N*-(4-methylthiazol-2-yl)propanamide,
(*S*)-*N*-(benzo[d]thiazol-2-yl)-3-(1*H*-indol-3-yl)-2-(4-methylphenylsulfonamido)propanamide,
(*S*)-2-(4-bromophenylsulfonamido)-3-(1*H*-indol-3-yl)propanoic acid,
(*S*)-4-methyl-2-(4-methylphenylsulfonamido)-*N*-(5-morpholinothiazol-2-yl)pentanamide,
(*S*)-*N*-(5-(4-methoxyphenyl)-1,3,4-thiadiazol-2-yl)-4-methyl-2-(4-methylphenylsulfonamido)pentanamide,
(*S*)-*N*-(5-(4-methoxyphenyl)isoxazol-3-yl)-4-methyl-2-(4-methylphenylsulfonamido)pentanamide,
(*S*)-*N*-(3-(4-methoxyphenyl)isoxazol-5-yl)-4-methyl-2-(4-methylphenylsulfonamido)pentanamide,
(*S*)-4-methyl-2-(4-methylphenylsulfonamido)-*N*-(4-(4-(trifluoromethyl)phenyl)thiazol-2-yl)pentanamide,
(*S*)-*N*-(4-(3,4-dimethoxyphenyl)thiazol-2-yl)-4-methyl-2-(4-methylphenylsulfonamido)pentanamide,
(*S*)-*N*-(4-(2-bromo-4-fluorophenyl)thiazol-2-yl)-4-methyl-2-(4-methylphenylsulfonamido)pentanamide,
(*S*)-4-(2-aminothiazol-4-yl)phenyl-4-methyl-2-(4-methylphenylsulfonamido)pentanoate,
(*S*)-*N*-(4-(4-fluorophenyl)thiazol-2-yl)-4-methyl-2-(4-methylphenylsulfonamido)pentanamide,
(*S*)-4-methyl-2-(4-methylphenylsulfonamido)-*N*-(4-(4-(trifluoromethoxy)phenyl)thiazol-2-yl)pentanamide,
(*S*)-*N*-(4-(4-hydroxyphenyl)thiazol-2-yl)-4-methyl-2-(4-methylphenylsulfonamido)pentanamide
(*S*)-4-methyl-2-(4-methylphenylsulfonamido)-*N*-(4-p-tolylthiazol-2-yl)pentanamide,
(*S*)-*N*-(4-(4-((*S*)-1-methoxypropan-2-yloxy)phenyl)thiazol-2-yl)-4-methyl-2-(4-methylphenylsulfonamido)pentanamide,
(*S*)-4-methyl-2-(4-methylphenylsulfonamido)-*N*-(4-(4-morpholinophenyl)thiazol-2-yl)pentanamide,
(S)-methyl-4-(2-(4-methyl-2-(4-methylphenylsulfonamido)pentanamido)thiazol-4-yl)benzoate,
(*S*)-4-(2-(4-methylphenylsulfonamido)-3-(4-morpholinophenylamino)-3-oxopropyl)phenyl 4-methylbenzenesulfonate,
(*S*)-2-(4-bromophenylsulfonamido)-3-(1*H*-imidazol-4-yl)-N-(4-(4-methoxyphenyl)thiazol-2-yl)propanamide,
(*S*)-N-methoxy-*N*,4-dimethyl-2-(4-methylphenylsulfonamido)pentanamide,
(*S*)-3-(1*H*-indol-3-yl)-N-methoxy-*N*-methyl-2-(4-methylphenylsulfonamido)propanamide,
(*S*)-*N*-3-(1*H*-indol-3-yL)-1-oxo-1 -(1 ,4,7-trioxa-1 0-azacyclododecan-1 0-yl)propan-2-yl)-4-methylbenzenesulfonamide,
(*S*)-2-(4-*tert*-butylphenylsulfonamido)-3-(1*H*-indol-3-yl)-*N*-(4-morpholinophenyl)propan amide,
(*S*)-2-(4-bromophenylsulfonamido)-*N*-(4-(2-chlorophenyl)thiazol-2-yl)-3-(1*H-*indol-3-yl)propanamide,
(*S*)-2-(4-bromophenylsulfonamido)-*N*-(4-(2,4-dimethoxyphenyl)thiazol-2-yl)-3-(1*H*-indol-3-yl)propanamide,
(*S*)-2-(4-bromophenylsulfonamido)-3-(1*H*-indol-3-yl)-*N*-(thiazol-2-yl)propanamide,
(*S*)-2-(4-bromophenylsulfonamido)-3-(1*H*-indol-3-yl)-*N*-(5-methylthiazol-2-yl)propanamide,
(*S*)-3-(1*H*-indol-3-yl)-2-(4-methylphenylsulfonamido)-*N*-(4-methylthiazol-2-yl)propanamide,
(*S*)-3-(1*H*-indol-3-yl)-2-(4-methylphenylsulfonamido)-*N*-(5-methylthiazol-2-yl)propanamide,
(*S*)-methyl 4-(*N*-(3-(1*H*-indol-3-yl)-1-(4-morpholinophenylamino)-1-oxopropan-2-yl)sulfamoyl)benzoate,
(*S*)-ethyl 4-(*N*-(3-(1*H*-indol-3-yl)-1-(4-morpholinophenylamino)-1-oxopropan-2-yl)sulfamoyl)benzoate,
(*S*)-3-(4-benzoylphenyl)-2-(4-methylphenylsulfonamido)-*N*-(4-morpholinophenyl)propanamide,
(*S*)-4-(*N*-(3-(1*H*-indol-3-yL)-1-(4-morpholinophenylamino)-1-oxopropan-2-yl)sulfamoyl)-*N*-methylbenzamide,
(*S*)-2-(4-methylphenylsulfonamido)-*N*-(4-morpholinophenyl)-3-(naphthalen-2-yl)propanamide,
(*S*)*-tert-*butyl 3-(1*H*-indol-3-yl)-1-(4-morpholinophenylamino)-1-oxopropan-2-ylcarbamate,
(*S*)-3-(1*H*-indol-3-yl)-2-(4-methylphenylsulfonamido)propanoic acid,
(*S*)-*tert*-butyl-4-(4-(3-(1*H*-indol-3-yl)-2-(4-methylphenylsulfonamido)propanamido)-3-methoxyphenyl)piperazine-1-carboxylate,
(*S*)-3-(1*H*-indol-3-yl)-*N*-(2-methoxy-4-(piperazin-1-yl)phenyl)-2-(4-methylphenylsulfonamido)propanamide,
(*S*)-*tert*-butyl-4-(4-(3-(1*H*-indol-3-yl)-2-(4-methylphenylsulfonamido)propanamido)phenyl)piperazine-1-carboxylate,
(*S*)-3-(1*H*-indol-3-yl)-*N*-(2-methoxy-4-morpholinophenyl)-2-(4-methylphenylsulfonamido)propanamide,
(*S*)-3-(1*H*-indo)-3-yl)-2-(4-methylphenylsulfonamido)-*N*-(4-(piperazin-1-yl)phenyl)propanamide,
(*S*)-3-(1-formyl-1*H*-indol-3-yl)-2-(4-methylphenylsulfonamido)-*N*-(4-morpholinophenyl)propanamide,
(*S*)-3-(1*H*-indol-3-yl)-*N*-(3-(4-methoxyphenyl)isoxazol-5-yl)-2-(4-methylphenylsulfonamido)propanamide,
(*S*)-benzyl 3-(1*H*-indol-3-yl)-2-(4-methylphenylsulfonamido)propanoate,
(*S*)-benzyl 3-(1*H*-indol-3-yl)-2-(4-methylbenzamido)propanoate,
(*S*)-*N*-3-(1*H*-indol-3-yl)-1-(4-morpholinophenylamino)-1-oxopropan-2-yl)-4-methylbenzamide,
(*S*)-3-hydroxy-2-(4-methylphenylsulfonamido)-*N*-(4-morpholinophenyl)propanamide,
(*S*)-3-methyl-2-(4-methylphenylsulfonamido)-*N*-(4-morpholinophenyl)butanamide,
(*S*)-1-(3-(1*H*-indol-3-yL)-1-morpholino-1-oxopropan-2-yl)-3-phenylurea,
(*S*)-3-(1*H*-indol-3-yl)-2-(4-methylphenylsulfonamido)-*N*-(6-morpholinopyridin-3-yl)propanamide,
(*S*)-3-(1*H*-indol-3-yl)-2-(4-methylphenylsulfonamido)-*N*-(2-morpholinopyrimidin-5-yl)propanamide,
(*S*)-2-(4-cyanophenylsulfonamido)-3-(1*H*-indol-3-yl)-*N*-(4-morpholinophenyl)propanamide,
(*S*)-3-(1*H*-indol-3-yl)-*N*-(4-morpholinophenyl)-2-(6-(trifluoromethyl)pyridine-3-sulfonamido)propanamide,
(S)-methyl 4-(4-methylphenylsulfonamido)-5-(4-morpholinophenylamino)-5-oxopentanoate,
(*S*)-3-(1*H*-benzo[*d*]imidazol-1-yl)-2-(4-methylphenylsulfonamido)-*N*-(4-morpholinophenyl)propanamide,
(R*)*-3-(2,3-dioxo-3,4-dihydroquinoxalin-1(2H)-yl)-2-(4-methylphenylsulfonamido)-*N*-(4-morpholinophenyl)propanamide,
(S*)*-*N*-(5-(4-methylphenylsulfonamido)-6-(4-morpholinophenylamino)-6-oxohexyl)-5-(trifluoromethyl)picolinamide,
(*S*)-benzyl 4-(4-methylphenylsulfonamido)-5-(4-morpholinophenylamino)-5-oxopentanoate,
(*S*)-benzyl 3-(4-methylphenylsulfonamido)-4-(4-morpholinophenylamino)-4-oxobutanoate,
(*S*)-4-methyl-*N*-(4-methyl-1-(4-morpholinophenylamino)-1-oxopentan-2-yl)benzamide,
2-(4-methylphenylsulfonamido)-*N-*(4-morpholinophenyl)acetamide,
(*S*)-3-(4-methylphenylsulfonamido)-4-(4-morpholinophenylamino)-4-oxobutanoic acid,
(*S*)-4-(4-methylphenylsulfonamido)-5-(4-morpholinophenylamino)-5-oxopentanoic acid,
(*S*)-2-(4-methylphenylsulfonamido)-*N*-(4-morpholinophenyl)-3-phenylpropanamide,
(*S*)-3-(1*H*-indol-3-yl)-2-(4-methylphenylsulfonamido)-*N*-phenylpropanamide,
(*S*)-2-(4-bromophenylsulfonamido)-3-(1*H*-indol-3-yl)-*N*-phenylpropanamide,
(*S*)-3-(1*H*-indol-3-yl)-2-(4-methylphenylsulfonamido)-*N*-(thiophen-2-ylmethyl)propanamide,
(*S*)-2-(4-bromophenylsulfonamido)-3-(1*H*-indol-3-yl)-*N*-(thiophen-2-ylmethyl)propanamide,
(*S*)-2-(4-bromophenylsulfonamido)-3-(1*H*-indol-3-yl)-*N*-(4-(2-methoxyphenyl)thiazol-2-yl)propanamide,
(*S*)-*N*-(3-(*N*-(4-methyl-1-morpholino-1-oxopentan-2-yl)sulfamoyl)phenyl)acetamide,
(S)-Benzyl 4-methyl-2-(3-phenylureido)pentanoate,
4-Methyl-*N*-(2-morpholino-2-oxoethyl)benzenesulfonamide,
4-Methyl-*N*-(1-(morpholine-4-carbonyl)cyclohexyl)benzenesulfonamide,
(*S*)-4-(*N*-(4-methyl-1-oxo-1-(1,4,7-trioxa-10-azacyclododecan-10-yl)pentan-2-yl)sulfamoyl)benzoic acid,
(*S*)-2-(4-Hydroxyphenylsulfonamido)-3-(1*H*-indol-3-yl)-*N*-(4-morpholinophenyl)propenamide,
(*S*)-4-(*N*-(3-(1*H*-indol-3-yl)-1-(4-morpholinophenylamino)-1-oxopropan-2-yl)sulfamoyl)benzoic acid,
(S)-2-Amino-3-(1*H*-indol-3-yl)-*N*-(4-morpholinophenyl)propenamide,
(*S*)-*N*-(4-(4-Acetylpiperazin-1-yl)-2-methoxyphenyl)-3-(1*H*-indol-3-yl)-2-(4-methylphenylsulfonamido)propenamide,
(*S*)-*N*-(4-(4-Acetylpiperazin-1-yl)phenyl)-3-(1*H*-indol-3-yl)-2-(4-methylphenylsulfonamido)propenamide,
(*S*)-*N*-3-(1*H*-indol-3-yl)-1-(2-(4-morpholinobenzoyl)hydrazinyl)-1-oxopropan-2-yl)-4-methylbenzenesulfonamide,
(*S*)-3-(1*H*-indol-3-yl)-2-(4-methylphenylsulfonamido)-*N*-(4-(4-morpholinophenyl)thiazol-2-yl)propanamide,
(*S*)-Methyl-4-(2-(3-(1*H*-indol-3-yl)-2-(4-methylphenylsulfonamido)propanamido)thiazol-4-yl)benzoate,
(*S*)-Benzyl-3-(1*H*-indol-3-yl)-2-(6-(trifluoromethyl)pyridine-3-sulfonamido)propanoate,
(*S*)-2-(4-Methylphenylsulfonamido)-*N*¹-(4-morpholinophenyl)-*N*⁵-(4-nitrophenyl)pentanediamide,
(S)-Benzyl-5-(4-methylphenylsulfonamido)-6-(4-morpholinophenylamino)-6-oxohexylcarbamate,
(*S*)-6-Amino-2-(4-methylphenylsulfonamido)-*N*-(4-morpholinophenyl)hexanamide,
(*S*)-3,5-Dimethoxy-*N*-(5-(4-methylphenylsulfonamido)-6-(4-morpholinophenylamino)-6-oxohexyl)benzamide,
(*S*)-*N*⁵-(4-Methoxybenzyl)-2-(4-methylphenylsulfonamido)-*N*¹-(4-morpholinophenyl)pentanediamide,
(*S*)-2-(4-Methylphenylsulfonamido)-*N*¹-(4-morpholinophenyl)-*N*⁵-phenylpentanediamide,
(*S*)-2-(4-Methylphenylsulfonamido)-5-morpholino-*N*-(4-morpholinophenyl)-5-oxopentanamide,
*S*)-*N*⁵-(4-fluorophenyl)-2-(4-methylphenylsulf onamido)-*N*¹-(4-morpholinophenyl)pentanediamide,
(*S*)*-N*⁵-(4*-tert-*butylphenyl)-2-(4-methylphenylsulfonamido)-*N*¹-(4-morpholinophenyl)pentanediamide,
(*S*)-2-(4-Methylphenylsulfonamido)-*N*¹-(4-morpholinophenyl)-*N*⁵-(4-(trifluoromethyl)phenyl)pentanediamide,
(*S*)-2-(4-Methylphenylsulfonamido)-*N*¹-(4-morpholinophenyl)-*N*⁴-(4-nitrophenyl)succinamide,
(*S*)-*N*-(furan-2-ylmethyl)-3-(1*H*-indol-3-yl)-2-(4-methylphenylsulfonamido)propanamide,
(*S*)-2-(4-Bromophenylsulfonamido)-*N*-(furan-2-ylmethyl)-3-(1*H*-indol-3-yl)propanamide,
(*S*)-3-(1*H*-indol-3-yl)-*N*-isopropyl-2-(4-methylphenylsulfonamido)propanamide,
(*S*)-2-(4-Bromophenylsulfonamido)-3-(1*H*-indol-3-yl)-*N*-isopropylpropanamide,
(*S*)-*N*-3-(1*H*-indol-3-yl)-1-(2-(4-methoxybenzoyl)hydrazinyl)-1-oxopropan-2-yl)-4-methylbenzenesulfonamide,
(*S*)-2-(3-(1*H*-indol-3-yl)-2-(4-methylphenylsulfonamido)propanoyl)-*N*-(4-methoxyphenyl)hydrazinecarboxamide,
(*S*)-4-Bromo-*N*-(1-(3,5-dimethyl-1*H*-pyrazol-1-yl)-3-(1*H*-indol-3-yl)-1-oxopropan-2-yl)benzenesulfonamide,
(*S*)-*N*-(3-(1*H*-indol-3-yl)-1-oxo-1-(3-phenyl-1*H*-pyrazol-1-yl)propan-2-yl)-4-bromobenzenesulfonamide,
*N*-((*S*)-1-(2-((3*S*,5*S*,7*S*)-adamantane-1-carbonyl)hydrazinyl)-3-(1*H*-indol-3-yL)-1-oxopropan-2-yl)-4-methylbenzenesulfonamide,
(S)-2-(4-Bromophenylsulfonamido)-3-(1*H*-indol-3-yl)-*N*-(4-(p-tolyl)thiazol-2-yl)propanamide,
(*S*)-3-(1*H*-indol-3-yl)-2-(4-methylphenylsulfonamido)-*N*-phenethylpropanamide,
(S)-2-(4-Bromophenylsulfonamido)-*N*-(4-(4-fluorophenyl)thiazol-2-yl)-3-(1*H-*indol-3-yl)propanamide,
(*S*)-2-(4-Bromophenylsulfonamido)-3-(1*H*-indol-3-yl)-*N*-(4-(4-nitrophenyl)thiazol-2-yl)propanamide,
(*S*)-*N*-(3-(1*H*-indol-3-yl)-1-(2-(4-methoxybenzoyl)hydrazinyl)-1-oxopropan-2-yl)-4-methylbenzenesulfonamide,
(S)-2-(4-Bromophenylsulfonamido)-*N*-(4-(4-cyanophenyl)thiazol-2-yl)-3-(1*H-*indol-3-yl)propanamide,
(*S*)-2-(4-Bromophenylsulfonamido)-3-(1*H*-indol-3-yl)-*N*-(4-(4-(trifluoromethyl)phenyl)thiazol-2-yl)propanamide,
(*S*)-4-Bromo-*N*-(1-(2-dodecanoylhydrazinyl)-3-(1*H*-indol-3-yl)-1-oxopropan-2-yl)benzenesulfonamide,
(*S*)-4-Bromo-*N*-(1-(2-decanoylhydrazinyl)-3-(1*H*-indol-3-yl)-1-oxopropan-2-yl)benzenesulfonamide,
(*S*)-2-(4-Bromophenylsulfonamido)-3-(1*H*-indol-3-yl)-*N*-phenethylpropanamide,
*N*-((*S*)-1-(2-((3*S*,5*S*,7*S*)-adamantane-1-carbonyl)hydrazinyl)-3-(1*H*-indol-3-yl)-1-oxopropan-2-yl)-4-bromobenzenesulfonamide,
(*S*)-2-(4-Bromophenylsulfonamido)-*N*-(4-fluorophenethyl)-3-(1*H*-indol-3-yl)propanamide,
(*S*)-2-(4-Bromophenylsulfonamido)-3-(1*H*-indol-3-yl)-*N*-(4-methoxyphenethyl)propanamide,
(*S*)-2-(4-Bromophenylsulfonamido)-3-(1*H*-indol-3-yl)-*N*-(3-phenylpropyl)propanamide,
(*S*)-*N*-((3*R*,5*R*,7*R*)-adamantan-1-yl)-2-(4-bromophenylsulfonamido)-3-(1*H*-indol-3-yl)propanamide,
(*S*)-*N*-(3-(1*H*-indol-3-yl)-1-(4-(4-methoxyphenyl)piperazin-1-yl)-1 -oxopropan-2-yl)-4-bromobenzenesulfonamide,
(*S*)-*N*-(3-(1*H*-indol-3-yl)-1-(4-(4-methoxyphenyl)piperazin-1 -yl)-1 -oxopropan-2-yl)-4-methylbenzenesulfonamide,
(*S*)-*N*-((3*R*,5*R*,7*R*)-adamantan-1-yl)-3-(1*H*-indol-3-yl)-2-(4-methylphenylsulfonamido)propanamide,
(*S*)-3-(1*H*-indol-3-yl)-*N*-(4-methoxyphenethyl)-2-(4-methylphenylsulfonamido)propanamide,
(*S*)-3-(1*H*-indol-3-yl)-2-(4-methylphenylsulfonamido)-*N*-(3-phenylpropyl)propanamide,
(*S*)-*N*-(4-fluorophenethyl)-3-(1*H*-indol-3-yl)-2-(4-methylphenylsulfonamido)propanamide,
(*S*)-3-(*1H*-indol-3-yl)-2-(4-methylphenylsulfonamido)-*N*-(4-(4-(trifluoromethyl)phenyl)thiazol-2-yl)propanamide,
(S)-*N*-(2-(1*H*-indol-3-yl)ethyl)-2-(4-bromophenylsulfonamido)-3-(1*H*-indol-3-yl)propanamide,
(*S*)-*N*-(3-(1*H*-indol-3-yl)-1-((4-(4-methoxyphenyl)thiazol-2-yl)amino)-1-oxopropan-2-yl)-4-methylbenzamide,
(*S*)-2-(4-Chlorophenylsulfonamido)-3-(1*H*-indol-3-yl)-*N*-(4-(4-methoxyphenyl)thiazol-2-yl)propanamide,
(*S*)-3-(1*H*-indol-3-yl)-*N*-(4-(4-methoxyphenyl)thiazol-2-yl)-2-(4-(trifluoromethyl)phenylsulfonamido)propanamide,
(*S*)-2-(4-Fluorophenylsulfonamido)-3-(1*H*-indol-3-yl)-*N*-(4-(4-methoxyphenyl)thiazol-2-yl)propanamide,
(*S*)-2-(4-Bromophenylsulfonamido)-*N*-((1*r*,3*R*,5*S*,7*S*)-3,5-dimethyladamantan-1-yl)-3-(1*H*-indol-3-yl)propanamide,
(*S*)-2-(4-Cyanophenylsulfonamido)-3-(1*H*-indol-3-yl)-*N*-(4-(4-methoxyphenyl)thiazol-2-yl)propanamide,
(*S*)-Benzyl 3-(1*H*-indol-3-yl)-2-(4-methylbenzamido)propanoate,
(*S*)-2-(4-Bromophenylsulfonamido)-3-(5-hydroxy-1*H*-indol-3-yl)-*N*-(4-(4-methoxyphenyl)thiazol-2-yl)propanamide,
(*S*)-2-(4-Bromophenylsulfonamido)-*N*-(4-(4-methoxyphenyl)thiazol-2-yl)-3-(5-methyl-1*H*-indol-3-yl)propenamide,
(S)-2-(4-Bromophenylsulfonamido)-3-(5-fluoro-1*H*-indol-3-yl)-*N*-(4-(4-methoxyphenyl)thiazol-2-yl)propanamide,
(*S*)-4-(2-(3-(1*H*-indol-3-yl)-2-(4-methylphenylsulfonamido)propanamido)ethyl)benzoic acid,
(*S*)-*N*-(4-bromophenethyl)-3-(1*H*-indol-3-yl)-2-(4-methylphenylsulfonamido)propanamide,
(*S*)-*N*-(2-(1*H*-indol-3-yl)ethyl)-3-(1*H*-indol-3-yl)-2-(4-methylphenylsulfonamido)propenamide,
(*S*)-3-(1*H*-indol-3-yl)-*N*-(4-methylphenethyl)-2-(4-methylphenylsulfonamido)propanamide,
Ethyl 2-((*S*)-3-(1*H*-indol-3-yl)-2-(4-methylphenylsulfonamido)propanamido)-3-(1*H*-indol-3-yl)propanoate,
(*S*)-*N*-(4-chlorophenethyl)-3-(1*H*-indol-3-yl)-2-(4-methylphenylsulfonamido)propanamide,
2-((*S*)-3-(1*H*-indol-3-yl)-2-(4-methylphenylsulfonamido)propanamido)-3-(1*H-*indol-3-yl)propanoic acid,
(*S*)-Methyl-4-(2-(3-(1*H*-indol-3-yl)-2-(4-methylphenylsulfonamido)propanamido)thiazol-4-yl)benzoate,
(*S*)-4-(2-(3-(1*H*-indol-3-yl)-2-(4-methylphenylsulfonamido)propanamido)thiazol-4-yl)benzoic acid,
(*S*)-3-(Benzo[*d*|oxazol-2-ylamino)-*N*-(4-(4-methoxyphenyl)thiazol-2-yl)-2-(4-methylphenylsulfonamido)propanamide,
(*S*)-*N*-(3-((4-(4-methoxyphenyl)thiazol-2-yl)amino)-2-(4-methylphenylsulfonamido)-3-oxopropyl)benzamide,
(*S*)-*N*-(3-((4-(4-methoxyphenyl)thiazol-2-yl)amino)-2-(4-methylphenylsulfonamido)-3-oxopropyl)nicotinamide or
(*S*)-*N*-(3-((4-(4-methoxyphenyl)thiazol-2-yl)amino)-2-(4-methylphenylsulfonamido)-3-oxopropyl)pyrimidine-5-carboxamide.

6. The compound according to any one of claims 1 to 5, wherein the compound inhibits the expression of AIMP2-DX2 or a pharmaceutically acceptable salt thereof.

7. A method for preparing the compound of claim 1 or a pharmaceutically acceptable salt thereof prepared by the following Reaction Formula: 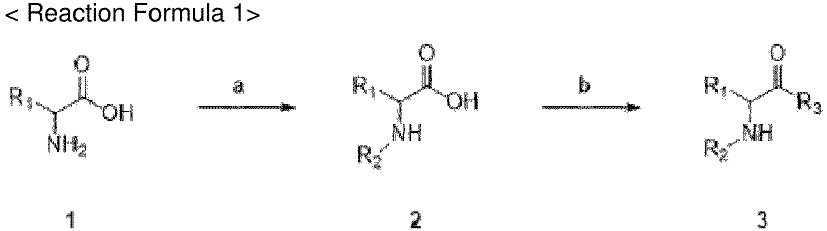 In Reaction Formula 1,
R1, R2 and R3 are as defined in claim 1.

8. A pharmaceutical composition for preventing or treating cancer comprising the compound of any one of claims 1 to 5 or a pharmaceutically acceptable salt thereof as an active ingredient.

9. The pharmaceutical composition according to claim 8, wherein the cancer is selected from the group consisting of leukemia, brain tumor, kidney cancer, stomach cancer, skin cancer, bladder cancer, breast cancer, uterine cancer, melanoma, thyroid cancer, head and neck cancer, lymphoma, gallbladder cancer, esophageal cancer, lung cancer, colon cancer, prostate cancer, ovarian cancer, liver cancer, colorectal cancer, peritoneal cancer, peritoneal metastatic cancer and pancreatic cancer.

10. A pharmaceutical composition for preventing or treating inflammatory diseases comprising the compound of any one of claims 1 to 5 or a pharmaceutically acceptable salt thereof as an active ingredient.

11. The pharmaceutical composition according to claim 10, wherein the inflammatory disease is selected from the group consisting of inflammatory skin diseases, crohn's disease, ulcerative colitis, peritonitis, osteomyelitis, cellulitis, meningitis, encephalitis, pancreatitis, trauma-induced shock, bronchial asthma, allergic rhinitis, cystic fibrosis, stroke, acute bronchitis, chronic bronchitis, acute bronchiolitis, chronic bronchiolitis, osteoarthritis, gout, spondyloarthropathy, ankylosing spondylitis, Reiter's syndrome, psoriatic arthropathy, enteric spondylitis, juvenile arthropathy, juvenile ankylosing spondylitis, reactive arthropathy, infectious arthritis, post-infectious arthritis, gonococcal arthritis, tuberculous arthritis, viral arthritis, fungal arthritis, syphilitic arthritis, Lyme disease, arthritis associated with 'vasculitis syndrome', polyarteritis nodosa, hypersensitivity vasculitis, Lou Gehrig's granulomatosis, polymyalgia rheumatica, articular cell arteritis, calcium crystals deposited arthropathy, pseudo gout, non-articular rheumatism, bursitis, tendinitis, epicondylitis (tennis elbow), neuropathic joint disease (charco and joint), hemarthrosic, Henoch-Schönlein purpura, hypertrophic osteoarthropathy, multicentral reticulocytoma, surcoilosis, hemochromatosis, sickle cell disease and other hemoglobinopathy, hyperproteinemia, hypogammaglobulinemia, familial thalassemia, Behat's disease, systemic lupus erythematosus, relapsing fever, psoriasis , multiple sclerosis, sepsis, septic shock, multiple organ dysfunction syndrome, acute respiratory distress syndrome, chronic obstructive pulmonary disease, rheumatoid arthritis, acute lung injury and broncho-pulmonary dysplasia.

12. Use of the compound of any one of claims 1 to 5 or a pharmaceutically acceptable salt thereof, for preparing an agent for preventing or treating cancer.

13. A method for treating cancer comprising administering to a subject in need thereof an effective amount of a composition comprising the compound of any one of claims 1 to 5 or a pharmaceutically acceptable salt thereof as an active ingredient.

14. Use of the compound of any one of claims 1 to 5 or a pharmaceutically acceptable salt thereof, for preparing an agent for preventing or treating inflammatory diseases.

15. A method for treating an inflammatory disease comprising administering to a subject in need thereof an effective amount of a composition comprising the compound of any one of claims 1 to 5 or a pharmaceutically acceptable salt thereof as an active ingredient.
